# EUROPEAN PATENT SPECIFICATION

(11) **EP 3 455 248 B1**
(45) Date of publication and mention of the grant of the patent: **30.06.2021**
(21) Application number: 17725542.9
(22) Date of filing: 11.05.2017
(51) Int. Cl.: C07K 14/705, A61K 39/395, C07K 16/28, C12N 15/62, A61P 35/00

(54) **ANTIGEN BINDING MOLECULES COMPRISING A TNF FAMILY LIGAND TRIMER AND PD1 BINDING MOIETY**
ANTIGENBINDENDE MOLEKÜLE MIT EINEM LIGANDENTRIMER DER TNF-FAMILIE UND PD1-BINDUNGSTEIL
MOLÉCULES DE LIAISON D'ANTIGÈNE COMPRENANT UN TRIMÈRE DE LIGAND DE LA FAMILLE TNF ET UN FRAGMENT DE LIAISON PD1

(30) Priority: 13.05.2016 EP 16169487
(43) Date of publication of application: 20.03.2019
(73) Proprietor: F. Hoffmann-La Roche AG, 4070 Basel (CH)
(72) Inventor: FERRARA KOLLER, Claudia, 8952 Schlieren (CH); CLAUS, Christina, 8952 Schlieren (CH); KLEIN, Christian, 8952 Schlieren (CH); SEEBER, Stefan, 82377 Penzberg (DE); AMANN, Maria, 8952 Schlieren (CH); GRAU-RICHARDS, Sandra, 8952 Schlieren (CH); BRUENKER, Peter, 8952 Schlieren (CH); UMANA, Pablo, 8952 Schlieren (CH); LEVITSKI, Viktor, 8952 Schlieren (CH); MOESSNER, Ekkehard, 8952 Schlieren (CH)
(74) Representative: Klostermeyer-Rauber, Dörte
(86) International application number: PCT/EP2017/061241
(87) International publication number: WO 2017/194641

(56) References cited:
- EP-A1- 3 455 254
- WO-A1-2010/010051
- WO-A1-2013/164694
- WO-A1-2016/075278
- WO-A2-2015/095423
- S. CHEN ET AL: "Combination of 4-1BB Agonist and PD-1 Antagonist Promotes Antitumor Effector/Memory CD8 T Cells in a Poorly Immunogenic Tumor Model", CANCER IMMUNOLOGY RESEARCH, vol. 3, no. 2, 11 November 2014 (2014-11-11), pages 149-160, XP055293412, US ISSN: 2326-6066, DOI: 10.1158/2326-6066.CIR-14-0118 cited in the application
- SHINDO Y ET AL: "Combination immunotherapy with 4-1BB activation and PD-1 blockade enhances antitumor efficacy in a mouse model of subcutaneous tumor", ANTICANCER RESEARCH - INTERNATIONAL JOURNAL OF CANCER RESEARCH AND TREATMENT, INTERNATIONAL INSTITUTE OF ANTICANCER RESEARCH, GR, vol. 35, no. 1, 1 January 2015 (2015-01-01), pages 129-136, XP002746546, ISSN: 0250-7005
- HORNIG NORA ET AL: "Combination of a Bispecific Antibody and Costimulatory Antibody-Ligand Fusion Proteins for Targeted Cancer Immunotherapy", JOURNAL OF IMMUNOTHERAPY, LIPPINCOTT WILLIAMS & WILKINGS INC, US, vol. 35, no. 5, 1 June 2012 (2012-06-01), pages 418-429, XP009163394, ISSN: 1524-9557, DOI: 10.1097/CJI.0B013E3182594387 cited in the application
- NORA HORNIG ET AL: "Evaluating combinations of costimulatory antibody-ligand fusion proteins for targeted cancer immunotherapy", CANCER IMMUNOLOGY, IMMUNOTHERAPY, vol. 62, no. 8, 1 August 2013 (2013-08-01) , pages 1369-1380, XP055184710, ISSN: 0340-7004, DOI: 10.1007/s00262-013-1441-7

## Description

### FIELD OF THE INVENTION

The invention relates to novel 4-1BB ligand (4-1BBL) trimer-containing antigen binding molecules comprising (a) at least one Fab molecule capable of specific binding to PD1 comprising a VH domain comprising (i) HVR-H1 comprising the amino acid sequence of SEQ ID NO: 13, (ii) HVR-H2 comprising the amino acid sequence of SEQ ID NO: 14, and (iii) HVR-H3 comprising the amino acid sequence of SEQ ID NO: 15, and a VL domain comprising (iv) HVR-L1 comprising the amino acid sequence of SEQ ID NO:16, (v) HVR-L2 comprising the amino acid sequence of SEQ ID NO: 17, and (vi) HVR-L3 comprising the amino acid sequence of SEQ ID NO: 18, and (b) a first polypeptide containing a first heavy chain constant domain (CH1) or a constant light chain domain (CL) and a second polypeptide containing a CL or CH1 domain, respectively, wherein the second polypeptide is linked to the first polypeptide by a disulfide bond between the CH1 and CL domain, wherein the antigen binding molecules are characterized in that the first polypeptide comprises two ectodomains of 4-1BBL comprising the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8 that are connected to each other and to the CH1 or CL domain by a peptide linker and in that the second polypeptide comprises one ectodomain of 4-1BBL comprising the amino acid sequence selected from the group consisting of SEQ ID NO: 1, SEQ ID NO: 2, SEQ ID NO:3, SEQ ID NO:4, SEQ ID NO:5, SEQ ID NO: 6, SEQ ID NO:7 and SEQ ID NO:8 connected via a peptide linker to the CL or CH1 domain of said polypeptide, and (c) an Fc domain composed of a first and a second subunit capable of stable association, wherein the Fc domain is an IgG1 Fc domain comprising the amino acid substitutions L234A, L235A and P329G (numbering according to Kabat EU index). The invention further relates to methods of producing these molecules and to methods of using the same.

### BACKGROUND

Ligands interacting with molecules of the TNF (tumor necrosis factor) receptor superfamily have pivotal roles in the organization and function of the immune system. While regulating normal functions such as immune responses, hematopoiesis and morphogenesis, the TNF family ligands (also called cytokines) play a role in tumorgenesis, transplant rejection, septic shock, viral replication, bone resorption, rheumatoid arthritis and diabetes (Aggarwal, 2003). The TNF ligand family comprises 18 genes encoding 19 type II (i.e. intracellular N terminus and extracellular C-terminus) transmembrane proteins, characterized by the presence of a conserved C-terminal domain coined the 'TNF homology domain' (THD). This domain is responsible for receptor binding and is thus critical for the biological activity of the TNF ligand family members. The sequence identity between family members is ∼20-30% (Bodmer, 2002). Members of the TNF ligand family exert their biological function as self-assembling, noncovalent trimers (Banner et al, Cell 1993, 73, 431-445). Thus, the TNF family ligands form a trimer that is able to bind to and to activate the corresponding receptors of TNFR superfamily.

4-1BB (CD137), a member of the TNF receptor superfamily, has been first identified as a molecule whose expression is induced by T-cell activation (Kwon and Weissman, 1989). Subsequent studies demonstrated expression of 4-1BB in T- and B-lymphocytes (Snell et al., 2011; Zhang et al., 2010), NK-cells (Lin et al., 2008), NKT-cells (Kim et al., 2008), monocytes (Kienzle and von Kempis, 2000; Schwarz et al., 1995), neutrophils (Heinisch et al., 2000), mast (Nishimoto et al., 2005) and dendritic cells as well as cells of non-hematopoietic origin such as endothelial and smooth muscle cells (Broll et al., 2001; Olofsson et al., 2008). Expression of 4-1BB in different cell types is mostly inducible and driven by various stimulatory signals, such as T-cell receptor (TCR) or B-cell receptor triggering, as well as signaling induced through co-stimulatory molecules or receptors of pro-inflammatory cytokines (Diehl et al., 2002; von Kempis et al., 1997; Zhang et al., 2010).

Expression of 4-1BB ligand (4-1BBL or CD137L) is more restricted and is observed on professional antigen presenting cells (APC) such as B-cells, dendritic cells (DCs) and macrophages. Inducible expression of 4-1BBL is characteristic for T-cells, including both αβ and γδ T-cell subsets, and endothelial cells (reviewed in Shao and Schwarz, 2011).

CD137 signaling is known to stimulate IFNγ secretion and proliferation of NK cells (Buechele et al., 2012; Lin et al., 2008; Melero et al., 1998) as well as to promote DC activation as indicated by their increased survival and capacity to secret cytokines and upregulate co-stimulatory molecules (Choi et al., 2009; Futagawa et al., 2002; Wilcox et al., 2002). However, CD137 is best characterized as a co-stimulatory molecule which modulates TCR-induced activation in both the CD4+ and CD8+ subsets of T-cells. In combination with TCR triggering, agonistic 4-1BB-specific antibodies enhance proliferation of T-cells, stimulate lymphokine secretion and decrease sensitivity of T-lymphocytes to activation-induced cells death (reviewed in (reviewed in Snell et al., 2011).

In line with these co-stimulatory effects of 4-1BB antibodies on T-cells in vitro, their administration to tumor bearing mice leads to potent anti-tumor effects in many experimental tumor models (Melero et al., 1997; Narazaki et al., 2010). However, 4-1BB usually exhibits its potency as an anti-tumor agent only when administered in combination with other immunomodulatory compounds (Curran et al., 2011; Guo et al., 2013; Morales-Kastresana et al., 2013; Teng et al., 2009; Wei et al., 2013), chemotherapeutic reagents (Ju et al., 2008; Kim et al., 2009), tumor-specific vaccination (Cuadros et al., 2005; Lee et al., 2011) or radiotherapy (Shi and Siemann, 2006). In vivo depletion experiments demonstrated that CD8+ T-cells play the most critical role in anti-tumoral effect of 4-1BB-specific antibodies. However, depending on the tumor model or combination therapy, which includes anti-4-1BB, contributions of other types of cells such as DCs, NK-cells or CD4+ T-cells have been reported (Melero et al., 1997; Murillo et al., 2009; Narazaki et al., 2010; Stagg et al., 2011).

In addition to their direct effects on different lymphocyte subsets, 4-1BB agonists can also induce infiltration and retention of activated T-cells in the tumor through 4-1BB-mediated upregulation of intercellular adhesion molecule 1 (ICAM1) and vascular cell adhesion molecule 1 (VCAM1) on tumor vascular endothelium (Palazon et al., 2011).

4-1BB triggering may also reverse the state of T-cell anergy induced by exposure to soluble antigen that may contribute to disruption of immunological tolerance in the tumor microenvironment or during chronic infections (Wilcox et al., 2004).

It appears that the immunomodulatory properties of 4-1BB agonistic antibodies in vivo require the presence of the wild type Fc-portion on the antibody molecule thereby implicating Fc-receptor binding as an important event required for the pharmacological activity of such reagents as has been described for agonistic antibodies specific to other apoptosis-inducing or immunomodulatory members of the TNFR-superfamily (Li and Ravetch, 2011; Teng et al., 2009). However, systemic administration of 4-1BB-specific agonistic antibodies with the functionally active Fc domain also induces expansion of CD8+ T-cells associated with liver toxicity (Dubrot et al., 2010) that is diminished or significantly ameliorated in the absence of functional Fc-receptors in mice. In human clinical trials (ClinicalTrials.gov, NCT00309023), Fc-competent 4-1BB agonistic antibodies (BMS-663513) administered once every three weeks for 12 weeks induced stabilization of the disease in patients with melanoma, ovarian or renal cell carcinoma. However, the same antibody given in another trial (NCT00612664) caused grade 4 hepatitis leading to termination of the trial (Simeone and Ascierto, 2012).

Collectively, the available pre-clinical and clinical data clearly demonstrate that there is a high clinical need for effective 4-1BB agonists. However, new generation drug candidates should not only effectively engage 4-1BB on the surface of hematopoietic and endothelial cells but also be capable of achieving that through mechanisms other than binding to Fc-receptors in order to avoid uncontrollable side effects. The latter may be accomplished through preferential binding to and oligomerization on tumor-specific or tumor-associated moieties.

Fusion proteins composed of one extracellular domain of a 4-1BB ligand and a single chain antibody fragment (Mueller et al., 2008; Hornig et al., J. Immunotherapy 2012, 35(5), 418-429; Hornig et al., Cancer Immunology 2013, 62(8), 1369-1380) or a single 4-1BB ligand fused to the C-terminus of a heavy chain (Zhang et al, 2007) have been made. WO 2010/010051 discloses the generation of fusion proteins that consist of three TNF ligand ectodomains linked to each other and fused to an antibody part. WO 2013/164694 describes fusion proteins wherein a single 4-1BB ligand is fused to the heavy chain of an antibody directed against HER2, EGFR1, CTLA4 or CD20. WO 2016/075278 discloses antigen binding molecules comprising a 4-1BBL trimer and an antigen binding domain against a target cell antigen such as FAP, CEA or CD19 and EP 3 455 254 A1 describes antigen binding molecules comprising a 4-1BBL trimer and an antigen binding domain against Tenascin-C. WO 2015/095423 discloses the combination therapy of an OX40 antibody with a PD-1 axis binding antagonist such as a PD-1 or PD-L1 antibody. Chen et al, Cancer Immunology Research 2014, 3(2), 149-160, disclose that the combination of a 4-1BB antibody with an anti-PD1 antibody in a melanoma mouse model promoted antitumor effector/memory CD8 T cells. Shindo et al., Anticancer Research 2015, 35(19; 129-136, also describe that combination of a 4-1BB antibody with PD1-blockade enhanced antitumor efficacy.

Programmed cell death protein 1 (PD1 or CD279) is an inhibitory member of the CD28 family of receptors, that also includes CD28, CTLA-4, ICOS and BTLA. PD1 is a cell surface receptor and is expressed on activated B cells, T cells, and myeloid cells (Okazaki et al (2002) Curr. Opin. Immunol. 14: 391779-82; Bennett et al. (2003) J Immunol 170:711-8). The structure of PD1 is a monomeric type 1 transmembrane protein, consisting of one immunoglobulin variable-like extracellular domain and a cytoplasmic domain containing an immunoreceptor tyrosine-based inhibitory motif (ITIM) and an immunoreceptor tyrosine-based switch motif (ITSM). Activated T cells transiently express PD1, but sustained hyperexpression of PD1 and its ligand PDL1 promote immune exhaustion, leading to persistence of viral infections, tumor evasion, increased infections and mortality. PD1 expression is induced by antigen recognition via the T-cell receptor and its expression is maintained primarily through continuous T-cell receptor signaling. After prolonged antigen exposure, the PD1 locus fails to be remethylated, which promotes continuous hyperexpression. Blocking the PD1 pathway can restore the exhausted T-cell functionality in cancer and chronic viral infections (Sheridan, Nature Biotechnology 30 (2012), 729-730). Monoclonal antibodies to PD1 have been described, for example, in WO 2003/042402, WO 2004/004771, WO 2004/056875, WO 2004/072286, WO 2004/087196, WO 2006/121168, WO 2006/133396, WO 2007/005874, WO 2008/083174, WO 2008/156712, WO 2009/024531, WO 2009/014708, WO 2009/101611, WO 2009/114335, WO 2009/154335, WO 2010/027828, WO 2010/027423, WO 2010/029434, WO 2010/029435, WO 2010/036959, WO 2010/063011, WO 2010/089411, WO 2011/066342, WO 2011/110604, WO 2011/110621, WO 2012/145493, WO 2013/014668, WO 2014/179664, and WO 2015/112900. Two PD1 monoclonal antibodies, Opdivo (nivolumab) and Keytruda (pembrolizumab), are already on the market for the treatment of certain cancers. However, despite of the compelling anti-tumor activity, relaps of tumor growth after therapeutic PD1 blockade has increasingly been observed (Koyama et al., Nature Communications 2016, DOI: 10.1038/ncomms10501).

It has been shown that dual targeting of CD137/PD1 in the tumor microenvironment abolishes the local suppression and leads to functional rescue and expansion of effector T cells that subsequently kill cancer cells (Wei et al., 2014). It has been shown in a mous model for subcutaneous tumor, that mice treated with a combination of an anti-4-1BB antibody and an anti-PD1 antibody had the best anti-tumor response compared to mice treated with one of the antibodies alone (Shindo et al., 2015). Administering the combination of an anti-PD1 antibody and a 4-1BB agonistic antibody in a tumor mouse model led to an effective long-lasting systemic antitumor response, however the known toxicity of the 4-1BB agonist as given alone was slightly increased (Chen et al., 2014). Thus, there is a need for new molecules that combine the positive effects of both the anti-PD1 antibody as well as the 4-1BB antibody with an improved safety profile.

The new antigen binding molecules of the present invention combine a PD1 moiety with a moiety that is capable of forming a costimulatory TNF ligand trimer and that is sufficiently stable to be pharmaceutically useful. Surprisingly, antigen binding molecules of the invention provide a trimeric and thus biologically active TNF ligand, although one of the trimerizing TNF ligand ectodomains is located on another polypeptide than the other two TNF ligand ectodomains of the molecule. Targeted by the anti-PD1 moiety the antigen binding molecules of the present invention have an increased activity in the tumor microenvironment and should thus impose less safety issues compared to conventional 4-1BB agonistic antibodies alone.

### SUMMARY OF THE INVENTION

In one aspect, the invention provides a 4-1BBL trimer-containing antigen binding molecule comprising
(a) at least one Fab molecule capable of specific binding to PD1 comprising a VH domain comprising (i) HVR-H1 comprising the amino acid sequence of SEQ ID NO:13, (ii) HVR-H2 comprising the amino acid sequence of SEQ ID NO:14, and (iii) HVR-H3 comprising the amino acid sequence of SEQ ID NO: 15, and a VL domain comprising (iv) HVR-L1 comprising the amino acid sequence of SEQ ID NO:16, (v) HVR-L2 comprising the amino acid sequence of SEQ ID NO: 17, and (vi) HVR-L3 comprising the amino acid sequence of SEQ ID NO: 18, and
(b) a first polypeptide containing a first heavy chain constant domain (CH1) or a constant light chain domain (CL) and a second polypeptide containing a CL or CH1 domain, respectively, wherein the second polypeptide is linked to the first polypeptide by a disulfide bond between the CH1 and CL domain,,
wherein the antigen binding molecule is characterized in that the first polypeptide comprises two ectodomains of 4-1BBL comprising the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8 that are connected to each other and to the CH1 or CL domain by a peptide linker and in that the second polypeptide comprises one ectodomain of 4-1BBL comprising the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8 connected via a peptide linker to the CL or CH1 domain of said polypeptide, and (c) an Fc domain composed of a first and a second subunit capable of stable association, wherein the Fc domain is an IgG1 Fc domain comprising the amino acid substitutions L234A, L235A and P329G (numbering according to Kabat EU index)..

In a further aspect, the ectodomain of 4-1BBL comprises the amino acid sequence selected from the group consisting of SEQ ID NO:1, SEQ ID NO: 2, SEQ ID NO:3, SEQ ID NO:4, SEQ ID NO:5, SEQ ID NO: 6, SEQ ID NO:7 and SEQ ID NO:8, particularly the amino acid sequence of SEQ ID NO:1 or SEQ ID NO:5. More particularly, the ectodomain of 4-1BBL comprises the amino acid sequence of SEQ ID NO:5.

In a further aspect, the TNF family ligand trimer-containing antigen binding molecule of the invention comprises (a) at least one Fab molecule capable of specific binding to PD1 comprising a VH domain comprising (i) HVR-H1 comprising the amino acid sequence of SEQ ID NO:13, (ii) HVR-H2 comprising the amino acid sequence of SEQ ID NO:14, and (iii) HVR-H3 comprising the amino acid sequence of SEQ ID NO: 15, and a VL domain comprising (iv) HVR-L1 comprising the amino acid sequence of SEQ ID NO: 16, (v) HVR-L2 comprising the amino acid sequence of SEQ ID NO: 17, and (vi) HVR-L3 comprising the amino acid sequence of SEQ ID NO: 18, and (b) a first and a second polypeptide that are linked to each other by a disulfide bond, wherein the antigen binding molecule is characterized in that the first polypeptide comprises the amino acid sequence selected from the group consisting of SEQ ID NO:9, SEQ ID NO:11, SEQ ID NO:12 and SEQ ID NO: 10 and in that the second polypeptide comprises the amino acid sequence selected from the group consisting of SEQ ID NO:1, SEQ ID NO:3, SEQ ID NO:4 and SEQ ID NO:5.

In one aspect, the 4-1BBL trimer-containing antigen binding molecule of the invention comprises
(a) at least one Fab molecule capable of specific binding to PD1 comprising a VH domain comprising (i) HVR-H1 comprising the amino acid sequence of SEQ ID NO:13, (ii) HVR-H2 comprising the amino acid sequence of SEQ ID NO:14, and (iii) HVR-H3 comprising the amino acid sequence of SEQ ID NO: 15, and a VL domain comprising (iv) HVR-L1 comprising the amino acid sequence of SEQ ID NO: 16, (v) HVR-L2 comprising the amino acid sequence of SEQ ID NO: 17, and (vi) HVR-L3 comprising the amino acid sequence of SEQ ID NO: 18, and
(b) a first and a second polypeptide that are linked to each other by a disulfide bond, wherein the antigen binding molecule is characterized in that the first polypeptide comprises the amino acid sequence of SEQ ID NO:10 and in that the second polypeptide comprises the amino acid sequence of SEQ ID NO:5.

The invention provides a 4-1BBL trimer-containing antigen binding molecule that comprises at least one Fab molecule capable of specific binding to PD1. In a particular aspect, the 4-1BBL trimer-containing antigen binding molecule comprises one Fab molecule capable of specific binding to PD1, meaning the 4-1BBL trimer-containing antigen binding molecule is monovalent. In another aspect, the invention provides a 4-1BBL trimer-containing antigen binding molecule comprising two Fab molecules capable of specific binding to PD1, meaning the 4-1BBL trimer-containing antigen binding molecule is bivalent.

In a particular aspect, the invention provides a 4-1BBL trimer-containing antigen binding molecule, wherein the Fab molecule capable of specific binding to PD1 comprises
(a) a VH domain comprising an amino acid sequence of SEQ ID NO:19 and a VL domain comprising an amino acid sequence of SEQ ID NO:20,
(b) a VH domain comprising an amino acid sequence of SEQ ID NO:21 and a VL domain comprising an amino acid sequence of SEQ ID NO:22,
(c) a VH domain comprising an amino acid sequence of SEQ ID NO:21 and a VL domain comprising an amino acid sequence of SEQ ID NO:23,
(d) a VH domain comprising an amino acid sequence of SEQ ID NO:21 and a VL domain comprising an amino acid sequence of SEQ ID NO:24, or
(e) a VH domain comprising an amino acid sequence of SEQ ID NO:21 and a VL domain comprising an amino acid sequence of SEQ ID NO:25.

In one aspect, provided is a 4-1BBL trimer-containing antigen binding molecule as defined herein before, wherein the moiety capable of specific binding to PD1 comprises a variable heavy chain comprising an amino acid sequence of SEQ ID NO:21 and a variable light chain comprising an amino acid sequence of SEQ ID NO:22 or wherein the Fab molecule capable of specific binding to PD1 comprises a variable heavy chain comprising an amino acid sequence of SEQ ID NO:21 and a variable light chain comprising an amino acid sequence of SEQ ID NO:24.

In a particular aspect, the invention relates to a 4-1BBL trimer-containing antigen binding molecule as defined above, wherein the peptide linker is (G4S)₂, i.e. a peptide linker of SEQ ID NO:117. In one aspect, the peptide linker in all instances is (G4S)₂.

The invention is further concerned with a 4-1BBL trimer-containing antigen binding molecule as defined herein before, comprising an Fc domain composed of a first and a second subunit capable of stable association.

More particularly, the Fc domain is an IgG1 Fc domain. In a particular aspect, the Fc domain comprises a modification promoting the association of the first and second subunit of the Fc domain. In a specific aspect, the invention provides a 4-1BBL trimer-containing antigen binding molecule, wherein the first subunit of the Fc domain comprises the amino acid substitutions S354C and T366W (numbering according to Kabat EU index) and the second subunit of the Fc domain comprises the amino acid substitutions Y349C, T366S, L368A and Y407V (numbering according to Kabat EU index).

Provided is further a 4-1BBL trimer-containing antigen binding molecule, wherein in the CL domain adjacent to the 4-1BBL the amino acid at position 123 (EU numbering) has been replaced by arginine (R) and the amino acid at position 124 (EU numbering) has been substituted by lysine (K), and wherein in the CH1 domain adjacent to the 4-1BBL the amino acids at position 147 (EU numbering) and at position 213 (EU numbering) have been substituted by glutamic acid (E).

In a further aspect, provided is a 4-1BBL trimer-containing antigen binding molecule as described herein before, wherein the antigen binding molecule comprises
(a) a first heavy chain and a first light chain, both comprising a Fab molecule capable of specific binding to PD1,
(b) a second heavy chain comprising an amino acid sequence selected from the group consisting of SEQ ID NO:9, SEQ ID NO:11, SEQ ID NO:12 and SEQ ID NO:10, and
a second light chain comprising an amino acid sequence selected from the group consisting of SEQ ID NO: 1, SEQ ID NO:3, SEQ ID NO:4 and SEQ ID NO:5.

In a particular aspect, provided is a 4-1BBL trimer-containing antigen binding molecule, wherein the antigen binding molecule comprises
(i) a first heavy chain comprising the VH domain comprising the amino acid sequence of SEQ ID NO:19 and a first light chain comprising the VL domain comprising the amino acid sequence of SEQ ID NO:20 or
   a first heavy chain comprising the VH domain comprising the amino acid sequence of SEQ ID NO:21 and a first light chain comprising the VL domain comprising the amino acid sequence selected from the group consisting of SEQ ID NO:22, SEQ ID NO:23, SEQ ID NO:24 and SEQ ID NO:25,
(ii) a second heavy chain comprising the amino acid sequence selected from the group consisting of SEQ ID NO:74, SEQ ID NO:76, SEQ ID NO:78 and SEQ ID NO:80, and
(iii) a second light chain comprising the amino acid sequence selected from the group consisting of SEQ ID NO:75, SEQ ID NO:77, SEQ ID NO:79 and SEQ ID NO:81..

In another aspect, the invention provides a 4-1BBL trimer-containing antigen binding molecule, wherein the antigen binding molecule comprises
(a) a first heavy chain comprising the amino acid sequence of SEQ ID NO:82, a first light chain comprising the amino acid sequence of SEQ ID NO:83 or SEQ ID NO: 171, a second heavy chain comprising the amino acid sequence of SEQ ID NO:74 and a second light chain comprising the amino acid sequence of SEQ ID NO:75,
(b) a first heavy chain comprising the amino acid sequence of SEQ ID NO:82, a first light chain comprising the amino acid sequence of SEQ ID NO:83 or SEQ ID NO: 171, a second heavy chain comprising the amino acid sequence of SEQ ID NO:76 and a second light chain comprising the amino acid sequence of SEQ ID NO:77,
(c) a first heavy chain comprising the amino acid sequence of SEQ ID NO:82, a first light chain comprising the amino acid sequence of SEQ ID NO:83 or SEQ ID NO: 171, a second heavy chain comprising the amino acid sequence of SEQ ID NO:78 and a second light chain comprising the amino acid sequence of SEQ ID NO:79, or
(d) a first heavy chain comprising the amino acid sequence of SEQ ID NO:82, a first light chain comprising the amino acid sequence of SEQ ID NO:83 or SEQ ID NO: 171, a second heavy chain comprising the amino acid sequence of SEQ ID NO:80 and a second light chain comprising the amino acid sequence of SEQ ID NO:81.

According to another aspect of the invention, there is provided an isolated polynucleotide encoding a 4-1BBL trimer-containing antigen binding molecule as defined herein before. The invention further provides a vector, particularly an expression vector, comprising the isolated polynucleotide of the invention and a host cell comprising the isolated polynucleotide or the vector of the invention. In some aspects the host cell is an eukaryotic cell, particularly a mammalian cell.

In another aspect, provided is a method for producing the 4-1BBL trimer-containing antigen binding molecule of the invention, comprising culturing the host cell of the invention under conditions suitable for expression of the 4-1BBL trimer-containing antigen binding molecule, and isolating the antigen 4-1BBL trimer-containing binding molecule. The invention also encompasses a 4-1BBL trimer-containing antigen binding molecule produced by the method of the invention.

The invention further provides a pharmaceutical composition comprising the 4-1BBL trimer-containing antigen binding molecule of the invention and at least one pharmaceutically acceptable excipient.

Also encompassed by the invention is the 4-1BBL trimer-containing antigen binding molecule of the invention, or the pharmaceutical composition of the invention, for use as a medicament. In one aspect is provided the 4-1BBL trimer-containing antigen binding molecule of the invention, or the pharmaceutical composition of the invention, for use in the treatment of a disease in an individual in need thereof. In a specific embodiment, provided is the 4-1BBL trimer-containing antigen binding molecule of the invention, or the pharmaceutical composition of the invention, for use in the treatment of cancer. In another aspect, provided is the 4-1BBL trimer-containing antigen binding molecule of the invention, or the pharmaceutical composition of the invention, for use in up-regulating or prolonging cytotoxic T cell activity.

In any of the above embodiments the individual is preferably a mammal, particularly a human.

### BRIEF DESCRIPTION OF THE DRAWINGS

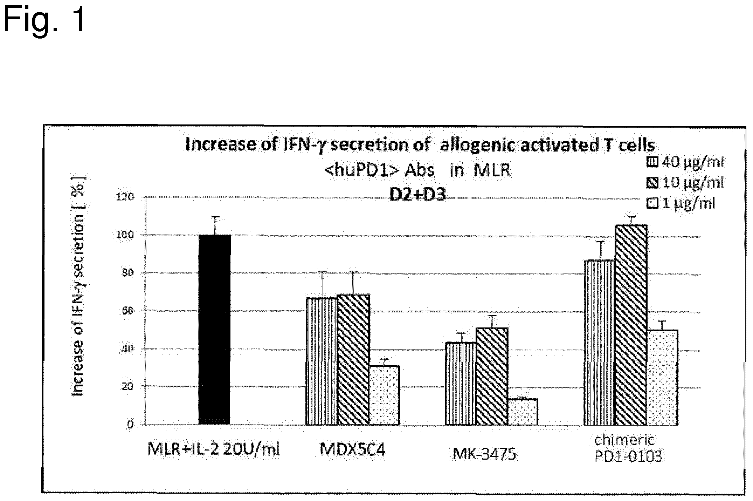

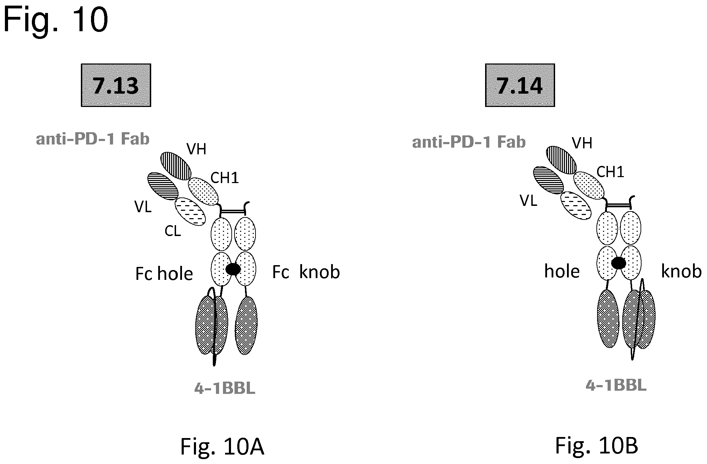

**Figure 1** shows that blockade of PD1 with chimeric antibody clone PD1-0103 strongly enhances IFN-gamma secretion by allogenic stimulated primary human T cells. IFN-gamma secretion is higher compared to reference antibodies MDX5C4 or MK-3475.

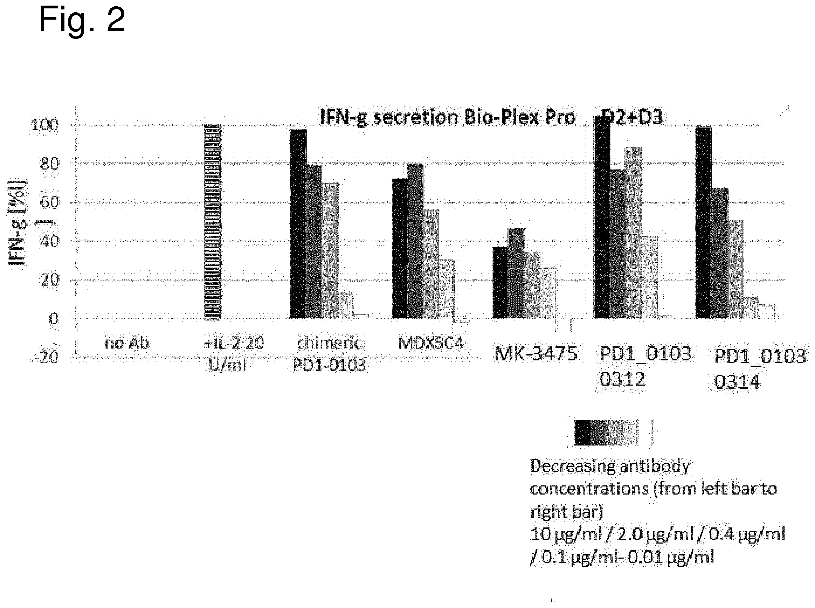

In **Figure 2** it is shown that blockade of PD1 humanized clones PD1-0103-0312 and PD1-103-314 even stronger increases interferon-gamma (IFN-g) secretion by allogenic stimulated primary human T cells as compared to chimeric clone PD1-0103.

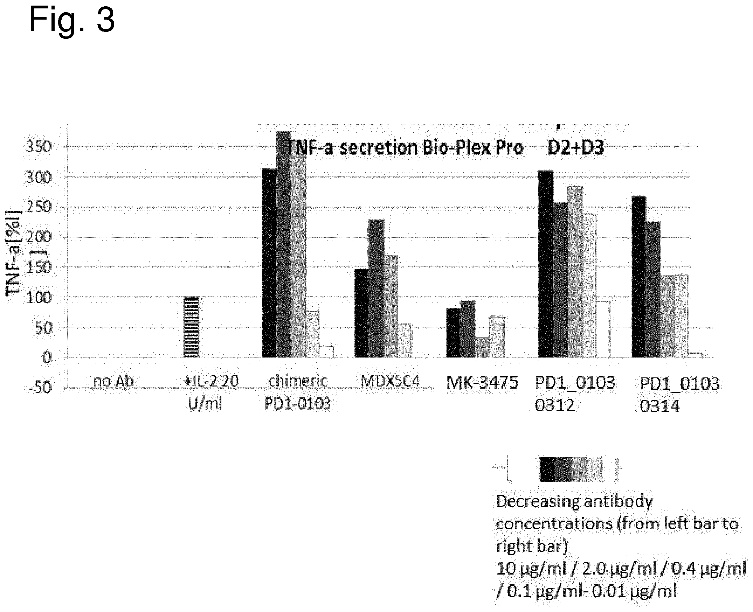

**Figure 3** demonstrates that the blockade of PD1 with chimeric antibody clone PD1-0103 and humanized clones PD1-0103-0312 and PD1-103-314 strongly increases tumor necrosis factor alpha (TNF) secretion by allogenic stimulated primary human T cells. TNF-alpha secretion is higher compared to reference antibodies MDX5C4 or MK-3475.

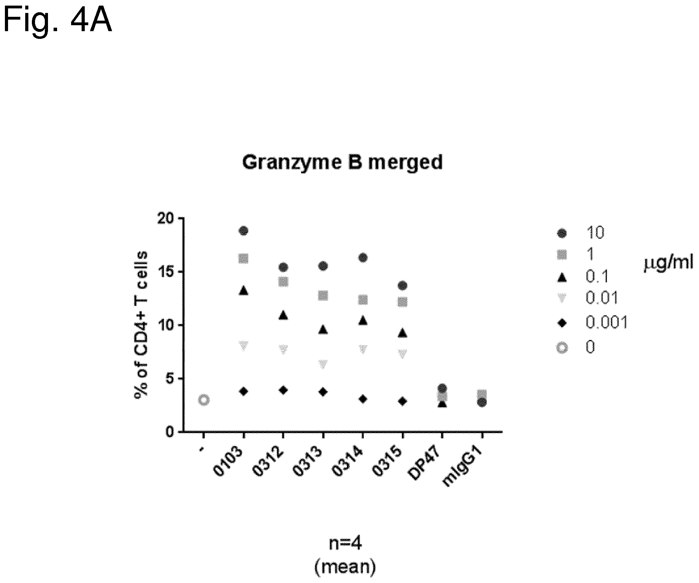

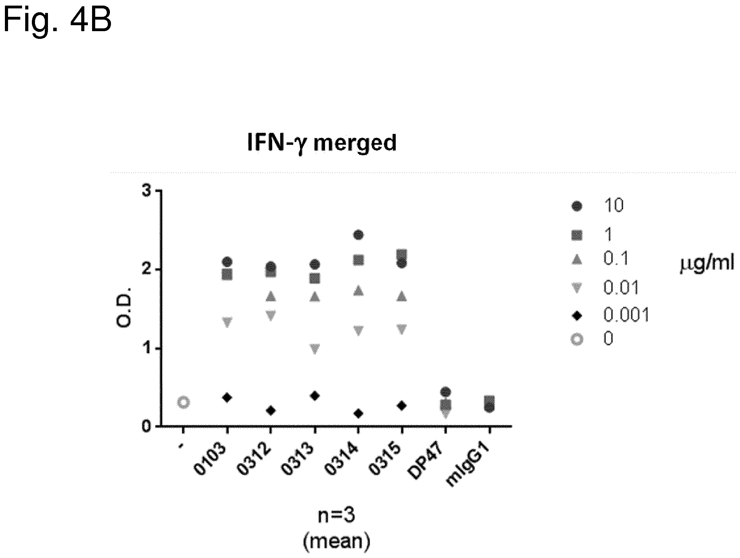

**Figure 4A** shows the frequency of CD4⁺ T cells producing Granzyme B in the presence of increasing concentrations of different anti-PD1 antibodies (chimeric antibody clone PD1-0103 and humanized clones PD1-0103-0312, PD1-0103-0313, PD1-0103-0314, PD1-0103-0315) and **Figure 4B** shows the amount of IFN-γ detected by absorbance (Optical Density, O.D.) in the supernatant of the MLR in presence of increasing concentrations of different anti-PD1 antibodies.

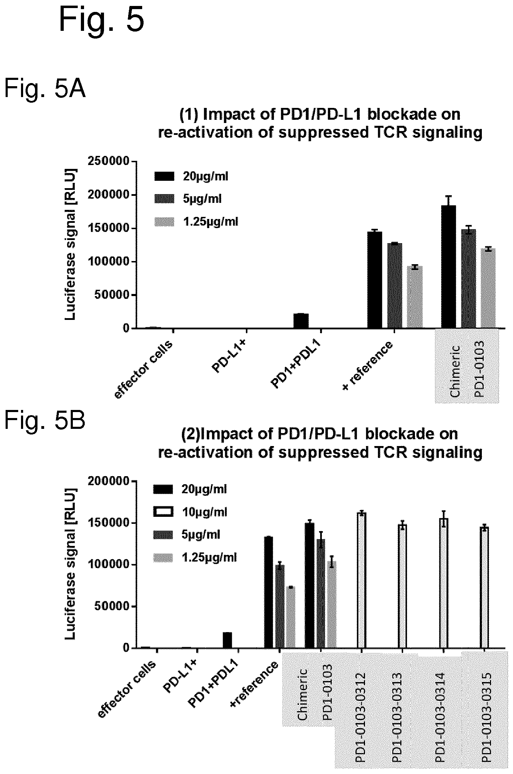

In **Figure 5A** is shown the impact of PD1/PD-L1 blockade on reactivation of suppressed T cell receptor signaling in presence of chimeric anti-PD1 antibody clone PD1-0103 in comparison to PDL1 alone and in **Figure 5B** the impact of PD1/PD-L1 blockade on reactivation of suppressed T cell receptor signaling in presence of different anti-PD1 antibodies (chimeric antibody clone PD1-0103 and humanized clones PD1-0103-0312, PD1-0103-0313, PD1-0103-0314, PD1-0103-0315) is presented.

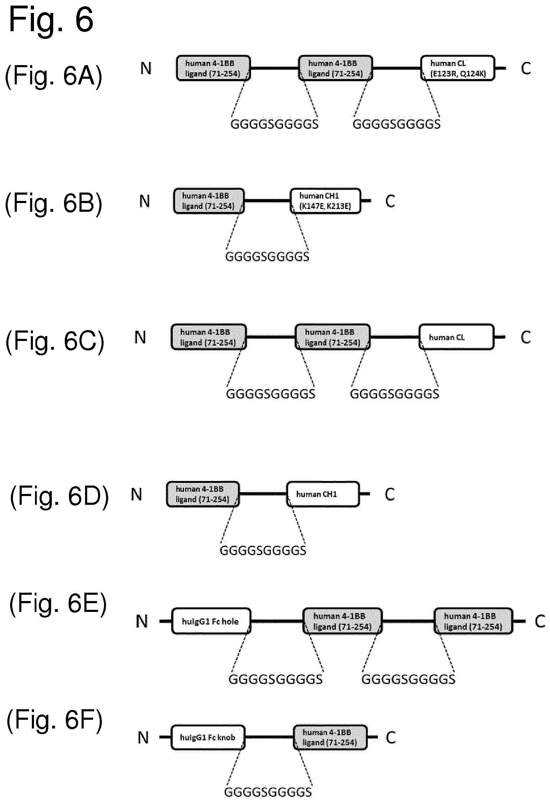

**Figure 6** shows the components for the assembly of split trimeric human 4-1BB ligands. **Fig. 6A** shows the dimeric 4-1BB(71-254) ligand that is fused at the C-terminus to a human CL domain with mutations E123R and Q124K (charged variant) and **Fig. 6B** shows the monomeric 4-1BB(71-254) ligand fused at its C-terminus to human CH1 domain with mutations K147E and K213E (charged variant). **Fig. 6C** shows the dimeric 4-1BB(71-254) ligand that is fused at the C-terminus to a human CL domain without the mutations and **Fig. 6D** shows the monomeric 4-1BB(71-254) ligand fused at its C-terminus to human CH1 domain without the mutations. **Fig. 1E** shows the dimeric 4-1BB(71-254)ligand that is fused at the N-terminus to a human IgG1 Fc hole domain and **Fig. 1F** shows the monomeric 4-1BB(71-254)ligand fused at the N-terminus to a human IgG1 Fc knob domain.

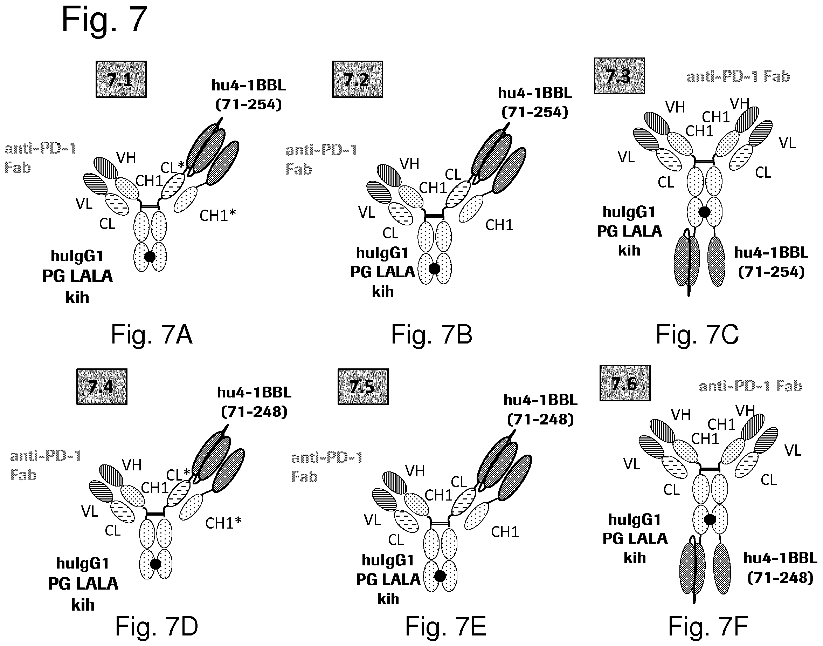

**Figure 7** illustrates schematically the structures of the 4-1BBL-trimer-containing antigen binding molecules Constructs 7.1 to 7.6 of the invention. The preparation and production of these constructs is described in Example 2. The VH and VL domains are those of the anti-PD1 antibody (in particular 0103-0314), the thick black point stands for the knob-into-hole modification. * symbolizes amino acid modifications in the CH1 and CL domain (so-called charged variant). **Fig. 7A** shows the construct 7.1 with charged variants and hu 4-1BBL(71-254), a corresponding construct without charged variants is shown in **Fig. 7B****.** Construct 7.4 as shown in **Fig. 7D** comprises three hu 4-1BBL(71-248) and charged variants, the corresponding construct without charged variants is illustrated in **Fig 7E. Fig. 7C** shows a bivalent construct with hu 4-1BBL (71-254) at the C-termini of the Fc chains and a corresponding construct with hu 4-1BBL(71-248) is shown in **Fig. 7F****.**

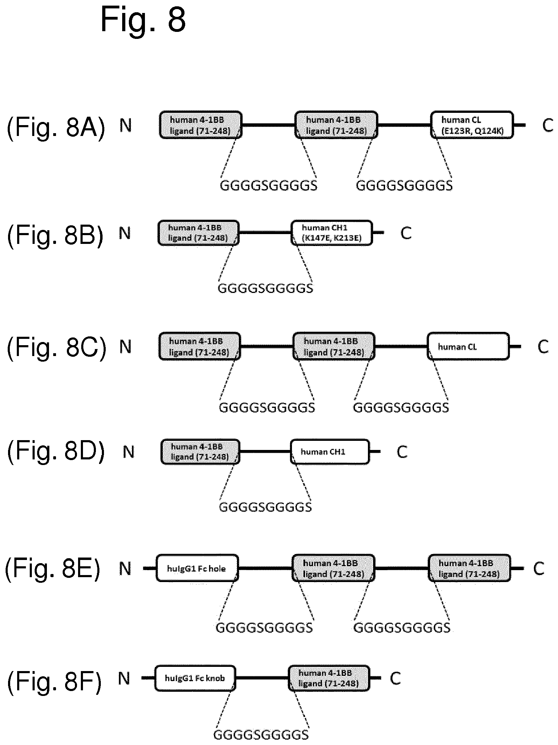

**Figure 8** shows the components for the assembly of split trimeric human 4-1BB ligands. **Fig. 8A** shows the dimeric 4-1BB(71-248) ligand that is fused at the C-terminus to a human CL domain with mutations E123R and Q124K (charged variant) and **Fig. 8B** shows the monomeric 4-1BB(71-248) ligand fused at its C-terminus to human CH1 domain with mutations K147E and K213E (charged variant). **Fig. 8C** shows the dimeric 4-1BB(71-248) ligand that is fused at the C-terminus to a human CL domain without the mutations and **Fig. 8D** shows the monomeric 4-1BB(71-248) ligand fused at its C-terminus to human CH1 domain without the mutations. **Fig. 8E** shows the dimeric 4-1BB(71-248) ligand that is fused at the N-terminus to a human IgG1 Fc hole domain and **Fig. 8F** shows the monomeric 4-1BB(71-248)ligand fused at the N-terminus to a human IgG1 Fc knob domain.

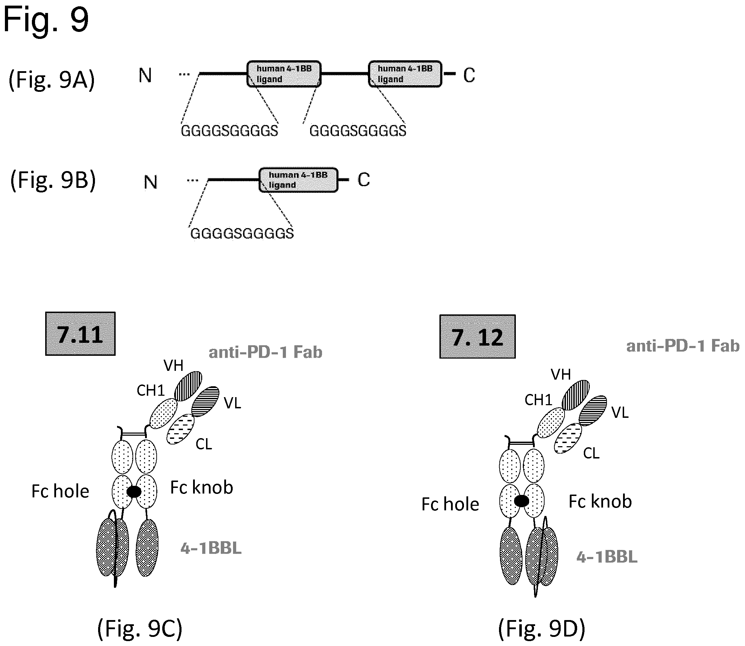

**Figure 9** shows the components for the assembly of split trimeric human 4-1BB ligand, wherein each of dimeric 4-1BB ligand and the monomeric 4-1BB ligand is fused at its N-terminus to another polypeptide chain, in particular one of the subunits of an Fc domain. **Fig. 9A** shows the dimeric ligand that is fused at the N-terminus and **Fig. 9B** shows the monomeric ligand fused at the N-terminus. Schematic drawings of the 4-1BBL-trimer-containing antigen binding molecules Constructs 7.11 and 7.12 are shown in **Fig. 9C** and **Fig. 9D****,** respectively. In Fig. 9C is shown an antigen binding molecule, wherein the Fab domain capable of specific binding to PD1 is attached to the Fc knob chain, the dimeric 4-1BBL is fused to the C-terminus of the Fc hole chain and the monomeric 4-1BBL is fused to the C-terminus of the Fc knob chain. Fig. 9D shows an antigen binding molecule, wherein the Fab domain capable of specific binding to PD1 is attached to the Fc knob chain, the dimeric 4-1BBL is also fused to the C-terminus of the Fc knob chain and the monomeric 4-1BBL is fused to the C-terminus of the Fc hole chain.
Schematic drawings of Constructs 7.13 and 7.14 are shown in **Fig. 10A** and **Fig. 10B****,** respectively. In Fig. 10A is shown an antigen binding molecule, wherein the Fab domain capable of specific binding to PD1 is attached to the Fc hole chain, the dimeric 4-1BBL is fused to the C-terminus of the Fc hole chain and the monomeric 4-1BBL is fused to the C-terminus of the Fc knob chain. In Fig. 10B is shown an antigen binding molecule, wherein wherein the Fab domain capable of specific binding to a target cell antigen is attached to the Fc hole chain, the dimeric 4-1BBL is also fused to the C-terminus of the Fc knob chain and the monomeric 4-1BBL is fused to the C-terminus of the Fc hole chain.

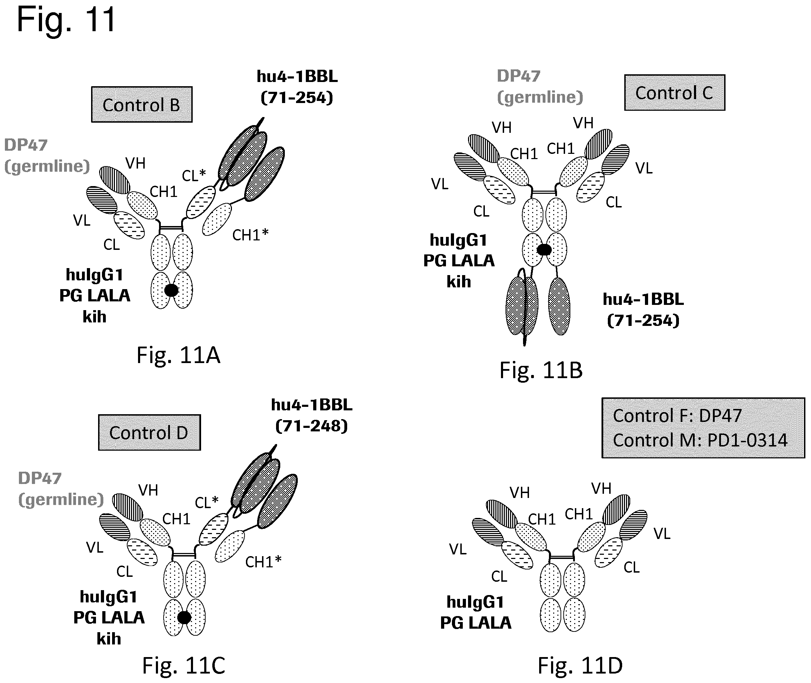

In **Figures 11A to 11D** schematic drawings of the control molecules are shown. **Fig. 11A** and **Fig. 11B** show the "untargeted" variants of Constructs 7.1 and 7.3, respectively, wherein the anti-PD1 Fab domains are replaced by a DP47 (germline) Fab domain. The molecules are named Control B and Control C, respectively. **Fig. 11C** shows the untargeted variant of Construct 7.4 named Control D. The preparation of the control molecules is described in Example 2.9. **Fig. 11D** is a drawing of monospecific IgG1 molecules with the PGLALA mutations, wherein the Fab domains are DP47 Fab domains (Control F) or PD1 0103-0314 Fab domains (Control M). These molecules are described in Example 2.10.

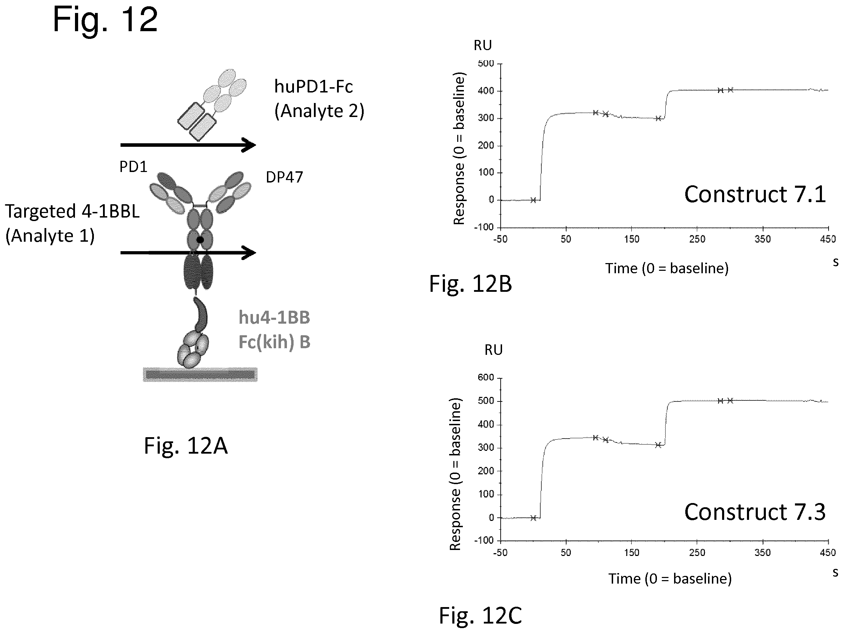

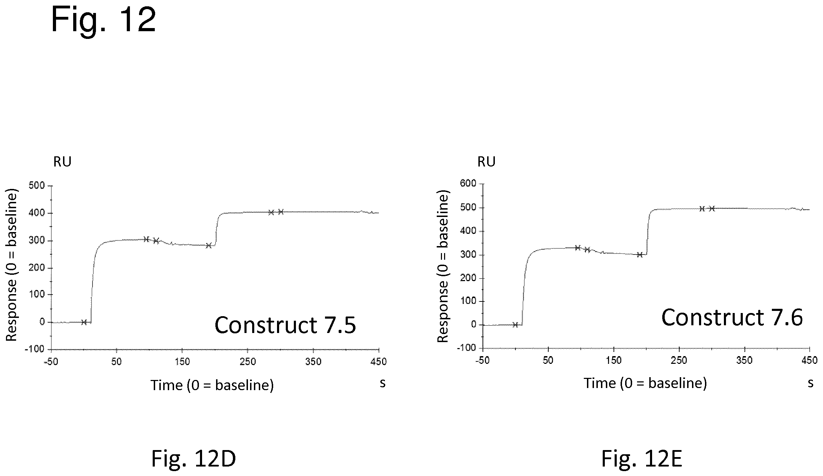

**Figure 12A** shows the setup of the SPR experiments for simultaneous binding of the PD1 targeted split trimeric C-terminal 4-1BB ligand-containing antigen binding molecules of the invention. The simultaneous binding to human 4-1BB and human PD1 of the PD1(0314)-targeted split 4-1BBL (71-254) Fc kih antigen binding molecule Construct 7.1 is shown in **Fig. 12B****.** Simultaneous binding to human 4-1BB and human PD1 of the bivalent PD1(0314) targeted split 4-1BBL (71-254) Fc kih antigen binding molecule (Construct 7.3) is shown in **Fig. 12C****.** Simultaneous binding to human 4-1BB and human PD1 of the PD1(0314) targeted split 4-1BBL (71-248) Fc kih antigen binding molecule (construct 7.5) is shown in **Fig. 12D** and simultaneous binding to human 4-1BB and human PD1 of the bivalent PD1(0314) targeted split 4-1BBL (71-248) Fc kih antigen binding molecule (Construct 7.6) is shown in **Fig. 12E****.**

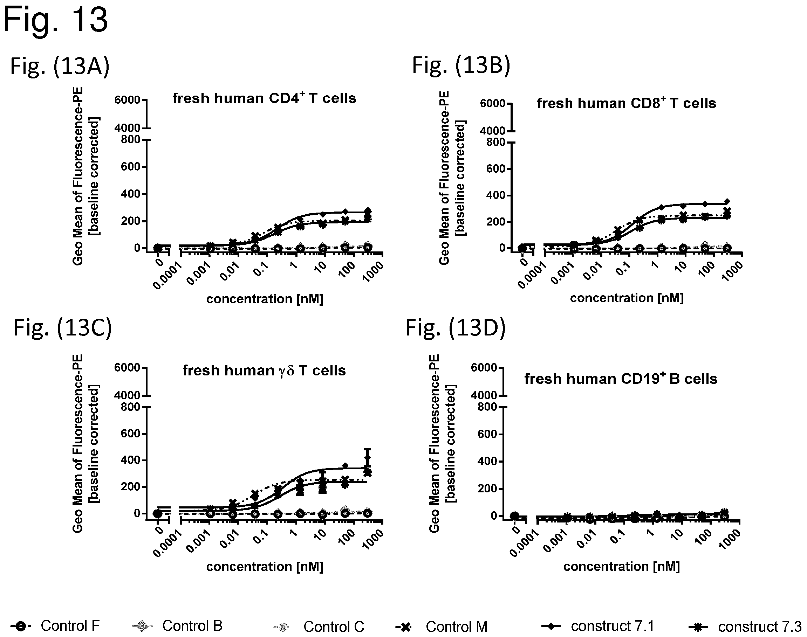

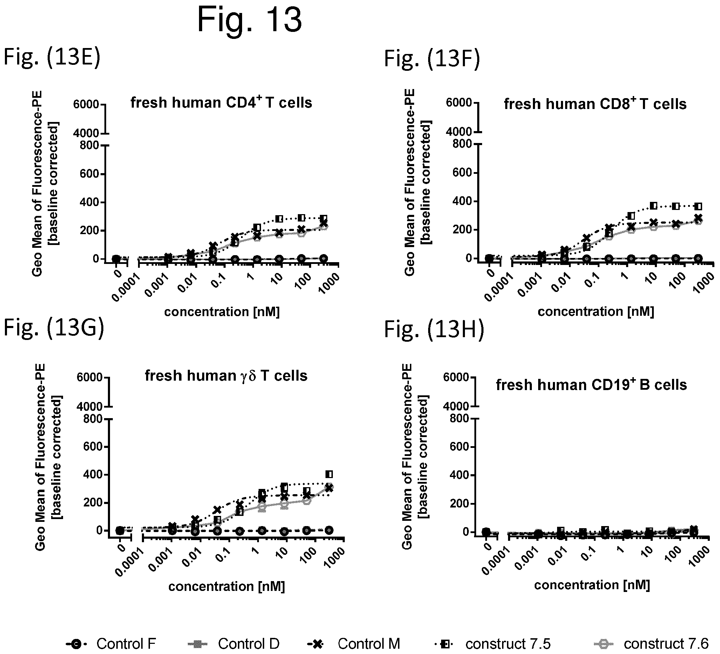

**Figures 13A to 13H** show the binding of PD1-targeted 4-1BB split trimeric ligand Fc fusion antigen binding molecules to freshly isolated PBMCs as described in Example 5.1. Shown is the geo mean of fluorescence intensity of a PE-conjugated secondary detection antibody (y-axis) versus the concentration of tested constructs (x-axis). For a better overview the graphs were split in two, Fig. (13A), (13B), (13C) and (13D) and Fig. (13E), (13F), (13G) and (13H), respectively, whereby the curves for Control F and Control M were shown in all graphs as references. Binding was monitored on fresh human CD45⁺ CD3⁺ CD4⁺ T cells (Figures (13A) and (13E)), fresh human CD45⁺ CD3⁺ CD8⁺ T cells (Figures (13B) and (13F)), fresh human CD45⁺ CD3⁺ CD4^{neg} CD8^{neg} γδ T cells (Figures (13C) and (13G)) and CD45⁺ CD3^{neg} CD19⁺ B cells (Figure (13D) and (13H)).

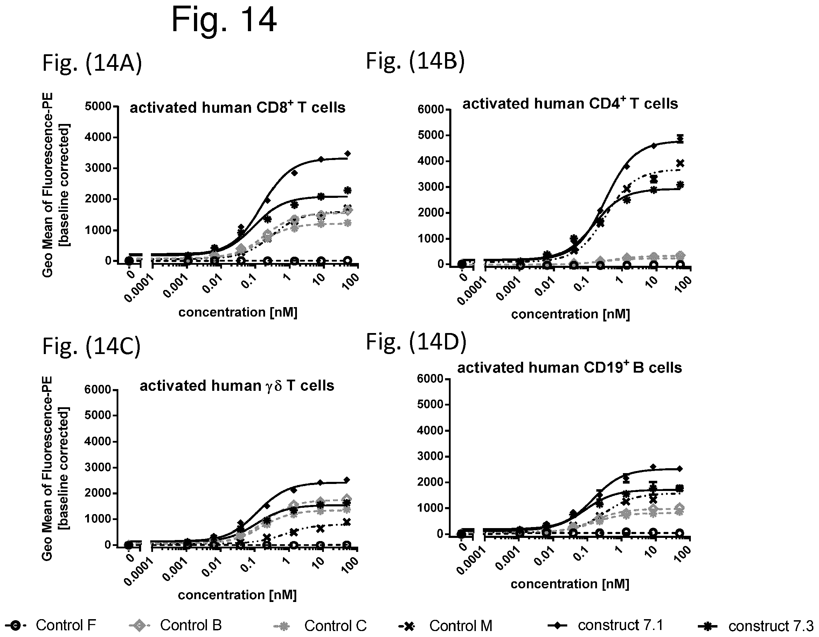

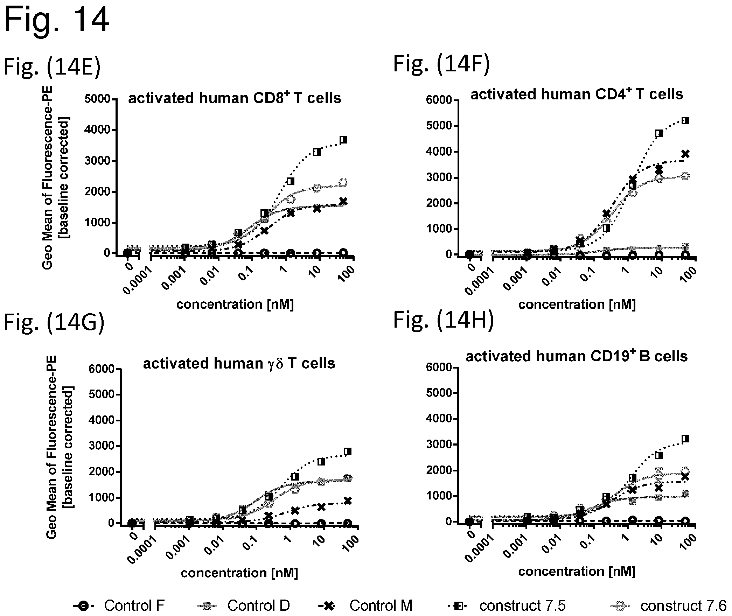

**Figures 14A to 14H** illustrate the binding of PD1-targeted split trimeric 4-1BB ligand Fc fusion antigen binding molecules to activated human PBMCs which was tested as described in Example 5.1. Shown is the geo mean of fluorescence intensity of PE-conjugated secondary detection antibody (y-axis) versus the concentration of tested constructs (x-axis). As negative control control F was used. For a better overview the graphs were split in two, i.e. in Figures (14A), (14B), (14C) and (14D) and in Figures (14E), (14F), (14G) and (14H) whereby the curves of Control F and Control M are shown in all graphs as references. After activation with agonistic anti-human CD28 and anti-human CD3 antibody 4-1BB expression is upregulated on the majority of CD45⁺ CD3⁺ CD8⁺ T cells (see Figures (14A) or (14E)) or CD45⁺ CD3⁺ CD4⁺ T cells (see Figures (14B) and (14F)) and to a lesser extend to the majority of human CD45⁺ CD3⁺ CD4^{neg} CD8^{neg} γδ T cells (Figures (14C) or (14G)) or CD45⁺ CD3^{neg} CD19⁺ B cells (Figures (14D) or (14H)

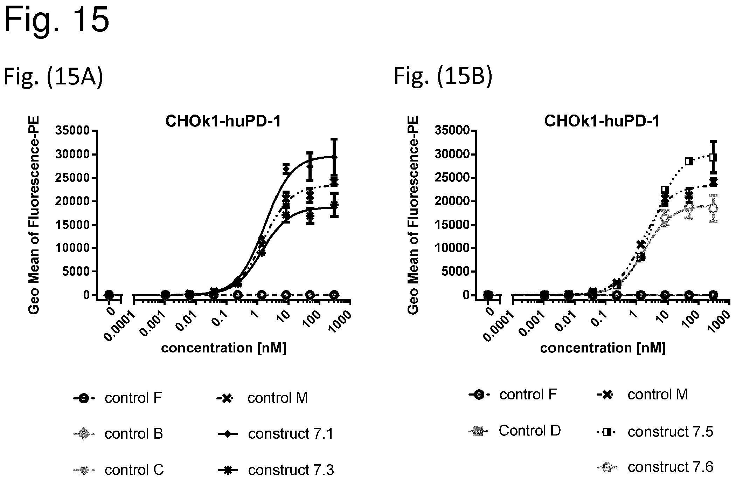

**Figures 15A and 15B** show the binding of PD1-targeted or untargeted split trimeric human 4-1BB ligand Fc (kih) fusion antigen binding molecules to human-PD1 expressing CHO-k1-huPD1 clone 5 cells. The method is described in Example 5.2. Binding was detected with R-Phycoerythrin-fluorochrome conjugated anti-human IgG Fcy-specific goat IgG F(ab')2 fragment and the geo mean measured by flow cytometry is shown on the y-axis. On the x-axis the used concentration of PD1 targeted 4-1BB split trimeric ligand Fc fusion antigen binding molecules and their controls are shown. For a better overview the graphs were split in two (Figure (15A) and Figure (15B)) whereby the curves for control F and control M are shown in both as references.

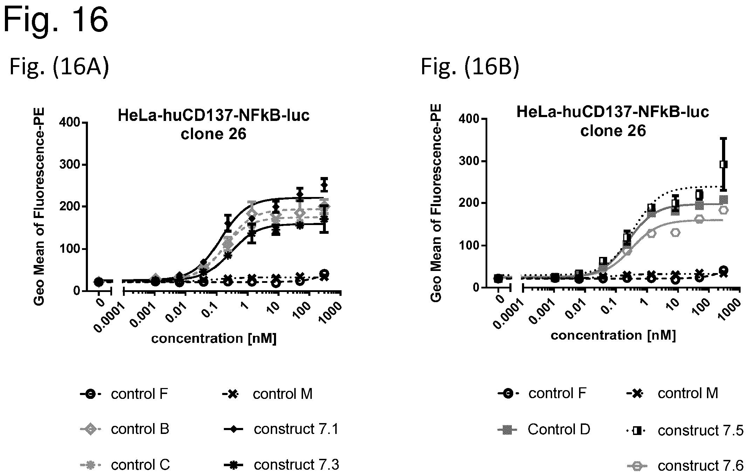

**Figures 16A and 16B** show the binding of PD1-targeted or untargeted split trimeric human 4-1BB ligand Fc (kih) fusion antigen binding molecules to human-4-1BB expressing HeLa-hu4-1BBL-NFkB-luc clone 26 cells as tested as described in Example 5.2. Binding was detected with R-Phycoerythrin-fluorochrome conjugated anti-human IgG Fcy-specific goat IgG F(ab')2 fragment and the geo mean measured by flow cytometry is shown on the y-axis. On the x-axis the used concentration of PD1 targeted 4-1BB split trimeric ligand Fc fusion antigen binding molecules and their controls are shown. For a better overview the graphs were split in two, named Figures (16A) and (16B), whereby the curves of control F and control M are shown in both graphs as references.

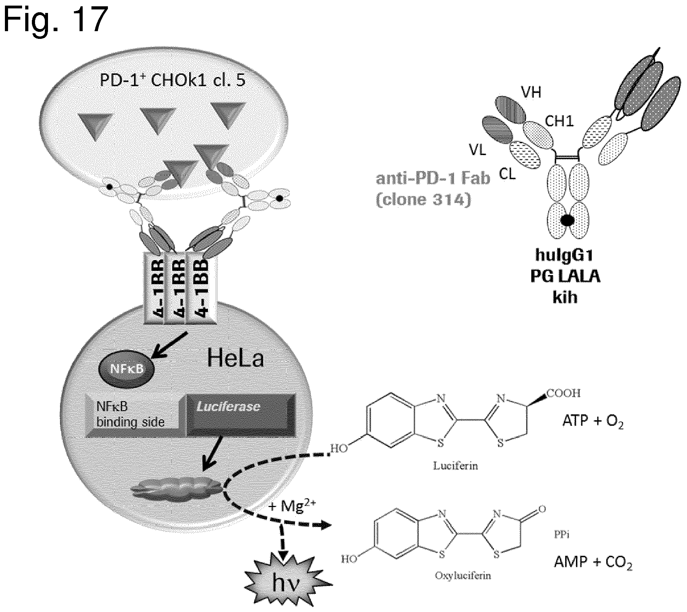

**Figure 17** shows a scheme that illustrates the general principal of the NFkB activity assay described in Example 6 using a reporter cell line. Shown is the activation assay set up with human 4-1BB expressing HeLa reporter cell line. A crosslinking of 4-1BB expressed on the reporter cells induces NFκB activation and NFκB-mediated luciferase expression. After lysis of the cells luciferase can catalyze the oxidation of luciferin to oxyluciferin. This chemical reaction correlates positively with the strength of NFκB-mediated luciferase expression and can be measured by the strength of light emission (units of released light).

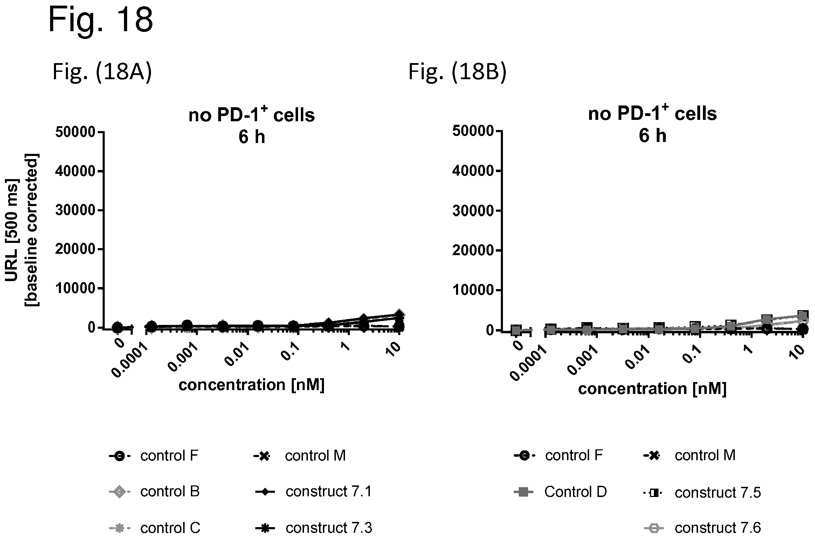

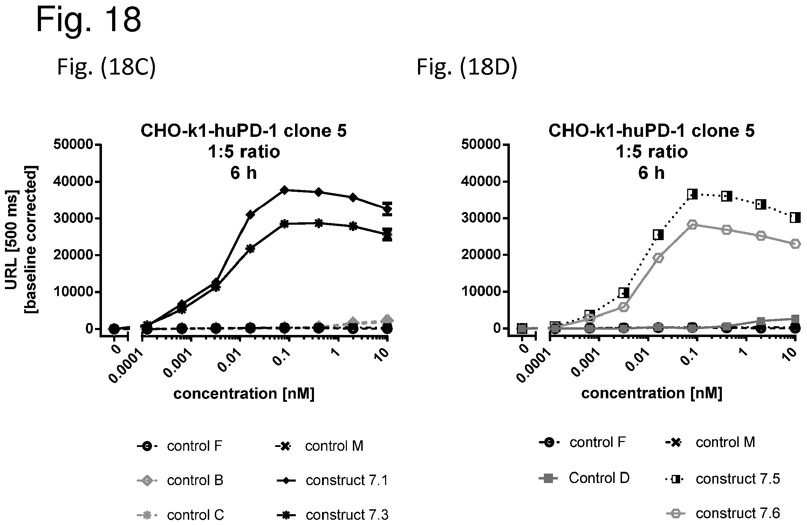

**Figures 18A to 18D** shows the NFκB-activation-induced luciferase expression and activity as measured with the assay described in Example 6. Units of released light (URL) are measured for 0.5 s/well and plotted against the used concentration of PD1-targeted or untargeted split trimeric human 4-1BB ligand Fc (kih) constructs. Human 4-1BB-expressing HeLa-reporter cells were incubated for 6 h in the absence (Figures (18A) and (18B)) or presence of crosslinking human-PD1 expressing CHO-k1-huPD1 clone 5 cells (Figure (18C) and (18D). The cell ratio of human 4-1BB-expressing HeLa reporter cell to CHO-k1-huPD1 clone 5 cells is indicated in each graph.

### DETAILED DESCRIPTION OF THE INVENTION

### Definitions

Unless defined otherwise, technical and scientific terms used herein have the same meaning as generally used in the art to which this invention belongs. For purposes of interpreting this specification, the following definitions will apply and whenever appropriate, terms used in the singular will also include the plural and vice versa.

As used herein, the term **"antigen binding molecule"** refers in its broadest sense to a molecule that specifically binds an antigenic determinant. Examples of antigen binding molecules are antibodies, antibody fragments and scaffold antigen binding proteins.

As used herein, the term **"moiety capable of specific binding to PD1"** or "antigen binding domain capable of specific binding to PD1" refers to a polypeptide molecule that specifically binds to PD1. In one aspect, the antigen binding moiety is able to activate signaling through PD1. In a particular aspect, the antigen binding moiety is able to direct the entity to which it is attached (e.g. the TNF family ligand trimer) to a target site, for example to a specific type of tumor cell bearing PD1 or on T cells bearing PD1. Moieties capable of specific binding to PD1 include antibodies and fragments thereof as further defined herein. In addition, moieties capable of specific binding to a target cell antigen include scaffold antigen binding proteins as further defined herein, e.g. binding domains which are based on designed repeat proteins or designed repeat domains (see e.g. WO 2002/020565).

In relation to an antibody or fragment thereof, the term "moiety capable of specific binding to a target cell antigen" refers to the part of the molecule that comprises the area which specifically binds to and is complementary to part or all of an antigen. A moiety capable of specific antigen binding may be provided, for example, by one or more antibody variable domains (also called antibody variable regions). Particularly, a moiety capable of specific antigen binding comprises an antibody light chain variable region (VL) and an antibody heavy chain variable region (VH).

The term **"antibody"** herein is used in the broadest sense and encompasses various antibody structures, including but not limited to monoclonal antibodies, polyclonal antibodies, monospecific and multispecific antibodies (e.g., bispecific antibodies), and antibody fragments so long as they exhibit the desired antigen-binding activity.

The term **"monoclonal antibody"** as used herein refers to an antibody obtained from a population of substantially homogeneous antibodies, i.e., the individual antibodies comprising the population are identical and/or bind the same epitope, except for possible variant antibodies, e.g. containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen.

The term **"monospecific"** antibody as used herein denotes an antibody that has one or more binding sites each of which bind to the same epitope of the same antigen. The term **"bispecific"** means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term **"valent"** as used within the current application denotes the presence of a specified number of binding sites in an antigen binding molecule. As such, the terms "bivalent", "tetravalent", and "hexavalent" denote the presence of two binding sites, four binding sites, and six binding sites, respectively, in an antigen binding molecule.

The terms "full length antibody", "intact antibody", and "whole antibody" are used herein interchangeably to refer to an antibody having a structure substantially similar to a native antibody structure. **"Native antibodies"** refer to naturally occurring immunoglobulin molecules with varying structures. For example, native IgG-class antibodies are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a light chain constant domain (CL), also called a light chain constant region. The heavy chain of an antibody may be assigned to one of five types, called α (IgA), δ (IgD), ε (IgE), γ (IgG), or µ (IgM), some of which may be further divided into subtypes, e.g. γ1 (IgG1), γ2 (IgG2), γ3 (IgG3), γ4 (IgG4), α1 (IgA1) and α2 (IgA2). The light chain of an antibody may be assigned to one of two types, called kappa (κ) and lambda (λ), based on the amino acid sequence of its constant domain.

An **"antibody fragment"** refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies, triabodies, tetrabodies, cross-Fab fragments; linear antibodies; single-chain antibody molecules (e.g. scFv); and single domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046. Diabodies are antibody fragments with two antigen-binding sites that may be bivalent or bispecific, see, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

Papain digestion of intact antibodies produces two identical antigen-binding fragments, called "Fab" fragments containing each the heavy- and light-chain variable domains and also the constant domain of the light chain and the first constant domain (CH1) of the heavy chain. As used herein, Thus, the term **"Fab fragment"** refers to an antibody fragment comprising a light chain fragment comprising a VL domain and a constant domain of a light chain (CL), and a VH domain and a first constant domain (CH1) of a heavy chain. Fab' fragments differ from Fab fragments by the addition of a few residues at the carboxy terminus of the heavy chain CH1 domain including one or more cysteins from the antibody hinge region. Fab'-SH are Fab' fragments in which the cysteine residue(s) of the constant domains bear a free thiol group. Pepsin treatment yields an F(ab')₂ fragment that has two antigen-combining sites (two Fab fragments) and a part of the Fc region.

The term **"cross-Fab fragment"** or "xFab fragment" or "crossover Fab fragment" refers to a Fab fragment, wherein either the variable regions or the constant regions of the heavy and light chain are exchanged. Two different chain compositions of a crossover Fab molecule are possible and comprised in the bispecific antibodies of the invention: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as CrossFab _{(VLVH)}. On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as CrossFab_{(CLCH1)}.

A "single chain Fab fragment" or **"scFab"** is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CH1-linker-VL-CL, b) VL-CL-linker-VH-CH1, c) VH-CL-linker-VL-CH1 or d) VL-CH1-linker-VH-CL; and wherein said linker is a polypeptide of at least 30 amino acids, preferably between 32 and 50 amino acids. Said single chain Fab fragments are stabilized via the natural disulfide bond between the CL domain and the CH1 domain. In addition, these single chain Fab molecules might be further stabilized by generation of interchain disulfide bonds via insertion of cysteine residues (e.g. position 44 in the variable heavy chain and position 100 in the variable light chain according to Kabat numbering).

A "crossover single chain Fab fragment" or **"x-scFab"** is a is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CL-linker-VL-CH1 and b) VL-CH1-linker-VH-CL; wherein VH and VL form together an antigen-binding site which binds specifically to an antigen and wherein said linker is a polypeptide of at least 30 amino acids. In addition, these x-scFab molecules might be further stabilized by generation of interchain disulfide bonds via insertion of cysteine residues (e.g. position 44 in the variable heavy chain and position 100 in the variable light chain according to Kabat numbering).

A **"single-chain variable fragment (scFv)"** is a fusion protein of the variable regions of the heavy (V_{H}) and light chains (V_{L}) of an antibody, connected with a short linker peptide of ten to about 25 amino acids. The linker is usually rich in glycine for flexibility, as well as serine or threonine for solubility, and can either connect the N-terminus of the V_{H} with the C-terminus of the V_{L}, or *vice versa.* This protein retains the specificity of the original antibody, despite removal of the constant regions and the introduction of the linker. scFv antibodies are, e.g. described in Houston, J.S., Methods in Enzymol. 203 (1991) 46-96). In addition, antibody fragments comprise single chain polypeptides having the characteristics of a VH domain, namely being able to assemble together with a VL domain, or of a VL domain, namely being able to assemble together with a VH domain to a functional antigen binding site and thereby providing the antigen binding property of full length antibodies.

**"Scaffold antigen binding proteins"** are known in the art, for example, fibronectin and designed ankyrin repeat proteins (DARPins) have been used as alternative scaffolds for antigen-binding domains, see, e.g., Gebauer and Skerra, Engineered protein scaffolds as next-generation antibody therapeutics. Curr Opin Chem Biol 13:245-255 (2009) and Stumpp et al., Darpins: A new generation of protein therapeutics. Drug Discovery Today 13: 695-701 (2008). In one aspect of the invention, a scaffold antigen binding protein is selected from the group consisting of CTLA-4 (Evibody), Lipocalins (Anticalin), a Protein A-derived molecule such as Z-domain of Protein A (Affibody), an A-domain (Avimer/Maxibody), a serum transferrin (*trans*-body); a designed ankyrin repeat protein (DARPin), a variable domain of antibody light chain or heavy chain (single-domain antibody, sdAb), a variable domain of antibody heavy chain (nanobody, aVH), V_{NAR} fragments, a fibronectin (AdNectin), a C-type lectin domain (Tetranectin); a variable domain of a new antigen receptor beta-lactamase (V_{NAR} fragments), a human gamma-crystallin or ubiquitin (Affilin molecules); a kunitz type domain of human protease inhibitors, microbodies such as the proteins from the knottin family, peptide aptamers and fibronectin (adnectin).

CTLA-4 (Cytotoxic T Lymphocyte-associated Antigen 4) is a CD28-family receptor expressed on mainly CD4+ T-cells. Its extracellular domain has a variable domain- like Ig fold. Loops corresponding to CDRs of antibodies can be substituted with heterologous sequence to confer different binding properties. CTLA-4 molecules engineered to have different binding specificities are also known as Evibodies (e.g. US7166697B1). Evibodies are around the same size as the isolated variable region of an antibody (e.g. a domain antibody). For further details see Journal of Immunological Methods 248 (1-2), 31-45 (2001).

Lipocalins are a family of extracellular proteins which transport small hydrophobic molecules such as steroids, bilins, retinoids and lipids. They have a rigid beta-sheet secondary structure with a number of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633.

An affibody is a scaffold derived from Protein A of Staphylococcus aureus which can be engineered to bind to antigen. The domain consists of a three-helical bundle of approximately 58 amino acids. Libraries have been generated by randomization of surface residues. For further details see Protein Eng. Des. Sel. 17, 455-462 (2004) and EP 1641818A1.

Avimers are multidomain proteins derived from the A-domain scaffold family. The native domains of approximately 35 amino acids adopt a defined disulfide bonded structure. Diversity is generated by shuffling of the natural variation exhibited by the family of A-domains. For further details see Nature Biotechnology 23(12), 1556 - 1561 (2005) and Expert Opinion on Investigational Drugs 16(6), 909-917 (June 2007).

A transferrin is a monomeric serum transport glycoprotein. Transferrins can be engineered to bind different target antigens by insertion of peptide sequences in a permissive surface loop. Examples of engineered transferrin scaffolds include the Trans-body. For further details see J. Biol. Chem 274, 24066-24073 (1999).

Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two alpha-helices and a beta-turn. They can be engineered to bind different target antigens by randomizing residues in the first alpha-helix and a beta-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1.

A single-domain antibody is an antibody fragment consisting of a single monomeric variable antibody domain. The first single domain were derived from the variable domain of the antibody heavy chain from camelids (nanobodies or V_{H}H fragments). Furthermore, the term single-domain antibody includes an autonomous human heavy chain variable domain (aVH) or V_{NAR} fragments derived from sharks.

Fibronectin is a scaffold which can be engineered to bind to antigen. Adnectins consists of a backbone of the natural amino acid sequence of the 10th domain of the 15 repeating units of human fibronectin type III (FN3). Three loops at one end of the beta-sandwich can be engineered to enable an Adnectin to specifically recognize a therapeutic target of interest. For further details see Protein Eng. Des. Sel. 18, 435- 444 (2005), US20080139791, WO2005056764 and US6818418B1.

Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005).

Microbodies are derived from naturally occurring microproteins of 25-50 amino acids in length which contain 3-4 cysteine bridges - examples of microproteins include KalataBI and conotoxin and knottins. The microproteins have a loop which can beengineered to include upto 25 amino acids without affecting the overall fold of the microprotein. For further details of engineered knottin domains, see WO2008098796.

An **"antigen binding molecule that binds to the same epitope"** as a reference molecule refers to an antigen binding molecule that blocks binding of the reference molecule to its antigen in a competition assay by 50% or more, and conversely, the reference molecule blocks binding of the antigen binding molecule to its antigen in a competition assay by 50% or more.

The term **"antigen binding domain"** refers to the part of an antigen binding molecule that comprises the area which specifically binds to and is complementary to part or all of an antigen. Where an antigen is large, an antigen binding molecule may only bind to a particular part of the antigen, which part is termed an epitope. An antigen binding domain may be provided by, for example, one or more variable domains (also called variable regions). Preferably, an antigen binding domain comprises an antibody light chain variable region (VL) and an antibody heavy chain variable region (VH).

As used herein, the term **"antigenic determinant"** is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins useful as antigens herein can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants.

The term **"capable of specific binding to PD1"** refers to an antigen binding molecule that is capable of binding PD1 with sufficient affinity such that the antigen binding molecule is useful as a diagnostic and/or therapeutic agent in targeting PD1. The antigen binding molecule includes but is not limited to, antibodies, Fab molecules, crossover Fab molecules, single chain Fab molecules, Fv molecules, scFv molecules, single domain antibodies, and VH and scaffold antigen binding protein. In one aspect, the extent of binding of an anti-PD1 antigen binding molecule to an unrelated, non-PD1 protein is less than about 10% of the binding of the antigen binding molecule to PD1 as measured, e.g., by a radioimmunoassay (RIA). In particular, an antigen binding molecule that is capable of specific binding to PD1 has a dissociation constant (K_{d}) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M). In certain embodiments, an anti-PD1 antigen binding molecule binds to PD1 from different species. In particular, the anti-PD1 antigen binding molecule binds to human and cynomolgus PD1.

By **"specific binding"** is meant that the binding is selective for the antigen and can be discriminated from unwanted or non-specific interactions. The ability of an antigen binding molecule to bind to a specific antigen can be measured either through an enzyme-linked immunosorbent assay (ELISA) or other techniques familiar to one of skill in the art, e.g. Surface Plasmon Resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding molecule to an unrelated protein is less than about 10% of the binding of the antigen binding molecule to the antigen as measured, e.g. by SPR. In certain embodiments, an molecule that binds to the antigen has a dissociation constant (Kd) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g. from 10⁻⁹ M to 10⁻¹³ M).

**"Affinity"** or "binding affinity" refers to the strength of the sum total of non-covalent interactions between a single binding site of a molecule (e.g. an antibody) and its binding partner (e.g. an antigen). Unless indicated otherwise, as used herein, "binding affinity" refers to intrinsic binding affinity which reflects a 1:1 interaction between members of a binding pair (e.g. antibody and antigen). The affinity of a molecule X for its partner Y can generally be represented by the dissociation constant (Kd), which is the ratio of dissociation and association rate constants (k_{off} and kₒₙ, respectively). Thus, equivalent affinities may comprise different rate constants, as long as the ratio of the rate constants remains the same. Affinity can be measured by common methods known in the art, including those described herein. A particular method for measuring affinity is Surface Plasmon Resonance (SPR).

A **"target cell antigen"** as used herein refers to an antigenic determinant presented on the surface of a target cell, for example a T-cell or B-cell, a cell in a tumor such as a cancer cell or a cell of the tumor stroma. In certain embodiments, the target cell antigen is an antigen on the surface of T cell. In one embodiment, the target cell antigen is PD1.

The term **"PD1",** also known as Programmed cell death protein 1, is a type I membrane protein of 288 amino acids that was first described in 1992 (Ishida et al., EMBO J., 11 (1992), 3887-3895). PD1 is a member of the extended CD28/CTLA-4 family of T cell regulators and has two ligands, PD-L1 (B7-H1, CD274) and PD-L2 (B7-DC, CD273). The protein's structure includes an extracellular IgV domain followed by a transmembrane region and an intracellular tail. The intracellular tail contains two phosphorylation sites located in an immunoreceptor tyrosine-based inhibitory motif and an immunoreceptor tyrosine-based switch motif, which suggests that PD1 negatively regulates TCR signals. This is consistent with binding of SHP-1 and SHP-2 phosphatases to the cytoplasmic tail of PD1 upon ligand binding. While PD1 is not expressed on naive T cells, it is upregulated following T cell receptor (TCR)-mediated activation and is observed on both activated and exhausted T cells (Agata et al., Int. Immunology 8 (1996), 765-772). These exhausted T-cells have a dysfunctional phenotype and are unable to respond appropriately. Although PD1 has a relatively wide expression pattern its most important role is likely as a coinhibitory receptor on T cells (Chinai et al, Trends in Pharmacological Sciences 36 (2015), 587-595). Current therapeutic approaches thus focus on blocking the interaction of PD1 with its ligands to enhance T cell response. The terms "Programmed Death 1, ""Programmed Cell Death 1," "Protein PD-1," "PD-1," PD1," "PDCD1," "hPD-1" and "hPD-I" can be used interchangeably, and include variants, isoforms, species homologs of human PD1, and analogs having at least one common epitope with PD1. The amino acid sequence of human PD1 is shown in UniProt (www.uniprot.org) accession no. Q15116 (SEQ ID NO:94).

The term **"variable region"** or "variable domain" refers to the domain of an antibody heavy or light chain that is involved in binding the antigen binding molecule to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen binding specificity.

The term **"hypervariable region"** or "HVR," as used herein refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (HI), 53-55 (H2), and 96-101 (H3). (Chothia and Lesk, J. Mol. Biol. 196:901-917 (1987)). Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins ofImmunological Interest, 5th Ed. Public Health Service, National Institutes of Health, Bethesda, MD (1991).) Hypervariable regions (HVRs) are also referred to as complementarity determining regions (CDRs), and these terms are used herein interchangeably in reference to portions of the variable region that form the antigen binding regions. This particular region has been described by Kabat et al., U.S. Dept. of Health and Human Services, "Sequences of Proteins of Immunological Interest" (1983) and by Chothia et al., J. Mol. Biol. 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table A as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE A. CDR Definitions¹**

| **CDR** | **Kabat** | **Chothia** | **AbM²** |
|---|---|---|---|
| V_{H} CDR1 | 31-35 | 26-32 | 26-35 |
| V_{H} CDR2 | 50-65 | 52-58 | 50-58 |
| V_{H} CDR3 | 95-102 | 95-102 | 95-102 |
| V_{L} CDR1 | 24-34 | 26-32 | 24-34 |
| V_{L} CDR2 | 50-56 | 50-52 | 50-56 |
| V_{L} CDR3 | 89-97 | 91-96 | 89-97 |

| | | | |
|---|---|---|---|
| ¹Numbering of all CDR definitions in Table A is according to the numbering conventions set forth by Kabat et al. (see below). ² "AbM" with a lowercase "b" as used in Table A refers to the CDRs as defined by Oxford Molecular's "AbM" antibody modeling software. | | | |

Kabat *et al.* also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra.*

As used herein, the term **"affinity matured"** in the context of antigen binding molecules (e.g., antibodies) refers to an antigen binding molecule that is derived from a reference antigen binding molecule, e.g., by mutation, binds to the same antigen, preferably binds to the same epitope, as the reference antibody; and has a higher affinity for the antigen than that of the reference antigen binding molecule. Affinity maturation generally involves modification of one or more amino acid residues in one or more CDRs of the antigen binding molecule. Typically, the affinity matured antigen binding molecule binds to the same epitope as the initial reference antigen binding molecule.

**"Framework"** or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

An **"acceptor human framework"** for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term **"chimeric"** antibody refers to an antibody in which a portion of the heavy and/or light chain is derived from a particular source or species, while the remainder of the heavy and/or light chain is derived from a different source or species.

The **"class"** of an antibody refers to the type of constant domain or constant region possessed by its heavy chain. There are five major classes of antibodies: IgA, IgD, IgE, IgG, and IgM, and several of these may be further divided into subclasses (isotypes), e.g. IgG₁, IgG₂, IgG₃, IgG₄, IgA₁, and IgA₂. The heavy chain constant domains that correspond to the different classes of immunoglobulins are called α, δ, ε, γ, and µ respectively..

A **"humanized"** antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A **"humanized form"** of an antibody, e.g., a non-human antibody, refers to an antibody that has undergone humanization. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

A **"human"** antibody is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

The term "Fc domain" or **"Fc region"** herein is used to define a C-terminal region of an antibody heavy chain that contains at least a portion of the constant region. The term includes native sequence Fc regions and variant Fc regions. An IgG Fc region comprises an IgG CH2 and an IgG CH3 domain. The "CH2 domain" of a human IgG Fc region usually extends from an amino acid residue at about position 231 to an amino acid residue at about position 340. In one embodiment, a carbohydrate chain is attached to the CH2 domain. The CH2 domain herein may be a native sequence CH2 domain or variant CH2 domain. The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protuberance" ("knob") in one chain thereof and a corresponding introduced "cavity" ("hole") in the other chain thereof; see US Patent No. 5,82 ,333) . Such variant CH3 domains may be used to promote heterodimerization of two non-identical antibody heavy chains as herein described. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The **"knob-into-hole"** technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine). The protuberance and cavity can be made by altering the nucleic acid encoding the polypeptides, e.g. by site-specific mutagenesis, or by peptide synthesis. In a specific embodiment a knob modification comprises the amino acid substitution T366W in one of the two subunits of the Fc domain, and the hole modification comprises the amino acid substitutions T366S, L368A and Y407V in the other one of the two subunits of the Fc domain. In a further specific embodiment, the subunit of the Fc domain comprising the knob modification additionally comprises the amino acid substitution S354C, and the subunit of the Fc domain comprising the hole modification additionally comprises the amino acid substitution Y349C. Introduction of these two cysteine residues results in the formation of a disulfide bridge between the two subunits of the Fc region, thus further stabilizing the dimer (Carter, J Immunol Methods 248, 7-15 (2001)). The numbering is according to EU index of Kabat et al, Sequences of Proteins of Immunological Interest, 5th Ed. Public Health Service, National Institutes of Health, Bethesda, MD, 1991.

A "region equivalent to the Fc region of an immunoglobulin" is intended to include naturally occurring allelic variants of the Fc region of an immunoglobulin as well as variants having alterations which produce substitutions, additions, or deletions but which do not decrease substantially the ability of the immunoglobulin to mediate effector functions (such as antibody-dependent cellular cytotoxicity). For example, one or more amino acids can be deleted from the N-terminus or C-terminus of the Fc region of an immunoglobulin without substantial loss of biological function. Such variants can be selected according to general rules known in the art so as to have minimal effect on activity (see, e.g., Bowie, J. U. et al., Science 247:1306-10 (1990)).

The term **"effector functions"** refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

An **"activating Fc receptor"** is an Fc receptor that following engagement by an Fc region of an antibody elicits signaling events that stimulate the receptor-bearing cell to perform effector functions. Activating Fc receptors include FcγRIIIa (CD16a), FcγRI (CD64), FcγRIIa (CD32), and FcaRI (CD89). A particular activating Fc receptor is human FcγRIIIa (see UniProt accession no. P08637, version 141).

The term **"TNF ligand family member"** or "TNF family ligand" refers to a proinflammatory cytokine. Cytokines in general, and in particular the members of the TNF ligand family, play a crucial role in the stimulation and coordination of the immune system. At present, nineteen cyctokines have been identified as members of the TNF (tumour necrosis factor) ligand superfamily on the basis of sequence, functional, and structural similarities. All these ligands are type II transmembrane proteins with a C-terminal extracellular domain (ectodomain), N-terminal intracellular domain and a single transmembrane domain. The C-terminal extracellular domain, known as TNF homology domain (THD), has 20-30% amino acid identity between the superfamily members and is responsible for binding to the receptor. The TNF ectodomain is also responsible for the TNF ligands to form trimeric complexes that are recognized by their specific receptors.

Members of the TNF ligand family are selected from the group consisting of Lymphotoxin α (also known as LTA or TNFSF1), TNF (also known as TNFSF2), LTβ (also known as TNFSF3), OX40L (also known as TNFSF4), CD40L (also known as CD154 or TNFSF5), FasL (also known as CD95L, CD178 or TNFSF6), CD27L (also known as CD70 or TNFSF7), CD30L (also known as CD153 or TNFSF8), 4-1BBL (also known as TNFSF9), TRAIL (also known as APO2L, CD253 or TNFSF10), RANKL (also known as CD254 or TNFSF11), TWEAK (also known as TNFSF12), APRIL (also known as CD256 or TNFSF13), BAFF (also known as CD257 or TNFSF13B), LIGHT (also known as CD258 or TNFSF14), TL1A (also known as VEGI or TNFSF15), GITRL (also known as TNFSF18), EDA-A1 (also known as ectodysplasin A1) and EDA-A2 (also known as ectodysplasin A2). The term refers to any native TNF family ligand from any vertebrate source, including mammals such as primates (e.g. humans), non-human primates (e.g. cynomolgus monkeys) and rodents (e.g. mice and rats), unless otherwise indicated. In specific embodiments of the invention, the TNF ligand family member is selected from the group consisting of OX40L, FasL, CD27L, TRAIL, 4-1BBL, CD40L and GITRL. In a particular embodiment, the TNF ligand family member is selected from 4-1BBL and OX40L.

Further information, in particular sequences, of the TNF ligand family members may be obtained from publically accessible databases such as Uniprot (www.uniprot.org). For instance, the human TNF ligands have the following amino acid sequences: human Lymphotoxin α (UniProt accession no. P01374, SEQ ID NO:95), human TNF (UniProt accession no. P01375, SEQ ID NO:96), human Lymphotoxin β (UniProt accession no. Q06643, SEQ ID NO:97), human OX40L (UniProt accession no. P23510, SEQ ID NO:98), human CD40L (UniProt accession no. P29965, SEQ ID NO:99), human FasL (UniProt accession no. P48023, SEQ ID NO: 100), human CD27L (UniProt accession no. P32970, SEQ ID NO:101), human CD30L (UniProt accession no. P32971, SEQ ID NO:102), 4-1BBL (UniProt accession no. P41273, SEQ ID NO:103), TRAIL (UniProt accession no. P50591, SEQ ID NO:104), RANKL (UniProt accession no. 014788, SEQ ID NO:105), TWEAK (UniProt accession no. O43508, SEQ ID NO:106), APRIL (UniProt accession no. O75888, SEQ ID NO:107), BAFF (UniProt accession no. Q9Y275, SEQ ID NO:108), LIGHT (UniProt accession no. O43557, SEQ ID NO:109), TL1A (UniProt accession no. 095150, SEQ ID NO:110), GITRL (UniProt accession no. Q9UNG2, SEQ ID NO:111) and ectodysplasin A (UniProt accession no. Q92838, SEQ ID NO:112).

An **"ectodomain"** is the domain of a membrane protein that extends into the extracellular space (i.e. the space outside the target cell). Ectodomains are usually the parts of proteins that initiate contact with surfaces, which leads to signal transduction. The ectodomain of TNF ligand family member as defined herein thus refers to the part of the TNF ligand protein that extends into the extracellular space (the extracellular domain), but also includes shorter parts or fragments thereof that are responsible for the trimerization and for the binding to the corresponding TNF receptor. The term "ectodomain of a TNF ligand family member or a fragment thereof' thus refers to the extracellular domain of the TNF ligand family member that forms the extracellular domain or to parts thereof that are still able to bind to the receptor (receptor binding domain).

The term **"costimulatory TNF ligand family member"** or "costimulatory TNF family ligand" refers to a subgroup of TNF ligand family members, which are able to costimulate proliferation and cytokine production of T-cells. These TNF family ligands can costimulate TCR signals upon interaction with their corresponding TNF receptors and the interaction with their receptors leads to recruitment of TNFR-associated factors (TRAF), which initiate signalling cascades that result in T-cell activation. Costimulatory TNF family ligands are selected from the group consisting of 4-1BBL, OX40L, GITRL, CD70, CD30L and LIGHT, more particularly the costimulatory TNF ligand family member is selected from 4-1BBL and OX40L.

As described herein before, 4-1BBL is a type II transmembrane protein and one member of the TNF ligand family. Complete or full length 4-1BBL having the amino acid sequence of SEQ ID NO:103 has been described to form trimers on the surface of cells. The formation of trimers is enabled by specific motives of the ectodomain of 4-1BBL. Said motives are designated herein as "trimerization region". The amino acids 50-254 of the human 4-1BBL sequence (SEQ ID NO:113) form the extracellular domain of 4-1BBL, but even fragments thereof are able to form the trimers. In specific embodiments of the invention, the term "ectodomain of 4-1BBL or a fragment thereof' refers to a polypeptide having an amino acid sequence selected from SEQ ID NO:4 (amino acids 52-254 of human 4-1BBL), SEQ ID NO:1 (amino acids 71-254 of human 4-1BBL), SEQ ID NO:3 (amino acids 80-254 of human 4-1BBL) and SEQ ID NO:2 (amino acids 85-254 of human 4-1BBL) or a polypeptide having an amino acid sequence selected from SEQ ID NO:5 (amino acids 71-248 of human 4-1BBL), SEQ ID NO:8 (amino acids 52-248 of human 4-1BBL), SEQ ID NO:7 (amino acids 80-248 of human 4-1BBL) and SEQ ID NO:6 (amino acids 85-248 of human 4-1BBL), but also other fragments of the ectodomain capable of trimerization are included herein.

As described herein before, OX40L is another type II transmembrane protein and a further member of the TNF ligand family. Complete or full length human OX40L has the amino acid sequence of SEQ ID NO:98. The amino acids 51-183 of the human OX40L sequence (SEQ ID NO:114) form the extracellular domain of OX40L, but even fragments thereof that are able to form the trimers. In specific embodiments of the invention, the term "ectodomain of OX40L or a fragment thereof' refers to a polypeptide having an amino acid sequence selected from SEQ ID NO:114 (amino acids 51-183 of human OX40L) or SEQ ID NO:115 (amino acids 52-183 of human OX40L), but also other fragments of the ectodomain capable of trimerization are included herein.

The term **"peptide linker"** refers to a peptide comprising one or more amino acids, typically about 2 to 20 amino acids. Peptide linkers are known in the art or are described herein. Suitable, non-immunogenic linker peptides are, for example, (G₄S)ₙ, (SG₄)ₙ or G₄(SG₄)ₙ peptide linkers, wherein "n" is generally a number between 1 and 10, typically between 1 and 4, in particular 2, i.e. the peptides selected from the group consisting of GGGGS (SEQ ID NO:116), GGGGSGGGGS (SEQ ID NO: 117), SGGGGSGGGG (SEQ ID NO: 118), (G₄S)₃ or GGGGSGGGGSGGGGS (SEQ ID NO:119), GGGGSGGGGSGGGG or G4(SG4)₂ (SEQ ID NO:120), and (G₄S)₄ or GGGGSGGGGSGGGGSGGGGS (SEQ ID NO:121), but also include the sequences GSPGSSSSGS (SEQ ID NO: 122), GSGSGSGS (SEQ ID NO:123), GSGSGNGS (SEQ ID NO:124), GGSGSGSG (SEQ ID NO:125), GGSGSG (SEQ ID NO:126), GGSG (SEQ ID NO:127), GGSGNGSG (SEQ ID NO: 128), GGNGSGSG (SEQ ID NO:129) and GGNGSG (SEQ ID NO:130). Peptide linkers of particular interest are (G4S)₁ or GGGGS (SEQ ID NO:86), (G₄S)₂ or GGGGSGGGGS (SEQ ID NO: 117) and GSPGSSSSGS (SEQ ID NO: 122), more particularly (G₄S)₂ or GGGGSGGGGS (SEQ ID NO:117).

The term **"amino acid"** as used within this application denotes the group of naturally occurring carboxy α-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

A **"fusion polypeptide"** or "single fusion polypeptide" as used herein refers to a single chain polypeptide composed of one or two ectodomains of said TNF ligand family member fused to a part of antigen binding moiety or Fc part. The fusion may occur by directly linking the N or C-terminal amino acid of the antigen binding moiety via a peptide linker to the C- or N-terminal amino acid of the ectodomain of said TNF ligand family member.

By "fused" or "connected" is meant that the components (e.g. a polypeptide and an ectodomain of said TNF ligand family member) are linked by peptide bonds, either directly or via one or more peptide linkers.

**"Percent (%) amino acid sequence identity"** with respect to a reference polypeptide (protein) sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN. SAWI or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

In certain embodiments, **amino acid sequence variants** of the TNF ligand trimer-containing antigen binding molecules provided herein are contemplated. For example, it may be desirable to improve the binding affinity and/or other biological properties of the TNF ligand trimer-containing antigen binding molecules. Amino acid sequence variants of the TNF ligand trimer-containing antigen binding molecules may be prepared by introducing appropriate modifications into the nucleotide sequence encoding the molecules, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding. Sites of interest for substitutional mutagenesis include the HVRs and Framework (FRs). Conservative substitutions are provided in Table B under the heading "Preferred Substitutions" and further described below in reference to amino acid side chain classes (1) to (6). Amino acid substitutions may be introduced into the molecule of interest and the products screened for a desired activity, e.g., retained/improved antigen binding, decreased immunogenicity, or improved ADCC or CDC.

**TABLE B**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

The term **"amino acid sequence variants"** includes substantial variants wherein there are amino acid substitutions in one or more hypervariable region residues of a parent antigen binding molecule (*e.g.* a humanized or human antibody). Generally, the resulting variant(s) selected for further study will have modifications (e.g., improvements) in certain biological properties (e.g., increased affinity, reduced immunogenicity) relative to the parent antigen binding molecule and/or will have substantially retained certain biological properties of the parent antigen binding molecule. An exemplary substitutional variant is an affinity matured antibody, which may be conveniently generated, e.g., using phage display-based affinity maturation techniques such as those described herein. Briefly, one or more HVR residues are mutated and the variant antigen binding molecules displayed on phage and screened for a particular biological activity (e.g. binding affinity). In certain embodiments, substitutions, insertions, or deletions may occur within one or more HVRs so long as such alterations do not substantially reduce the ability of the antigen binding molecule to bind antigen. For example, conservative alterations (e.g., conservative substitutions as provided herein) that do not substantially reduce binding affinity may be made in HVRs. A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as Arg, Asp, His, Lys, and Glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antigen binding molecule complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include TNF family ligand trimer-containing antigen binding molecule with an N-terminal methionyl residue. Other insertional variants of the molecule include the fusion to the N- or C-terminus to a polypeptide which increases the serum half-life of the TNF ligand trimer-containing antigen binding molecules.

In certain aspects, the TNF family ligand trimer-containing antigen binding molecules provided herein are altered to increase or decrease the extent to which the antibody is glycosylated. Glycosylation variants of the molecules may be conveniently obtained by altering the amino acid sequence such that one or more glycosylation sites is created or removed. Where the TNF ligand trimer-containing antigen binding molecule comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in TNF family ligand trimer-containing antigen binding molecule may be made in order to create variants with certain improved properties. In one aspect, variants of TNF family ligand trimer-containing antigen binding molecules are provided having a carbohydrate structure that lacks fucose attached (directly or indirectly) to an Fc region. Such fucosylation variants may have improved ADCC function, see e.g. US Patent Publication Nos. US 2003/0157108 (Presta, L.) or US 2004/0093621 (Kyowa Hakko Kogyo Co., Ltd). Further variants of the TNF family ligand trimer-containing antigen binding molecules of the invention include those with bisected oligosaccharides, e.g., in which a biantennary oligosaccharide attached to the Fc region is bisected by GlcNAc. Such variants may have reduced fucosylation and/or improved ADCC function., see for example WO 2003/011878 (Jean-Mairet et al.); US Patent No. 6,602,684 (Umana et al.); and US 2005/0123546 (Umana et al.). Variants with at least one galactose residue in the oligosaccharide attached to the Fc region are also provided. Such antibody variants may have improved CDC function and are described, e.g., in WO 1997/30087 (Patel et al.); WO 1998/58964 (Raju, S.); and WO 1999/22764 (Raju, S.).

In certain aspects, it may be desirable to create **cysteine engineered variants** of the TNF family ligand trimer-containing antigen binding molecule of the invention, e.g., "thioMAbs," in which one or more residues of the molecule are substituted with cysteine residues. In particular aspects, the substituted residues occur at accessible sites of the molecule. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain aspects, any one or more of the following residues may be substituted with cysteine: V205 (Kabat numbering) of the light chain; A118 (EU numbering) of the heavy chain; and S400 (EU numbering) of the heavy chain Fc region. Cysteine engineered antigen binding molecules may be generated as described, e.g., in U.S. Patent No. 7,521,541.

In certain aspects, the TNF family ligand trimer-containing antigen binding molecules provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the bispecific antibody derivative will be used in a therapy under defined conditions, etc. In another aspect, conjugates of an antibody and non-proteinaceous moiety that may be selectively heated by exposure to radiation are provided. In one aspect, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

In another aspect, immunoconjugates of the TNF family ligand trimer-containing antigen binding molecules provided herein maybe obtained. An **"immunoconjugate"** is an antibody conjugated to one or more heterologous molecule(s), including but not limited to a cytotoxic agent.

The term **"polynucleotide"** refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By **"isolated"** nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present invention. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include in vivo or in vitro RNA transcripts of the present invention, as well as positive and negative strand forms, and double-stranded forms. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present invention can be determined conventionally using known computer programs, such as the ones discussed above for polypeptides (e.g. ALIGN-2).

The term **"expression cassette"** refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain aspects, the expression cassette of the invention comprises polynucleotide sequences that encode bispecific antigen binding molecules of the invention or fragments thereof.

The term **"vector"** or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector of the present invention comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one aspect, the expression vector of the invention comprises an expression cassette that comprises polynucleotide sequences that encode bispecific antigen binding molecules of the invention or fragments thereof.

The terms **"host cell",** "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules of the present invention. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An **"effective amount"** of an agent refers to the amount that is necessary to result in a physiological change in the cell or tissue to which it is administered.

A **"therapeutically effective amount"** of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An **"individual"** or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term **"pharmaceutical composition"** refers to a preparation which is in such form as to permit the biological activity of an active ingredient contained therein to be effective, and which contains no additional components which are unacceptably toxic to a subject to which the formulation would be administered.

A **"pharmaceutically acceptable excipient"** refers to an ingredient in a pharmaceutical composition, other than an active ingredient, which is nontoxic to a subject. A pharmaceutically acceptable excipient includes, but is not limited to, a buffer, a stabilizer, or a preservative.

The term **"package insert"** is used to refer to instructions customarily included in commercial packages of therapeutic products, that contain information about the indications, usage, dosage, administration, combination therapy, contraindications and/or warnings concerning the use of such therapeutic products.

As used herein, **"treatment"** (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some aspects, the molecules of the invention are used to delay development of a disease or to slow the progression of a disease.

The term **"cancer"** as used herein refers to proliferative diseases, such as lymphomas, carcinoma, lymphoma, blastoma, sarcoma, leukemia, lymphocytic leukemias, lung cancer, non-small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colorectal cancer (CRC), pancreatic cancer, breast cancer, triple-negative breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma and Ewings sarcoma, melanoma, multiple myeloma, B-cell cancer (lymphoma), chronic lymphocytic leukemia (CLL), acute lymphoblastic leukemia (ALL), hairy cell leukemia, chronic myeloblastic leukemia, including refractory versions of any of the above cancers, or a combination of one or more of the above cancers.

### 4-1BB ligand (4-1BBL) trimer-containing antigen binding molecules

The disclosure provides novel 4-1BBL trimer-containing antigen binding molecules with particularly advantageous properties such as producibility, stability, binding affinity, biological activity, targeting efficiency and reduced toxicity.

In a first aspect, the disclosure provides a 4-1BBL trimer-containing antigen binding molecule comprising
(a) at least one moiety capable of specific binding to PD1 and
(b) a first and a second polypeptide that are linked to each other by a disulfide bond,
wherein the antigen binding molecule is characterized in that the first polypeptide comprises two ectodomains of 4-1BBL or a fragment thereof that are connected to each other by a peptide linker and in that the second polypeptide comprises only one ectodomain of said 4-1BBL or a fragment thereof.

In a particular aspect, the disclosure provides a 4-1BBL trimer-containing antigen binding molecule comprising
(a) at least one moiety capable of specific binding to PD1,
(b) a first and a second polypeptide that are linked to each other by a disulfide bond,
   wherein the antigen binding molecule is characterized in that the first polypeptide comprises two ectodomains of 4-1BBL or a fragment thereof that are connected to each other by a peptide linker and in that the second polypeptide comprises only one ectodomain of said 4-1BBL or a fragment thereof, and
(c) an Fc domain composed of a first and a second subunit capable of stable association.

In a particular aspect, the 4-1BBL trimer-containing antigen binding molecule comprises (a) at least one moiety capable of specific binding to PD1 and
(b) a first and a second polypeptide that are linked to each other by a disulfide bond,
wherein the antigen binding molecule is characterized in that the first polypeptide comprises two ectodomains of 4-1BBL or two fragments thereof that are connected to each other by a peptide linker and in that the second polypeptide comprises only one ectodomain of said 4-1BBL or a fragment thereof, wherein the 4-1BBL costimulates human T-cell activation.

In another particular aspect, the 4-1BBL trimer-containing antigen binding molecule comprises (a) at least one moiety capable of specific binding to PD1 and (b) a first and a second polypeptide that are linked to each other by a disulfide bond, wherein the antigen binding molecule is characterized in that the first polypeptide comprises two ectodomains of 4-1BBL or two fragments thereof that are connected to each other by a peptide linker and in that the second polypeptide comprises only one ectodomain of said 4-1BBL or a fragment thereof, wherein the ectodomains of 4-1BBL are identical in all instances.

In a further aspect, provided is a 4-1BBL trimer-containing antigen binding molecule as defined herein before, comprising
(a) at least one moiety capable of specific binding to a PD1 and
(b) a first and a second polypeptide that are linked to each other by a disulfide bond, wherein the antigen binding molecule is characterized in that
   (i) the first polypeptide contains a CH1 or CL domain and the second polypeptide contains a CL or CH1 domain, respectively, wherein the second polypeptide is linked to the first polypeptide by a disulfide bond between the CH1 and CL domain, and wherein the first polypeptide comprises two ectodomains of 4-1BBL or two fragments thereof that are connected to each other and to the CH1 or CL domain by a peptide linker and wherein the second polypeptide comprises one ectodomain of said 4-1BBL or a fragment thereof connected via a peptide linker to the CL or CH1 domain of said polypeptide, or
   (ii) the first polypeptide contains a CH3 domain and the second polypeptide contains a CH3 domain, respectively, and wherein the first polypeptide comprises two ectodomains of 4-1BBL or two fragments thereof that are connected to each other and to the C-terminus of the CH3 domain by a peptide linker and wherein the second polypeptide comprises only one ectodomain of said 4-1BBL or a fragment thereof connected via a peptide linker to C-terminus of the CH3 domain of said polypeptide, or
   (iii) the first polypeptide contains a VH-CL or a VL-CH1 domain and the second polypeptide contains a VL-CH1 domain or a VH-CL domain, respectively, wherein the second polypeptide is linked to the first polypeptide by a disulfide bond between the CH1 and CL domain, and wherein the first polypeptide comprises two ectodomains of 4-1BBL or fragments thereof that are connected to each other and to to VH or VL by a peptide linker and wherein the second polypeptide comprises one ectodomain of said 4-1BBL or a fragment thereof connected via a peptide linker to VL or VH of said polypeptide.

In another aspect, provided is a 4-1BBL trimer-containing antigen binding molecule of as defined herein before, comprising
(a) at least one moiety capable of specific binding to a PD1 and
(b) a first and a second polypeptide that are linked to each other by a disulfide bond,
   wherein the antigen binding molecule is characterized in that
   (i) the first polypeptide contains a CH1 or CL domain and the second polypeptide contains a CL or CH1 domain, respectively, wherein the second polypeptide is linked to the first polypeptide by a disulfide bond between the CH1 and CL domain, and wherein the first polypeptide comprises two ectodomains of 4-1BBL or two fragments thereof that are connected to each other and to the CH1 or CL domain by a peptide linker and wherein the second polypeptide comprises one ectodomain of said 4-1BBL or a fragment thereof connected via a peptide linker to the CL or CH1 domain of said polypeptide, or
   (ii) the first polypeptide contains a CH3 domain and the second polypeptide contains a CH3 domain, respectively, and wherein the first polypeptide comprises two ectodomains of 4-1BBL or two fragments thereof that are connected to each other and to the C-terminus of the CH3 domain by a peptide linker and wherein the second polypeptide comprises only one ectodomain of said 4-1BBL or a fragment thereof connected via a peptide linker to C-terminus of the CH3 domain of said polypeptide.

In another aspect, wherein the ectodomain of 4-1BBL comprises the amino acid sequence selected from the group consisting of SEQ ID NO: 1, SEQ ID NO: 2, SEQ ID NO:3, SEQ ID NO:4, SEQ ID NO:5, SEQ ID NO: 6, SEQ ID NO:7 and SEQ ID NO:8, particularly the amino acid sequence of SEQ ID NO:1 or SEQ ID NO:5. More particularly, the ectodomain of 4-1BBL comprises the amino acid sequence of SEQ ID NO:5.

In a further aspect, the 4-1BBL trimer-containing antigen binding molecule comprises
(a) at least one moiety capable of specific binding to PD1 and
(b) a first and a second polypeptide that are linked to each other by a disulfide bond,
wherein the antigen binding molecule is characterized in that the first polypeptide comprises the amino acid sequence selected from the group consisting of SEQ ID NO:9, SEQ ID NO:11, SEQ ID NO:12 and SEQ ID NO:10 and in that the second polypeptide comprises the amino acid sequence selected from the group consisting of SEQ ID NO:1, SEQ ID NO:3, SEQ ID NO:4 and SEQ ID NO:5.

In one aspect, the 4-1BBL trimer-containing antigen binding molecule comprises
(a) at least one moiety capable of specific binding to PD1 and
(b) a first and a second polypeptide that are linked to each other by a disulfide bond,
wherein the antigen binding molecule is characterized in that the first polypeptide comprises the amino acid sequence of SEQ ID NO:10 and in that the second polypeptide comprises the amino acid sequence of SEQ ID NO:5.

In a further aspect, the 4-1BBL trimer-containing antigen binding molecule comprises
(a) at least one moiety capable of specific binding to PD1 and
(b) a first and a second polypeptide that are linked to each other by a disulfide bond,
wherein the antigen binding molecule is characterized in that the first polypeptide comprises the amino acid sequence of SEQ ID NO:9 and in that the second polypeptide comprises the amino acid sequence of SEQ ID NO:1.

In another aspect, the 4-1BBL trimer-containing antigen binding molecule comprises
(a) at least one moiety capable of specific binding to PD1,
(b) a first polypeptide containing a CH1 or CL domain and a second polypeptide containing a CL or CH1 domain, respectively, wherein the second polypeptide is linked to the first polypeptide by a disulfide bond between the CH1 and CL domain,
and wherein the antigen binding molecule is characterized in that the first polypeptide comprises two ectodomains of 4-1BBL or two fragments thereof that are connected to each other and to the CH1 or CL domain by a peptide linker and in that the second polypeptide comprises only one ectodomain of said 4-1BBL or a fragment thereof connected by a peptide linker to the CL or CH1 domain of said polypeptide.

In one aspect, provided is a 4-1BBL trimer-containing antigen binding molecule comprising
(a) at least one moiety capable of specific binding to PD1,
(b) a first polypeptide containing a CH1 domain and a second polypeptide containing a CL domain, wherein the second polypeptide is linked to the first polypeptide by a disulfide bond between the CH1 and CL domain,
and wherein the antigen binding molecule is characterized in that the first polypeptide comprises two ectodomains of 4-1BBL or fragments thereof that are connected to each other and to the CH1 domain by a peptide linker and in that the second polypeptide comprises one ectodomain of said 4-1BBL or a fragment thereof connected via a peptide linker to the CL domain of said polypeptide.

In another aspect, provided is a 4-1BBL trimer-containing antigen binding molecule comprising
(a) at least one moiety capable of specific binding to PD1,
(b) a first polypeptide containing a CL domain and a second polypeptide containing a CH1 domain, wherein the second polypeptide is linked to the first polypeptide by a disulfide bond between the CH1 and CL domain,
and wherein the antigen binding molecule is characterized in that the first polypeptide comprises two ectodomains of 4-1BBL or fragments thereof that are connected to each other and to the CL domain by a peptide linker and in that the second polypeptide comprises one ectodomain of said 4-1BBL or a fragment thereof connected via a peptide linker to the CH1 domain of said polypeptide.

In another aspect, the disclosure provides a 4-1BBL trimer-containing antigen binding molecule comprising
(a) one moiety capable of specific binding to PD1 and
(b) a first and a second polypeptide that are linked to each other by a disulfide bond,
wherein the antigen binding molecule is characterized in that the first polypeptide comprises two ectodomains of 4-1BBL or two fragments thereof that are connected to each other by a peptide linker and in that the second polypeptide comprises only one ectodomain of said 4-1BBL or a fragment thereof.

In yet another aspect, the disclosure provides a 4-1BBL trimer-containing antigen binding molecule comprising
(a) more than one moiety capable of specific binding to PD1 and
(b) a first and a second polypeptide that are linked to each other by a disulfide bond,
wherein the antigen binding molecule is characterized in that the first polypeptide comprises two ectodomains of 4-1BBL or two fragments thereof that are connected to each other by a peptide linker and in that the second polypeptide comprises only one ectodomain of said 4-1BBL or a fragment thereof connected via a peptide linker to said polypeptide.

In one aspect, the disclosure provides a 4-1BBL trimer-containing antigen binding molecule comprising
(a) two moities capable of specific binding to PD1 and
(b) a first and a second polypeptide that are linked to each other by a disulfide bond,
wherein the antigen binding molecule is characterized in that the first polypeptide comprises two ectodomains of 4-1BBL or two fragments thereof that are connected to each other by a peptide linker and in that the second polypeptide comprises only one ectodomain of said 4-1BBL or a fragment thereof.

In a particular aspect, provided is a 4-1BBL trimer-containing antigen binding molecule, comprising
(a) at least one moiety capable of specific binding to PD1, and
(b) a first and a second polypeptide that are linked to each other by a disulfide bond,
wherein the antigen binding molecule is characterized in that the first polypeptide contains a CH3 domain and the second polypeptide contains a CH3 domain, respectively, and wherein the first polypeptide comprises two ectodomains of 4-1BBL or fragments thereof that are connected to each other and to the C-terminus of the CH3 domain by a peptide linker and wherein the second polypeptide comprises one ectodomain of said 4-1BBL or a fragment thereof connected via a peptide linker to C-terminus of the CH3 domain of said polypeptide. Particularly, such 4-1BBL trimer-containing antigen binding molecule comprises two moieties capable of specific binding to a target cell antigen.

In one aspect, the disclosure provides a 4-1BBL trimer-containing antigen binding molecule comprising
(a) two moities capable of specific binding to PD1 and
(b) a first and a second polypeptide that are linked to each other by a disulfide bond,
wherein the antigen binding molecule is characterized in that the first polypeptide comprises two ectodomains of 4-1BBL or two fragments thereof that are connected to each other by a peptide linker and in that the second polypeptide comprises one ectodomain of said 4-1BBL or a fragment thereof,
wherein the two moieties capable of specific binding to a target cell antigen bind to two different target cell antigens.

In a further aspect, the disclosure provides a 4-1BBL trimer-containing antigen binding molecule as defined herein before, wherein the moiety capable of specific binding to a target cell antigen is selected from the group consisting of an antibody, an antibody fragment and a scaffold antigen binding protein.

In one aspect, provided is a 4-1BBL trimer-containing antigen binding molecule as described herein before, wherein the moiety capable of specific binding to PD1 is selected from the group consisting of an antibody fragment, a Fab molecule, a crossover Fab molecule, a single chain Fab molecule, a Fv molecule, a scFv molecule, a single domain antibody, an aVH and a scaffold antigen binding protein. In one aspect, the moiety capable of specific binding to PD1 is an aVH or a scaffold antigen binding protein. In one aspect, the moiety capable of specific binding to a target cell antigen is a scaffold antigen binding protein capable of specific binding to a target cell antigen.

In particular, the 4-1BBL trimer-containing antigen binding molecule comprises one or two moieties capable of specific binding to PD1.

In a particular aspect, provided is a 4-1BBL trimer-containing antigen binding molecule, wherein the moiety capable of specific binding to PD1 is a Fab molecule or a crossover Fab molecule capable of specific binding to PD1. In particular, the moiety capable of specific binding to a target cell antigen is a Fab capable of specific binding to PD1.

In a further aspect, provided is a 4-1BBL trimer-containing antigen binding molecule, wherein a peptide comprising two ectodomains of 4-1BBL or fragments thereof connected to each other by a first peptide linker is fused at its C-terminus to the CH1 domain of a heavy chain by a second peptide linker and wherein one ectodomain of said 4-1BBL or a fragment thereof is fused at the its C-terminus to the CL domain on a light chain by a third peptide linker.

In another aspect, provided is a 4-1BBL trimer-containing antigen binding molecule according to the invention, wherein a peptide comprising two ectodomains of 4-1BBL or fragments thereof connected to each other by a first peptide linker is fused at its C-terminus to the CL domain of a heavy chain by a second peptide linker and wherein one ectodomain of said 4-1BBL or a fragment thereof is fused at the its C-terminus to the CH1 domain on a light chain by a third peptide linker.

In a further aspect, the disclosure is concerned with a 4-1BBL trimer-containing antigen binding molecule, wherein a peptide comprising two ectodomains of 4-1BBL or fragments thereof connected to each other by a first peptide linker is fused at its C-terminus to the CL domain of a light chain by a second peptide linker and wherein one ectodomain of said 4-1BBL or a fragment thereof is fused at the its C-terminus to the CH1 domain of the heavy chain by a third peptide linker.

In a particular aspect, the disclosure relates to a 4-1BBL trimer-containing antigen binding molecule as defined above, wherein the peptide linker is (G4S)₂. In one aspect, the first peptide linker is (G4S)₂ (SEQ ID NO: 117), the second peptide linker is GSPGSSSSGS (SEQ ID NO: 122) and the third peptide linker is (G4S)₂ (SEQ ID NO:117). In particular, the disclosure relates to a 4-1BBL trimer-containing antigen binding molecule as defined above, wherein the first peptide linker is (G4S)₂ (SEQ ID NO: 117), the second peptide linker is (G4S)₂ (SEQ ID NO:117), and the third peptide linker is (G4S)₂ (SEQ ID NO:117).

In another aspect, the 4-1BBL trimer-containing antigen binding molecule as defined herein before comprises an Fc domain composed of a first and a second subunit capable of stable association.

In particular, the 4-1BBL trimer-containing antigen binding molecule comprises (a) a Fab molecule capable of specific binding to PD1, wherein the Fab heavy chain is fused at the C-terminus to the N-terminus of a CH2 domain in the Fc domain and (c) an Fc domain composed of a first and a second subunit capable of stable association.

In a further aspect, the Fc domain is an IgG, particularly an IgG1 Fc domain or an IgG4 Fc domain. More particularly, the Fc domain is an IgG1 Fc domain. In a particular aspect, the Fc domain comprises a modification promoting the association of the first and second subunit of the Fc domain.

### Fc domain modifications reducing Fc receptor binding and/or effector function

The Fc domain of the 4-1BBL trimer-containing antigen binding molecules of the invention consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other.

The Fc domain confers favorable pharmacokinetic properties to the antigen binding molecules, including a long serum half-life which contributes to good accumulation in the target tissue and a favorable tissue-blood distribution ratio. At the same time it may, however, lead to undesirable targeting of the bispecific antibodies to cells expressing Fc receptors rather than to the preferred antigen-bearing cells. Accordingly, in particular aspects, the Fc domain of the 4-1BBL trimer-containing antigen binding molecule exhibits reduced binding affinity to an Fc receptor and/or reduced effector function, as compared to a native IgG1 Fc domain. In one aspect, the Fc does not substantially bind to an Fc receptor and/or does not induce effector function. In a particular aspect the Fc receptor is an Fcγ receptor. In one aspect, the Fc receptor is a human Fc receptor. In a specific aspect, the Fc receptor is an activating human Fcγ receptor, more specifically human FcγRIIIa, FcγRI or FcγRIIa, most specifically human FcγRIIIa. In one aspect, the Fc domain does not induce effector function. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced dendritic cell maturation, or reduced T cell priming.

In certain aspects, one or more amino acid modifications may be introduced into the Fc region of a 4-1BBL trimer-containing antigen binding molecule provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In a particular aspect, the disclosure provides a 4-1BBL trimer-containing antigen binding molecule comprising
(a) at least one moiety capable of specific binding to PD1,
(b) a first and a second polypeptide that are linked to each other by a disulfide bond,
   wherein the antigen binding molecule is characterized in that the first polypeptide comprises two ectodomains of 4-1BBL or two fragments thereof that are connected to each other by a peptide linker and in that the second polypeptide comprises only one ectodomain of said 4-1BBL or a fragment thereof, and
(c) an Fc domain composed of a first and a second subunit capable of stable association, wherein the Fc domain comprises one or more amino acid substitution that reduces binding to an Fc receptor, in particular towards Fcγ receptor.

In one aspect, the Fc domain of the 4-1BBL trimer-containing antigen binding molecule comprises one or more amino acid mutation that reduces the binding affinity of the Fc domain to an Fc receptor and/or effector function. Typically, the same one or more amino acid mutation is present in each of the two subunits of the Fc domain. In particular, the Fc domain comprises an amino acid substitution at a position of E233, L234, L235, N297, P331 and P329 (EU numbering). In particular, the Fc domain comprises amino acid substitutions at positions 234 and 235 (EU numbering) and/or 329 (EU numbering) of the IgG heavy chains. More particularly, provided is a trimeric TNF family ligand-containing antigen binding molecule which comprises an Fc domain with the amino acid substitutions L234A, L235A and P329G ("P329G LALA", EU numbering) in the IgG heavy chains. The amino acid substitutions L234A and L235A refer to the so-called LALA mutation. The "P329G LALA" combination of amino acid substitutions almost completely abolishes Fcγ receptor binding of a human IgG1 Fc domain and is described in International Patent Appl. Publ. No. WO 2012/130831 A1 which also describes methods of preparing such mutant Fc domains and methods for determining its properties such as Fc receptor binding or effector functions. "EU numbering" refers to the numbering according to EU index of Kabat et al, Sequences of Proteins of Immunological Interest, 5th Ed. Public Health Service, National Institutes of Health, Bethesda, MD, 1991.

Fc domains with reduced Fc receptor binding and/or effector function also include those with substitution of one or more of Fc domain residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

In another aspect, the Fc domain is an IgG4 Fc domain. IgG4 antibodies exhibit reduced binding affinity to Fc receptors and reduced effector functions as compared to IgG1 antibodies. In a more specific aspect, the Fc domain is an IgG4 Fc domain comprising an amino acid substitution at position S228 (Kabat numbering), particularly the amino acid substitution S228P. In a more specific aspect, the Fc domain is an IgG4 Fc domain comprising amino acid substitutions L235E and S228P and P329G (EU numbering). Such IgG4 Fc domain mutants and their Fcγ receptor binding properties are also described in WO 2012/130831.

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing FcγIIIa receptor.

Effector function of an Fc domain, or bispecific antibodies comprising an Fc domain, can be measured by methods known in the art. A suitable assay for measuring ADCC is described herein. Other examples of in vitro assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed in vivo, e.g. in a animal model such as that disclosed in Clynes et al., Proc Natl Acad Sci USA 95, 652-656 (1998).

In some aspects, binding of the Fc domain to a complement component, specifically to C1q, is reduced. Accordingly, in some aspects wherein the Fc domain is engineered to have reduced effector function, said reduced effector function includes reduced CDC. C1q binding assays may be carried out to determine whether the bispecific antibodies is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

In a particular aspect, the Fc domain comprises a modification promoting the association of the first and second subunit of the Fc domain.

### Fc domain modifications promoting heterodimerization

In one aspect, the 4-1BBL trimer-containing antigen binding molecules comprise (a) at least one moiety capable of specific binding to a PD1,
(b) a first and a second polypeptide that are linked to each other by a disulfide bond, wherein the antigen binding molecule is characterized in that the first polypeptide comprises two ectodomains of 4-1BBL or two fragments thereof that are connected to each other by a peptide linker and in that the second polypeptide comprises only one ectodomain of said 4-1BBL or a fragment thereof, and (c) an Fc domain composed of a first and a second subunit capable of stable association, wherein the Fc domain comprises one or more amino acid substitution that reduces binding to an Fc receptor, in particular towards Fcγ receptor. Thus, they comprise different moieties, fused to one or the other of the two subunits of the Fc domain that are typically comprised in two non-identical polypetide chains ("heavy chains"). Recombinant co-expression of these polypeptides and subsequent dimerization leads to several possible combinations of the two polypeptides. To improve the yield and purity of the 4-1BBL trimer-containing antigen binding molecules in recombinant production, it will thus be advantageous to introduce in the Fc domain of the 4-1BBL trimer-containing antigen binding molecules a modification promoting the association of the desired polypeptides.

Accordingly, the Fc domain of the 4-1BBL trimer-containing antigen binding molecules comprises a modification promoting the association of the first and the second subunit of the Fc domain. The site of most extensive protein-protein interaction between the two subunits of a human IgG Fc domain is in the CH3 domain of the Fc domain. Thus, said modification is particularly in the CH3 domain of the Fc domain.

In a specific aspect, said modification is a so-called "knob-into-hole" modification, comprising a "knob" modification in one of the two subunits of the Fc domain and a "hole" modification in the other one of the two subunits of the Fc domain. Thus, in a particular aspect, the disclosure relates to a 4-1BBL trimer-containing antigen binding molecule as described herein before which comprises an IgG molecule, wherein the Fc part of the first heavy chain comprises a first dimerization module and the Fc part of the second heavy chain comprises a second dimerization module allowing a heterodimerization of the two heavy chains of the IgG molecule and the first dimerization module comprises knobs and the second dimerization module comprises holes according to the knob into hole technology.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular aspect, in the CH3 domain of the first subunit of the Fc domain of the 4-1BBL trimer-containing antigen binding molecules an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

The protuberance and cavity can be made by altering the nucleic acid encoding the polypeptides, e.g. by site-specific mutagenesis, or by peptide synthesis.

In a specific aspect, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). More particularly, in the second subunit of the Fc domain additionally the threonine residue at position 366 is replaced with a serine residue (T366S) and the leucine residue at position 368 is replaced with an alanine residue (L368A). More particularly, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). The introduction of these two cysteine residues results in the formation of a disulfide bridge between the two subunits of the Fc domain. The disulfide bridge further stabilizes the dimer (Carter, J Immunol Methods 248, 7-15 (2001)).

In an alternative aspect, a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

### Modifications in the CH1/CL domains

To further improve correct pairing, the 4-1BBL trimer-containing antigen binding molecules can contain different charged amino acid substitutions (so-called "charged residues"). These modifications are introduced in the crossed or non-crossed CH1 and CL domains. In a particular aspect, the disclosure relates to a TNF family ligand trimer-containing antigen binding molecule, wherein in one of CL domains the amino acid at position 123 (EU numbering) has been replaced by arginine (R) and the amino acid at position 124 (EU numbering) has been substituted by lysine (K) and wherein in one of the CH1 domains the the amino acids at position 147 (EU numbering) and at position 213 (EU numbering) have been substituted by glutamic acid (E).

More particularly, the disclosure relates to a 4-1BBL trimer-containing antigen binding molecule, wherein in the CL domain adjacent to the TNF ligand family member the amino acid at position 123 (EU numbering) has been replaced by arginine (R) and the amino acid at position 124 (EU numbering) has been substituted by lysine (K), and wherein in the CH1 domain adjacent to the TNF ligand family member the amino acids at position 147 (EU numbering) and at position 213 (EU numbering) have been substituted by glutamic acid (E).

Thus, in a particular aspect, provided is a 4-1BBL trimer-containing antigen binding molecule comprising
(a) at least one moiety capable of specific binding to PD1,
(b) a first polypeptide containing a CL domain comprising the amino acid mutations E123R and Q124K and a second polypeptide containing a CH1 domain comprising the amino acid mutations K147E and K213E, wherein the second polypeptide is linked to the first polypeptide by a disulfide bond between the CH1 and CL domain,
and wherein the antigen binding molecule is characterized in that the first polypeptide comprises two ectodomains of 4-1BBL or fragments thereof that are connected to each other and to the CL domain by a peptide linker and in that the second polypeptide comprises one ectodomain of said 4-1BBL or a fragment thereof connected via a peptide linker to the CH1 domain of said polypeptide.

### Particular 4-1BBL trimer-containing antigen binding molecules

In another aspect, the disclosure provides a 4-1BBL trimer-containing antigen binding molecule, wherein the antigen binding molecule comprises
a first heavy chain and a first light chain, both comprising a Fab molecule capable of specific binding to PD1,
a first peptide comprising two ectodomains of 4-1BBL or fragments thereof connected to each other by a first peptide linker fused at its C-terminus by a second peptide linker to a second heavy or light chain,
and a second peptide comprising one ectodomain of said 4-1BBL fused at its C-terminus by a third peptide linker to a second light or heavy chain, respectively.

In a further aspect, provided is a 4-1BBL trimer-containing antigen binding molecule, wherein the first peptide comprising two ectodomains of 4-1BBL or fragments thereof connected to each other by a first peptide linker is fused at its C-terminus by a second peptide linker to a CH1 domain that is part of a heavy chain, and the second peptide comprising one ectodomain of said 4-1BBL or a fragment thereof is fused at its C-terminus by a third peptide linker to a CL domain that is part of a light chain.

In yet another aspect, provided is a 4-1BBL trimer-containing antigen binding molecule, wherein the first peptide comprising two ectodomains of 4-1BBL or fragments thereof connected to each other by a first peptide linker is fused at its C-terminus by a second peptide linker to a CL domain that is part of a heavy chain,
and the second peptide comprising one ectodomain of said 4-1BBL or a fragment thereof that is fused at its C-terminus by a third peptide linker to a CH1 domain that is part of a light chain.

In one aspect, the disclosure provides a 4-1BBL trimer-containing antigen binding molecule, wherein in the CL domain adjacent to the 4-1BBL the amino acid at position 123 (EU numbering) has been replaced by arginine (R) and the amino acid at position 124 (EU numbering) has been substituted by lysine (K), and wherein in the CH1 domain adjacent to the TNF ligand family member the amino acids at position 147 (EU numbering) and at position 213 (EU numbering) have been substituted by glutamic acid (E). These modifications lead to so-called charged residues with advantageaous properties that avoid undesired effects such as for example mispairing.

In particular, the CL domain comprises the amino acid mutations E123R and Q124K and the CH1 domain comprises the amino acid mutations K147E and K213E.

In one aspect, the disclosure provides a 4-1BBL trimer-containing antigen binding molecule as defined herein before, wherein the moiety capable of specific binding to PD1 is a scaffold antigen binding protein.

In a particular aspect, the disclosure is concerned with a 4-1BBL trimer-containing antigen binding molecule as defined above, wherein the moiety capable of specific binding to PD1 is a Fab molecule capable of specific binding to a target cell antigen.

The disclosure provides a 4-1BBL trimer-containing antigen binding molecule that comprises at least one moiety capable of specific binding to PD1. In a particular aspect, the 4-1BBL trimer-containing antigen binding molecule comprises one moiety capable of specific binding to PD1, meaning the T4-1BBL trimer-containing antigen binding molecule is monovalent. In another aspect, the disclosure provides a 4-1BBL trimer-containing antigen binding molecule comprising two moieties capable of specific binding to PD1, meaning the 4-1BBL trimer-containing antigen binding molecule is bivalent.

In one aspect, the disclosure provides a 4-1BBL trimer-containing antigen binding molecule, wherein the moiety capable of specific binding to PD1 comprises
(a) a VH domain comprising (i) HVR-H1 comprising the amino acid sequence of SEQ ID NO: 13, (ii) HVR-H2 comprising the amino acid sequence of SEQ ID NO: 14, and (iii) HVR-H3 comprising the amino acid sequence of SEQ ID NO: 15, and a VL domain comprising (iv) HVR-LI comprising the amino acid sequence of SEQ ID NO:16 (v) HVR-L2 comprising the amino acid sequence of SEQ ID NO: 17, and (vi) HVR-L3 comprising the amino acid sequence of SEQ ID NO:18,
(b) a VH domain comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:28, and a VL domain comprising (iv) HVR-L1 comprising the amino acid sequence of SEQ ID NO:29 (v) HVR-L2 comprising the amino acid sequence of SEQ ID NO:30, and (vi) HVR-L3 comprising the amino acid sequence of SEQ ID NO:31,
(c) a VH domain comprising (i) HVR-H1 comprising the amino acid sequence of SEQ ID NO:34, (ii) HVR-H2 comprising the amino acid sequence of SEQ ID NO:35, and (iii) HVR-H3 comprising the amino acid sequence of SEQ ID NO:36, and a VL domain comprising (iv) HVR-L1 comprising the amino acid sequence of SEQ ID NO:37 (v) HVR-L2 comprising the amino acid sequence of SEQ ID NO:38, and (vi) HVR-L3 comprising the amino acid sequence of SEQ ID NO:39,
(d) a VH domain comprising (i) HVR-H1 comprising the amino acid sequence of SEQ ID NO:42, (ii) HVR-H2 comprising the amino acid sequence of SEQ ID NO:43, and (iii) HVR-H3 comprising the amino acid sequence of SEQ ID NO:44, and a VL domain comprising (iv) HVR-L1 comprising the amino acid sequence of SEQ ID NO:45 (v) HVR-L2 comprising the amino acid sequence of SEQ ID NO:46, and (vi) HVR-L3 comprising the amino acid sequence of SEQ ID NO:47,
(e) a VH domain comprising (i) HVR-H1 comprising the amino acid sequence of SEQ ID NO:50, (ii) HVR-H2 comprising the amino acid sequence of SEQ ID NO:51, and (iii) HVR-H3 comprising the amino acid sequence of SEQ ID NO:52, and a VL domain comprising (iv) HVR-L1 comprising the amino acid sequence of SEQ ID NO:53 (v) HVR-L2 comprising the amino acid sequence of SEQ ID NO:54, and (vi) HVR-L3 comprising the amino acid sequence of SEQ ID NO:55,
(f) a VH domain comprising (i) HVR-H1 comprising the amino acid sequence of SEQ ID NO:58, (ii) HVR-H2 comprising the amino acid sequence of SEQ ID NO:59, and (iii) HVR-H3 comprising the amino acid sequence of SEQ ID NO:60, and a VL domain comprising (iv) HVR-L1 comprising the amino acid sequence of SEQ ID NO:61 (v) HVR-L2 comprising the amino acid sequence of SEQ ID NO:62, and (vi) HVR-L3 comprising the amino acid sequence of SEQ ID NO:63, or
(g) a VH domain comprising (i) HVR-H1 comprising the amino acid sequence of SEQ ID NO:66, (ii) HVR-H2 comprising the amino acid sequence of SEQ ID NO:67, and (iii) HVR-H3 comprising the amino acid sequence of SEQ ID NO:68, and a VL domain comprising (iv) HVR-L1 comprising the amino acid sequence of SEQ ID NO:69 (v) HVR-L2 comprising the amino acid sequence of SEQ ID NO:70, and (vi) HVR-L3 comprising the amino acid sequence of SEQ ID NO:71.

In a further aspect, the disclosure provides a a 4-1BBL trimer-containing antigen binding molecule, wherein the moiety capable of specific binding to PD1 comprises
(a) a VH domain comprising an amino acid sequence of SEQ ID NO:19 and a VL domain comprising an amino acid sequence of SEQ ID NO:20,
(b) a VH domain comprising an amino acid sequence of SEQ ID NO:21 and a VL domain comprising an amino acid sequence of SEQ ID NO:22,
(c) a VH domain comprising an amino acid sequence of SEQ ID NO:21 and a VL domain comprising an amino acid sequence of SEQ ID NO:23,
(d) a VH domain comprising an amino acid sequence of SEQ ID NO:21 and a VL domain comprising an amino acid sequence of SEQ ID NO:24,
(e) a VH domain comprising an amino acid sequence of SEQ ID NO:21 and a VL domain comprising an amino acid sequence of SEQ ID NO:25,
(f) a VH domain comprising an amino acid sequence of SEQ ID NO:32 and a VL domain comprising an amino acid sequence of SEQ ID NO:33,
(g) a VH domain comprising an amino acid sequence of SEQ ID NO:40 and a VL domain comprising an amino acid sequence of SEQ ID NO:41,
(h) a VH domain comprising an amino acid sequence of SEQ ID NO:48 and a VL domain comprising an amino acid sequence of SEQ ID NO:49,
(i) a VH domain comprising an amino acid sequence of SEQ ID NO:56 and a VL domain comprising an amino acid sequence of SEQ ID NO:57,
(k) a VH domain comprising an amino acid sequence of SEQ ID NO:64 and a VL domain comprising an amino acid sequence of SEQ ID NO:65, or
(l) a VH domain comprising an amino acid sequence of SEQ ID NO:72 and a VL domain comprising an amino acid sequence of SEQ ID NO:73.

In one aspect, the disclosure provides a 4-1BBL trimer-containing antigen binding molecule, wherein the moiety capable of specific binding to PD1 comprises (a) a VH domain comprising (i) HVR-H1 comprising the amino acid sequence of SEQ ID NO: 13, (ii) HVR-H2 comprising the amino acid sequence of SEQ ID NO:14, and (iii) HVR-H3 comprising the amino acid sequence of SEQ ID NO:15, and a VL domain comprising (iv) HVR-L1 comprising the amino acid sequence of SEQ ID NO:16, (v) HVR-L2 comprising the amino acid sequence of SEQ ID NO: 17, and (vi) HVR-L3 comprising the amino acid sequence of SEQ ID NO:18.

In a particular aspect, the disclosure provides a 4-1BBL trimer-containing antigen binding molecule, wherein the moiety capable of specific binding to PD1 comprises
(a) a VH domain comprising an amino acid sequence of SEQ ID NO: 19 and a VL domain comprising an amino acid sequence of SEQ ID NO:20,
(b) a VH domain comprising an amino acid sequence of SEQ ID NO:21 and a VL domain comprising an amino acid sequence of SEQ ID NO:22,
(c) a VH domain comprising an amino acid sequence of SEQ ID NO:21 and a VL domain comprising an amino acid sequence of SEQ ID NO:23,
(d) a VH domain comprising an amino acid sequence of SEQ ID NO:21 and a VL domain comprising an amino acid sequence of SEQ ID NO:24, or
(e) a VH domain comprising an amino acid sequence of SEQ ID NO:21 and a VL domain comprising an amino acid sequence of SEQ ID NO:25.

In a further aspect, the moiety capable of specific binding to PD1 comprises a heavy chain variable region comprising an amino acid sequence that is at least about 95%, 96%, 97%, 98%, 99% or 100% identical to the amino acid sequence of SEQ ID NO:21 and a light chain variable region comprising an amino acid sequence that is at least about 95%, 96%, 97%, 98%, 99% or 100% identical to the amino acid sequence of SEQ ID NO:22.

In another aspect, the moiety capable of specific binding to PD1 comprises a heavy chain variable region comprising an amino acid sequence that is at least about 95%, 96%, 97%, 98%, 99% or 100% identical to the amino acid sequence of SEQ ID NO:21 and a light chain variable region comprising an amino acid sequence that is at least about 95%, 96%, 97%, 98%, 99% or 100% identical to the amino acid sequence of SEQ ID NO:24.

In one aspect, provided is a 4-1BBL trimer-containing antigen binding molecule as defined herein before, wherein the moiety capable of specific binding to PD1 comprises a variable heavy chain comprising an amino acid sequence of SEQ ID NO:21 and a variable light chain comprising an amino acid sequence of SEQ ID NO:22 or wherein the moiety capable of specific binding to FAP comprises a variable heavy chain comprising an amino acid sequence of SEQ ID NO:21 and a variable light chain comprising an amino acid sequence of SEQ ID NO:24.

In a particular aspect, the moiety capable of specific binding to PD1 comprises a heavy chain variable region comprising the amino acid sequence of SEQ ID NO:21 and a light chain variable region comprising the amino acid sequence of SEQ ID NO:22. In another particular aspect, the moiety capable of specific binding to PD1 comprises a heavy chain variable region comprising the amino acid sequence of SEQ ID NO:21 and a light chain variable region comprising the amino acid sequence of SEQ ID NO:24 In a specific aspect, the moiety capable of specific binding to PD1 comprises a VH domain consisting of amino acid sequence of SEQ ID NO:21 and a VL domain consisting of the amino acid sequence of SEQ ID NO:22 or a VH domain consisting of amino acid sequence of SEQ ID NO:21 and a VL domain consisting of the amino acid sequence of SEQ ID NO:24.

In a further aspect, the 4-1BBL trimer-containing antigen binding molecule comprises
(a) at least one moiety capable of specific binding to PD1 comprising a heavy chain variable region comprising the amino acid sequence of SEQ ID NO:21 and a light chain variable region comprising the amino acid sequence of SEQ ID NO:22 or a heavy chain variable region comprising the amino acid sequence of SEQ ID NO:21 and a light chain variable region comprising the amino acid sequence of SEQ ID NO:24, and
(b) a second heavy chain comprising an amino acid sequence selected from the group consisting of SEQ ID NO:9, SEQ ID NO:11, SEQ ID NO:12 and SEQ ID NO:10, and
a second light chain comprising an amino acid sequence selected from the group consisting of SEQ ID NO:1, SEQ ID NO:3, SEQ ID NO:4 and SEQ ID NO:5.

In a particular aspect, the 4-1BBL trimer-containing antigen binding molecule comprises
(a) at least one moiety capable of specific binding to PD1 comprising a heavy chain variable region comprising the amino acid sequence of SEQ ID NO:21 and a light chain variable region comprising the amino acid sequence of SEQ ID NO:22 or a heavy chain variable region comprising the amino acid sequence of SEQ ID NO:21 and a light chain variable region comprising the amino acid sequence of SEQ ID NO:24, and
(b) a first and a second polypeptide that are linked to each other by a disulfide bond,
wherein the antigen binding molecule is characterized in that the first polypeptide comprises the the amino acid sequence of SEQ ID NO:10 and the second polypeptide comprises the amino acid sequence of SEQ ID NO:5.

In a further aspect, provided is a 4-1BBL trimer-containing antigen binding molecule as described herein before, wherein the antigen binding molecule comprises
(a) a first heavy chain and a first light chain, both comprising a Fab molecule capable of specific binding to PD1,
(b) a second heavy chain comprising an amino acid sequence selected from the group consisting of SEQ ID NO:9, SEQ ID NO:11, SEQ ID NO:12 and SEQ ID NO:10, and
a second light chain comprising an amino acid sequence selected from the group consisting of SEQ ID NO: 1, SEQ ID NO:3, SEQ ID NO:4 and SEQ ID NO:5.

In one aspect, the disclosureprovides a 4-1BBL trimer-containing antigen binding molecule, wherein the antigen binding molecule comprises
(a) a Fab molecule capable of specific binding to PD1 comprising a heavy chain variable region comprising the amino acid sequence of SEQ ID NO:21 and a light chain variable region comprising the amino acid sequence of SEQ ID NO:22 or a heavy chain variable region comprising the amino acid sequence of SEQ ID NO:21 and a light chain variable region comprising the amino acid sequence of SEQ ID NO:24, and
(b) a second heavy chain comprising an amino acid sequence selected from the group consisting of SEQ ID NO:9, SEQ ID NO:11, SEQ ID NO:12 and SEQ ID NO:10, and
a second light chain comprising an amino acid sequence selected from the group consisting of SEQ ID NO: 1, SEQ ID NO:3, SEQ ID NO:4 and SEQ ID NO:5.

In one aspect, the disclosure provides a 4-1BBL trimer-containing antigen binding molecule, wherein the antigen binding molecule comprises
(a) (i) a first heavy chain comprising the VH domain comprising the amino acid sequence of SEQ ID NO: 19 and a first light chain comprising the VL domain comprising the amino acid sequence of SEQ ID NO:20 or
   a first heavy chain comprising the VH domain comprising the amino acid sequence of SEQ ID NO:21 and a first light chain comprising the VL domain comprising the amino acid sequence selected from the group consisting of SEQ ID NO:22, SEQ ID NO:23, SEQ ID NO:24 and SEQ ID NO:25, and
(b) a second heavy chain comprising an amino acid sequence selected from the group consisting of SEQ ID NO:9, SEQ ID NO:11, SEQ ID NO:12 and SEQ ID NO:10, and
a second light chain comprising an amino acid sequence selected from the group consisting of SEQ ID NO: 1, SEQ ID NO:3, SEQ ID NO:4 and SEQ ID NO:5.

In a particular aspect, provided is a 4-1BBL trimer-containing antigen binding molecule, wherein the antigen binding molecule comprises
(i) a first heavy chain comprising the VH domain comprising the amino acid sequence of SEQ ID NO: 19 and a first light chain comprising the VL domain comprising the amino acid sequence of SEQ ID NO:20 or
   a first heavy chain comprising the VH domain comprising the amino acid sequence of SEQ ID NO:21 and a first light chain comprising the VL domain comprising the amino acid sequence selected from the group consisting of SEQ ID NO:22, SEQ ID NO:23, SEQ ID NO:24 and SEQ ID NO:25,
(ii) a second heavy chain comprising the amino acid sequence selected from the group consisting of SEQ ID NO:74, SEQ ID NO:76, SEQ ID NO:78 and SEQ ID NO:80, and
(iii) a second light chain comprising the amino acid sequence selected from the group consisting of SEQ ID NO:75, SEQ ID NO:77, SEQ ID NO:79 and SEQ ID NO:81..

In another aspect, the disclosure provides a 4-1BBL trimer-containing antigen binding molecule, wherein the antigen binding molecule comprises
(a) a first heavy chain comprising the amino acid sequence of SEQ ID NO:82, a first light chain comprising the amino acid sequence of SEQ ID NO:83, a second heavy chain comprising the amino acid sequence of SEQ ID NO:74 and a second light chain comprising the amino acid sequence of SEQ ID NO:75,
(b) a first heavy chain comprising the amino acid sequence of SEQ ID NO:82, a first light chain comprising the amino acid sequence of SEQ ID NO:83, a second heavy chain comprising the amino acid sequence of SEQ ID NO:76 and a second light chain comprising the amino acid sequence of SEQ ID NO:77,
(c) a first heavy chain comprising the amino acid sequence of SEQ ID NO:82, a first light chain comprising the amino acid sequence of SEQ ID NO:83, a second heavy chain comprising the amino acid sequence of SEQ ID NO:78 and a second light chain comprising the amino acid sequence of SEQ ID NO:79, or
(d) a first heavy chain comprising the amino acid sequence of SEQ ID NO:82, a first light chain comprising the amino acid sequence of SEQ ID NO:83, a second heavy chain comprising the amino acid sequence of SEQ ID NO:80 and a second light chain comprising the amino acid sequence of SEQ ID NO:81.

In a further aspect, the disclosure provides a 4-1BBL trimer-containing antigen binding molecule, wherein the antigen binding molecule comprises
(a) a first heavy chain comprising the amino acid sequence of SEQ ID NO:82, a first light chain comprising the amino acid sequence of SEQ ID NO:171, a second heavy chain comprising the amino acid sequence of SEQ ID NO:74 and a second light chain comprising the amino acid sequence of SEQ ID NO:75,
(b) a first heavy chain comprising the amino acid sequence of SEQ ID NO:82, a first light chain comprising the amino acid sequence of SEQ ID NO:171, a second heavy chain comprising the amino acid sequence of SEQ ID NO:76 and a second light chain comprising the amino acid sequence of SEQ ID NO:77,
(c) a first heavy chain comprising the amino acid sequence of SEQ ID NO:82, a first light chain comprising the amino acid sequence of SEQ ID NO:171, a second heavy chain comprising the amino acid sequence of SEQ ID NO:78 and a second light chain comprising the amino acid sequence of SEQ ID NO:79, or
(d) a first heavy chain comprising the amino acid sequence of SEQ ID NO:82, a first light chain comprising the amino acid sequence of SEQ ID NO:171, a second heavy chain comprising the amino acid sequence of SEQ ID NO:80 and a second light chain comprising the amino acid sequence of SEQ ID NO:81.

In particular, provided is a 4-1BBL trimer-containing antigen binding molecule, wherein the antigen binding molecule comprises a first heavy chain comprising the amino acid sequence of SEQ ID NO:82, a first light chain comprising the amino acid sequence of SEQ ID NO:83, a second heavy chain comprising the amino acid sequence of SEQ ID NO:78 and a second light chain comprising the amino acid sequence of SEQ ID NO:79.

In a further particular aspect, provided is a 4-1BBL trimer-containing antigen binding molecule, wherein the antigen binding molecule comprises a first heavy chain comprising the amino acid sequence of SEQ ID NO:82, a first light chain comprising the amino acid sequence of SEQ ID NO: 171, a second heavy chain comprising the amino acid sequence of SEQ ID NO:78 and a second light chain comprising the amino acid sequence of SEQ ID NO:79.

In yet another aspect, the disclosure provides a 4-1BBL trimer-containing antigen binding molecule, comprising
(a) at least one moiety capable of specific binding to PD1, and
(b) a first and a second polypeptide that are linked to each other by a disulfide bond,
wherein the antigen binding molecule is characterized in that the first polypeptide contains a CH3 domain and the second polypeptide contains a CH3 domain, respectively, and wherein the first polypeptide comprises two ectodomains of 4-1BBL or fragments thereof that are connected to each other and to the C-terminus of the CH3 domain by a peptide linker and wherein the second polypeptide comprises one ectodomain of said 4-1BBL or a fragment thereof connected via a peptide linker to C-terminus of the CH3 domain of said polypeptide.

In particular, such a 4-1BBL trimer-containing antigen binding molecule comprises two Fab domains capable of specific binding to PD1.

In particular, provided is a 4-1BBL trimer-containing antigen binding molecule as described herein before comprises
(a) a first heavy chain comprising the amino acid sequence of SEQ ID NO:84, a second heavy chain comprising the amino acid sequence of SEQ ID NO:85, and two light chains comprising the amino acid sequence of SEQ ID NO:83, or
(b) a first heavy chain comprising the amino acid sequence of SEQ ID NO:86, a second heavy chain comprising the amino acid sequence of SEQ ID NO:87, and two light chains comprising the amino acid sequence of SEQ ID NO:83.

In a further aspect, provided is a 4-1BBL trimer-containing antigen binding molecule as described herein before comprises
(a) a first heavy chain comprising the amino acid sequence of SEQ ID NO:84, a second heavy chain comprising the amino acid sequence of SEQ ID NO:85, and two light chains comprising the amino acid sequence of SEQ ID NO: 171, or
(b) a first heavy chain comprising the amino acid sequence of SEQ ID NO:86, a second heavy chain comprising the amino acid sequence of SEQ ID NO:87, and two light chains comprising the amino acid sequence of SEQ ID NO: 171.

In another particular aspect, the disclosure provides a 4-1BBL trimer-containing antigen binding molecule as described herein before, wherein the 4-1BBL trimer-containing antigen binding molecule comprises one Fab domain capable of specific binding to PD1.

In such aspect, provided is a 4-1BBL trimer-containing antigen binding molecule, wherein the antigen binding molecule comprises
(i) a fusion polypeptide comprising a first subunit of a Fc domain and two ectodomains of 4-1BBL or fragments thereof that are connected to each other and to the C-terminus of the CH3 domain by a peptide linker, (ii) a heavy chain comprising the VH domain of the Fab domain capable of specific binding to PD1, a second subunit of the Fc domain and one ectodomain of said 4-1BBL or a fragment thereof connected via a peptide linker to C-terminus of the CH3 domain, and (iii) a light chain comprising the VL domain of the Fab domain capable of specific binding to PD1, or
(i) a fusion polypeptide comprising a first subunit of a Fc domain and one ectodomain of 4-1BBL or a fragment thereof that is connected to the C-terminus of the CH3 domain by a peptide linker, (ii) a heavy chain comprising the VH domain of the Fab domain capable of specific binding to PD1, a second subunit of the Fc domain and two ectodomains of said 4-1BBL or fragments thereof that are connected to each other and to the C-terminus of the CH3 domain via a peptide linker, and (iii) a light chain comprising the VL domain of the Fab domain capable of specific binding to PD1.

In a particular aspect, provided is a 4-1BBL trimer-containing antigen binding molecule, wherein the antigen binding molecule comprises
(a) a fusion polypeptide comprising the amino acid sequence of SEQ ID NO:88, a heavy chain comprising the amino acid sequence of SEQ ID NO:87 and a light chain comprising the amino acid sequence of SEQ ID NO:83,
(b) a fusion polypeptide comprising the amino acid sequence of SEQ ID NO:89, a heavy chain comprising the amino acid sequence of SEQ ID NO:90 and a light chain comprising the amino acid sequence of SEQ ID NO:83,
(c) a fusion polypeptide comprising the amino acid sequence of SEQ ID NO:91, a heavy chain comprising the amino acid sequence of SEQ ID NO:86 and a light chain comprising the amino acid sequence of SEQ ID NO:83, or
(d) a fusion polypeptide comprising the amino acid sequence of SEQ ID NO:93, a heavy chain comprising the amino acid sequence of SEQ ID NO:92 and a light chain comprising the amino acid sequence of SEQ ID NO:83.

In another particular aspect, provided is a 4-1BBL trimer-containing antigen binding molecule, wherein the antigen binding molecule comprises
(a) a fusion polypeptide comprising the amino acid sequence of SEQ ID NO:88, a heavy chain comprising the amino acid sequence of SEQ ID NO:87 and a light chain comprising the amino acid sequence of SEQ ID NO: 171,
(b) a fusion polypeptide comprising the amino acid sequence of SEQ ID NO:89, a heavy chain comprising the amino acid sequence of SEQ ID NO:90 and a light chain comprising the amino acid sequence of SEQ ID NO: 171,
(c) a fusion polypeptide comprising the amino acid sequence of SEQ ID NO:91, a heavy chain comprising the amino acid sequence of SEQ ID NO:86 and a light chain comprising the amino acid sequence of SEQ ID NO: 171, or
(d) a fusion polypeptide comprising the amino acid sequence of SEQ ID NO:93, a heavy chain comprising the amino acid sequence of SEQ ID NO:92 and a light chain comprising the amino acid sequence of SEQ ID NO: 171.

### Polynucleotides

The disclosure further provides isolated polynucleotides encoding a 4-1BBL trimer-containing antigen binding molecule as described herein or a fragment thereof.

The isolated polynucleotides encoding 4-1BBL trimer-containing antigen binding molecules may be expressed as a single polynucleotide that encodes the entire antigen binding molecule or as multiple (e.g., two or more) polynucleotides that are co-expressed. Polypeptides encoded by polynucleotides that are co-expressed may associate through, e.g., disulfide bonds or other means to form a functional antigen binding molecule. For example, the light chain portion of an immunoglobulin may be encoded by a separate polynucleotide from the heavy chain portion of the immunoglobulin. When co-expressed, the heavy chain polypeptides will associate with the light chain polypeptides to form the immunoglobulin.

In some aspects, the isolated polynucleotide encodes the entire 4-1BBL trimer-containing antigen binding molecule as described herein. In parrticular, the isolated polynucleotide encodes a polypeptide comprised in the 4-1BBL trimer-containing antigen binding molecule as described herein.

In one aspect, the present disclosure is directed to isolated polynucleotides encoding a 4-1BBL trimer-containing antigen binding molecule, wherein the polynucleotide comprises (a) a sequence that encodes a moiety capable of specific binding to a PD1, (b) a sequence that encodes a polypeptide comprising two ectodomains of 4-1BBL or two fragments thereof that are connected to each other by a peptide linker and (c) a sequence that encodes a polypeptide comprising one ectodomain of said 4-1BBL or a fragment thereof.

In another aspect, provided is an isolated polynucleotide encoding a 4-1BB ligand trimer-containing antigen binding molecule, wherein the polynucleotide comprises (a) a sequence that encodes a moiety capable of specific binding to PD1, (b) a sequence that encodes a polypeptide comprising two ectodomains of 4-1BBL or two fragments thereof that are connected to each other by a peptide linker and (c) a sequence that encodes a polypeptide comprising one ectodomain of 4-1BBL or a fragment thereof.

In a further aspect, the disclosure is directed to an isolated polynucleotide comprising a sequence that encodes a polypeptide comprising two 4-1BBL fragments comprising an amino acid sequence that is at least about 90%, 95%, 98% or 100% identical to an amino acid sequence shown in SEQ ID NO:1, SEQ ID NO:2, SEQ ID NO:3, SEQ ID NO:4, SEQ ID NO:5, SEQ ID NO:6, SEQ ID NO:7 or SEQ ID NO:8, and to a polynucleotide comprising a sequence that encodes a polypeptide comprising one 4-1BBL fragment comprising an amino acid sequence that is is at least about 90%, 95%, 98% or 100% identical to an amino acid sequence shown in SEQ ID NO:1, SEQ ID NO:2, SEQ ID NO:3, SEQ ID NO:4, SEQ ID NO:5, SEQ ID NO:6, SEQ ID NO:7 or SEQ ID NO:8.

In further aspects, the disclosure relates to the polynucleotides comprising a sequence that is at least about 90%, 95%, 98% or 100% identical to the specific cDNA sequences disclosed herein. In a particular aspect, the disclosure relates to a polynucleotide comprising a sequence that is identical to one of the specific cDNA sequences disclosed herein.

In other aspects, the nucleic acid molecule comprises or consists of a nucleotide sequence that encodes an amino acid sequence as set forth in any one of SEQ ID NOs: 19, 20, 21, 22, 23, 24, 25, 32, 33, 40, 41, 48, 49, 56, 57, 64, 65, 72 or 73. In a further aspect, the nucleic acid molecule comprises or consists of a nucleotide sequence that encodes an amino acid sequence as set forth in any one of SEQ ID NOs:9, 10, 11 or 12.

In still other aspects, the nucleic acid molecule comprises or consists of a nucleotide sequence selected from the group consisting of SEQ ID NOs: 131, 132, 133, 134, 135, 136, 137, 138, 139, 140, 141, 142, 143, 144, 145, 146, 147, 148, 149 or 150.

In certain aspects, the polynucleotide or nucleic acid is DNA. In other aspects, a polynucleotide of the present disclosure is RNA, for example, in the form of messenger RNA (mRNA). RNA may be single stranded or double stranded.

### Recombinant Methods

4-1BBL trimer-containing antigen binding molecules of the disclosure may be obtained, for example, by solid-state peptide synthesis (e.g. Merrifield solid phase synthesis) or recombinant production. For recombinant production one or more polynucleotide encoding the 4-1BBL trimer-containing antigen binding molecule or polypeptide fragments thereof, e.g., as described above, is isolated and inserted into one or more vectors for further cloning and/or expression in a host cell. Such polynucleotide may be readily isolated and sequenced using conventional procedures. In one aspect, a vector, preferably an expression vector, comprising one or more of the polynucleotides of the disclosure is provided. Methods which are well known to those skilled in the art can be used to construct expression vectors containing the coding sequence of the 4-1BBL trimer-containing antigen binding molecule (fragment) along with appropriate transcriptional/translational control signals. These methods include in vitro recombinant DNA techniques, synthetic techniques and in vivo recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y. (1989). The expression vector can be part of a plasmid, virus, or may be a nucleic acid fragment. The expression vector includes an expression cassette into which the polynucleotide encoding the 4-1BBL trimer-containing antigen binding molecule or polypeptide fragments thereof (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector of the present disclosure may encode one or more polypeptides, which are post- or co-translationally separated into the final proteins via proteolytic cleavage. In addition, a vector, polynucleotide, or nucleic acid may encode heterologous coding regions, either fused or unfused to a polynucleotide encoding the 4-1BBL trimer-containing antigen binding molecule or polypeptide fragments thereof, or variants or derivatives thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription.

Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions of the present disclosure may be associated with additional coding regions which encode secretory or signal peptides, which direct the secretion of a polypeptide encoded by a polynucleotide of the present disclosure. For example, if secretion of the 4-1BBL trimer-containing antigen binding molecule or polypeptide fragments thereof is desired, DNA encoding a signal sequence may be placed upstream of the nucleic acid encoding a 4-1BBL trimer-containing antigen binding molecule of the invention or polypeptide fragments thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain aspects, the native signal peptide, e.g. an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase.

DNA encoding a short protein sequence that could be used to facilitate later purification (e.g. a histidine tag) or assist in labeling the fusion protein may be included within or at the ends of the polynucleotide encoding a 4-1BBL trimer-containing antigen binding molecule or polypeptide fragments thereof.

In a further aspect, a host cell comprising one or more polynucleotides of the disclosure is provided. In certain aspects a host cell comprising one or more vectors of the disclosure is provided. The polynucleotides and vectors may incorporate any of the features, singly or in combination, described herein in relation to polynucleotides and vectors, respectively. In one aspect, a host cell comprises (e.g. has been transformed or transfected with) a vector comprising a polynucleotide that encodes (part of) a 4-1BBL trimer-containing antigen binding molecule. As used herein, the term "host cell" refers to any kind of cellular system which can be engineered to generate the fusion proteins or fragments thereof. Host cells suitable for replicating and for supporting expression of antigen binding molecules are well known in the art. Such cells may be transfected or transduced as appropriate with the particular expression vector and large quantities of vector containing cells can be grown for seeding large scale fermenters to obtain sufficient quantities of the antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006).

Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr- CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one aspect, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell). Standard technologies are known in the art to express foreign genes in these systems. Cells expressing a polypeptide comprising either the heavy or the light chain of an immunoglobulin, may be engineered so as to also express the other of the immunoglobulin chains such that the expressed product is an immunoglobulin that has both a heavy and a light chain.

In one aspect, a method of producing a 4-1BBL trimer-containing antigen binding molecule or polypeptide fragments thereof is provided, wherein the method comprises culturing a host cell comprising polynucleotides encoding the 4-1BBL trimer-containing antigen binding molecule or polypeptide fragments thereof, as provided herein, under conditions suitable for expression of the TNF family ligand trimer-containing antigen binding molecule of the invention or polypeptide fragments thereof, and recovering the 4-1BBL trimer-containing antigen binding molecule or polypeptide fragments thereof from the host cell (or host cell culture medium).

In the 4-1BBL trimer-containing antigen binding molecule, the components (at least one moiety capable of specific binding to a target cell antigen, one polypeptide comprising two ectodomains of 4-1BBL or fragments thereof and a polypeptide comprising one ectodomain of said 4-1BBL or a fragment thereof) are not genetically fused to each other. The polypeptides are designed such that its components (two ectodomains of a TNF ligand family member or fragments thereof and other components such as CH or CL) are fused to each other directly or through a linker sequence. The composition and length of the linker may be determined in accordance with methods well known in the art and may be tested for efficacy. Examples of linker sequences between different components of the antigen binding molecules are found in the sequences provided herein. Additional sequences may also be included to incorporate a cleavage site to separate the individual components of the fusion protein if desired, for example an endopeptidase recognition sequence.

In certain aspects the moieties capable of specific binding to a target cell antigen (e.g. Fab fragments) forming part of the antigen binding molecule comprise at least an immunoglobulin variable region capable of binding to an antigen. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of immunoglobulin can be used. Non-limiting immunoglobulins useful can be of murine, primate, or human origin. If the fusion protein is intended for human use, a chimeric form of immunoglobulin may be used wherein the constant regions of the immunoglobulin are from a human. A humanized or fully human form of the immunoglobulin can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Particular immunoglobulins are human immunoglobulins. Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain aspects, the moieties capable of specific binding to a target cell antigen (e.g. Fab fragments) comprised in the antigen binding molecules are engineered to have enhanced binding affinity according to, for example, the methods disclosed in PCT publication WO 2012/020006 (see Examples relating to affinity maturation) or U.S. Pat. Appl. Publ. No. 2004/0132066. The ability of the antigen binding molecules to bind to a specific antigenic determinant can be measured either through an enzyme-linked immunosorbent assay (ELISA) or other techniques familiar to one of skill in the art, e.g. surface plasmon resonance technique (Liljeblad, et al., Glyco J 17, 323-329 (2000)), and traditional binding assays (Heeley, Endocr Res 28, 217-229 (2002)). Competition assays may be used to identify an antigen binding molecule that competes with a reference antibody for binding to a particular antigen. In certain aspects, such a competing antigen binding molecule binds to the same epitope (e.g. a linear or a conformational epitope) that is bound by the reference antigen binding molecule. Detailed exemplary methods for mapping an epitope to which an antigen binding molecule binds are provided in Morris (1996) "Epitope Mapping Protocols", in Methods in Molecular Biology vol. 66 (Humana Press, Totowa, NJ). In an exemplary competition assay, immobilized antigen is incubated in a solution comprising a first labeled antigen binding molecule that binds to the antigen and a second unlabeled antigen binding molecule that is being tested for its ability to compete with the first antigen binding molecule for binding to the antigen. The second antigen binding molecule may be present in a hybridoma supernatant. As a control, immobilized antigen is incubated in a solution comprising the first labeled antigen binding molecule but not the second unlabeled antigen binding molecule. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antigen binding molecule is competing with the first antigen binding molecule for binding to the antigen. See Harlow and Lane (1988) Antibodies: A Laboratory Manual ch.14 (Cold Spring Harbor Laboratory, Cold Spring Harbor, NY).

4-1BBL trimer-containing antigen binding molecules prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the 4-1BBL trimer-containing antigen binding molecule binds. For example, for affinity chromatography purification of fusion proteins of the disclosure, a matrix with protein A or protein G may be used. Sequential Protein A or G affinity chromatography and size exclusion chromatography can be used to isolate an antigen binding molecule essentially as described in the Examples. The purity of the 4-1BBL trimer-containing antigen binding molecule or fragments thereof can be determined by any of a variety of well-known analytical methods including gel electrophoresis, high pressure liquid chromatography, and the like. For example, the 4-1BBL trimer-containing antigen binding molecules expressed as described in the Examples were shown to be intact and properly assembled as demonstrated by reducing and non-reducing SDS-PAGE.

### Assays

The antigen binding molecules provided herein may be identified, screened for, or characterized for their physical/chemical properties and/or biological activities by various assays known in the art. Biological activity may include, e.g., the ability to enhance the activation and/or proliferation of different immune cells especially T-cells. E.g. they enhance secretion of immunomodulating cytokines (e.g. interferon-gamma (IFN-gamma) and/or tumor necrosis factor alpha (TNF alpha)). Other immunomodulating cytokines which are or can be enhanced are e.g IL12, Granzyme B etc. Biological activity may also include, cynomolgus binding crossreactivity, as well as binding to different cell types. Antigen binding molecules having such biological activity in vivo and/or in vitro are also provided.

### 1. Affinity assays

The affinity of the 4-1BBL trimer-containing antigen binding molecule provided herein for the corresponding receptor can be determined in accordance with the methods set forth in the Examples by surface plasmon resonance (SPR), using standard instrumentation such as a BIAcore instrument (GE Healthcare), and receptors or target proteins such as may be obtained by recombinant expression. The affinity of the 4-1BBL trimer-containing antigen binding molecule for PD1 can also be determined by surface plasmon resonance (SPR), using standard instrumentation such as a BIAcore instrument (GE Healthcare), and receptors or target proteins such as may be obtained by recombinant expression. A specific illustrative and exemplary embodiment for measuring binding affinity is described in Example 4. According to one aspect, K_{D} is measured by surface plasmon resonance using a BIACORE® T100 machine (GE Healthcare) at 25 °C.

### 2. Binding assays and other assays

Binding of the 4-1BBL trimer-containing antigen binding molecule provided herein to the corresponding receptor expressing cells may be evaluated using cell lines expressing the particular receptor or target antigen, for example by flow cytometry (FACS). In one aspect, fresh peripheral blood mononuclear cells (PBMCs) expressing the TNF receptor are used in the binding assay. These cells are used directly after isolation (naive PMBCs) or after stimulation (activated PMBCs). In another aspect, activated mouse splenocytes (expressing the receptor molecule) were used to demonstrate the binding of the 4-1BBL trimer-containing antigen binding molecule of the disclosure to the corresponding TNF receptor expressing cells.

In a further aspect, cell lines expressing PD1 were used to demonstrate the binding of the antigen binding molecules to this target cell antigen.

In another aspect, competition assays may be used to identify an antigen binding molecule that competes with a specific antibody or antigen binding molecule for binding to the target or TNF receptor, respectively. In certain aspects, such a competing antigen binding molecule binds to the same epitope (e.g., a linear or a conformational epitope) that is bound by a specific anti-target antibody or a specific anti-TNF receptor antibody. Detailed exemplary methods for mapping an epitope to which an antibody binds are provided in Morris (1996) "Epitope Mapping Protocols," in Methods in Molecular Biology vol. 66 (Humana Press, Totowa, NJ).

### 3. Activity assays

In one aspect, assays are provided for identifying 4-1BBL trimer-containing antigen binding molecules that bind to a specific target cell antigen and to a specific TNF receptor having biological activity. Biological activity may include, e.g., agonistic signalling through the TNF receptor on cells expressing the target cell antigen. 4-1BBL trimer-containing antigen binding molecules identified by the assays as having such biological activity in vitro are also provided.

In certain aspects, a 4-1BBL trimer-containing antigen binding molecule of the invention is tested for such biological activity. Assays for detecting the biological activity of the molecules are those described in Examples 5 and 6. Furthermore, assays for detecting cell lysis (e.g. by measurement of LDH release), induced apoptosis kinetics (e.g. by measurement of Caspase 3/7 activity) or apoptosis (e.g. using the TUNEL assay) are well known in the art. In addition the biological activity of such complexes can be assessed by evaluating their effects on survival, proliferation and lymphokine secretion of various lymphocyte subsets such as NK cells, NKT-cells or γδ T-cells or assessing their capacity to modulate phenotype and function of antigen presenting cells such as dendritic cells, monocytes/macrophages or B-cells.

### Pharmaceutical Compositions, Formulations and Routes of Administation

In a further aspect, the disclosure provides pharmaceutical compositions comprising any of the 4-1BBL trimer-containing antigen binding molecules provided herein, e.g., for use in any of the below therapeutic methods. In one aspect, a pharmaceutical composition comprises any of the 4-1BBL trimer-containing antigen binding molecules provided herein and at least one pharmaceutically acceptable excipient. In another aspect, a pharmaceutical composition comprises any of the 4-1BBL trimer-containing antigen binding molecules provided herein and at least one additional therapeutic agent, e.g., as described below.

Pharmaceutical compositions of the present disclosure comprise a therapeutically effective amount of one or more 4-1BBL trimer-containing antigen *binding* molecules dissolved or dispersed in a pharmaceutically acceptable excipient. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one 4-1BBL trimer-containing antigen binding molecule and optionally an additional active ingredient will be known to those of skill in the art in light of the present disclosure, as exemplified by Remington's Pharmaceutical Sciences, 18th Ed. Mack Printing Company, 1990. In particular, the compositions are lyophilized formulations or aqueous solutions. As used herein, "pharmaceutically acceptable excipient" includes any and all solvents, buffers, dispersion media, coatings, surfactants, antioxidants, preservatives (e.g. antibacterial agents, antifungal agents), isotonic agents, salts, stabilizers and combinations thereof, as would be known to one of ordinary skill in the art.

Parenteral compositions include those designed for administration by injection, e.g. subcutaneous, intradermal, intralesional, intravenous, intraarterial intramuscular, intrathecal or intraperitoneal injection. For injection, the 4-1BBL trimer-containing antigen binding molecules may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the fusion proteins may be in powder form for constitution with a suitable vehicle, e.g., sterile pyrogen-free water, before use. Sterile injectable solutions are prepared by incorporating the fusion proteins in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable excipients include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular aspects, prolonged absorption of an injectable composition can be brought about by the use in the compositions of agents delaying absorption, such as, for example, aluminum monostearate, gelatin or combinations thereof.

Exemplary pharmaceutically acceptable excipients herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

In addition to the compositions described previously, the fusion proteins may also be formulated as a depot preparation. Such long acting formulations may be administered by implantation (for example subcutaneously or intramuscularly) or by intramuscular injection. Thus, for example, the fusion proteins may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising the fusion proteins may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The 4-1BBL trimer-containing antigen binding molecules may be formulated into a composition in a free acid or base, neutral or salt form. Pharmaceutically acceptable salts are salts that substantially retain the biological activity of the free acid or base. These include the acid addition salts, e.g. those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

The composition herein may also contain more than one active ingredients as necessary for the particular indication being treated, preferably those with complementary activities that do not adversely affect each other. Such active ingredients are suitably present in combination in amounts that are effective for the purpose intended.

The formulations to be used for in vivo administration are generally sterile. Sterility may be readily accomplished, e.g., by filtration through sterile filtration membranes.

### Therapeutic methods and compositions

Any of the 4-1BBL trimer-containing antigen binding molecules provided herein may be used in therapeutic methods.

For use in therapeutic methods, 4-1BBL trimer-containing antigen binding molecules can be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one aspect, 4-1BBL trimer-containing antigen binding molecules of the disclosure for use as a medicament are provided. In further aspects, 4-1BBL trimer-containing antigen binding molecules for use in treating a disease, in particular for use in the treatment of cancer, are provided. In certain aspects, 4-1BBL trimer-containing antigen binding molecules for use in a method of treatment are provided. In one aspect, the disclosure provides a 4-1BBL trimer-containing antigen binding molecule as described herein for use in the treatment of a disease in an individual in need thereof. In certain aspects, the disclosure provides a 4-1BBL trimer-containing antigen binding molecule for use in a method of treating an individual having a disease comprising administering to the individual a therapeutically effective amount of the fusion protein. In certain aspects, the disease to be treated is cancer. Examples of cancers include solid tumors, bladder cancer, renal cell carcinoma, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer, melanoma, B-cell lymphoma, B-cell leukemia, non-Hodgkin lymphoma and acute lymphoblastic leukemia. Thus, a 4-1BBL trimer-containing antigen binding molecule as described herein for use in the treatment of cancer is provided. The subject, patient, or "individual" in need of treatment is typically a mammal, more specifically a human.

In another aspect, provided is a 4-1BBL trimer-containing antigen binding molecule as described herein for use in the treatment of infectious diseases, in particular for the treatment of viral infections. In a further aspect, provided is a 4-1BBL trimer-containing antigen binding molecule as described herein for use in the treatment of autoimmune diseases such as for example Lupus disease.

In a further aspect, the disclosure relates to the use of a 4-1BBL trimer-containing antigen binding molecule in the manufacture or preparation of a medicament for the treatment of a disease in an individual in need thereof. In one aspect, the medicament is for use in a method of treating a disease comprising administering to an individual having the disease a therapeutically effective amount of the medicament. In certain aspects the disease to be treated is a proliferative disorder, particularly cancer. Thus, in one aspect, the disclosure relates to the use of a 4-1BBL trimer-containing antigen binding molecule in the manufacture or preparation of a medicament for the treatment of cancer. Examples of cancers include solid tumors, bladder cancer, renal cell carcinoma, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer, melanoma, B-cell lymphoma, B-cell leukemia, non-Hodgkin lymphoma and acute lymphoblastic leukemia.. Other cell proliferation disorders that can be treated using a 4-1BBL trimer-containing antigen binding molecule as disclosed herein include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases. In certain aspects the cancer is chosen from the group consisting of renal cell cancer, skin cancer, lung cancer, colorectal cancer, breast cancer, brain cancer, head and neck cancer. A skilled artisan may recognize that in some cases the 4-1BBL trimer-containing antigen binding molecule may not provide a cure but may only provide partial benefit. In some aspects, a physiological change having some benefit is also considered therapeutically beneficial. Thus, in some aspects, an amount of 4-1BBL trimer-containing antigen binding molecule that provides a physiological change is considered an "effective amount" or a "therapeutically effective amount".

In a further aspect, the disclosure relates to the use of a TNF family ligand trimer-containing antigen binding molecule as described herein in the manufacture or preparation of a medicament for the treatment of infectious diseases, in particular for the treatment of viral infections or for the treatment of autoimmune diseases, for example Lupus disease.

For the prevention or treatment of disease, the appropriate dosage of a 4-1BBL trimer-containing antigen binding molecule as disclosed herein (when used alone or in combination with one or more other additional therapeutic agents) will depend on the type of disease to be treated, the route of administration, the body weight of the patient, the type of antigen binding molecule, the severity and course of the disease, whether the fusion protein is administered for preventive or therapeutic purposes, previous or concurrent therapeutic interventions, the patient's clinical history and response to the fusion protein, and the discretion of the attending physician. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The 4-1BBL trimer-containing antigen binding molecule is suitably administered to the patient at one time or over a series of treatments. Depending on the type and severity of the disease, about 1 µg/kg to 15 mg/kg (e.g. 0.1 mg/kg - 10 mg/kg) of TNF family ligand trimer-containing antigen binding molecule can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the fusion protein would be in the range from about 0.005 mg/kg to about 10 mg/kg. In other examples, a dose may also comprise from about 1 µg/kg body weight, about 5 µg/kg body weight, about 10 µg/kg body weight, about 50 µg/kg body weight, about 100 µg/kg body weight, about 200 µg/kg body weight, about 350 µg/kg body weight, about 500 µg/kg body weight, about 1 mg/kg body weight, about 5 mg/kg body weight, about 10 mg/kg body weight, about 50 mg/kg body weight, about 100 mg/kg body weight, about 200 mg/kg body weight, about 350 mg/kg body weight, about 500 mg/kg body weight, to about 1000 mg/kg body weight or more per administration, and any range derivable therein. In examples of a derivable range from the numbers listed herein, a range of about 5 mg/kg body weight to about 100 mg/kg body weight, about 5 µg/kg body weight to about 500 mg/kg body weight etc., can be administered, based on the numbers described above. Thus, one or more doses of about 0.5 mg/kg, 2.0 mg/kg, 5.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the fusion protein). An initial higher loading dose, followed by one or more lower doses may be administered. However, other dosage regimens may be useful. The progress of this therapy is easily monitored by conventional techniques and assays.

The 4-1BBL trimer-containing antigen binding molecules will generally be used in an amount effective to achieve the intended purpose. For use to treat or prevent a disease condition, the 4-1BBL trimer-containing antigen binding molecules, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from in vitro assays, such as cell culture assays. A dose can then be formulated in animal models to achieve a circulating concentration range that includes the IC₅₀ as determined in cell culture. Such information can be used to more accurately determine useful doses in humans.

Initial dosages can also be estimated from in vivo data, e.g., animal models, using techniques that are well known in the art. One having ordinary skill in the art could readily optimize administration to humans based on animal data.

Dosage amount and interval may be adjusted individually to provide plasma levels of the 4-1BBL trimer-containing antigen binding molecules which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the 4-1BBL trimer-containing antigen binding molecule may not be related to plasma concentration. One skilled in the art will be able to optimize therapeutically effective local dosages without undue experimentation.

A therapeutically effective dose of the 4-1BBL trimer-containing antigen binding molecules described herein will generally provide therapeutic benefit without causing substantial toxicity. Toxicity and therapeutic efficacy of a fusion protein can be determined by standard pharmaceutical procedures in cell culture or experimental animals. Cell culture assays and animal studies can be used to determine the LD₅₀ (the dose lethal to 50% of a population) and the ED₅₀ (the dose therapeutically effective in 50% of a population). The dose ratio between toxic and therapeutic effects is the therapeutic index, which can be expressed as the ratio LD₅₀/ED₅₀. 4-1BBL trimer-containing antigen binding molecules that exhibit large therapeutic indices are preferred. In one aspect, the 4-1BBL trimer-containing antigen binding molecule exhibits a high therapeutic index. The data obtained from cell culture assays and animal studies can be used in formulating a range of dosages suitable for use in humans. The dosage lies preferably within a range of circulating concentrations that include the ED₅₀ with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition (see, e.g., Fingl et al., 1975, in: The Pharmacological Basis of Therapeutics, Ch. 1, p. 1).

The attending physician for patients treated with fusion proteins of the disclosure would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

### Other agents and treatments

The 4-1BBL trimer-containing antigen binding molecules may be administered in combination with one or more other agents in therapy. For instance, a fusion protein of the disclosure may be co-administered with at least one additional therapeutic agent. The term "therapeutic agent" encompasses any agent that can be administered for treating a symptom or disease in an individual in need of such treatment. Such additional therapeutic agent may comprise any active ingredients suitable for the particular indication being treated, preferably those with complementary activities that do not adversely affect each other. In certain aspects, an additional therapeutic agent is another anti-cancer agent.

Such other agents are suitably present in combination in amounts that are effective for the purpose intended. The effective amount of such other agents depends on the amount of fusion protein used, the type of disorder or treatment, and other factors discussed above. The 4-1BBL trimer-containing antigen binding molecules are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the 4-1BBL trimer-containing antigen binding molecule can occur prior to, simultaneously, and/or following, administration of the additional therapeutic agent and/or adjuvant.

### Articles of Manufacture

In another aspect, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper that is pierceable by a hypodermic injection needle). At least one active agent in the composition is a 4-1BBL trimer-containing antigen binding molecule as described herein.

The label or package insert indicates that the composition is used for treating the condition of choice. Moreover, the article of manufacture may comprise (a) a first container with a composition contained therein, wherein the composition comprises a 4-1BBL trimer-containing antigen binding molecule; and (b) a second container with a composition contained therein, wherein the composition comprises a further cytotoxic or otherwise therapeutic agent. The article of manufacture in this aspect of the disclosure may further comprise a package insert indicating that the compositions can be used to treat a particular condition.

Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**Table C (Sequences):**

| SEQ ID NO: | Description | Sequence |
|---|---|---|
| 1 | Human (hu) 4-1BBL (71-254) | |
| 2 | hu 4-1BBL (85-254) | |
| 3 | hu 4-1BBL (80-254) | |
| 4 | hu 4-1BBL (52-254) | |
| 5 | Human (hu) 4-1BBL (71-248) | |
| 6 | hu 4-1BBL (85-248) | |
| 7 | hu 4-1BBL (80-248) | |
| 8 | hu 4-1BBL (52-248) | |
| | | |
| 9 | dimeric hu 4-1BBL (71-254) connected by (G4S)₂ linker | |
| 10 | dimeric hu 4-1BBL (71-248) connected by (G4S)₂ linker | |
| 11 | dimeric hu 4-1BBL (80-254) connected by (G4S)₂ linker | |
| 12 | dimeric hu 4-1BBL (52-254) connected by (G4S)₂ linker | |
| | | |
| 13 | heavy chain HVR-H1, PD1-0103 | GFSFSSY |
| 14 | heavy chain HVR-H2, PD1-0103 | GGR |
| 15 | heavy chain HVR-H3, PD1-0103 | TGRVYFALD |
| 16 | light chain HVR-L1, PD1-0103 | SESVDTSDNSF |
| 17 | light chain HVR-L2, PD1-0103 | RSS |
| 18 | light chain HVR-L3, PD 1-0103 | NYDVPW |
| 19 | heavy chain variable domain VH, PD1-0103 | |
| 20 | light chain variable domain VL, PD 1-0103 | |
| 21 | humanized variant -heavy chain variable domain VH of PD1-0103-0312, -0313, - 0314 and -0315 | |
| 22 | humanized variant -light chain variable domain VL of PD1-0103-0312 | |
| 23 | humanized variant -light chain variable domain VL of PD1-0103-0313 | |
| 24 | humanized variant -light chain variable domain VL of PD1-0103-0314 | |
| 25 | humanized variant -light chain variable domain VL of PD1-0103-0315 | |
| 26 | heavy chain HVR-H1, PD1-0098 | GYSITSDY |
| 27 | heavy chain HVR-H2, PD1-0098 | YSG |
| 28 | heavy chain HVR-H3, PD1-0098 | HGSAPWYFD |
| 29 | light chain HVR-L1, PD1-0098 | SQNIVHSDGNTY |
| 30 | light chain HVR-L2, PD1-0098 | KVS |
| 31 | light chain HVR-L3, PD1-0098 | GSHFPL |
| 32 | heavy chain variable domain VH, PD1-0098 | |
| | | |
| 33 | light chain variable domain VL, PD 1-0098 | |
| 34 | heavy chain HVR-H1, PD1-0050 | GYSITSDY |
| 35 | heavy chain HVR-H2, PD1-0050 | YTG |
| 36 | heavy chain HVR-H3, PD1-0050 | MDYYGSTLD |
| 37 | light chain HVR-L1, PD1-0050 | SESVDRYGNSF |
| 38 | light chain HVR-L2, PD1-0050 | RAS |
| 39 | light chain HVR-L3, PD1-0050 | NNEDPY |
| 40 | heavy chain variable domain VH, PD1-0050 | |
| 41 | light chain variable domain VL, PD 1-0050 | |
| 42 | heavy chain HVR-H1, PD1-0069 | GYTFTDY |
| 43 | heavy chain HVR-H2, PD1-0069 | YSG |
| 44 | heavy chain HVR-H3, PD1-0069 | GITTGFA |
| 45 | light chain HVR-L1, PD1-0069 | SKGVSTSSYSF |
| 46 | light chain HVR-L2, PD1-0069 | YAS |
| 47 | light chain HVR-L3, PD1-0069 | SREFPW |
| 48 | heavy chain variable domain VH, PD1-0069 | |
| 49 | light chain variable domain VL, PD1-0069 | |
| 50 | heavy chain HVR-H1, PD1-0073 | GFTFSNY |
| 51 | heavy chain HVR-H2, PD1-0073 | GGR |
| 52 | heavy chain HVR-H3, PD 1-0073 | YYGID |
| 53 | light chain HVR-L1, PD1-0073 | SQDVTTA |
| 54 | light chain HVR-L2, PD1-0073 | WAS |
| 55 | light chain HVR-L3, PD1-0073 | HYSIPW |
| 56 | heavy chain variable domain VH, PD1-0073 | |
| 57 | light chain variable domain VL, PD1-0073 | |
| 58 | heavy chain HVR-H1, PD1-0078 | GYTFTST |
| 59 | heavy chain HVR-H2, PD 1-0078 | SDS |
| 60 | heavy chain HVR-H3, PD 1-0078 | PFD |
| 61 | light chain HVR-L1, PD 1-0078 | SQDVSTA |
| 62 | light chain HVR-L2, PD 1-0078 | SAS |
| 63 | light chain HVR-L3, PD 1-0078 | HYSHPF |
| 64 | heavy chain variable domain VH, PD1-0078 | |
| 65 | light chain variable domain VL, PD1-0078 | |
| 66 | heavy chain HVR-H1, PD1-0102 | GYSITSGY |
| 67 | heavy chain HVR-H2, PD1-0102 | SSG |
| 68 | heavy chain HVR-H3, PD1-0102 | RNWYFD |
| 69 | light chain HVR-L1, PD1-0102 | SQSLLNSGTQKNY |
| 70 | light chain HVR-L2, PD1-0102 | WAS |
| 71 | light chain HVR-L3, PD1-0102 | DYTFPL |
| 72 | heavy chain variable domain VH, PD1-0102 | |
| 73 | light chain variable domain VL, PD1-0102 | |
| 74 | Dimeric 4-1BB ligand (71-254) - CL* Fc knob chain | see Table 8 |
| 75 | Monomeric 4-1BB ligand (71-254)-CH1* | see Table 8 |
| 76 | Dimeric 4-1BB ligand (71-254) - CL Fc knob chain | see Table 9 |
| 77 | Monomeric 4-1BB ligand (71-254) -CH1 | see Table 9 |
| 78 | Dimeric 4-1BB ligand (71-248) - CL* Fc knob chain | see Table 11 |
| 79 | Monomeric 4-1BB ligand (71-248)-CH1* | see Table 11 |
| 80 | Dimeric 4-1BB ligand (71-248) - CL Fc knob chain | see Table 12 |
| 81 | Monomeric 4-1BB ligand (71-248)-CH1 | see Table 12 |
| 82 | anti-PD1(314) Fc hole chain | see Table 8 |
| 83 | anti-PD1(314) light chain | see Table 8 |
| 84 | anti-PD1(314) Fc hole dimeric 4-1BB ligand (71-254) | see Table 10 |
| 85 | anti-PD1(314) Fc knob monomeric 4-1BB ligand (71-254) | see Table 10 |
| 86 | anti-PD1(314) Fc hole dimeric 4-1BB ligand (71-248) | see Table 13 |
| 87 | anti-PD1(314) Fc knob monomeric 4-1BB ligand (71-248) | see Table 13 |
| 88 | Fc hole dimeric 4-1BB ligand (71-248) | see Table 14 |
| 89 | Fc hole monomeric 4-1BB ligand (71-248) | see Table 15 |
| 90 | anti-PD1(314) Fc knob dimeric 4-1BB ligand (71-248) | see Table 15 |
| 91 | Fc knob chain fused to monomeric 4-1BB ligand (71-248) chain | see Table 16 |
| 92 | anti-PD1(314) Fc hole monomeric 4-1BB ligand (71-248) chain | see Table 17 |
| 93 | Fc knob dimeric 4-1BB ligand (71-248) | see Table 17 |
| 94 | Human PD 1 | UniProt no. Q15116 |
| 95 | human Lymphotoxin α | UniProt no. P01374 |
| 96 | human TNF | UniProt no. P01375 |
| 97 | human Lymphotoxin β | UniProt no. Q06643 |
| 98 | human OX40L | UniProt no. P23510 |
| 99 | human CD40L | UniProt no. P29965 |
| 100 | human FasL | UniProt no. P48023 |
| 101 | human CD27L | UniProt no. P32970 |
| 102 | human CD30L | UniProt no. P32971 |
| 103 | human 4-1BBL | UniProt no. P41273 |
| 104 | human TRAIL | UniProt no. P50591 |
| 105 | human RANKL | UniProt no. 014788 |
| 106 | human TWEAK | UniProt no. O43508 |
| 107 | human APRIL | UniProt no. O75888 |
| 108 | human BAFF | UniProt no. Q9Y275 |
| 109 | human LIGHT | UniProt no. O43557 |
| 110 | human TL1A | UniProt no. 095150 |
| 111 | human GITRL | UniProt no. Q9UNG2 |
| 112 | human ectodysplasin A | UniProt no. Q92838 |
| 113 | human 4-1BBL(50-254) | |
| 114 | human OX40L (51-183) | |
| | | |
| 115 | human OX40L (52-183) | |
| 116 | Peptide linker G4S | GGGGS |
| 117 | Peptide linker (G4S)₂ | GGGGSGGGGS |
| 118 | Peptide linker (SG4)₂ | SGGGGSGGGG |
| 119 | Peptide linker (G₄S)₃ | GGGGSGGGGSGGGGS |
| 120 | Peptide linker G4(SG4)₂ | GGGGSGGGGSGGGG |
| 121 | Peptide linker (G₄S)₄ | GGGGSGGGGSGGGGSGGGGS |
| 122 | Peptide linker | GSPGSSSSGS |
| 123 | Peptide linker | GSGSGSGS |
| 124 | Peptide linker | GSGSGNGS |
| 125 | Peptide linker | GGSGSGSG |
| 126 | Peptide linker | GGSGSG |
| 127 | Peptide linker | GGSG |
| 128 | Peptide linker | GGSGNGSG |
| 129 | Peptide linker | GGNGSGSG |
| 130 | Peptide linker | GGNGSG |
| 131 | nucleotide sequence of dimeric hu 4-1BBL (71-254) - CL* Fc knob chain | see Table 8 |
| 132 | nucleotide sequence of monomeric hu 4-1BBL (71-254) - CH1* | see Table 8 |
| 133 | nucleotide sequence of anti-PD1(0314) Fc hole chain | see Table 8 |
| 134 | nucleotide sequence of anti-PD1(0314) light chain | see Table 8 |
| 135 | nucleotide sequence of dimeric hu 4-1BBL (71-254) - CL Fc knob chain | see Table 9 |
| 136 | nucleotide sequence of monomeric hu 4-1BBL (71-254) - CH1 | see Table 9 |
| 137 | nucleotide sequence of anti-PD1(0314) Fc hole chain fused to dimeric hu 4-1BBL (71-254) | see Table 10 |
| 138 | nucleotide sequence of anti-PD1(0314) Fc knob chain fused to monomeric hu 4-1BBL (71-254) | see Table 10 |
| 139 | nucleotide sequence of dimeric hu 4-1BBL (71-248) - CL* Fc knob chain | see Table 11 |
| 140 | nucleotide sequence of monomeric hu 4-1BBL (71-248) - CH1* | see Table 11 |
| 141 | nucleotide sequence of dimeric hu 4-1BBL (71-248) - CL Fc knob chain | see Table 12 |
| 142 | nucleotide sequence of monomeric hu 4-1BBL (71-248) - CH1 | see Table 12 |
| 143 | nucleotide sequence of anti-PD1(0314) Fc hole chain fused to dimeric hu 4-1BBL (71-248) | see Table 13 |
| 144 | nucleotide sequence of anti-PD1(0314) Fc knob chain fused to monomeric hu 4-1BBL (71-248) | see Table 13 |
| 145 | nucleotide sequence of Fc hole dimeric ligand (71-248) chain | see Table 14 |
| 146 | nucleotide sequence of Fc hole monomeric ligand (71-248) chain | see Table 15 |
| 147 | nucleotide sequence of anti-PD1(0314) Fc knob chain fused to dimeric hu 4-1BBL (71-248) | see Table 15 |
| 148 | nucleotide sequence of Fc knob chain fused to monomeric hu 4-1BBL (71-248) | see Table 16 |
| 149 | nucleotide sequence of anti-PD1(0314) Fc hole monomeric ligand (71-248) chain | see Table 17 |
| 150 | nucleotide sequence of Fc knob chain fused to dimeric hu 4-1BBL (71-248) | see Table 17 |
| 151 | nucleotide sequence of DP47 Fc hole chain | see Table 18 |
| 152 | nucleotide sequence of DP47 light chain | see Table 18 |
| 153 | DP47 Fc hole chain | see Table 18 |
| 154 | DP47 light chain | see Table 18 |
| 155 | nucleotide sequence of DP47 Fc hole chain fused to dimeric hu 4-1BBL (71-254) | see Table 19 |
| 156 | nucleotide sequence of DP47 Fc knob chain fused to monomeric hu 4-1BBL (71-254) | see Table 19 |
| 157 | DP47 Fc hole chain fused to dimeric hu 4-1BBL (71-254) | see Table 19 |
| 158 | DP47 Fc knob chain fused to monomeric hu 4-1BBL (71-254) | see Table 19 |
| 159 | nucleotide sequence of DP47 heavy chain (hu IgG1 PGLALA) | see Table 21 |
| 160 | DP47 heavy chain (hu IgG1 PGLALA) | see Table 21 |
| 161 | nucleotide sequence of PD1(0314) heavy chain (huIgG1 PGLALA) | see Table 22 |
| 162 | PD1(0314) heavy chain (huIgG1 PGLALA) | see Table 22 |
| 163 | human 4-1BB ECD, aa 24-186 of Q07011 | |
| 164 | Nucleotide sequence of Fc hole chain | see Table 24 |
| 165 | Nucleotide sequence of human 4-1BB antigen Fc knob chain | see Table 24 |
| 166 | Fc hole chain | see Table 24 |
| 167 | human 4-1BB antigen Fc knob chain | see Table 24 |
| 168 | avi tag | GLNDIFEAQKIEWHE |
| 169 | nucleotide sequence of human 4-1BB His | see Table 25 |
| 170 | human 4-1BB His | see Table 25 |
| 171 | anti-PD1 (0312) light chain | |

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered and referred to according to the EU numbering systems according to Kabat (Kabat, E.A., et al., Sequences of Proteins of Immunological Interest, 5th ed., Public Health Service, National Institutes of Health, Bethesda, MD (1991)) as defined above.

### EXAMPLES

### Recombinant DNA techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturer's instructions. General information regarding the nucleotide sequences of human immunoglobulin light and heavy chains is given in: Kabat, E.A. et al., (1991) Sequences of Proteins of Immunological Interest, Fifth Ed., NIH Publication No 91-3242.

### DNA sequencing

DNA sequences were determined by double strand sequencing.

### Gene synthesis

Desired gene segments were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells.

### Cell culture techniques

Standard cell culture techniques were used as described in Current Protocols in Cell Biology (2000), Bonifacino, J.S., Dasso, M., Harford, J.B., Lippincott-Schwartz, J. and Yamada, K.M. (eds.), John Wiley & Sons, Inc.

### Protein purification

Proteins were purified from filtered cell culture supernatants referring to standard protocols. In brief, antibodies were applied to a Protein A Sepharose column (GE healthcare) and washed with PBS. Elution of antibodies was achieved at pH 2.8 followed by immediate neutralization of the sample. Aggregated protein was separated from monomeric antibodies by size exclusion chromatography (Superdex 200, GE Healthcare) in PBS or in 20 mM Histidine, 150 mM NaCl pH 6.0. Monomeric antibody fractions were pooled, concentrated (if required) using e.g., a MILLIPORE Amicon Ultra (30 MWCO) centrifugal concentrator, frozen and stored at -20°C or -80°C. Part of the samples were provided for subsequent protein analytics and analytical characterization e.g. by SDS-PAGE, size exclusion chromatography (SEC) or mass spectrometry.

### SDS-PAGE

The NuPAGE® Pre-Cast gel system (Invitrogen) was used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer was used.

### Analytical size exclusion chromatography

Size exclusion chromatography (SEC) for the determination of the aggregation and oligomeric state of antibodies was performed by HPLC chromatography. Briefly, Protein A purified antibodies were applied to a Tosoh TSKgel G3000SW column in 300 mM NaCl, 50 mM KH₂PO₄/K₂HPO₄, pH 7.5 on an Agilent HPLC 1100 system or to a Superdex 200 column (GE Healthcare) in 2 x PBS on a Dionex HPLC-System. The eluted protein was quantified by UV absorbance and integration of peak areas. BioRad Gel Filtration Standard 151-1901 served as a standard.

### Mass spectrometry

This section describes the characterization of the multispecific antibodies with VH/VL exchange (VH/VL CrossMabs) with emphasis on their correct assembly. The expected primary structures were analyzed by electrospray ionization mass spectrometry (ESI-MS) of the deglycosylated intact CrossMabs and deglycosylated/plasmin digested or alternatively deglycosylated/limited LysC digested CrossMabs.

The VH/VL CrossMabs were deglycosylated with N-Glycosidase F in a phosphate or Tris buffer at 37°C for up to 17 h at a protein concentration of 1 mg/ml. The plasmin or limited LysC (Roche) digestions were performed with 100 µg deglycosylated VH/VL CrossMabs in a Tris buffer pH 8 at room temperature for 120 hours and at 37°C for 40 min, respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

### Determination of binding and binding affinity of multispecific antibodies to the respective antigens using surface plasmon resonance (SPR) (BIACORE)

Binding of the generated antibodies to the respective antigens is investigated by surface plasmon resonance using a BIACORE instrument (GE Healthcare Biosciences AB, Uppsala, Sweden). Briefly, for affinity measurements Goat-Anti-Human IgG, JIR 109-005-098 antibodies are immobilized on a CM5 chip via amine coupling for presentation of the antibodies against the respective antigen. Binding is measured in HBS buffer (HBS-P (10 mM HEPES, 150 mM NaCl, 0.005% Tween 20, ph 7.4), 25°C (or alternatively at 37°C). Antigen (R&D Systems or in house purified) was added in various concentrations in solution. Association was measured by an antigen injection of 80 seconds to 3 minutes; dissociation was measured by washing the chip surface with HBS buffer for 3 - 10 minutes and a KD value was estimated using a 1:1 Langmuir binding model. Negative control data (e.g. buffer curves) are subtracted from sample curves for correction of system intrinsic baseline drift and for noise signal reduction. The respective Biacore Evaluation Software is used for analysis of sensorgrams and for calculation of affinity data.

### Example 1

### 1.1 Generation of anti-PD1 antibodies

### Immunization of mice

NMRI mice were immunized genetically, using a plasmid expression vector coding for full-length human PD1 by intradermal application of 100 ug vector DNA (plasmid15300_hPD1-fl), followed by Electroporation (2 square pulses of 1000 V/cm, duration 0.1 ms, interval 0.125 s; followed by 4 square pulses of 287.5 V/cm, duration 10 ms, interval 0.125 s. Mice received either 6 consecutive immunizations at days 0, 14, 28, 42, 56, 70, and 84. Blood was taken at days 36, 78 and 92 and serum prepared, which was used for titer determination by ELISA (see below). Animals with highest titers were selected for boosting at day 96, by intravenous injection of 50 ug of recombinant human PD1 human Fc chimera, and monoclonal antibodies were isolated by hybridoma technology, by fusion of splenocytes to myeloma cell line 3 days after boost.

### Determination of serum titers (ELISA)

Human recombinant PD1 human Fc chimera was immobilized on a 96-well NUNC Maxisorp plate at 0.3 ug/ml, 100 ul/well, in PBS, followed by: blocking of the plate with 2% Crotein C in PBS, 200 ul/well; application of serial dilutions of antisera, in duplicates, in 0.5% Crotein C in PBS, 100 ul/well; detection with HRP-conjugated goat anti-mouse antibody (Jackson Immunoresearch/Dianova 115-036-071; 1/16 000). For all steps, plates were incubated for 1 h at 37° C. Between all steps, plates were washed 3 times with 0.05% Tween 20 in PBS. Signal was developed by addition of BM Blue POD Substrate soluble (Roche), 100 ul/well; and stopped by addition of 1 M HCl, 100 ul/well. Absorbance was read out at 450 nm, against 690 nm as reference. Titer was defined as dilution of antisera resulting in half-maximal signal.

### 1.2 Characterization anti-PDl antibodies/ Binding of anti-PDl antibodies to human PD1

### Elisa for hu PD1

Nunc maxisorp streptavidin coated plates (MicroCoat #11974998001) were coated with 25 µl/well biotinylated PD1-ECD-AviHis and incubated at 4°C over night. After washing (3x90 µl/well with PBST-buffer) 25 µl anti PD1 samples or reference antibodies (human anti PD1; Roche/mouse anti PD1; Biolegend; cat.:329912) were added and incubated 1h at RT. After washing (3x90 µl/well with PBST-buffer) 25µl/well goat-anti-human H+L-POD (JIR, JIR109-036-088)/ Sheep-anti-mouse-POD (GE Healthcare; NA9310) was added in 1:2000/1:1000 dilution and incubated at RT for 1 h on shaker. After washing (3x90 µl/well with PBST-buffer) 25 µl/well TMB substrate (Roche Catalogue No. 11835033001) was added and incubated until OD 2 - 3. Measurement took place at 370/492 nm.

ELISA results are listed as EC₅₀-values [ng/ml] in Summary Table 1 and 2 below.

### Cell ELISA for PD1

Adherent CHO-K1 cell line stably transfected with plasmid 15311_hPD1-fl_pUC_Neo coding for full-length human PD1 and selection with G418 (Neomycin restistance marker on plasmid) were seeded at a concentration of 0.01x10E6 cells/well in 384-well flat bottom plates and grown over night.

The next day 25 µl/well PD1 sample or human anti PD1 (Roche)/mouse anti PD1(Biolegend; cat.:329912) reference antibody were added and incubated for 2h at 4°C (to avoid internalization). After washing carefully (1x90µl/well PBST) cells were fixed by adding 30µl/well 0,05% Glutaraldehyde (Sigma, Cat.No: G5882, 25%)diluted in 1xPBS-buffer and incubated for 10min at RT. After washing (3x90µl/well PBST) 25 µl/well secondary antibody was added for detection: goat-anti-human H+L-POD (JIR, JIR109-036-088)/Sheep-anti-mouse-POD (GE NA9310) followed by 1h incubation at RT on shaker. After washing (3x90µl/well PBST) 25 µl/well TMB substrate solution (Roche 11835033001) was added and incubated until OD 1.0 - 2.0. Plates were measured at 370/492 nm.

Cell ELISA results are listed as "EC₅₀ CHO-PD1"-values [ng/ml] in Summary Table 2 below.

### ELISA for cyno PD1

Nunc maxisorp streptavidin coated plates (MicroCoat #11974998001) were coated with 25 µl/well biotinylated cyno PD1-ECD-Biotin and incubated at 4°C over night. After washing (3x90 µl/well with PBST-buffer) 25 µl anti PD1 samples or reference antibodies (human anti PD1; Roche) were added and incubated 1h at RT on shaker. After washing (3x90 µl/well with PBST-buffer) 25µl/well goat-anti-human H+L-POD (JIR, JIR109-036-088) was added in 1:1000 dilution and incubated at RT for 1 h on shaker. After washing (3x90 µl/well with PBST-buffer) 25 µl/well TMB substrate (Roche, 11835033001) was added and incubated until OD 2 - 3. Measurement took place at 370/492 nm.

ELISA results are listed as EC₅₀-values [ng/ml] in Summary Tables 1 and 2 below.

### PD Ligand 1 replacing assay

Nunc maxisorp streptavidin coated plates (MicroCoat #11974998001) were coated with 25 µl/well biotinylated PD1-ECD-AviHis and incubated at 4°C over night. After washing (3x90 µl/well with PBST-buffer) 25 µl anti PD1 samples or reference antibodies (mouse anti PD1; Biolegend; cat.:329912) were added and incubated 1h at RT on shaker. After washing (3x90 µl/well with PBST-buffer) 25µl/well PD-L1 (Recombinant human B7-H1/PD-L1 Fc Chimera; 156-B7, R&D) was added and incubated 1h at RT on shaker. After washing (3x90 µl/well with PBST-buffer) 25µl/well goat-anti-human H+L-POD (JIR, 109-036-088) was added in 1:1000 dilution and incubated at RT for 1 h on shaker. After washing (3x90 µl/well with PBST-buffer) 25 µl/well TMB substrate (Roche, 11835033001) was added and incubated until OD 2 - 3. Measurement took place at 370/492 nm.

ELISA results are listed as IC₅₀-values [ng/ml] in Summary Table 1 below.

### PD Ligand 2 replacing assay

Nunc maxisorp streptavidin coated plates (MicroCoat #11974998001) were coated with 25 µl/well biotinylated PD1-ECD-AviHis and incubated at 4°C over night. After washing (3x90 µl/well with PBST-buffer) 25 µl anti PD1 samples or reference antibodies (mouse anti huPD1; Roche) were added and incubated 1h at RT on shaker. After washing (3x90 µl/well with PBST-buffer) 25µl/well PD-L2 (Recombinant human B7-DC/PD-L2 Fc Chimera; 1224-PL-100, R&D) was added and incubated 1h at RT on shaker. After washing (3x90 µl/well with PBST-buffer) 25µl/well goat-anti-human H+L-POD (JIR, 109-036-088) was added in 1:2000 dilution and incubated at RT for 1 h on shaker. After washing (3x90 µl/well with PBST-buffer) 25 µl/well TMB substrate (Roche, 11835033001) was added and incubated until OD 2 - 3. Measurement took place at 370/492 nm.

ELISA results are listed as IC₅₀-values [ng/ml] in summary Table 1 below.

### Epitope mapping ELISA/ Binding competition assay

Nunc maxisorp plates (Nunc #464718) were coated with 25 µl/well capture antibody (goat anti mouse IgG; JIR; 115-006-071) and incubated for 1h at RT on shaker. After washing (3x90µl/well with PBST-buffer) plates were blocked for 1h with 2% BSA containing PBS buffer at RT on shaker. After washing (3x90µl/well with PBST-buffer) 25µl mouse anti PD1 samples were added and incubated 1h at RT on shaker. After washing (3x90µl/well with PBST-buffer) capture antibody was blocked by 30µl/well mouse IgG (JIR; 015-000-003) for 1h at RT on shaker. At the same time biotinylated PD1-ECD-AviHis was preincubated with second sample antibody for 1h at RT on shaker. After washing assay plate (3x90 µl/well with PBST-buffer) the PD1 antibody mix was transferred to assay plate and incubated at RT for 1h on shaker. After washing (3x90 µl/well with PBST-buffer) 25µl/well streptavidin POD (Roche, #11089153001) was added in 1:4000 dilution and incubated at RT for 1 h on shaker. After washing (3x90 µl/well with PBST-buffer) 25 µl/well TMB substrate (Roche, #11089153001) was added and incubated until OD 1.5 - 2.5. Measurement took place at 370/492 nm. Epitope groups were defined by hierarchical clustering against reference antibodies.

**Table 1: Binding, PD-L1 inhibition and epitope region groups of exemplary PD1 antibodies (ELISA)**

| **Antibody** | **ELISA huPD1 EC₅₀ [ng/ml]** | **ELISA cyPD1 EC₅₀ [ng/ml]** | **ELISA PD-L1 inhibition IC₅₀ [ng/ml]** | **ELISA PD-L2 inhibition IC₅₀ [ng/ml]** | **Epitope region group (by competition assay)** |
|---|---|---|---|---|---|
| PD1-0050 | 17.9 | 9.8 | 128 | 34 | 1 |
| PD1-0069 | 45.7 | 22.7 | 225 | 89 | 6 |
| PD1-0073 | 15.1 | 8.3 | 124 | 65 | 5 |
| PD1-0078 | 26.3 | 22.4 | x | 86 | 2 |
| PD1-0098 | 50.8 | 54.6 | 174 | 45 | 5 |
| PD1-0102 | 34.2 | 52.7 | >35.5 µg/ml | 140 | 4 |
| PD1-0103 | 33.7 | 36.9 | 182 | 51 | 5 |

**Table 2: Biochemial- and Cell-binding of humanized PD1 antibodies derived from parental mouse antibody PD1-0103 (ELISA)**

| **Humanized antibody** | **ELISA huPD1 EC₅₀ [ng/ml]** | **ELISA cyPD1 EC₅₀ [ng/ml]** | **ELISA CHO-PD1 EC₅₀ [ng/ml]** |
|---|---|---|---|
| PD1-103-0312 | 11 | 8.3 | 10.1 |
| PD1-103-0313 | 15 | 11 | 10.8 |
| PD1-103-0314 | 11 | 8.3 | 7.7 |
| PD1-103-0315 | 10 | 7.9 | 7.3 |

### Biacore characterization of the humanized anti-PD1 antibodies

A surface plasmon resonance (SPR) based assay has been used to determine the kinetic parameters of the binding between several murine PD1 binders as well as commercial human PD1 binding references. Therefore, an anti-human IgG was immobilized by amine coupling to the surface of a (Biacore) CM5 sensor chip. The samples were then captured and hu PD1-ECD was bound to them. The sensor chip surface was regenerated after each analysis cycle. The equilibrium constant and kinetic rate constants were finally gained by fitting the data to a 1:1 langmuir interaction model.

About 2000 response units (RU) of 20 µg/ml anti-human IgG (GE Healthcare #BR-1008-39) were coupled onto the flow cells 1 and 2 (alternatively: 3 and 4) of a CM5 sensor chip in a Biacore T200 at pH 5.0 by using an amine coupling kit supplied by GE Healthcare.

The sample and running buffer was HBS-EP+ (0.01 M HEPES, 0.15 M NaCl, 3 mM EDTA, 0.05 % v/v Surfactant P20, pH 7.4). Flow cell temperature was set to 25 °C and sample compartment temperature to 12 °C. The system was primed with running buffer.

The samples were injected for 20 seconds with a concentration of 10 nM and bound to the second flow cell. Then a complete set of human PD1-ECD concentrations (144 nM, 48 nM, 16 nM, 5.33 nM, 1.78 nM, 0.59 nM, 0.20 nM and 0 nM) was injected over each sample for 120s followed by a dissociation time of 30/300s and two 20s regeneration steps with 3 M MgCl₂, of which the last one contained an "extra wash after injection" with running buffer.

Finally the double referenced data was fitted to a 1:1 langmuir interaction model with the Biacore T200 Evaluation Software. Resulting *K*_{D}*, k*ₐ and *k*_{d} values are shown in Table 3.

**Table 3: Kinetic rate constants and equilibrium constant for chimeric PD1-0103 and humanized PD1-Abs determined by Biacore**

| Ligand | *k*ₐ [M⁻¹s⁻¹] | *k*_{d} [s⁻¹] | ***K*_{D} [nM]** |
|---|---|---|---|
| chimeric PD1-0103 | 3.86E+05 | 3.07E-04 | **0.8** |
| PD1-0103-0312 | 1.95E+05 | 3.45E-04 | **1.8** |
| PD1-0103-0313 | 1.60E+05 | 3.67E-04 | **2.3** |
| PD1-0103-0314 | 1.87E+05 | 2.79E-04 | **1.5** |
| PD1-0103-0315 | 1.89E+05 | 2.91E-04 | **1.5** |

As shown in Table 3, all the humanized versions of chimeric PD1-0103 (generation see Example 1.3) display kinetic properties similar to the parental antibody (chimeric PD1-0103).

### Kinetics

A CM5 sensor series S was mounted into the Biacore 4000 System and the detection spots were hydrodynamically addressed according to the manufacturer's instructions.

The polyclonal rabbit IgG antibody <IgGFCγM>R (Jackson ImmunoResearch Laboratories Inc.) was immobilized at 10 000 Ru on the detection spots 1 and 5 in the flow cells 1,2,3 and 4. Coupling was done via EDC/NHS chemistry according to the manufacturer's instructions. The remaining spots in the flow cells served as a reference. The sample buffer was the system buffer supplemented with 1 mg/ml carboxymethyldextrane.

In one embodiment the assay was driven at 25 °C. In another embodiment the assay was driven at 37 °C. 50 nM of each murine monoclonal antibody was captured on the sensor surface by a 1 min injection at 10 µl/min. Subsequently the respective antigens were injected in a concentration series of 100 nM, 2x 33 nM, 11 nM, 4 nM, 1 nM and system buffer 0 nM at 30µl/min for 4 min association phase time. The dissociation was monitored for another 4 min. The capture system was regenerated using a 3 min injection of 10 mM glycine pH 1.5 at 30 µl/min. Relevant kinetic data was calculated using the Biacore evaluation software according to the manufacturer's instructions.

### Epitope Mapping

A Biacore 4000 instrument was mounted with a Biacore CAP sensor and was prepared like recommended by the manufacturer. The instrument buffer was HBS-ET (10 mM HEPES pH 7.4, 150 mM NaCl, 3 mM EDTA, 0.005% w/v Tween 20). The instrument was running at 25 °C.

All samples were diluted in system buffer. A 35kDa biotinylated antigen PD1-ECD-AviHis was captured at 200 RU on the CAP sensor surface by a 1 min injection at 30µl/min in the flow cells 1, 2, 3 and 4 in the spots 1 and 5. Spots 2, 3 and 4 served as a reference. In another embodiment, a 35 kDa biotinylated antigen PDI-ECD-AviHis was captured at 200 RU on the CAP sensor in the same manner.

Subsequently a primary antibody was injected at 100 nM for 3 min at 30 µl/min followed by the injection of a secondary antibody at 100 nM for 3 min at 30 µl/min. The primary antibody was injected until full saturation of the surface presented antigen. At the end of the primary and secondary antibody injection phases report points " Binding Late" (BL) were set to monitor the binding response of the respective antibodies. The Molar Ratio, a quotient between the secondary antibody binding response "BL2" and the primary antibody response "BL1" was calculated. The Molar Ratio was used as an indicator of the antigen accessibility of the secondary antibody, when the antigen was already complexed by the primary antibody.

The complexes were completely removed from the sensor surface by an injection for 2 min at 30µl/min 2M guanidine-HCL 250 mM NaOH regeneration buffer as recommended by the manufacturer, followed by a 1 min injection at 30µl /min of system buffer.

### 1.3 Effect of different anti-PDl Antibodies on Cytokine Production in a Mixed Lymphocyte Reaction (MLR)

3A) The Mixed Lymphocyte Reaction (MLR) is a immune cell assay which measures the activation of lymphocytes from one individual (donor X) to lymphocytes from another individual (donor Y). A mixed lymphocyte reaction was used to demonstrate the effect of blocking the PD1 pathway to lymphocyte effector cells. T cells in the assay were tested for activation and theier IFN-gamma secretion in the presence or absence of an anti-PDl mAbs.

To perform an allogeneic MLR, peripheral blood mononuclear cells (PBMCs) from at least four healthy donors of unknown HLA type were isolated by density gradient centrifugation using Leukosep (Greiner Bio One, 227 288). Briefly, heparinized blood samples were diluted with the three fold volume of PBS and 25 ml aliquots of the diluted blood were layered in 50 ml Leukosep tubes. After centrifugation at 800 x g for 15 min at room temperature (w/o break) the lymphocyte containing fractions were harvested, washed in PBS and used directly in functional assay or resuspended in freezing medium (10% DMSO, 90 %FCS) at 1.0E+07 cells/ml and stored in liquid nitrogen. Individual 2-way MLR reactions were set up by mixing PBMCs from two different donors at a 1:1 stimulator/responder cell ratio and co-cultures were done at least in duplicate in flat-bottomed 96-well plates for 6 days at 37oC, 5% CO2, in the presence or w/o of a different concentration range of purified anti-PDl monoclonal antibodies PD1-0050, PD1-0069, PD1-0073, PD1-0078, PD1-0098, PD1-0102, PD1-0103. As reference anti-PD1 antibodies antibodies comprising the VH and VL domains of either nivolumab (also known as MDX-5C4 or MDX-1106) or pembrolizumab (also known as MK-3475 or Org 1.09A) were synthesized and cloned with backbones of human IgG1 (with mutations L234A, L235A and P329G (EU index of Kabat)). Either no antibody or an isotype control antibody was used as a negative control and rec hu IL-2 (20 EU/ml) was used as positive control. After day 6 100 µl of medium was taken from each culture for cytokine measurement. The levels of IFN-gamma were measured using OptEIA ELISA kit (BD Biosciences).

The results are shown in Table 4 (IFN-γ secretion/release). The anti-PDl monoclonal antibodies promoted T cell activation and IFN-gamma secretion in concentration dependent manner. The value of % increase of IFNg secretion was calculated in relation to IFN-γ production of MLR w/o adding of any blocking mAbs (basal allogeneic stimulation induced IFNg value as E-c) and MLR with adding of 20 EU/ml rec hu IL-2 (positive control = 100% IFNg value as E+c) and was calculated according to formula: Rel. Stimulation [%] = ((Example-E-c)/(E+c-E-c)^{∗}100).

**Table 4: Percentage of of IFN gamma secretion after allogenic stimulation and treatment with anti-PD1 antibody in comparison to effect of recombinant human IL-2 treatment (20 EU/ml) (= 100% increase) as positive control**

| | Concentration (µg/ml) | 1:12 | 1:120 | 1:1200 | Effect in MLR |
|---|---|---|---|---|---|
| PD1-0050 | 44 | 136 | 96 | 33 | +++ |
| PD1-0069 | 60 | 76 | 71 | 55 | +++ |
| PD 1-0073 | 43 | 103 | 63 | 38 | ++ |
| PD1-0078 | 64 | 99 | 72 | 21 | ++ |

Several PD1 blocking antibodies PD1-0050, PD1-0069, PD1-0073, PD1-0078, PD1-0098, PD1-0102, PD1-0103 demonstrated strong immune modulating activity by enhancing secretion of interferon gamma (IFN-γ) (data not shown for all antibodies).

**3B)** In a further experiment chimeric PD1-0103 (human IgG1 isotype with mutations L234A, L235A and P329G (EU index of Kabat)) was evaluated. Blockade of PD1 with chimeric PD1-0103 strongly enhances IFN-gamma secretion by allogenic stimulated primary human T cells. Chimeric PD1-0103 is more potent than reference anti-PDl antibodies (see **Figure 1**).

For comparison the reference anti-PDl antibodies comprising the VH and VL domains of either nivolumab (also known as MDX5C4 or MDX-1106) and pembrolizumab (also known as MK-3475 or Org 1.09A) were synthesized and cloned with backbones of human IgG1 (with mutations L234A, L235A and P329G (EU index of Kabat)) were used.

**3C)** In additional experiments the immune modulating activity of the humanized variants of anti-PDl antibody PD1-0103 (humanized antibodies PD1-0103-0312, PD1-0103-0314, in figures 2 and 3) the a) IFNγ release (secretion) b) TNF-alpha release (secretion) was evaluated in MLR as described above. The effect of the chimeric PD1-0103 antibody and its humanized versions were compared to the reference anti-PD1 antibodies comprising the VH and VL domains of either nivolumab (also known as MDX5C4 or MDX-1106) and pembrolizumab (also known as MK-3475 or Org 1.09A) with backbones of human IgG1 (with mutations L234A, L235A and P329G (EU index of Kabat)). After 6 days of MLR culture 50 µl of supernatant was taken and multiple cytokines were measured in a single culture using Bio-Plex Pro™ Human Cytokine Th1/Th2 Assay (Bio-Rad Laboratories Inc.). (data not shown for all cytokines).

The chimeric PD1-0103 antibody and its humanized versions (PD1-0103_0312 and PD1-0103_0314) were more potent compared to the reference anti-PD1 antibodies in enhancing the T cell activation and IFN-gamma secretion (see **Figure 2**).

Furthermore, the chimeric PD1-0103 antibody and its humanization variants increase tumor necrosis factor alpha (TNF alpha) (see **Figure 3**) and IL-12 (data not shown) secretion by antigen presenting cells and encance capacity of monocytes /macrophages or antigen presenting cells to stimulate a T cell.

### 1.4 Effect of anti-PDl blockade on cytotoxic Granzyme B release and IFN-γ secretion by human CD4 T cells cocultured with allogeneic mature dendritic cells

To further investigate the effect of anti-PDl treatment in an allogeneic setting we developed an assay in which freshly purified CD4 T cells are cocultured for 5 days in presence of monocyte-derived allogeneic mature dendritic cells (mDCs). Monocytes were isolated from fresh PBMCs one week before through plastic adherence followed by the removal of the non-adherent cells. We then generated immature DCs from the monocytes by culturing them for 5 days in media containing GM-CSF (50 ng/ml) and IL-4 (100 ng/ml). To induce iDCs maturation, we added TNF-α, IL-1β and IL-6 (50 ng/ml each) to the culturing media for 2 additional days. We then assessed DCs maturation by measuring their surface expression of Major Histocompatibility Complex Class II (MHCII), CD80, CD83 and CD86 thorugh flow cytometry (LSRFortessa, BD Biosciences).

On the day of the minimal mixed lymphocyte reaction (mMLR), CD4 T cells were enriched via a microbead kit (Miltenyi Biotec) from 10⁸ PBMCs obtained from an unrelated donor. Prior culture, CD4 T cells were labeled with 5µM of Carboxy-Fluorescein-Succinimidyl Esther (CFSE). 10⁵ CD4 T cells were then plated in a 96 well plate together with mature allo-DCs (5:1) in presence or absence of blocking anti-PD1 antibody (either PD1-0103, chimeric PD1-0103, or humanized antibodies PD1-0103-0312, PD1-0103-0313, PD1-0103-0314, PD1-0103-0315, abbreviated as 0312, 0313, 0314, 0315 in **figures 4A** **and** **4** **B**), at the concentration of 10 µg/ml if not differentely indicated in the figures.

Five days later we collected the cell-culture supernatants, used later to measure the IFN-γ levels by ELISA (R&D systems), and left the cells at 37 C degrees for additional 5 hours in presence of Golgi Plug (Brefeldin A) and Golgi Stop (Monensin). The cells were then washed, stained on the surface with anti-human CD4 antibody and the Live/Dead fixable dye Aqua (Invitrogen) before being fixed/permeabilized with Fix/Perm Buffer (BD Bioscience). We performed intracellular staining for Granzyme B (BD Bioscience), IFN-γand IL-2 (both from eBioscience).

We also tested different concentrations of the humanized variants PD1-0103 (humanized antibodies PD1-0103-0312, PD1-0103-0313, PD1-0103-0314, PD1-0103-0315, abbreviated as 0312, 0313, 0314, 0315 in the figures, see also Example 1.6 below) and found them to be equally good in enhancing granzyme B and interferon gamma. DP47 is a non binding human IgG with a LALA mutation in the Fc portion to avoid recognition by FcyR and was used as negative control. Results are shown in **Figures 4A** **and** **4** **B.**

### 1.5 Chimeric antibodies derivatives

Chimeric PD1 antibodies were generated by amplifying the variable heavy and light chain regions of the anti-PDl mouse antibodies PD1-0098, PD1-0103 via PCR and cloning them into heavy chain expression vectors as fusion proteins with human IgG1 backbones / human CH1-Hinge-CH2-CH3 with mutations L234A, L235A and P329G (EU index of Kabat)) (Leucine 234 to Alanine, Leucine 235 to Alanine, Proline 329 to Glycine) abrogating effector functions and light chain expression vectors as fusion proteins to human C-kappa. LC and HC Plasmids were then cotransfected into HEK293 and purified after 7 days from supertnatants by standard methods for antibody purification. The chimeric PD1-antibodies were renamed chimeric chiPD1-0098 (chiPD1-0098) and chimeric PD1-0103 (chiPD1-0103). For comparison the reference anti-PDl antibodies comprising the VH and VL domains of either nivolumab (also known as MDX-5C4 or MDX-1106) and pembrolizumab (also known as MK-3475 or Org 1.09A) were synthesized and cloned with backbones of human IgG1 (with mutations L234A, L235A and P329G (EU index of Kabat)) were used.

### 1.6 Generation, Expression and Purification of humanized variants of anti-PDl antibody PD-0103 (huMab PD-0103) and characterization

### Humanization of the VH and VL domains of murine anti-PD1 antibody 0103

Based upon the amino acid sequence of the murine VH and VL domains of murine anti-PD1 antibody PD1-0103 (SEQ ID NO: 43 and 44), humanized anti- anti-PDl antibody variants were generated.

The humanized VH-variant is based on the human germline IMGT_hVH_3_23 in combination with the human J-element germline IGHJ5-01 with several mutations. (resulting in SEQ ID NO: 45).

The humanized variants of VL are based on the human germlines IMGT_hVK_4_1, IMGT_hVK_2_30, IMGT_hVK_3_11 and IMGT_hVK_1_39 in combination with the human J-element germline IGKJ1-01. Different muations resulted in humanized variants of SEQ ID NO: 46 to SEQ ID NO: 49.

The humanized amino acid sequences for heavy and light chain variable regions of PD1-0103 were backtranslated in to DNA and the resulting cNDA were synthesized (GenArt) and then cloned into heavy chain expression vectors as fusion proteins with human IgG1 backbones /human CH1-Hinge-CH2-CH3 with LALA and PG mutations (Leucine 234 to Alanine, Leucine 235 to Alanine, Proline 329 to Glycine) abrogating effector functions or into light chain expression vectors as fusion proteins to human C-kappa. LC and HC Plasmids were then cotransfected into HEK293 and purified after 7 days from supertnatants by standard methods for antibody purification. The resulting humanized PD1-antibodies named as follows:

**Table 5: VH and VL sequences of humanized variant antibodies of PD1-0103**

| **Humanized antibodies of PD1-0103** | **humanized variant of VH/SEQ ID NO:** | **humanized variant of VL/SEQ ID NO:** |
|---|---|---|
| PD1-0103-0312 | SEQ ID NO: 21 | SEQ ID NO: 22 |
| PD1-0103-0313 | SEQ ID NO: 21 | SEQ ID NO: 23 |
| PD1-0103-0314 | SEQ ID NO: 21 | SEQ ID NO: 24 |
| PD1-0103-0315 | SEQ ID NO: 21 | SEQ ID NO: 25 |

**Table 6: HVR sequences of humanized variant antibodies of PD1-0103**

| **Humanized antibodies of PD1-0103** | **HVR-H1, HVR-H2, and HVR-H3 of humanized variant/SEQ ID NO:** | **HVR-L1, HVR-L2, and HVR-L3 of humanized variant/SEQ ID NO:** |
|---|---|---|
| PD-0103-0312 | SEQ ID NOs: 13, 14 and 15 | SEQ ID NOs: 16, 17 and 18 |
| PD-0103-0313 | SEQ ID NOs: 13, 14 and 15 | SEQ ID NOs: 16, 17 and 18 |
| PD-0103-0314 | SEQ ID NOs: 13, 14 and 15 | SEQ ID NOs: 16, 17 and 18 |
| PD-0103-0315 | SEQ ID NOs: 13, 14 and 15 | SEQ ID NOs: 16, 17 and 18 |

Humanized PD1-0103 antibody variants and parental chimeric PD1-0103 were characterized as descibed above. Results are shown in Table 7.

**Table 7: Summary of results for humanized PD1-0103 antibody variants and parental chimeric PD1-0103**

| **Assay** | **chimeric PD1-0103** | **PD-0103-0312** | **PD-0103-0313** | **PD-0103-0314** | **PD-0103-0315** |
|---|---|---|---|---|---|
| Affinity *K*_{D} [nM] at 37°C | 2.0/0.8 | 1.5/1.8 | 1.9/2.3 | 1.6/1.5 | 1.7/1.5 |
| ELISA EC₅₀ [nM] | 0,2 | 0,1 | 0,07 | 0,07 | 0,06 |
| CHO-PD1 EC₅₀ | + | + | + | + | + |
| IC₅₀ PD-L1, 2 [nM] | 1.35 | tbd | tbd | tbd | tbd |
| Mixed Lymphocyte Reaction assay | +++ | +++ | +++ | ++++ | ++ |
| cynomolgus crossreactivity (EC₅₀ [nm] | + | 0,08 | 0,06 | 0,05 | 0,04 |

### 1.7 Neutralizing potency of PD1 antibodies

To test the neutralizing potency of inhouse generated PD1 antibodies in mimicking a restoration of a suppressed T cell response in vitro a commercially available PD1/PD-L1 reporter assay (Promega) was used. This system consists of PD1+ NFAT Jurkat cells and a PD-L1+ CHO counterpart, which also gives the activation signal. In principle, the reporter system is based on three steps: (1) TCR-mediated NFAT activation, (2) inhibition of NFAT signal upon activation by the PD-1/PD-L1 axis and (3) recovery of the NFAT signal by PD1 blocking antibodies.

Material and Methods:
- PD-L1 Medium: PAN Biotech (#P04-03609); FBS (10%) and L-Gln (4mM)
- Assay Medium: RPMI 1640 (#31870; Invitrogen), 25mM HEPES, 2mM L-Gln, FBS (2%)
- Cells used for this assay (both cell types purchased by Promega):
   PD-L1+ CHO cells (batch no. #139147): 2-3x104 cells/96well
   PD1+NFAT Jurkat cells (batch no. #133024: 3.5x104 cells/well

On day 1, PD-L1+ cells were thawed, seeded at the indicated cell concentration in the above mentioned medium and cultured over night at 37°C and 5% CO₂. On the next day, medium was removed and PD-L1+ cells were incubated with the prepared antibodies at indicated concentrations (in Assay Medium). In parallel, PD1+ NFAT Jurkat cells were thawed and above mentioned cell numbers were transferred to and co-cultured with the PD-L1+ cells. After an incubation of 6 hrs at 37 °C and 5% CO₂, Bio-Glo substrate was warmed to room temperature (1-2 hrs prior addition). The cell culture plate was removed from the incubator and adjusted to room temperature (10min) before 80µl Bio-Glo solution was added per well, incubated for 5-10 min before the luminescence was measured at a Tecan Infinite reader according to the kit's manufacturer's recommendation. Results can be seen in the Figures 5A and 5B where the restoration of a PD1/PD-L1 mediated suppression of the NFAT signal by different PD1 antibodies upon TCR stimulation is shown: **Figure 5** **A:** Chimeric PD1_0103 showed a reproducibly superior effect when compared to a reference antibody. As reference an anti-PD1 antibody comprising the VH and VL domains nivolumab (also known as MDX-5C4 or MDX-1106) was synthesized and cloned with backbones of human IgG1 (with mutations L234A, L235A and P329G (EU index of Kabat)). **Figure 5B****:** The four humanized variants of PD1_0103 demonstrated a similar in vitro potency to the lead antibody and were also slightly superior to the reference antibody.

### Example 2

### Preparation of PD1- targeted 4-1BB ligand trimer-containing Fc fusion antigen binding molecules

Different fragments of the DNA sequence encoding part of the ectodomain (amino acids 71-254 and 71-248) of human 4-1BB ligand were synthetized according to the P41273 sequence of Uniprot database (SEQ ID NO:103).

### 2.1 Preparation of monovalent PD1 (0314) targeted 4-1BB ligand (71-254) trimer-containing Fc (kih) fusion antigen binding molecule with crossed CH1-CL domains with charged residues (Construct 7.1)

A polypeptide containing two ectodomains of 4-1BB ligand (71-254), separated by (G4S)2 linkers, and fused to the human IgG1-CL domain, was cloned as depicted in **Figure 6A****:** human 4-1BB ligand, (G4S)2 connector, human 4-1BB ligand, (G4S)2 connector, human CL.

A polypeptide containing one ectodomain of 4-1BB ligand (71-254) and fused to the human IgG1-CH1 domain, was cloned as described in **Figure 6B****:** human 4-1BB ligand, (G4S)2 connector, human CH.

To improve correct pairing the following mutations have been introduced in the crossed CH-CL. In the dimeric 4-1BB ligand fused to human CL the mutations E123R and Q124K were introduced. In the monomeric 4-1BB ligand fused to human CHI, the mutations K147E and K213E were cloned into the human CH1 domain as described in International Patent Appl. Publ. No. WO 2015/150447.

The variable region of heavy and light chain DNA sequences encoding a binder specific for PD1, clone 0314 (or 0103-0314), were subcloned in frame with either the constant heavy chain of the hole or the constant light chain of human IgG1. The generation and preparation of the PD1 binders is described in Example 1.

The Pro329Gly, Leu234Ala and Leu235Ala mutations have been introduced in the constant region of the knob and hole heavy chains to abrogate binding to Fc gamma receptors according to the method described in International Patent Appl. Publ. No. WO 2012/130831.

For all constructs the knobs into hole heterodimerization technology was used with the the S354C/T366W mutations in the knob chain and the corresponding Y349C/T366S/L368A/Y407V mutations in the hole chain.

Combination of the dimeric ligand-Fc knob chain containing the S354C/T366W mutations, the monomeric CHI fusion, the targeted anti-PD1-Fc hole chain containing the Y349C/T366S/L368A/Y407V mutations and the anti-PD1 light chain allows generation of a heterodimer, which includes an assembled trimeric 4-1BB ligand and a PD1 binding Fab (**Figure 7**, Construct 7.1).

Table 8 shows the cDNA and amino acid sequences of the monovalent PD1(0314)-human 4-1BB ligand (71-254) Fc (kih) fusion antigen binding molecule containing CH1-CL crossover and charged residues (Construct 7.1).

**Table 8: Sequences of monovalent PD1(0314)-targeted human 4-1BB ligand (71-254) containing Fc (kih) fusion molecule Construct 7.1**

| SEQ ID NO: | Description | Sequence |
|---|---|---|
| 131 | nucleotide sequence of dimeric hu 4-1BBL (71-254)-CL^{∗} Fc knob chain | |
| | | |
| 132 | nucleotide sequence of monomeric hu 4-1BBL (71-254)-CH1^{∗} | |
| | | |
| 133 | nucleotide sequence of anti-PD1(0314) Fc hole chain | |
| 134 | nucleotide sequence of anti-PD1 (0314) light chain | |
| | | |
| 74 | Dimeric hu 4-1BBL (71-254)-CL^{∗} Fc knob chain | |
| 75 | Monomeric hu 4-1BBL (71-254)-CH1^{∗} | |
| 82 | anti-PDl (0314) Fc hole chain | |
| 83 | anti-PDl (0314) light chain | |

### 2. 2 Preparation of monovalent PD1 (0314) targeted 4-1BB ligand (71-254) trimer-containing Fc (kih) fusion antigen binding molecule with crossed CH1-CL domains without charged residues (Construct 7.2)

A polypeptide containing two ectodomains of 4-1BB ligand (71-254), separated by (G4S)2 linkers, and fused to the human IgG1-CL domain, was cloned as depicted in **Figure 6C****:** human 4-1BB ligand, (G4S)2 connector, human 4-1BB ligand, (G4S)2 connector, human CL.

A polypeptide containing one ectodomain of 4-1BB ligand (71-254) and fused to the human IgG1-CH1 domain, was cloned as described in **Figure 6D****:** human 4-1BB ligand, (G4S)2 connector, human CH1.

The variable region of heavy and light chain DNA sequences encoding a binder specific for PD1, clone 0103-0314, were subcloned in frame with either the constant heavy chain of the hole or the constant light chain of human IgG1.

The Pro329Gly, Leu234Ala and Leu235Ala mutations have been introduced in the constant region of the knob and hole heavy chains to abrogate binding to Fc gamma receptors (WO 2012/130831).

Combination of the dimeric ligand-Fc knob chain containing the S354C/T366W mutations, the monomeric CH1 fusion, the targeted anti-FAP-Fc hole chain containing the Y349C/T366S/L368A/Y407V mutations and the anti-FAP light chain allows generation of a heterodimer, which includes an assembled trimeric 4-1BB ligand and a FAP binding Fab (**Figure 7****,** Construct 7.2).

Table 9 shows the cDNA and amino acid sequences of the monovalent anti-PD1(0314) human 4-1BB ligand (71-254) Fc (kih) fusion antigen binding molecule containing CH1-CL crossover without charged residues (Construct 7.2).

**Table 9: Sequences of monovalent PD1(0314)-targeted human 4-1BB ligand (71-254) containing Fc (kih) fusion molecule Construct 7.2**

| SEQ ID NO: | Description | Sequence |
|---|---|---|
| 135 | nucleotide sequence of dimeric hu 4-1BBL (71-254)-CL Fc knob chain | |
| | | |
| 136 | nucleotide sequence of monomeric hu 4-1BBL (71-254)-CH1 | |
| | | |
| 133 | nucleotide sequence of anti-PD1(0314) Fc hole chain | see Table 8 |
| 134 | nucleotide sequence of anti-PD 1(0314) light chain | see Table 8 |
| 76 | Dimeric hu 4-1BBL (71-254)-CL Fc knob chain | |
| 77 | Monomeric hu 4-1BBL (71-254)-CH1 | |
| 82 | anti-PD1(0314) Fc hole chain | see Table 8 |
| 83 | anti-PD1(0314) light chain | see Table 8 |

### 2.3 Preparation of bivalent PD1(0314) targeted 4-1BB ligand (71-254) trimer-containing Fc (kih) fusion antigen binding molecule with the dimeric and monomeric 4-1BB ligands fused at the C-terminus of each heavy chain (Construct 7.3)

A polypeptide containing two ectodomains of 4-1BB ligand (71-254), separated by (G4S)2 linkers was fused to the C-terminus of human IgG1 Fc hole chain, as depicted in **Figure 6E****:** human IgG1 Fc hole, (G4S)2 connector, human 4-1BB ligand, (G4S)2 connector, human 4-1BB ligand. A polypeptide containing one ectodomain of 4-1BB ligand (71-254) was fused to the C-terminus of human IgG1 Fc knob chain as described in **Figure 6F****:** human IgG1 Fc knob, (G4S)2 connector, human 4-1BB ligand.

The variable region of heavy and light chain DNA sequences encoding a binder specific for PD1, clone 0103-0314, were subcloned in frame with either the constant heavy chain of the hole, the knob or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the anti-FAP huIgG1 hole dimeric ligand chain containing the Y349C/T366S/L368A/Y407V mutations, the anti-FAP huIgG1 knob monomeric ligand chain containing the S354C/T366W mutations and the anti-FAP light chain allows generation of a heterodimer, which includes an assembled trimeric 4-1BB ligand and two PD1 binding Fabs (**Figure 7****,** Construct 7.3)

Table 10 shows the cDNA and amino acid sequences of the bivalent PD1 (0314)-targeted 4-1BB ligand trimer-containing Fc (kih) fusion molecule Construct 7.3 (PD1 split trimer with 2 anti-PDl Fabs, dimeric and monomeric 4-1BB ligand fused at the C-terminus of each heavy chain, respectively).

**Table 10: Sequences of bivalent PD1(0314)-targeted human 4-1BB ligand (71-254) containing Fc (kih) fusion molecule Construct 7.3**

| SEQ ID NO: | Description | Sequence |
|---|---|---|
| 137 | nucleotide sequence of anti-PD1(0314) Fc hole chain fused to dimeric hu 4-1BBL (71-254) | |
| | | |
| 138 | nucleotide sequence of anti-PD1(0314) Fc knob chain fused to monomeric hu 4-1BBL (71-254) | |
| | | |
| 134 | nucleotide sequence of anti-PD 1(0314) light chain | see Table 8 |
| 84 | anti-PD1(0314) Fc hole chain fused to dimeric hu 4-1BBL (71-254) | |
| 85 | anti-PD1(0314) Fc knob chain | |
| | fused to monomeric hu 4-1BBL (71-254) | |
| 83 | anti-PD1(0314) light chain | see Table 21 |

### 2.4 Preparation of monovalent PD1(0314) targeted 4-1BB ligand (71-248) trimer-containing Fc (kih) fusion antigen binding molecule with crossed CH1-CL domains with charged residues (Construct 7.4)

A polypeptide containing two ectodomains of 4-1BB ligand (71-248), separated by (G4S)2 linkers, and fused to the human IgG1-CL domain, was cloned as depicted in **Figure 8A****:** human 4-1BB ligand, (G4S)2 connector, human 4-1BB ligand, (G4S)2 connector, human CL. A polypeptide containing one ectodomain of 4-1BB ligand (71-248) and fused to the human IgG1-CH domain, was cloned as described in **Figure 8B****:** human 4-1BB ligand, (G4S)2 connector, human CH1.

To improve correct pairing the following mutations have been introduced in the crossed CH-CL. In the dimeric 4-1BB ligand fused to human CL the mutations E123R and Q124K were introduced. In the monomeric 4-1BB ligand fused to human CHI, the mutations K147E and K213E were cloned into the human CH1 domain as described in International Patent Appl. Publ. No. WO 2015/150447.

The variable region of heavy and light chain DNA sequences encoding a binder specific for PD1, clone 0103-0314, were subcloned in frame with either the constant heavy chain of the hole or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the dimeric ligand-Fc knob chain containing the S354C/T366W mutations, the monomeric CH1 fusion, the targeted anti-PD1(0314)-Fc hole chain containing the Y349C/T366S/L368A/Y407V mutations and the anti-PD1(0314) light chain allows generation of a heterodimer, which includes an assembled trimeric 4-1BB ligand and a FAP binding Fab (**Figure 7****,** Construct 7.4).

**Table 11** shows the cDNA and amino acid sequences of the monovalent PD1(0314)-human 4-1BB ligand (71-248) Fc (kih) fusion antigen binding molecule containing CH1-CL crossover with charged residues (Construct 7.4).

**Table 11: Sequences of monovalent PD1(0314)-targeted human 4-1BB ligand (71-248) containing Fc (kih) fusion molecule Construct 7.4**

| SEQ ID NO: | Description | Sequence |
|---|---|---|
| 139 | nucleotide sequence of dimeric hu 4-1BBL (71-248)-CL^{∗} Fc knob chain | |
| | | |
| 140 | nucleotide sequence of monomeric hu 4-1BBL (71-248)-CH1^{∗} | |
| 133 | nucleotide sequence of anti-PD1(0314) Fc hole chain | see Table 8 |
| 134 | nucleotide sequence of anti-PD 1(0314) light chain | see Table 8 |
| 78 | Dimeric hu 4-1BBL (71-248)-CL^{∗} Fc knob chain | |
| 79 | Monomeric hu 4-1BBL (71-248)-CH1^{∗} | |
| 82 | anti-PD1(0314) Fc hole chain | see Table 8 |
| 83 | anti-PD1(0314) light chain | see Table 8 |

### 2.5 Preparation of monovalent PD1(0314) targeted 4-1BB ligand (71-248) trimer-containing Fc (kih) fusion antigen binding molecule with crossed CH1-CL domains without charged residues (Construct 7.5)

A polypeptide containing two ectodomains of 4-1BB ligand (71-248), separated by (G4S)2 linkers, and fused to the human IgG1-CL domain, was cloned as depicted in **Figure 8C****:** human 4-1BB ligand, (G4S)2 connector, human 4-1BB ligand, (G4S)2 connector, human CL. A polypeptide containing one ectodomain of 4-1BB ligand (71-248) and fused to the human IgG1-CH domain, was cloned as described in **Figure 8D****:** human 4-1BB ligand, (G4S)2 connector, human CH.

The variable region of heavy and light chain DNA sequences encoding a binder specific for PD1, clone 0103-0314, were subcloned in frame with either the constant heavy chain of the hole or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the dimeric ligand-Fc knob chain containing the S354C/T366W mutations, the monomeric CH1 fusion, the targeted anti-PDl Fc hole chain containing the Y349C/T366S/L368A/Y407V mutations and the anti-PD1 light chain allows generation of a heterodimer, which includes an assembled trimeric 4-1BB ligand and a FAP binding Fab (**Figure 7****,** Construct 7.5)

Table 12 shows the cDNA and amino acid sequences of the monovalent PD1(0314)-human 4-1BB ligand (71-248) Fc (kih) fusion antigen binding molecule containing CH1-CL crossover without charged residues (Construct 7.5).

**Table 12: Sequences of monovalent PD1(0314)-targeted human 4-1BB ligand (71-248) containing Fc (kih) fusion molecule Construct 7.5**

| SEQ ID NO: | Description | Sequence |
|---|---|---|
| 141 | nucleotide sequence of dimeric hu 4-1BBL (71-248)-CL Fc knob chain | |
| | | |
| 142 | nucleotide sequence of monomeric hu 4-1BBL (71-248)-CH1 | |
| | | |
| 133 | nucleotide sequence of anti-PD1(0314) Fc hole chain | see Table 8 |
| 134 | nucleotide sequence of anti-PD 1(0314) light chain | see Table 8 |
| 80 | Dimeric hu 4-1BBL (71-248)-CL Fc knob chain | |
| 81 | Monomeric hu 4-1BBL (71-248)-CH1 | |
| 82 | anti-PD1(0314) Fc hole chain | see Table 8 |
| 83 | anti-PD1(0314) light chain | see Table 8 |

### 2.6 Preparation of bivalent PD1(0314) targeted 4-1BB ligand (71-248) trimer-containing Fc (kih) fusion antigen binding molecule with the dimeric and monomeric 4-1BB ligands fused at the C-terminus of each heavy chain (Construct 7.6)

A polypeptide containing two ectodomains of 4-1BB ligand (71-248), separated by (G4S)2 linkers was fused to the C-terminus of human IgG1 Fc hole chain, as depicted in **Figure 8E****:** human IgG1 Fc hole, (G4S)2 connector, human 4-1BB ligand, (G4S)2 connector, human 4-1BB ligand. A polypeptide containing one ectodomain of 4-1BB ligand (71-248) and fused to the C-terminus of human IgG1 Fc knob chain as described in **Figure 8F****:** human IgG1 Fc knob, (G4S)2 connector, human 4-1BB ligand.

The variable region of heavy and light chain DNA sequences encoding a binder specific for PD1, clone 0103-0314, were subcloned in frame with either the constant heavy chain of the hole, the knob or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the anti-PD1 huIgG1 hole dimeric ligand chain containing the Y349C/T366S/L368A/Y407V mutations, the anti-PD1 huIgG1 knob monomeric ligand chain containing the S354C/T366W mutations and the anti-PD1 light chain allows generation of a heterodimer, which includes an assembled trimeric 4-1BB ligand and two PD1 binding Fabs (**Figure 7****,** Construct 7.6).

Table 13 shows the cDNA and amino acid sequences of the bivalent PD1(0314)-targeted 4-1BB ligand trimer-containing Fc (kih) fusion molecule Construct 7.6 (PD1 split trimer with 2 anti-PD1 Fabs, dimeric and monomeric 4-1BB ligand fused at the C-terminus of each heavy chain, respectively).

**Table 13: Sequences of bivalent PD1(0314)-targeted human 4-1BB ligand (71-248) containing Fc (kih) fusion molecule (Construct 7.6)**

| SEQ ID NO: | Description | Sequence |
|---|---|---|
| 143 | nucleotide sequence of anti-PD1(0314) Fc hole chain fused to dimeric hu 4-1BBL (71-248) | |
| | | |
| 144 | nucleotide sequence of anti-PD1(0314) Fc knob chain fused to monomeric hu 4-1BBL (71-248) | |
| | | |
| 134 | nucleotide sequence of anti-PD 1(0314) light chain | see Table 8 |
| 86 | anti-PD1(0314) Fc hole chain fused to dimeric hu 4-1BBL (71-248) | |
| 87 | anti-PD1(0314) Fc knob chain fused to monomeric hu 4-1BBL (71-248) | |
| | | |
| 83 | anti-PD1(0314) light chain | see Table 8 |

### 2.7 Preparation of monovalent PD1(0314) targeted 4-1BB ligand (71-248) trimer-containing Fc (kih) fusion antigen binding molecules with the dimeric and monomeric 4-1BB ligands fused at the C-terminus of each heavy chain (anti-PD1 on the knob chain, Constructs 7.11 and 7.12)

A polypeptide containing two ectodomains of 4-1BB ligand, separated by (G4S)2 linkers was subcloned in frame to the C-terminus of human IgG1 Fc hole or knob chain, as depicted in **Figure 9A****:** human IgG1 Fc, (G4S)2 connector, human 4-1BB ligand, (G4S)2 connector, human 4-1BB ligand. A polypeptide containing one ectodomain of 4-1BB ligand was subcloned in frame to the C-terminus of human IgG1 Fc knob or hole chain as described in **Figure 9B****:** human IgG1 Fc, (G4S)2 connector, human 4-1BB ligand..

The variable region of heavy and light chain DNA sequences encoding a binder specific for PD1, clone 0314, were subcloned in frame with either the constant heavy chain of the knob or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the huIgG1 hole chain containing the Y349C/T366S/L368A/Y407V mutations, the anti-PD1 huIgG1 knob chain containing the S354C/T366W mutations and the anti-PD1 light chain allows generation of a heterodimer, which includes an assembled trimeric 4-1BB ligand and one PD1 binding Fab (**Figures 9C and D**, Constructs 7.11 and 7.12).

Table 14 shows the cDNA and amino acid sequences of the monovalent PD1(0314)-targeted 4-1BB ligand trimer-containing Fc (kih) fusion molecule Construct 7.11 (PD1 split trimer with one anti-PD1 Fab, dimeric 4-1BB ligand fused at the C-terminus of the Fc hole chain and monomeric 4-1BB ligand fused at the C-terminus of the Fc knob chain).

**Table 14: Sequences of monovalent PD1(0314)-targeted human 4-1BB ligand (71-248) containing Fc (kih) fusion molecule (Construct 7.11)**

| SEQ ID NO: | Description | Sequence |
|---|---|---|
| 145 | nucleotide sequence of Fc hole dimeric ligand (71-248) chain | |
| | | |
| 144 | nucleotide sequence of anti-PD1(0314) Fc knob chain fused to monomeric hu 4-1BBL (71-248) | see Table 13 |
| 134 | nucleotide sequence of anti-PD 1(0314) light chain | see Table 8 |
| 88 | Fc hole dimeric ligand (71-248) chain | |
| 87 | anti-PD1(0314) Fc knob chain fused to monomeric hu 4-1BBL (71-248) | see Table 13 |
| 83 | anti-PD1(0314) light chain | see Table 8 |

Table 15 shows the cDNA and amino acid sequences of the monovalent PD1(0314)-targeted 4-1BB ligand trimer-containing Fc (kih) fusion molecule Construct 7.12 (PD1 split trimer with one anti-PDl Fab, dimeric 4-1BB ligand fused at the C-terminus of the Fc knob chain and monomeric 4-1BB ligand fused at the C-terminus of the Fc hole chain).

**Table 15: Sequences of monovalent PD1(0314)-targeted human 4-1BB ligand (71-248) containing Fc (kih) fusion molecule (Construct 7.12)**

| SEQ ID NO: | Description | Sequence |
|---|---|---|
| 146 | nucleotide sequence of Fc hole monomeric ligand (71-248) chain | |
| 147 | nucleotide sequence of anti-PD1(0314) Fc knob chain fused to dimeric hu 4-1BBL (71-248) | |
| | | |
| 134 | nucleotide sequence of anti-PD 1(0314) light chain | see Table 8 |
| 89 | Fc hole monomeric ligand (71-248) chain | |
| 90 | anti-PD1(0314) Fc knob chain fused to dimeric hu 4-1BBL (71-248) | |
| 83 | anti-PD1(0314) light chain | see Table 8 |

### 2.8 Preparation of monovalent PD1(0314) targeted 4-1BB ligand (71-248) trimer-containing Fc (kih) fusion antigen binding molecules with the dimeric and monomeric 4-1BB ligands fused at the C-terminus of each heavy chain (anti-PD1 on the hole chain, Constructs 7.13 and 7.14)

A polypeptide containing two ectodomains of 4-1BB ligand, separated by (G4S)2 linkers was subcloned in frame to the C-terminus of human IgG1 Fc hole or knob chain, as depicted in **Figure 9A****:** human IgG1 Fc, (G4S)2 connector, human 4-1BB ligand, (G4S)2 connector, human 4-1BB ligand. A polypeptide containing one ectodomain of 4-1BB ligand was subcloned in frame to the C-terminus of human IgG1 Fc knob or hole chain as described in **Figure 9B****:** human IgG1 Fc, (G4S)2 connector, human 4-1BB ligand..

The variable region of heavy and light chain DNA sequences encoding a binder specific for PD1, clone 0314, were subcloned in frame with either the constant heavy chain of the hole or the constant light chain of human IgG1.

The Pro329Gly, Leu234Ala and Leu235Ala mutations have been introduced in the constant region of the knob and hole heavy chains to abrogate binding to Fc gamma receptors according to the method described in WO 2012/130831.

Combination of the anti-PDl huIgG1 hole chain containing the Y349C/T366S/L368A/ Y407V mutations, the huIgG1 knob chain containing the S354C/T366W mutations and the anti-PD1 light chain allows generation of a heterodimer, which includes an assembled trimeric 4-1BB ligand and one PD1 binding Fab (**Figures 10A and B**, Constructs 7.13 and 7.14).

Table 16 shows the cDNA and amino acid sequences of the monovalent PD1(0314)-targeted 4-1BB ligand trimer-containing Fc (kih) fusion molecule Construct 7.13 (PD1 split trimer with one anti-PDl Fab, dimeric 4-1BB ligand fused at the C-terminus of the Fc hole chain and monomeric 4-1BB ligand fused at the C-terminus of the Fc knob chain).

**Table 16: Sequences of monovalent PD1(0314)-targeted human 4-1BB ligand (71-248) containing Fc (kih) fusion molecule (Construct 7.13)**

| SEQ ID NO: | Description | Sequence |
|---|---|---|
| 143 | nucleotide sequence of anti-PD1(0314) Fc hole dimeric ligand (71-248) chain | see Table 13 |
| 148 | nucleotide sequence of Fc knob chain fused to monomeric hu 4-1BBL (71-248) | |
| | | |
| 134 | nucleotide sequence of anti-PD 1(0314) light chain | see Table 8 |
| 86 | anti-PD1(0314) Fc hole dimeric ligand (71-248) chain | see Table 13 |
| 91 | Fc knob chain fused to monomeric hu 4-1BBL (71-248) | |
| 83 | anti-PD1(0314) light chain | see Table 8 |

Table 17 shows the cDNA and amino acid sequences of the monovalent PD1(0314)-targeted 4-1BB ligand trimer-containing Fc (kih) fusion molecule Construct 7.14 (PD1 split trimer with one anti-PDl Fab, dimeric 4-1BB ligand fused at the C-terminus of the Fc knob chain and monomeric 4-1BB ligand fused at the C-terminus of the Fc hole chain).

**Table 17: Sequences of monovalent PD1(0314)-targeted human 4-1BB ligand (71-248) containing Fc (kih) fusion molecule (Construct 7.14)**

| SEQ ID NO: | Description | Sequence |
|---|---|---|
| 149 | nucleotide sequence of anti-PD1(0314) Fc hole monomeric ligand (71-248) chain | |
| | | |
| 150 | nucleotide sequence of Fc knob chain fused to dimeric hu 4-1BBL (71-248) | |
| | | |
| 134 | nucleotide sequence of anti-PD 1(0314) light chain | see Table 8 |
| 92 | anti-PD1(0314) Fc hole monomeric ligand (71-248) chain | |
| 93 | Fc knob chain fused to dimeric hu 4-1BBL (71-248) | |
| 83 | anti-PD1(0314) light chain | see Table 8 |

### 2.9 Preparation of untargeted human 4-1BB ligand trimer-containing Fc fusion antigen binding molecules (Control molecules)

The control molecules were prepared as described above for the PD1-targeted antigen binding molecules, with the only difference that the anti-PDl binder (VH and VL) was replaced by a germline control, termed DP47, not binding to the antigen.

Control B is an untargeted monovalent split trimeric human 4-1BB ligand (71-254) Fc (kih) antigen binding molecule (**Figure 11A**) containing a CH-CL crossover with charged residues. It corresponds to Construct 7.1, however the variable region of heavy and light chain DNA sequences of the PD1 binder were replaced with those of the germline control (DP47) and subcloned in frame with either the constant heavy chain of the hole or the constant light chain of human IgG1.

A bivalent variant Control C (**Figure 11B**) was prepared in analogy to the bivalent Constructs 7.3 and 7.6 and a monovalent variant Control D (**Figure 11C**) was prepared in analogy to Construct 7.3 (containing a 4-1BB ligand (71-248) trimer), with the only difference that the anti-PDl binder (VH and VL) was replaced by a germline control, termed DP47, not binding to the antigen.

Table 18 shows the cDNA and amino acid sequences of the DP47-untargeted 4-1BB ligand (71-254) trimer-containing Fc (kih) fusion antigen binding molecule with CH1-CL crossover and charged residues in the 4-1BB ligand containing arms (Control B).

**Table 18: Sequences of DP47 untargeted 4-1BB ligand (71-254) trimer-containing Fc (kih) fusion antigen binding molecule (DP47 split 4-1BBL trimer) Control B**

| SEQ ID NO: | Description | Sequence |
|---|---|---|
| 131 | nucleotide sequence of dimeric hu 4-1BBL (71-254)-CL^{∗} Fc knob chain | see Table 8 |
| 132 | nucleotide sequence of monomeric hu 4-1BBL (71-254) - CH1^{∗} | see Table 8 |
| 151 | nucleotide sequence of DP47 Fc hole chain | |
| | | |
| 152 | nucleotide sequence of DP47 light chain | |
| 74 | Dimeric hu 4-1BBL (71-254)-CL^{∗} Fc knob chain | see Table 8 |
| 75 | Monomeric hu 4-1BBL (71-254)-CH1^{∗} | see Table 8 |
| 153 | DP47 Fc hole chain | |
| 154 | DP47 light chain | |

Table 19 shows the cDNA and amino acid sequences of the bivalent DP47-untargeted split trimeric 4-1BB ligand (71-254) Fc (kih) fusion molecule Control C.

**Table 19: Sequences of bivalent DP47-untargeted human 4-1BB ligand (71-254) containing Fc (kih) fusion molecule Control C**

| SEQ ID NO: | Description | Sequence |
|---|---|---|
| 155 | nucleotide sequence of DP47 Fc hole chain fused to dimeric hu 4-1BBL (71-254) | |
| | | |
| 156 | nucleotide sequence of DP47 Fc knob chain fused to monomeric hu 4-1BBL (71-254) | |
| | | |
| 152 | nucleotide sequence of DP47 light chain | see Table 18 |
| 157 | DP47 Fc hole chain fused to dimeric hu 4-1BBL (71-254) | |
| | | |
| 158 | DP47 Fc knob chain fused to monomeric hu 4-1BBL (71-254) | |
| 154 | DP47 light chain | see Table 18 |

Table 20 shows, respectively, the cDNA and amino acid sequences of the monovalent DP47-untargeted split trimeric 4-1BB ligand (71-248) Fc (kih) fusion containing CH-CL cross with charged residues, control D.

**Table 20: cDNA and amino acid sequences of monovalent DP47 untargeted split trimeric human 4-1BB ligand (71-248) Fc (kih) fusion with CH-CL cross and with charged residues (control D). ^{*} charged residues**

| SEQ ID NO: | Description | Sequence |
|---|---|---|
| 139 | nucleotide sequence of dimeric hu 4-1BBL (71-248) - CL^{∗} Fc knob chain | see Table 11 |
| 140 | nucleotide sequence of monomeric hu 4-1BBL (71-248) - CH1^{∗} | see Table 11 |
| 151 | nucleotide sequence DP47 Fc hole chain | see Table 18 |
| 152 | nucleotide sequence DP47 light chain | see Table 18 |
| 78 | Dimeric hu 4-1BBL (71-254) - CL^{∗} Fc knob chain | see Table 11 |
| 79 | Monomeric hu 4-1BBL (71-254) - CH1^{∗} | see Table 11 |
| 153 | DP47 Fc hole chain | see Table 18 |
| 154 | DP47 light chain | see Table 18 |

### 2.10 Preparation of human IgG1 antigen binding molecules as controls

An additional control molecule (Control F) used in the assays was an untargeted DP47, germline control, human IgG1, containing the Pro329Gly, Leu234Ala and Leu235Ala mutations, to abrogate binding to Fc gamma receptors according to the method described in International Patent Appl. Publ. No. WO 2012/130831).

Table 21 shows the cDNA and amino acid sequences of the cDNA and amino acid sequences of the untargeted DP47 huIgG1 PGLALA (Control F).

**Table 21: Sequences of untargeted DP47 huIgG1 (Control F)**

| SEQ ID NO: | Description | Sequence |
|---|---|---|
| 159 | nucleotide sequence of DP47 heavy chain (hu IgG1 PGLALA) | |
| | | |
| 152 | nucleotide sequence of DP47 light chain | see Table 18 |
| 160 | DP47 heavy chain (hu IgG1 PGLALA) | |
| 154 | DP47 light chain | see Table 18 |

**Table 22** shows the cDNA and amino acid sequences of the anti-PD1 huIgG1 PGLALA (clone 0314), i.e. control M.

**Table 22: cDNA and amino acid sequences of anti-PD1(0314) huIgG1 PGLALA (control M)**

| SEQ ID NO: | Description | Sequence |
|---|---|---|
| 161 | nucleotide sequence of PD 1(0314) heavy chain (huIgG1 PGLALA) | |
| | | |
| 134 | nucleotide sequence of anti-PD1(0314) light chain | see Table 8 |
| 162 | PD 1(0314) heavy chain (huIgG1 PGLALA) | |
| 83 | anti-PD1(0314) light chain | see Table 8 |

### Example 3

### Production and Purification of monovalent and bivalent anti-PDl targeted split trimeric 4-1BB ligand Fc fusion antigen binding molecules and control molecules

The targeted and untargeted split trimeric 4-1BB ligand Fc (kih) fusion molecule encoding sequences were cloned into a plasmid vector, which drives expression of the insert from an MPSV promoter and contains a synthetic polyA sequence located at the 3' end of the CDS. In addition, the vector contains an EBV OriP sequence for episomal maintenance of the plasmid.

The split trimeric 4-1BB ligand Fc (kih) fusion molecules were produced by co-transfecting HEK293-EBNA cells with the mammalian expression vectors using polyethylenimine. The cells were transfected with the corresponding expression vectors. For constructs 7.1, 7.2, 7.4, 7.6 and the corresponding control molecules control B and D, at a 1:1:1:1 ratio ("vector Fc hole chain": "vector PD1 light chain": "vector Fc knob chain": "vector 4-1BBL light chain"). For constructs 7.3, 7.6, 7.11, 7.12, 7.13, 7.14 and the control C, at a 1:1:1 ratio ("vector Fc hole chain": "vector Fc knob chain": "vector anti-PD1 light chain"). Human IgGs, used as control in the assay, were produced as for the bispecific constructs (for transfection only a vector for light and a vector for heavy chain were used at a 1:1 ratio).

For production in 500 mL shake flasks, 300 million HEK293 EBNA cells were seeded 24 hours before transfection. For transfection cells were centrifuged for 10 minutes at 210 x g, and the supernatant was replaced by 20mL pre-warmed CD CHO medium. Expression vectors (200 µg of total DNA) were mixed in 20 mL CD CHO medium. After addition of 540 µL PEI, the solution was vortexed for 15 seconds and incubated for 10 minutes at room temperature. Afterwards, cells were mixed with the DNA/PEI solution, transferred to a 500 mL shake flask and incubated for 3 hours at 37 °C in an incubator with a 5% CO₂ atmosphere. After the incubation, 160 mL of Excell medium supplemented with 6mM L-Glutamine, 5g/L PEPSOY and 1.2mM valproic acid was added and cells were cultured for 24 hours. One day after transfection 12% Feed 7 and Glucose (final conc. 3g/L) were added. After culturing for 7 days, the supernatant was collected by centrifugation for 30-40 minutes at least 400 x g. The solution was sterile filtered (0.22 µm filter), supplemented with sodium azide to a final concentration of 0.01 % (w/v), and kept at 4 °C.

Secreted proteins were purified from cell culture supernatants by affinity chromatography using Protein A, followed by size exclusion chromatography. For affinity chromatography, the supernatant was loaded on a MabSelect Sure column (CV = 5-15 mL, resin from GE Healthcare) equilibrated with Sodium Phosphate (20 mM), Sodium Citrate (20 mM) buffer (pH 7.5). Unbound protein was removed by washing with at least 6 column volumes of the same buffer. The bound protein was eluted using either a linear gradient (20 CV) or a step elution (8 CV) with 20 mM sodium citrate, 100 mM Sodium chloride, 100 mM Glycine buffer (pH 2.5). For the linear gradient an additional 4 column volumes step elution was applied.

The pH of collected fractions was adjusted by adding 1/10 (v/v) of 0.5M sodium phosphate, pH8.0. The protein was concentrated prior to loading on a HiLoad Superdex 200 column (GE Healthcare) equilibrated with 20 mM Histidine, 140 mM sodium chloride, 0.01% (v/v) Tween20 solution of pH 6.0.

The protein concentration was determined by measuring the optical density (OD) at 280 nm, using a molar extinction coefficient calculated on the basis of the amino acid sequence. Purity and molecular weight of the targeted trimeric 4-1BB ligand Fc (kih) fusion was analyzed by SDS-PAGE in the presence and absence of a reducing agent (5 mM 1,4-dithiotreitol) and staining with Coomassie SimpleBlue™ SafeStain (Invitrogen USA) or CE-SDS using Caliper LabChip GXII (Perkin Elmer). The aggregate content of samples was analyzed using a TSKgel G3000 SW XL analytical size-exclusion column (Tosoh) equilibrated in 25 mM K₂HPO₄, 125 mM NaCl, 200 mM L-Arginine Monohydrocloride, 0.02 % (w/v) NaN3, pH 6.7 running buffer at 25 °C.

Table 23 summarizes the yield and final monomer content of the FAP (4B9) targeted and untargeted 4-1BB ligand trimer-containing Fc (kih) fusion antigen binding molecules and control molecules.

**Table 23 Biochemical analysis of PD1 targeted and untargeted 4-1BB ligand trimer-containing Fc (kih) fusion antigen binding molecules and control molecules**

| Construct | Monomer [%] (SEC) | Yield [mg/l] |
|---|---|---|
| Construct 7.1 | 97.2 | 110.2 |
| Construct 7.3 | 96.6 | 8.9 |
| Construct 7.5 | 85 | 5.1 |
| Construct 7.6 | 100 | 16.5 |
| Control B | 99 | 15.4 |
| Control C | 98 | 12.6 |
| Control D | 99.5 | 25.9 |
| Control M | 99 | 34.4 |
| Control F | 100 | 50 |

### Example 4

### Simultaneous binding of PD1-targeted 4-1BB split trimeric ligand Fc fusion antigen binding molecules by surface plasmon resonance

### 4.1 Preparation, purification and characterization of antigens for surface plasmon resonance

### Preparation of 4-1BB Fc (kih) fusion molecule

A DNA sequence encoding the ectodomain of human 4-1BB (amino acids 24 to 186 of human 4-1BB according to Q07011, SEQ ID NO: 163) were subcloned in frame with the human IgG1 heavy chain CH2 and CH3 domains on the knob. An AcTEV protease cleavage site was introduced between an antigen ectodomain and the Fc of human IgG1. An Avi tag for directed biotinylation was introduced at the C-terminus of the antigen-Fc knob. Combination of the antigen-Fc knob chain containing the S354C/T366W mutations, with a Fc hole chain containing the Y349C/T366S/L368A/Y407V mutations allows generation of a heterodimer which includes a single copy of 4-1BB ectodomain containing chain, thus creating a monomeric form of Fc-linked antigen. Table 24 shows the cDNA and amino acid sequences of the antigen Fc-fusion construct.

**Table 24: cDNA and Amino acid sequences of monomeric antigen Fc(kih) fusion molecules**

| SEQ ID NO: | Antigen | Sequence |
|---|---|---|
| 164 | Nucleotide sequence of Fc hole chain | |
| | | |
| 165 | Nucleotide sequence of human 4-1BB antigen Fc knob chain | |
| 166 | Fc hole chain | |
| 167 | human 4-1BB antigen Fc knob chain | |

All 4-1BB-Fc-fusion molecule encoding sequences were cloned into a plasmid vector, which drives expression of the insert from an MPSV promoter and contains a synthetic polyA signal sequence located at the 3' end of the CDS. In addition, the vector contains an EBV OriP sequence for episomal maintenance of the plasmid.

For preparation of the biotinylated monomeric antigen/Fc fusion molecule, exponentially growing suspension HEK293 EBNA cells were co-transfected with three vectors encoding the two components of fusion protein (knob and hole chains) as well as BirA, an enzyme necessary for the biotinylation reaction. The corresponding vectors were used at a 2 : 1 : 0.05 ratio ("antigen ECD-AcTEV- Fc knob" : "Fc hole" : "BirA").

For protein production in 500 ml shake flasks, 400 million HEK293 EBNA cells were seeded 24 hours before transfection. For transfection cells were centrifuged for 5 minutes at 210 g, and the supernatant was replaced by pre-warmed CD CHO medium. Expression vectors were resuspended in 20 mL of CD CHO medium containing 200 µg of vector DNA. After addition of 540 µL of polyethylenimine (PEI), the solution was vortexed for 15 seconds and incubated for 10 minutes at room temperature. Afterwards, cells were mixed with the DNA/PEI solution, transferred to a 500 mL shake flask and incubated for 3 hours at 37°C in an incubator with a 5 % CO₂ atmosphere. After the incubation, 160 mL of F17 medium was added and cells were cultured for 24 hours. One day after transfection, 1 mM valproic acid and 7 % Feed 1 with supplements were added to the culture. After 7 days of culturing, the cell supernatant was collected by spinning down cells for 15 min at 210 g. The solution was sterile filtered (0.22 µm filter), supplemented with sodium azide to a final concentration of 0.01 % (w/v), and kept at 4°C.

Secreted proteins were purified from cell culture supernatants by affinity chromatography using Protein A, followed by size exclusion chromatography. For affinity chromatography, the supernatant was loaded on a HiTrap ProteinA HP column (CV = 5 mL, GE Healthcare) equilibrated with 40 mL 20 mM sodium phosphate, 20 mM sodium citrate pH 7.5. Unbound protein was removed by washing with at least 10 column volumes of 20 mM sodium phosphate, 20 mM sodium citrate, 0.5 M sodium chloride containing buffer (pH 7.5). The bound protein was eluted using a linear pH-gradient of sodium chloride (from 0 to 500 mM) created over 20 column volumes of 20 mM sodium citrate, 0.01 % (v/v) Tween-20, pH 3.0. The column was then washed with 10 column volumes of 20 mM sodium citrate, 500 mM sodium chloride, 0.01 % (v/v) Tween-20, pH 3.0.

The pH of collected fractions was adjusted by adding 1/40 (v/v) of 2M Tris, pH8.0. The protein was concentrated and filtered prior to loading on a HiLoad Superdex 200 column (GE Healthcare) equilibrated with 2mM MOPS, 150 mM sodium chloride, 0.02 % (w/v) sodium azide solution of pH 7.4.

For affinity determination to the human receptor, the ectodomain of human 4-1BB was also subcloned in frame with an avi (GLNDIFEAQKIEWHE, SEQ ID NO: 168) and a hexahistidine tag.

Protein production was performed as described above for the Fc-fusion protein. Secreted proteins were purified from cell culture supernatants by chelating chromatography, followed by size exclusion chromatography. The first chromatographic step was performed on a NiNTA Superflow Cartridge (5ml, Qiagen) equilibrated in 20 mM sodium phosphate, 500 nM sodium chloride, pH7.4. Elution was performed by applying a gradient over 12 column volume from 5 % to 45 % of elution buffer (20 mM sodium phosphate, 500 nM sodium chloride, 500 mM Imidazole, pH7.4). The protein was concentrated and filtered prior to loading on a HiLoad Superdex 75 column (GE Healthcare) equilibrated with 2 mM MOPS, 150 mM sodium chloride, 0.02 % (w/v) sodium azide solution of pH 7.4 (Table 25).

**Table 25: Sequences of monomeric human 4-1BB His molecule**

| SEQ ID NO: | antigen | Sequence |
|---|---|---|
| 169 | nucleotide sequence of human 4-1BB His | |
| 170 | human 4-1BB His | |

Human PD1 Fc fusion antigen was bought from R&D systems (Cat N° 1086-PD-050).

### 4.2 Determination of simultaneous binding of targeted C-terminal 4-1BB split trimeric ligand Fc fusion antigen binding molecules by surface plasmon resonance

The capacity of binding simultaneously to human 4-1BB Fc(kih) and human PD1, was assessed by surface plasmon resonance (SPR). All SPR experiments were performed on a Biacore T200 at 25 °C with HBS-EP as running buffer (0.01 M HEPES pH 7.4, 0.15 M NaCl, 3 mM EDTA, 0.005% Surfactant P20, Biacore, Freiburg/Germany).

Biotinylated human 4-1BB Fc (kih) was directly coupled to a flow cell of a streptavidin (SA) sensor chip. Immobilization levels up to 200 resonance units (RU) were used. The targeted trimeric C-terminal 4-1BB ligand Fc (kih) fusion molecules were passed at a concentration range of 200 nM with a flow of 30 µL/minute through the flow cells over 90 seconds and dissociation was set to zero sec. Human PD1-Fc (R&D systems) was injected as second analyte with a flow of 30 µL/minute through the flow cells over 90 seconds at a concentration of 500 nM (**Figure 12A**). The dissociation was monitored for 120 sec. Bulk refractive index differences were corrected for by subtracting the response obtained in a reference flow cell, where no protein was immobilized. All bispecific constructs could bind simultaneously human 4-1BB and human PD1 **(****Figures 12B, 12C****,** **12D and 12E****)**.

### Example 5

### Functional characterization of the PD1 targeted 4-1BB ligand trimer-containing Fc fusion antigen binding molecules

### 5.1. Binding on fresh and activated human PMBCs of the PD1 targeted 4-1BB ligand trimer-containing Fc (kih) fusion antigen binding molecules

Buffy coats were obtained from the Zurich blood donation center. To isolate fresh peripheral blood mononuclear cells (PBMCs) the buffy coat was diluted with the same volume of DPBS (Gibco by Life Technologies, Cat. No. 14190 326). 50 mL polypropylene centrifuge tubes (TPP, Cat.-No. 91050) were supplied with 15 mL Histopaque 1077 (SIGMA Life Science, Cat.-No. 10771, polysucrose and sodium diatrizoate, adjusted to a density of 1.077 g/mL) and the diluted buffy coat contents were layered above the Histopaque 1077. The tubes were centrifuged for 30 min at 450 x g. PBMCs were then collected from the interface, washed three times with DPBS and resuspended in T cell medium consisting of RPMI 1640 medium (Gibco by Life Technology, Cat. No. 42401-042) supplied with 10 % (v/v) Fetal Bovine Serum (FBS, US-origin, PAN biotech, P30-2009, Lot P150307GI, gamma irradiated mycoplasma free, heat inactivated 35 min 56 °C), 1 % (v/v) GlutaMAX I (GIBCO by Life Technologies, Cat. No. 35050 038), 1 mM Sodium-Pyruvat (SIGMA, Cat. No. S8636), 1 % (v/v) MEM non-essential amino acids (SIGMA, Cat.-No. M7145) and 50 µM β-Mercaptoethanol (SIGMA, M3148).

PBMCs were used directly after isolation (binding on fresh human PBMCs) or they were stimulated to induce 4-1BB expression on the cell surface of T cells (binding on activated human PBMCs). To induce 4-1BB upregulation on human T cells, PBMCs were cultured for 3 days in RPMI 1640 (GIBCO by life technologies, Cat.-No. 42401-042) supplied with 10% Fetal Bovine Serum (FBS, US-origin, PAN biotech, P30-2009, Lot P150307GI, gamma irradiated mycoplasma free, heat inactivated 35 min 56 °C), 1% GlutaMAX-I (GIBCO by life technologies, Cat.-No. 35050-038), 200 U/mL Proleukin (Novartis Pharma Schweiz AG, CHCLB-P-476-700-10340, Lot AA4493BAL) and 2 ug/mL PHA-L (SIGMA, Cat.-No. L2769) at a concentration of 1x10⁶ cells/mL. After three days pre-activated human PBMCs were harvested, resupended in RPMI 1640 supplied with 10 % FBS, 1 mM Sodium-Pyruvat (SIGMA, Cat.-No. S8636), 1% Gluta-MAX-I, 1% MEM-non essential amino acid Solution (SIGMA, Cat.-No. M7145) and 50 µM beta-Mercaptoethanol (Sigma M3148) to a final concentration of 1x10⁶ cells/mL. Afterwards cells were seeded in 6-well tissue culture plates (TTP, Cat.-No. 92006) which had been coated overnight at 4°C with 10 ug/mL anti-human CD3 (clone OKT3, Mouse IgG2a, BioLegend, Cat.-No. 317315) and 2 ug/mL anti-human CD28 (clone CD28.2, Mouse IgG1, BioLegend, Cat.-No. 302923). Cells were further incubated for 2 days at 37°C and 5% CO₂
For the binding assay 0.1 x 10⁶ fresh or activated PBMCs were added to each well of a round-bottom suspension cell 96-well plates (greiner bio-one, cellstar, Cat. No. 650185). Plates were centrifuged 5 minutes with 350 x g and at 4 °C and supernatant were discarded. Afterwards cells were stained in 100 µL/well DPBS containing 1:5000 diluted fixable viability dye eF660 (eBioscience, Cat.-No. 65-0864-18) for 30 minutes at 4°C in the dark. Cells were washed once with 200 µL ice cold cold DPBS buffer. Next, 50 µL/well of 4 °C cold FACS buffer (DPBS supplied with 2 % FBS, 5 mM EDTA pH8 (Amresco, Cat. No. E177) and 7.5 mM Sodium azide (Sigma-Aldrich, Cat.-No. S2002)) containing titrated concentrations of construct 7.1, construct 7.3, construct 7.5 and construct 7.6 and as controls control B, control C, control D, control M and control F were added and cells were incubated for 60 minutes at 4 °C, washed three times with 200 µL/well 4 °C FACS buffer. Cells were further resuspended in 50 µL/well of 4 °C cold FACS buffer containing 0.67 µg/mL anti-human CD45-AF488 (clone HI30, mouse igG1k, BioLegend, Cat.-No. 304019), 0.33 µg/mL anti-human CD8a-BV510 (clone SK1, mouse IgG1k, BioLegend, Cat.-No. 344732), 0.23 µg/mL anti-human CD4-BV421 (clone OKT4, mouse IgG2bκ, BioLegend, Cat.-No. 317434), 0.67 µg/mL anti-human CD3-PerCP-Cy5.5 (clone UCHT1, mouse IgG1k, BioLegend, Cat.-No. 300430), 0.67 µg/mL anti-human CD19-PE/Cy7 (clone HIB19, mouse IgG1k, BioLegend, Cat.-No. 302216), 5 µg/mL PE-conjugated AffiniPure anti-human IgG F(ab')2-fragment-specific goat F(ab")2 fragment (Jackson ImmunoResearch, Cat. No. 109 116 098) and incubated for 30 min at 4°C. Cells were washed twice with 200 µL/well 4 °C FACS buffer and then fixed with DPBS supplied with 1 % Formaldehyde (Sigma, HT501320-9.5L). Cells were resuspended in 100 µL/well FACS-buffer and acquired using the MACS Quant Analyzer 10 (Miltenyi Biotech). Data was analyzed using FlowJo VI0 (FlowJo, LLC) and GraphPad Prism 6.04 (GraphPad Software, Inc)
Gates were set on living CD45⁺ CD3⁺ CD19^{neg} CD8^{neg} CD4⁺ T cells, CD45⁺ CD3⁺ CD19 neg CD4^{neg} CD8⁺ T cells, CD45⁺ CD3⁺ CD4^{neg} CD8^{neg} γδ T cells and CD45⁺ CD3^{neg} CD19⁺ B cells and geo means of fluorescence intensity of PE-conjugated AffiniPure anti-human IgG IgG Fcy-fragment-specific goat F(ab')2 fragment were blotted against the titrated concentration of PD1-targeted or DP47-untargeted 4-1BB split trimeric ligand Fc fusion antigen binding molecules. As shown in **Figures 13A-H**, on resting fresh isolated human PBMCs from healthy donors only the PD1 targeted 4-1BB split trimeric ligand Fc fusion antigen binding molecules (construct 7.1., construct 7.3, construct 7.5 and construct 7.6) and the PD1 targeted human IgG control M bound to CD3⁺ CD4⁺, CD3⁺ CD8⁺ and CD3⁺ CD4^{neg} CD8^{neg} γδ T cells. Whereas the DP47-untargeted 4-1BB split trimeric ligand Fc fusion antigen binding molecules (control B, C and D) did not bind to any freshly isolated PBMCs. Therefore on freshly isolated human PBMCs the majority of cells do not express 4-1BB whereas a population of T cells expresses a detectable amount of PD1. In Table 26 the EC₅₀ values are summarized for the binding curves to freshly isolated human PBMCs.

**Table 26: EC₅₀ values of binding curves to freshly isolated resting human PBMCs**

| | **Control M** | **Construct 7.1** | **Construct 7.3** | **Construct 7.5** | **Construct 7.6** |
|---|---|---|---|---|---|
| **ECso [nM] on activated CD4 T cells** | 0.03 | 0.13 | 0.11 | 0.28 | 0.11 |
| **ECso [nM] on activated CD8 T cells** | 0.07 | 0.21 | 0.17 | 0.38 | 0.19 |
| **ECso [nM] on activated γδ T cells** | 0.03 | 0.34 | 0.13 | 0.42 | 0.27 |

As shown in **Figures 14A-H**, after activation living CD45⁺ CD3⁺ CD19^{neg} CD8^{neg} CD4⁺ T cells, CD45⁺ CD3⁺ CD19^{neg} CD4^{neg} CD8⁺ T cells, CD45⁺ CD3⁺ CD4neg CD8^{neg} γδ T cells and CD45⁺ CD3^{neg} CD19⁺ B cells express human 4-1BB and human PD1. The only PD1 binding molecules (control M) or only human 4-1BB binding molecules (controls B, control C and control D) proof thereby, that PD1 is highly upregulated on activated CD4⁺ T cells, CD8⁺ T cells, γδ T cells and B cells, whereas 4-1BB is mainly upregulated on CD8⁺ T cells, γδ T cells and B cells and to a lesser extent on CD4⁺ T cells. PD1-targeted 4-1BB split trimeric ligand Fc fusion antigen binding molecules (construct 7.1., construct 7.3, construct 7.5 and construct 7.6) bind to PD1 and human 4-1BB and this is reflected by higher geo means of fluorescence intensity compared to the fitting controls. In Table 27 the EC₅₀ values are summarized for the binding curves to activated human PBMCs.

**Table 27: EC₅₀ values of binding curves to activated human PBMCs**

| | Control B | Control C | Control D | Control M | Construct 7.1 | Construct 7.3 | Construct 7.5 | Construct 7.6 |
|---|---|---|---|---|---|---|---|---|
| EC₅₀ [nM] on v | 0.19 | 0.15 | 0.08 | 0.30 | 0.15 | 0.09 | 0.62 | 0.25 |
| EC₅₀ [nM] on activated CD4 T cells | 0.43 | 0.25 | 0.15 | 0.33 | 0.32 | 0.15 | 1.32 | 0.33 |
| EC₅₀ [nM] on activated γδ T cells | 0.20 | 0.16 | 0.09 | 0.71 | 0.12 | 0.12 | 0.52 | 0.32 |
| EC₅₀ [nM] on activated CD19 cells | 0.15 | 0.15 | 0.08 | 0.33 | 0.15 | 0.08 | 1.13 | 0.40 |

### 5.2 Binding of human PD1 and human 4-1BB transfected tumor cells

For binding assays on PD1 and 4-1BB expressing tumor cells two genetically modified tumor cells were used. The generation of human expressing HeLa-hu4-1BB-NFκB-luc clone 26 cells will be described in Example 5.3.1. For human PD1 expressing tumor cell lines ,CHO-k1 cells were transfected with human PD1 as following: parental adherent CHO-K1 cell line (ATCC CCL-61) was stably transfected with plasmid 15311_hPD1-fl_pUC_Neo coding for full-length human PD1 and selection with G418 (Neomycin resistance marker on plasmid).

0.1 x 10⁶ tumor cells resuspended in DPBS (Gibco by Life Technologies, Cat. No. 14190 326) were added to each well of a round-bottom suspension cell 96-well plates (greiner bio-one, cellstar, Cat. No. 650185). Cells were washed once with 200 µL DPBS. Cells were resuspended in 100 µL/well of 4 °C cold DPBS buffer containing 1:5000 diluted Fixable Viability Dye eFluor 660 (eBioscience, Cat. No. 65-0864-18) and plates were incubated for 30 minutes at 4°C. Cells were washed once with 200 µL/well 4 °C cold DPBS buffer and resuspended in 50 µL/well of 4 °C cold FACS buffer (DPBS supplied with 2 % FBS, 5 mM EDTA pH8 (Amresco, Cat. No. E177) and 7.5 mM Sodium azide (Sigma-Aldrich S2002)) containing PD1-targeted 4-1BB split trimeric ligand Fc fusion antigen binding molecules at a series of concentrations, followed by incubation for 1 hour at 4 °C. After extensive washing, cells were further stained with 50 µL/well of 4 °C cold FACS buffer containing 5 µg/mL PE-conjugated AffiniPure anti-human IgG F(ab')2-fragment-specific goat F(ab')2 fragment (Jackson ImmunoResearch, Cat. No. 109 116 098) for 30 minutes at 4 °C. Cells were washed twice with 200 µL/well 4 °C FACS buffer and cells were fixed in 50 µL/well DPBS containing 1 % Formaldehyde (Sigma, HT501320-9.5L). Cells were resuspended in 100 µL/well FACS-buffer and acquired using the MACS Quant Analyzer 10 (Miltenyi Biotech). Data was analyzed using FlowJo V10 (FlowJo, LLC) and GraphPad Prism 6.04 (GraphPad Software, Inc).

Gates were set on living tumor cells and geo means of fluorescence intensity of PE-conjugated AffiniPure anti-human IgG IgG Fcy-fragment-specific goat F(ab')2 fragment were blotted against the titrated concentration of PD1-targeted 4-1BB split trimeric ligand Fc fusion antigen binding molecules or their controls. As shown in **Figures 15A and 15B****,** only the PD1-targeted split trimeric 4-1BB ligand fusion molecules construct 7.1., construct 7.3, construct 7.5 and construct 7.6 and control M bound to PD1-expressing CHO-k1-huPD1 clone 5 whereas the not PD1 targeted control molecules control B, control C, control D and control F did not bind to the tumor cells. In **Figures 16A and 16B****,** only the split trimeric 4-1BB ligand containing fusion molecules construct 7.1., construct 7.3, construct 7.5 and construct 7.6 and control B, control C and control D bound to human 4-1BB-expressing HeLa-hu4-1BB-NFkB-luc clone 26 whereas the controls containing no fused split trimeric 4-1BB ligand (control F and control M) did not bind to the human 4-1BB expressing tumor cells. In Table 28 the EC₅₀ values of the curves are listed.

**Table 28: EC₅₀ values of binding curves to human PD1 or human 4-1BB expressing tumor cells**

| | Control C | Control B | Control D | Control M | Construct 7.1 | Construct 7.3 | Construct 7.5 | Construct 7.6 |
|---|---|---|---|---|---|---|---|---|
| EC₅₀ [nM] on HeLa-huCD137-NFkB-luc clone 26 cells | 0.16 | 0.19 | 0.25 | n.d. | 0.14 | 0.28 | 0.36 | 0.32 |
| EC₅₀ [nM] on CHO-K1-huPD1 clone 5 cells | n.d. | n.d. | n.d. | 1.59 | 1.73 | 1.43 | 3.33 | 1.82 |

### Example 6

### Biological activity of the PD1 targeted 4-1BB ligand trimer-containing Fc fusion antigen binding molecules

### NF-κB activation of human 4-1BB expressing HeLa reporter cell line

### 6.1 Generation of HeLa cells expressing human 4-1BB and NF-κB-luciferase

The human-papilloma-virus-18-induced cervix carcinoma cell line HeLa (ATCC CCL-2) was transduced with a plasmid based on the expression vector pETR10829, which contains the sequence of human 4-1BB (Uniprot accession Q07011) under control of a CMV-promoter and a puromycin resistance gene. Cells were cultured in DMEM-medium (Gibco by Life Technologies Cat. No. 42430-025) supplied with 10% Fetal Bovine Serum (FBS, GIBCO by Life Technologies, Cat. No. 16000-044, Lot. No. 941273, gamma-irradiated mycoplasma free, heat inactivated at 56 °C for 35 minutes), 1 % GlutaMAX-I (GIBCO by Life Technologies, Cat.-No. 35050-038) and 3 µg/mL Puromycin (InvivoGen, Cat No. ant-pr-1).

4-1BB-transduced HeLa cells were tested for 4-1BB expression by flow cytometry: 0.2 x 10⁶ living cells were resuspended in 100 µL FACS buffer (DPBS supplied with 2 % FBS, 5 mM EDTA pH 8 (Amresco, Cat. No. E177) and 7.5 mM Sodium Azide (Sigma-Aldrich, Cat. No. S2002)) containing 0.1 µg PerCP/Cy5.5 conjugated anti-human 4-1BB mouse IgG1κ clone 4B4-1 (BioLegend Cat. No. 309814) or its isotype control (PerCP/Cy5.5 conjugated mouse IgG1κ isotype control antibody clone MOPC 21, BioLegend Cat. No. 400150) and incubated for 30 minutes at 4 °C. Cells were washed twice with FACS buffer, resuspended in 300 µL FACS buffer containing 0.06 µg DAPI (Santa Cruz Biotec, Cat. No. Sc-3598) and acquired using a 5-laser LSR-Fortessa (BD Bioscience, DIVA software). Limited dilutions were performed to generate single clones as following: Human-4-1BB transduced HeLa cells were resuspended in medium to a concentration of 10, 5 and 2.5 cells/mL and 200 µL of cell suspensions were transferred to round bottom tissue-culture treated 96-well plates (6 plates/cell concentration, TPP Cat. No. 92697). Single clones were harvested, expanded and tested for 4-1BB expression as described above. The clone with the highest expression of 4-1BB (clone 5) was chosen for subsequent transfection with the NFκB-luciferase expression-vector 5495p Tranlucent HygB. The vector confers transfected cells both with resistance to hygromycin B and capacity to express luciferase under control of NFkB-response element (Panomics, Cat. No. LR0051). Human-4-1BB HeLa clone 5 cells were cultured to 70% confluence. 50 µg (40 µL) linearized (restriction enzymes AseI and SalI) 5495p Tranlucent HygB expression vector were added to a sterile 0.4 cm Gene Pulser/MicroPulser Cuvette (Biorad, Cat.-No, 165-2081). 2.5 x 10⁶ human-4-1BB HeLa clone 5 cells in 400 µl supplement-free DMEM were added and mixed carefully with the plasmid solution. Transfection of cells was performed using a Gene Pulser Xcell total system (Biorad, Cat No. 165 2660) under the following settings: exponential pulse, capacitance 500 µF, voltage 160 V, resistance ∞. Immediately after the pulse transfected cells were transferred to a tissue culture flask 75 cm² (TPP, Cat. No. 90075) with 15 mL 37 °C warm DMEM-Medium (Gibco by Life Technologies Cat. No. 42430-025) supplied with 10 % FBS and 1 % GlutaMAX I (GIBCO by Life Technologies, Cat. No. 35050 038). Next day, culture medium containing 3 µg/mL Puromycin (InvivoGen, Cat No. ant-pr-1) and 200 µg/mL Hygromycin B (Roche, Cat. No. 10843555001) was added. Surviving cells were expanded and limited dilution was performed as described above to generate single clones. Clones were tested for 4-1BB expression as described above and for NFκB-Luciferase activity as following: Clones were harvested in selection medium and counted using a Cell Counter Vi-cell xr 2.03 (Beckman Coulter, Cat. No. 731050). Cells were set to a cell density of 0.33 x 10⁶ cells/mL and 150 µL of this cell suspension were transferred to each well of a sterile white 96-well flat bottom tissue culture plate with lid (greiner bio one, Cat. No. 655083) and as a control to normal 96 well flat bottom tissue culture plate (TPP Cat. No. 92096) to test survival and cell density the next day. Cells were incubated at 37°C and 5 % CO₂ overnight. The next day 50 µL of medium containing different concentrations of recombinant human tumor necrosis factor alpha (rhTNF α, PeproTech, Cat.-No. 300 01A) were added to each well of a 96-well plate resulting in final concentration of rhTNF α of 100, 50, 25, 12.5, 6.25 and 0 ng/well. Cells were incubated for 6 hours at 37 °C and 5 % CO₂ and then washed three times with 200 µL/well DPBS. Reporter Lysis Buffer (Promega, Cat-No: E3971) was added to each well (30 µl) and the plates were stored over night at -20 °C. The next day frozen cell plates and Detection Buffer (Luciferase 1000 Assay System, Promega, Cat. No. E4550) were thawed to room temperature. 100 uL of detection buffer were added to each well and the plate was measured as fast as possible using a SpectraMax M5/M5e microplate reader and the SoftMax Pro Software (Molecular Devices). Measured URLs above control (no rhTNF α added) were taken as luciferase activity. The NFκB-luc-4-1BB-HeLa clone 26 was chosen for further use exhibiting the highest luciferase activity and a considerable level of 4-1BB-expression.

### 6.2 NF-κB activation of HeLa reporter cells expressing human 4-1BB co-cultured with PD1-expressing CHO-k1-huPD1 clone 5 cells

NFκB-luc-4-1BB-HeLa clone 26 cells were washed with DPBS (Gibco by Life Technologies, Cat. No. 14190 326) and treated with 0.05 % Trypsin EDTA (Gibco by Life Technologies Cat. No. 25300 054) for 5 min at 37 °C. Cells were harvested and resuspended in DMEM medium (Gibco by Life Technologies Cat. No. 42430 025) supplied with 10 % Fetal Calf Serum (FBS, US-origin, PAN biotech, P30-2009, Lot P150307GI, gamma irradiated mycoplasma free, heat inactivated 35 min 56 °C) and 1 % GlutaMAX-I (GIBCO by Life Technologies, Cat. No. 35050 038). Cells were counted using Cell Counter Vi-cell xr 2.03 (Beckman Coulter, Cat. No. 731050) and set to a cell density of 0.2 x 10⁶ cells/mL. 100 µL (2 x 10⁴ cells) of this cell suspension were transferred to each well of a sterile white 96-well flat bottom tissue culture plate with lid (greiner bio one, Cat. No. 655083) and the plates were incubated at 37 °C and 5 % CO₂ overnight. The next day 50 µL of medium containing different titrated split trimeric 4-1BB ligand containing fusion molecules construct 7.1., construct 7.3, construct 7.5 and construct 7.6 or the fitting controls control B, control C, control D, control F and control M.For PD1-binding-mediated crosslinking the PD1-expressing CHO-k1-huPD1 clone 5 cells were used.

Adherent CHO-k1-huPD1 clone 5 cells were washed with DPBS (Gibco by Life Technologies, Cat. No. 14190 326) and treated with enzyme-free, PBS-based Cell Dissociation Buffer (Gibco by Life Technologies, Cat.-No. 13151-014) for 10 minutes at 37 °C. Afterwards cell were harvested and counted using Cell Counter Vi-cell xr 2.03. Cells were resuspended in DEM supplied with 10% FBS and 1% L-GlutaMAX-I to 2 x 10⁶ cells/mL and 50 µL were added/well. After adding crosslinking PD1-expressing CHO-k1-huPD1 clone 5 cells, plates were incubated for 6 hours at 37 °C and 5 % CO₂. White flat bottom 96-well plates were washed three times with 200 µL/well DPBS. 40 µl fresh prepared Reporter Lysis Buffer (Promega, Cat-No: E3971) were added to each well and the plate were stored over night at -20 °C. The next day frozen plates and detection buffer (Luciferase 1000 Assay System, Promega, Cat. No. E4550) were thawed to room temperature. 100 uL of detection buffer were added to each well and plates were measured as fast as possible using the SpectraMax M5/M5e microplate reader and SoftMax Pro Software (Molecular Devices) with following settings: for luciferase (RLUs), 500ms integration time, no filter, collecting all wave length and top reading.

PD1-targeted 4-1BB ligand trimer-containing Fc fusion antigen binding molecule (PD1 split 4-1BBL trimer) triggered activation of the NFκB signaling pathway in the reporter cell line in the presence of PD1-expressing tumor cells. In contrast, the untargeted variant of the same molecule failed to trigger such an effect at any of the tested concentrations (Figures 17C and 17D). This activity of targeted 4-1BBL was strictly dependent on the expression of PD1 at the cell surface of tumor cells as no NF-kB activation could be detected upon culturing of the NF-kB reporter cell line with PD1-negative tumor cells even in the presence of PD1-targeted 4-1BB ligand trimer-containing Fc fusion antigen binding molecule (**Figures 17A and 17B**). The activities as measured for Constructs 7.1, 7.3, 7.5 and 7.6 are shown. The EC₅₀ values as measured in the presence of PD1 expressing tumor cells are given in Table 29 below.

**Table 29: EC₅₀ values of NFκB-activation-induced Luciferase activity-curves**

| | Constuct 7.1 | Construct 7.3 | Construct 7.5 | Construct 7.6 |
|---|---|---|---|---|
| EC₅₀ [nM] with CHO-k1-huPD1 clone 5 | 0.004 | 0.004 | 0.006 | 0.007 |

### Citations:

Ascierto, P. A., E. Simeone, M. Sznol, Y. X. Fu, and I. Melero (2010), Clinical experiences with anti-CD137 and anti-PDl therapeutic antibodies. Semin Oncol 37:508-516.
Aggarwal B.B. (2003), Signalling pathways of the TNF superfamily: a double-edged sword. Nat. Rev. Immunol. 3(9),745-56.
Banner D. et al (1993), Crystal structure of the soluble human 55 kd TNF receptor-human TNF beta complex: implications for TNF receptor activation. Cell 73, 431-445.
Bodmer J., Schneider P. and Tschopp, J. (2002), The molecular architecture of the TNF superfamily. Trends in Biochemical Sciences 27(1), 19-26.
Broll, K., Richter, G., Pauly, S., Hofstaedter, F., and Schwarz, H. (2001). CD137 expression in tumor vessel walls. High correlation with malignant tumors. Am J Clin Pathol 115, 543-549.
Buechele, C., Baessler, T., Schmiedel, B.J., Schumacher, C.E., Grosse-Hovest, L., Rittig, K., and Salih, H.R. (2012). 4-1BB ligand modulates direct and Rituximab-induced NK-cell reactivity in chronic lymphocytic leukemia. Eur J Immunol 42, 737-748.
Chen S., Lee L., Fisher T., Jessen B., Elliott M., Evering W., Logronio K., Tu K.H., Tsaparikos K., Li X., Wang H., Ying C., Xiong M.., Van Arsdale T., and. Lin J.C. (2015), Combination of 4-1BB Agonist and PD-1 Antagonist Promotes Antitumor Effector/Memory CD8 T Cells in a Poorly Immunogenic Tumor Model. Cancer Immunology Research 3(2), 149-160. Published online 11 November 2014.
Choi, B.K., Kim, Y.H., Kwon, P.M., Lee, S.C., Kang, S.W., Kim, M.S., Lee, M.J., and Kwon, B.S. (2009). 4-1BB functions as a survival factor in dendritic cells. J Immunol 182, 4107-4115.
Cuadros, C., Dominguez, A.L., Lollini, P.L., Croft, M., Mittler, R.S., Borgstrom, P., and Lustgarten, J. (2005). Vaccination with dendritic cells pulsed with apoptotic tumors in combination with anti-OX40 and anti-4-1BB monoclonal antibodies induces T cell-mediated protective immunity in Her-2/neu transgenic mice. Int J Cancer 116, 934-943.
Curran, M.A., Kim, M., Montalvo, W., Al-Shamkhani, A., and Allison, J.P. (2011). Combination CTLA-4 blockade and 4-1BB activation enhances tumor rejection by increasing T-cell infiltration, proliferation, and cytokine production. PLoS One 6, e19499.
Diehl, L., van Mierlo, G.J., den Boer, A.T., van der Voort, E., Fransen, M., van Bostelen, L., Krimpenfort, P., Melief, C.J., Mittler, R., Toes, R.E., and Offringa, R. (2002). In vivo triggering through 4-1BB enables Th-independent priming of CTL in the presence of an intact CD28 costimulatory pathway. J Immunol 168, 3755-3762.
Dubrot, J., Milheiro, F., Alfaro, C., Palazon, A., Martinez-Forero, I., Perez-Gracia, J.L., Morales-Kastresana, A., Romero-Trevejo, J.L., Ochoa, M.C., Hervas-Stubbs, S., et al. (2010). Treatment with anti-CD137 mAbs causes intense accumulations of liver T cells without selective antitumor immunotherapeutic effects in this organ. Cancer Immunol Immunother 59, 1223-1233.
Futagawa, T., Akiba, H., Kodama, T., Takeda, K., Hosoda, Y., Yagita, H., and Okumura, K. (2002). Expression and function of 4-1BB and 4-1BB ligand on murine dendritic cells. Int Immunol 14, 275-286.
Guo, Z., Cheng, D., Xia, Z., Luan, M., Wu, L., Wang, G., and Zhang, S. (2013). Combined TIM-3 blockade and CD137 activation affords the long-term protection in a murine model of ovarian cancer. J Transl Med 11, 215.
Heinisch, I.V., Daigle, I., Knopfli, B., and Simon, H.U. (2000). CD137 activation abrogates granulocyte-macrophage colony-stimulating factor-mediated anti-apoptosis in neutrophils. Eur J Immunol 30, 3441-3446.
Hornig, N., Kermer, V., Frey, K., Diebolder, P., Kontermann, R.E., Mueller, D. (2012), Combination of a bispecific antibody and costimulatory antibody-ligand fusion proteins for targeted cancer immunotherapy. J. Immunother. 35, 418-429.
Ju, S.A., Cheon, S.H., Park, S.M., Tam, N.Q., Kim, Y.M., An, W.G., and Kim, B.S. (2008). Eradication of established renal cell carcinoma by a combination of 5-fluorouracil and anti-4-1BB monoclonal antibody in mice. Int J Cancer 122, 2784-2790.
Kienzle, G., and von Kempis, J. (2000). CD137 (ILA/4-1BB), expressed by primary human monocytes, induces monocyte activation and apoptosis of B lymphocytes. Int Immunol 12, 73-82.
Kim, D.H., Chang, W.S., Lee, Y.S., Lee, K.A., Kim, Y.K., Kwon, B.S., and Kang, C.Y. (2008). 4-1BB engagement costimulates NKT cell activation and exacerbates NKT cell ligand-induced airway hyperresponsiveness and inflammation. J Immunol 180, 2062-2068.
Kim, Y.H., Choi, B.K., Oh, H.S., Kang, W.J., Mittler, R.S., and Kwon, B.S. (2009). Mechanisms involved in synergistic anticancer effects of anti-4-1BB and cyclophosphamide therapy. Mol Cancer Ther 8, 469-478.
Kwon, B.S., and Weissman, S.M. (1989). cDNA sequences of two inducible T-cell genes. Proc Natl Acad Sci U S A 86, 1963-1967.
Lee, H., Park, H.J., Sohn, H.J., Kim, J.M., and Kim, S.J. (2011). Combinatorial therapy for liver metastatic colon cancer: dendritic cell vaccine and low-dose agonistic anti-4-1BB antibody costimulatory signal. J Surg Res 169, e43-50.
Levitsky, V., de Campos-Lima, P.O., Frisan, T., and Masucci, M.G. (1998). The clonal composition of a peptide-specific oligoclonal CTL repertoire selected in response to persistent EBV infection is stable over time. J Immunol 161, 594-601.
Li, F., and Ravetch, J.V. (2011). Inhibitory Fcgamma receptor engagement drives adjuvant and anti-tumor activities of agonistic CD40 antibodies. Science 333, 1030-1034.
Lin, W., Voskens, C.J., Zhang, X., Schindler, D.G., Wood, A., Burch, E., Wei, Y., Chen, L., Tian, G., Tamada, K., et al. (2008). Fc-dependent expression of CD137 on human NK cells: insights into "agonistic" effects of anti-CD137 monoclonal antibodies. Blood 112, 699-707.
Melero, I., Johnston, J.V., Shufford, W.W., Mittler, R.S., and Chen, L. (1998). NK1.1 cells express 4-1BB (CDw137) costimulatory molecule and are required for tumor immunity elicited by anti-4-1BB monoclonal antibodies. Cell Immunol 190, 167-172.
Melero, I., Shuford, W.W., Newby, S.A., Aruffo, A., Ledbetter, J.A., Hellstrom, K.E., Mittler, R.S., and Chen, L. (1997). Monoclonal antibodies against the 4-1BB T-cell activation molecule eradicate established tumors. Nat Med 3, 682-685.
Merchant, A.M., Zhu, Z., Yuan, J.Q., Goddard, A., Adams, C.W., Presta, L.G., and Carter, P. (1998). An efficient route to human bispecific IgG. Nat Biotechnol 16, 677-681.
Morales-Kastresana, A., Sanmamed, M.F., Rodriguez, I., Palazon, A., Martinez-Forero, I., Labiano, S., Hervas-Stubbs, S., Sangro, B., Ochoa, C., Rouzaut, A., et al. (2013). Combined immunostimulatory monoclonal antibodies extend survival in an aggressive transgenic hepatocellular carcinoma mouse model. Clin Cancer Res 19, 6151-6162.
Mueller, D., Frey, K., Kontermann, R.E. (2008), A novel antibody-4-1BB1 fusion protein for targeted costimulation in cancer immunotherapy, J. Immunother. 31, 714-722.
Murillo, O., Dubrot, J., Palazon, A., Arina, A., Azpilikueta, A., Alfaro, C., Solano, S., Ochoa, M.C., Berasain, C., Gabari, I., et al. (2009). In vivo depletion of DC impairs the anti-tumor effect of agonistic anti-CD137 mAb. Eur J Immunol 39, 2424-2436.
Narazaki, H., Zhu, Y., Luo, L., Zhu, G., and Chen, L. (2010). CD137 agonist antibody prevents cancer recurrence: contribution of CD137 on both hematopoietic and nonhematopoietic cells. Blood 115, 1941-1948.
Nishimoto, H., Lee, S.W., Hong, H., Potter, K.G., Maeda-Yamamoto, M., Kinoshita, T., Kawakami, Y., Mittler, R.S., Kwon, B.S., Ware, C.F., et al. (2005). Costimulation of mast cells by 4-1BB, a member of the tumor necrosis factor receptor superfamily, with the high-affinity IgE receptor. Blood 106, 4241-4248.
Olofsson, P.S., Soderstrom, L.A., Wagsater, D., Sheikine, Y., Ocaya, P., Lang, F., Rabu, C., Chen, L., Rudling, M., Aukrust, P., et al. (2008). CD137 is expressed in human atherosclerosis and promotes development of plaque inflammation in hypercholesterolemic mice. Circulation 117, 1292-1301.
Palazon, A., Teijeira, A., Martinez-Forero, I., Hervas-Stubbs, S., Roncal, C., Penuelas, I., Dubrot, J., Morales-Kastresana, A., Perez-Gracia, J.L., Ochoa, M.C., et al. (2011). Agonist anti-CD137 mAb act on tumor endothelial cells to enhance recruitment of activated T lymphocytes. Cancer Res 71, 801-811.
Schwarz, H., Valbracht, J., Tuckwell, J., von Kempis, J., and Lotz, M. (1995). ILA, the human 4-1BB homologue, is inducible in lymphoid and other cell lineages. Blood 85, 1043-1052.
Shao, Z., and Schwarz, H. (2011). CD137 ligand, a member of the tumor necrosis factor family, regulates immune responses via reverse signal transduction. J Leukoc Biol 89, 21-29.
Shi, W., and Siemann, D.W. (2006). Augmented antitumor effects of radiation therapy by 4-1BB antibody (BMS-469492) treatment. Anticancer Res 26, 3445-3453.
Shindo Y., Yoshimura K., Kuramasu A., Watanabe Y., Ito H.; Kondo T., et al. (2015). Combination Immunotherapy with 4-1BB activation and PD-1 blockade enhances antitumor efficacy in a mouse model of subcutaneous tumor. Anticancer Res. 35(1), 129-136.
Simeone, E., and Ascierto, P.A. (2012). Immunomodulating antibodies in the treatment of metastatic melanoma: the experience with anti-CTLA-4, anti-CD137, and anti-PD1. J Immunotoxicol 9, 241-247.
Snell, L.M., Lin, G.H., McPherson, A.J., Moraes, T.J., and Watts, T.H. (2011). T-cell intrinsic effects of GITR and 4-1BB during viral infection and cancer immunotherapy. Immunol Rev 244, 197-217.
Stagg, J., Loi, S., Divisekera, U., Ngiow, S.F., Duret, H., Yagita, H., Teng, M.W., and Smyth, M.J. (2011). Anti-ErbB-2 mAb therapy requires type I and II interferons and synergizes with anti-PD-1 or anti-CD137 mAb therapy. Proc Natl Acad Sci U S A 108, 7142-7147.
Teng, M.W., Sharkey, J., McLaughlin, N.M., Exley, M.A., and Smyth, M.J. (2009). CD1d-based combination therapy eradicates established tumors in mice. J Immunol 183, 1911-1920.
von Kempis, J., Schwarz, H., and Lotz, M. (1997). Differentiation-dependent and stimulus-specific expression of ILA, the human 4-1BB-homologue, in cells of mesenchymal origin. Osteoarthritis Cartilage 5, 394-406.
Wei, H., Zhao, L., Li, W., Fan, K., Qian, W., Hou, S., Wang, H., Dai, M., Hellstrom, I., Hellstrom, K.E., and Guo, Y. (2013). Combinatorial PD-1 blockade and CD137 activation has therapeutic efficacy in murine cancer models and synergizes with cisplatin. PLoS One 8, e84927.
Wei H., Zhao L., Hellstrom I., Hellstrom K.E. and Guo Y. (2014). Dual targeting of CD137 costimulatory and PD-1 co-inhibitory molecules for ovarian cancer immunotherapy, OncoImmunology, 3:4, e28248, DOI: 10.4161/onci.28248.
Wilcox, R.A., Chapoval, A.I., Gorski, K.S., Otsuji, M., Shin, T., Flies, D.B., Tamada, K., Mittler, R.S., Tsuchiya, H., Pardoll, D.M., and Chen, L. (2002). Cutting edge: Expression of functional CD137 receptor by dendritic cells. J Immunol 168, 4262-4267.
Wilcox, R.A., Tamada, K., Flies, D.B., Zhu, G., Chapoval, A.I., Blazar, B.R., Kast, W.M., and Chen, L. (2004). Ligation of CD137 receptor prevents and reverses established anergy of CD8+ cytolytic T lymphocytes in vivo. Blood 103, 177-184.
Zhang, N., Sadun, R.E., Arias, R.S., Flanagan, M.L., Sachsman, S.M., Nien, Y, Khawli, L.A., Hu, P., Epstein, A.L. (2007). Targeted and untargeted CD137L fusion proteins for the immunotherapy of experimental solid tumors. Clin. Cancer Res. 13, 2758-2767.
Zhang, X., Voskens, C.J., Sallin, M., Maniar, A., Montes, C.L., Zhang, Y., Lin, W., Li, G., Burch, E., Tan, M., et al. (2010). CD137 promotes proliferation and survival of human B cells. J Immunol 184, 787-795.

## Claims

1. A 4-1BB ligand (4-1BBL) trimer-containing antigen binding molecule comprising
(a) at least one Fab molecule capable of specific binding to PD1 comprising a VH domain comprising (i) HVR-H1 comprising the amino acid sequence of SEQ ID NO:13, (ii) HVR-H2 comprising the amino acid sequence of SEQ ID NO:14, and (iii) HVR-H3 comprising the amino acid sequence of SEQ ID NO:15, and a VL domain comprising (iv) HVR-L1 comprising the amino acid sequence of SEQ ID NO:16, (v) HVR-L2 comprising the amino acid sequence of SEQ ID NO:17, and (vi) HVR-L3 comprising the amino acid sequence of SEQ ID NO:18, and
(b) a first polypeptide containing a first heavy chain constant domain (CH1) or a constant light chain domain (CL) and a second polypeptide containing a CL or CH1 domain, respectively, wherein the second polypeptide is linked to the first polypeptide by a disulfide bond between the CH1 and CL domain,
wherein the antigen binding molecule is **characterized in that** the first polypeptide comprises two ectodomains of 4-1BBL comprising the amino acid sequence selected from the group consisting of SEQ ID NO:1, SEQ ID NO:2, SEQ ID NO:3, SEQ ID NO:4, SEQ ID NO:5, SEQ ID NO:6, SEQ ID NO:7 and SEQ ID NO:8 that are connected to each other and to the CH1 or CL domain by a peptide linker and **in that** the second polypeptide comprises one ectodomain of 4-1BBL comprising the amino acid sequence selected from the group consisting of SEQ ID NO:1, SEQ ID NO:2, SEQ ID NO:3, SEQ ID NO:4, SEQ ID NO:5, SEQ ID NO:6, SEQ ID NO:7 and SEQ ID NO:8 connected via a peptide linker to the CL or CH1 domain of said polypeptide, and
(c) an Fc domain composed of a first and a second subunit capable of stable association, wherein the Fc domain is an IgG1 Fc domain comprising the amino acid substitutions L234A, L235A and P329G (numbering according to Kabat EU index).

2. The 4-1BBL trimer-containing antigen binding molecule of claim 1, wherein the ectodomain of 4-1BBL comprises the amino acid sequence of SEQ ID NO:1 or SEQ ID NO:5.

3. The 4-1BBL trimer-containing antigen binding molecule of claim 1, comprising
(a) at least one Fab molecule capable of specific binding to PD1 comprising a VH domain comprising (i) HVR-H1 comprising the amino acid sequence of SEQ ID NO:13, (ii) HVR-H2 comprising the amino acid sequence of SEQ ID NO:14, and (iii) HVR-H3 comprising the amino acid sequence of SEQ ID NO:15, and a VL domain comprising (iv) HVR-L1 comprising the amino acid sequence of SEQ ID NO:16, (v) HVR-L2 comprising the amino acid sequence of SEQ ID NO:17, and (vi) HVR-L3 comprising the amino acid sequence of SEQ ID NO:18, and
(b) a first and a second polypeptide that are linked to each other by a disulfide bond, wherein the antigen binding molecule is **characterized in that** the first polypeptide comprises the amino acid sequence selected from the group consisting of SEQ ID NO:9, SEQ ID NO:11, SEQ ID NO:12 and SEQ ID NO:10 and **in that** the second polypeptide comprises the amino acid sequence selected from the group consisting of SEQ ID NO:1, SEQ ID NO:3, SEQ ID NO:4 and SEQ ID NO:5.

4. The 4-1BBL trimer-containing antigen binding molecule of any one of claims 1 to 3, wherein the Fab molecule capable of specific binding to PD1 comprises
(a) a VH domain comprising an amino acid sequence of SEQ ID NO:19 and a VL domain comprising an amino acid sequence of SEQ ID NO:20,
(b) a VH domain comprising an amino acid sequence of SEQ ID NO:21 and a VL domain comprising an amino acid sequence of SEQ ID NO:22,
(c) a VH domain comprising an amino acid sequence of SEQ ID NO:21 and a VL domain comprising an amino acid sequence of SEQ ID NO:23,
(d) a VH domain comprising an amino acid sequence of SEQ ID NO:21 and a VL domain comprising an amino acid sequence of SEQ ID NO:24, or
(e) a VH domain comprising an amino acid sequence of SEQ ID NO:21 and a VL domain comprising an amino acid sequence of SEQ ID NO:25.

5. The 4-1BBL trimer-containing antigen binding molecule of any one of claims 1 to 4, wherein the antigen binding molecule comprises
a first heavy chain and a first light chain, both comprising the Fab molecule capable of specific binding to PD1,
a first peptide comprising two ectodomains of 4-1BBL comprising the amino acid sequence selected from the group consisting of SEQ ID NO:1, SEQ ID NO:2, SEQ ID NO:3, SEQ ID NO:4, SEQ ID NO:5, SEQ ID NO:6, SEQ ID NO:7 and SEQ ID NO:8 connected to each other by a first peptide linker fused at its C-terminus by a second peptide linker to a second heavy or light chain,
and a second peptide comprising one ectodomain of 4-1BBL comprising the amino acid sequence selected from the group consisting of SEQ ID NO:1, SEQ ID NO:2, SEQ ID NO:3, SEQ ID NO:4, SEQ ID NO:5, SEQ ID NO:6, SEQ ID NO:7 and SEQ ID NO:8 fused at its C-terminus by a third peptide linker to a second light or heavy chain, respectively.

6. The 4-1BBL trimer-containing antigen binding molecule of any one of claims 1 to 4, wherein the first peptide comprising two ectodomains of 4-1BBL comprising the amino +acid sequence selected from the group consisting of SEQ ID NO:1, SEQ ID NO:2, SEQ ID NO:3, SEQ ID NO:4, SEQ ID NO:5, SEQ ID NO:6, SEQ ID NO:7 and SEQ ID NO:8 connected to each other by a first peptide linker is fused at its C-terminus by a second peptide linker to a CL domain that is part of a heavy chain,
and the second peptide comprising one ectodomain of 4-1BBL comprising the amino acid sequence selected from the group consisting of SEQ ID NO:1, SEQ ID NO:2, SEQ ID NO:3, SEQ ID NO:4, SEQ ID NO:5, SEQ ID NO:6, SEQ ID NO:7 and SEQ ID NO:8 is fused at its C-terminus by a third peptide linker to a CH1 domain that is part of a light chain.

7. The 4-1BBL trimer-containing antigen binding molecule of any one of claims 1 to 6, wherein the Fc domain comprises knob-into-hole modifications promoting association of the first and the second subunit of the Fc domain.

8. The 4-1BBL trimer-containing antigen binding molecule of any one of claims 1 to 7, wherein the antigen binding molecule comprises
(i) a first heavy chain comprising the VH domain comprising the amino acid sequence of SEQ ID NO:19 and a first light chain comprising the VL domain comprising the amino acid sequence of SEQ ID NO:20 or
a first heavy chain comprising the VH domain comprising the amino acid sequence of SEQ ID NO:21 and a first light chain comprising the VL domain comprising the amino acid sequence selected from the group consisting of SEQ ID NO:22, SEQ ID NO:23, SEQ ID NO:24 and SEQ ID NO:25,
(ii) a second heavy chain comprising the amino acid sequence selected from the group consisting of SEQ ID NO:74, SEQ ID NO:76, SEQ ID NO:78 and SEQ ID NO:80, and
(iii) a second light chain comprising the amino acid sequence selected from the group consisting of SEQ ID NO:75, SEQ ID NO:77, SEQ ID NO:79 and SEQ ID NO:81.

9. The 4-1BBL trimer-containing antigen binding molecule of any one of claims 1 to 8, wherein the antigen binding molecule comprises
(a) a first heavy chain comprising the amino acid sequence of SEQ ID NO:82, a first light chain comprising the amino acid sequence of SEQ ID NO:83 or SEQ ID NO:171, a second heavy chain comprising the amino acid sequence of SEQ ID NO:74 and a second light chain comprising the amino acid sequence of SEQ ID NO:75,
(b) a first heavy chain comprising the amino acid sequence of SEQ ID NO:82, a first light chain comprising the amino acid sequence of SEQ ID NO:83 or SEQ ID NO:171, a second heavy chain comprising the amino acid sequence of SEQ ID NO:76 and a second light chain comprising the amino acid sequence of SEQ ID NO:77,
(c) a first heavy chain comprising the amino acid sequence of SEQ ID NO:82, a first light chain comprising the amino acid sequence of SEQ ID NO:83 or SEQ ID NO:171, a second heavy chain comprising the amino acid sequence of SEQ ID NO:78 and a second light chain comprising the amino acid sequence of SEQ ID NO:79, or
(d) a first heavy chain comprising the amino acid sequence of SEQ ID NO:82, a first light chain comprising the amino acid sequence of SEQ ID NO:83 or SEQ ID NO:171, a second heavy chain comprising the amino acid sequence of SEQ ID NO:80 and a second light chain comprising the amino acid sequence of SEQ ID NO:81.

10. An isolated polynucleotide encoding the 4-1BBL trimer-containing antigen binding molecule of any one of claims 1 to 9.

11. A vector, particularly an expression vector, comprising the isolated polynucleotide of claim 10.

12. A host cell comprising the isolated polynucleotide of claim 10 or the vector of claim 11.

13. A method of producing the 4-1BBL trimer-containing antigen binding molecule of any one of claims 1 to 9, comprising culturing the host cell of claim 12 under conditions suitable for expression of the 4-1BBL trimer-containing antigen binding molecule, and isolating the 4-1BBL trimer-containing antigen binding molecule.

14. A pharmaceutical composition comprising the 4-1BBL trimer-containing antigen binding molecule of any one of claims 1 to 9 and at least one pharmaceutically acceptable excipient.

15. The 4-1BBL trimer-containing antigen binding molecule of any one of claims 1 to 9, or the pharmaceutical composition of claim 14, for use as a medicament.

16. The 4-1BBL trimer-containing antigen binding molecule of any one of claims 1 to 9 for use in the treatment of cancer.

## Patentansprüche

1. 4-1BB-Liganden-(4-1BBL)-Trimer-enthaltendes antigenbindendes Molekül, umfassend
(a) mindestens ein Fab-Molekül, das spezifisch an PD1 binden kann, das eine VH-Domäne, umfassend (i) HVR-H1, umfassend die Aminosäuresequenz von SEQ ID NO:13, (ii) HVR-H2, umfassend die Aminosäuresequenz von SEQ ID NO:14, und (iii) HVR-H3, umfassend die Aminosäuresequenz von SEQ ID NO:15, und eine VL-Domäne, umfassend (iv) HVR-L1, umfassend die Aminosäuresequenz von SEQ ID NO:16, (v) HVR-L2, umfassend die Aminosäuresequenz von SEQ ID NO:17, und (vi) HVR-L3, umfassend die Aminosäuresequenz von SEQ ID NO:18, umfasst und
(b) ein erstes Polypeptid, enthaltend eine erste konstante Domäne der schweren Kette (CH1) oder eine konstante Domäne der leichten Kette (CL), und ein zweites Polypeptid, enthaltend eine CL- bzw. CH1-Domäne, wobei das zweite Polypeptid durch eine Disulfidbindung zwischen der CH1- und CL-Domäne mit dem ersten Polypeptid verknüpft ist,
wobei das antigenbindende Molekül **dadurch gekennzeichnet ist, dass** das erste Polypeptid zwei Ektodomänen von 4-1BBL umfasst, die die Aminosäuresequenz, ausgewählt aus der Gruppe, bestehend aus SEQ ID NO:1, SEQ ID NO:2, SEQ ID NO:3, SEQ ID NO:4, SEQ ID NO:5, SEQ ID NO:6, SEQ ID NO:7 und SEQ ID NO:8, umfassen und miteinander und mit der CH1- oder CL-Domäne durch einen Peptidlinker verbunden sind, und dadurch, dass das zweite Polypeptid eine Ektodomäne von 4-1BBL umfasst, die die Aminosäuresequenz, ausgewählt aus der Gruppe, bestehend aus SEQ ID NO:1, SEQ ID NO:2, SEQ ID NO:3, SEQ ID NO:4, SEQ ID NO:5, SEQ ID NO:6, SEQ ID NO:7 und SEQ ID NO:8, umfasst und über einen Peptidlinker mit der CL- oder CH1-Domäne des Polypeptids verbunden ist, und
(c) eine Fc-Domäne, bestehend aus einer ersten und einer zweiten Untereinheit, die zu stabiler Assoziation fähig sind, wobei die Fc-Domäne eine IgGl-Fc-Domäne ist, die die Aminosäuresubstitutionen L234A, L235A und P329G (Nummerierung gemäß Kabat-EU-Index) umfasst.

2. 4-1BBL-Trimer-enthaltendes antigenbindendes Molekül nach Anspruch 1, wobei die Ektodomäne von 4-1BBL die Aminosäuresequenz von SEQ ID NO:1 oder SEQ ID NO:5 umfasst.

3. 4-1BBL-Trimer-enthaltendes antigenbindendes Molekül nach Anspruch 1, umfassend
(a) mindestens ein Fab-Molekül, das spezifisch an PD1 binden kann, das eine VH-Domäne, umfassend (i) HVR-H1, umfassend die Aminosäuresequenz von SEQ ID NO:13, (ii) HVR-H2, umfassend die Aminosäuresequenz von SEQ ID NO:14, und (iii) HVR-H3, umfassend die Aminosäuresequenz von SEQ ID NO:15, und eine VL-Domäne, umfassend (iv) HVR-L1, umfassend die Aminosäuresequenz von SEQ ID NO:16, (v) HVR-L2, umfassend die Aminosäuresequenz von SEQ ID NO:17, und (vi) HVR-L3, umfassend die Aminosäuresequenz von SEQ ID NO:18, umfasst und
(b) ein erstes und ein zweites Polypeptid, die durch eine Disulfidbindung miteinander verknüpft sind, wobei das antigenbindende Molekül **dadurch gekennzeichnet ist, dass** das erste Polypeptid die Aminosäuresequenz, ausgewählt aus der Gruppe, bestehend aus SEQ ID NO:9, SEQ ID NO:11, SEQ ID NO:12 und SEQ ID NO:10, umfasst und dadurch, dass das zweite Polypeptid die Aminosäuresequenz, ausgewählt aus der Gruppe, bestehend aus SEQ ID NO:1, SEQ ID NO:3, SEQ ID NO:4 und SEQ ID NO:5, umfasst.

4. 4-1BBL-Trimer-enthaltendes antigenbindendes Molekül nach einem der Ansprüche 1 bis 3, wobei das Fab-Molekül, das spezifisch an PD1 binden kann, Folgendes umfasst
(a) eine VH-Domäne, umfassend eine Aminosäuresequenz von SEQ ID NO:19, und eine VL-Domäne, umfassend eine Aminosäuresequenz von SEQ ID NO:20,
(b) eine VH-Domäne, umfassend eine Aminosäuresequenz von SEQ ID NO:21, und eine VL-Domäne, umfassend eine Aminosäuresequenz von SEQ ID NO:22,
(c) eine VH-Domäne, umfassend eine Aminosäuresequenz von SEQ ID NO:21, und eine VL-Domäne, umfassend eine Aminosäuresequenz von SEQ ID NO:23,
(d) eine VH-Domäne, umfassend eine Aminosäuresequenz von SEQ ID NO:21, und eine VL-Domäne, umfassend eine Aminosäuresequenz von SEQ ID NO:24, oder
(e) eine VH-Domäne, umfassend eine Aminosäuresequenz von SEQ ID NO:21, und eine VL-Domäne, umfassend eine Aminosäuresequenz von SEQ ID NO:25.

5. 4-1BBL-Trimer-enthaltendes antigenbindendes Molekül nach einem der Ansprüche 1 bis 4, wobei das antigenbindende Molekül Folgendes umfasst
eine erste schwere Kette und eine erste leichte Kette, die beide das Fab-Molekül, das spezifisch an PD1 binden kann, umfassen,
ein erstes Peptid, umfassend zwei Ektodomänen von 4-1BBL, die die Aminosäuresequenz, ausgewählt aus der Gruppe, bestehend aus SEQ ID NO:1, SEQ ID NO:2, SEQ ID NO:3, SEQ ID NO:4, SEQ ID NO:5, SEQ ID NO:6, SEQ ID NO:7 und SEQ ID NO:8, umfassen und durch einen ersten Peptidlinker, der an seinem C-Terminus durch einen zweiten Peptidlinker an eine zweite schwere oder leichte Kette fusioniert ist, miteinander verbunden sind,
und ein zweites Peptid, umfassend eine Ektodomäne von 4-1BBL, die die Aminosäuresequenz, ausgewählt aus der Gruppe, bestehend aus SEQ ID NO:1, SEQ ID NO:2, SEQ ID NO:3, SEQ ID NO:4, SEQ ID NO:5, SEQ ID NO:6, SEQ ID NO:7 und SEQ ID NO:8, umfasst und an ihrem C-Terminus durch einen dritten Peptidlinker an eine zweite leichte bzw. schwere Kette fusioniert ist.

6. 4-1BBL-Trimer-enthaltendes antigenbindendes Molekül nach einem der Ansprüche 1 bis 4, wobei das erste Peptid, das zwei Ektodomänen von 4-1BBL umfasst, die die Aminosäuresequenz, ausgewählt aus der Gruppe, bestehend aus SEQ ID NO:1, SEQ ID NO:2, SEQ ID NO:3, SEQ ID NO:4, SEQ ID NO:5, SEQ ID NO:6, SEQ ID NO:7 und SEQ ID NO:8, umfassen und durch einen ersten Peptidlinker miteinander verbunden sind, an seinem C-Terminus durch einen zweiten Peptidlinker an eine CL-Domäne fusioniert ist, die Teil einer schweren Kette ist,
und das zweite Peptid, das eine Ektodomäne von 4-1BBL umfasst, die die Aminosäuresequenz, ausgewählt aus der Gruppe, bestehend aus SEQ ID NO:1, SEQ ID NO:2, SEQ ID NO:3, SEQ ID NO:4, SEQ ID NO:5, SEQ ID NO:6, SEQ ID NO:7 und SEQ ID NO:8, umfasst, an seinem C-Terminus durch einen dritten Peptidlinker an eine CH1-Domäne fusioniert ist, die Teil einer leichten Kette ist.

7. 4-1BBL-Trimer-enthaltendes antigenbindendes Molekül nach einem der Ansprüche 1 bis 6, wobei die Fc-Domäne Knopf-in-Loch-Modifikationen umfasst, die die Assoziation der ersten und der zweiten Untereinheit der Fc-Domäne unterstützen.

8. 4-1BBL-Trimer-enthaltendes antigenbindendes Molekül nach einem der Ansprüche 1 bis 7, wobei das antigenbindende Molekül Folgendes umfasst
(i) eine erste schwere Kette, die die VH-Domäne umfasst, die die Aminosäuresequenz von SEQ ID NO:19 umfasst, und eine erste leichte Kette, die die VL-Domäne umfasst, die die Aminosäuresequenz von SEQ ID NO:20 umfasst, oder eine erste schwere Kette, die die VH-Domäne umfasst, die die Aminosäuresequenz von SEQ ID NO:21 umfasst, und eine erste leichte Kette, die die VL-Domäne umfasst, die die Aminosäuresequenz, ausgewählt aus der Gruppe, bestehend aus SEQ ID NO:22, SEQ ID NO:23, SEQ ID NO:24 und SEQ ID NO:25, umfasst,
(ii) eine zweite schwere Kette, die die Aminosäuresequenz, ausgewählt aus der Gruppe, bestehend aus SEQ ID NO:74, SEQ ID NO:76, SEQ ID NO:78 und SEQ ID NO:80, umfasst, und
(iii) eine zweite leichte Kette, die die Aminosäuresequenz, ausgewählt aus der Gruppe, bestehend aus SEQ ID NO:75, SEQ ID NO:77, SEQ ID NO:79 und SEQ ID NO:81, umfasst.

9. 4-1BBL-Trimer-enthaltendes antigenbindendes Molekül nach einem der Ansprüche 1 bis 8, wobei das antigenbindende Molekül Folgendes umfasst
(a) eine erste schwere Kette, die die Aminosäuresequenz von SEQ ID NO:82 umfasst, eine erste leichte Kette, die die Aminosäuresequenz von SEQ ID NO:83 oder SEQ ID NO:171 umfasst, eine zweite schwere Kette, die die Aminosäuresequenz von SEQ ID NO:74 umfasst, und eine zweite leichte Kette, die die Aminosäuresequenz von SEQ ID NO:75 umfasst,
(b) eine erste schwere Kette, die die Aminosäuresequenz von SEQ ID NO:82 umfasst, eine erste leichte Kette, die die Aminosäuresequenz von SEQ ID NO:83 oder SEQ ID NO:171 umfasst, eine zweite schwere Kette, die die Aminosäuresequenz von SEQ ID NO:76 umfasst, und eine zweite leichte Kette, die die Aminosäuresequenz von SEQ ID NO:77 umfasst,
(c) eine erste schwere Kette, die die Aminosäuresequenz von SEQ ID NO:82 umfasst, eine erste leichte Kette, die die Aminosäuresequenz von SEQ ID NO:83 oder SEQ ID NO:171 umfasst, eine zweite schwere Kette, die die Aminosäuresequenz von SEQ ID NO:78 umfasst, und eine zweite leichte Kette, die die Aminosäuresequenz von SEQ ID NO:79 umfasst, oder
(d) eine erste schwere Kette, die die Aminosäuresequenz von SEQ ID NO:82 umfasst, eine erste leichte Kette, die die Aminosäuresequenz von SEQ ID NO:83 oder SEQ ID NO:171 umfasst, eine zweite schwere Kette, die die Aminosäuresequenz von SEQ ID NO:80 umfasst, und eine zweite leichte Kette, die die Aminosäuresequenz von SEQ ID NO:81 umfasst.

10. Isoliertes Polynukleotid, das für das 4-1BBL-Trimer-enthaltende antigenbindende Molekül nach einem der Ansprüche 1 bis 9 kodiert.

11. Vektor, insbesondere Expressionsvektor, umfassend das isolierte Polynukleotid nach Anspruch 10.

12. Wirtszelle, umfassend das isolierte Polynukleotid nach Anspruch 10 oder den Vektor nach Anspruch 11.

13. Verfahren zum Produzieren des 4-1BBL-Trimer-enthaltenden antigenbindenden Moleküls nach einem der Ansprüche 1 bis 9, umfassend das Kultivieren der Wirtszelle nach Anspruch 12 unter Bedingungen, die zur Expression des 4-1BBL-Trimerenthaltenden antigenbindenden Moleküls geeignet sind, und Isolieren des 4-1BBL-Trimer-enthaltenden antigenbindenden Moleküls.

14. Pharmazeutische Zusammensetzung, umfassend das 4-1BBL-Trimer-enthaltende antigenbindende Molekül nach einem der Ansprüche 1 bis 9 und mindestens einen pharmazeutisch unbedenklichen Hilfsstoff.

15. 4-1BBL-Trimer-enthaltendes antigenbindendes Molekül nach einem der Ansprüche 1 bis 9 oder pharmazeutische Zusammensetzung nach Anspruch 14 zur Verwendung als ein Medikament.

16. 4-1BBL-Trimer-enthaltendes antigenbindendes Molekül nach einem der Ansprüche 1 bis 9 zur Verwendung bei der Behandlung von Krebs.

## Revendications

1. Molécule de liaison d'antigène contenant un trimère de ligand 4-1BB (4-1BBL) comprenant
(a) au moins une molécule Fab capable de se lier spécifiquement à PD1 comprenant un domaine VH comprenant (i) une HVR-H1 comprenant la séquence d'acides aminés de SEQ ID NO:13, (ii) une HVR-H2 comprenant la séquence d'acides aminés de SEQ ID NO:14 et (iii) une HVR-H3 comprenant la séquence d'acides aminés de SEQ ID NO:15, et un domaine VL comprenant (iv) une HVR-L1 comprenant la séquence d'acides aminés de SEQ ID NO:16, (v) une HVR-L2 comprenant la séquence d'acides aminés de SEQ ID NO:17 et (vi) une HVR-L3 comprenant la séquence d'acides aminés de SEQ ID NO:18, et
(b) un premier polypeptide contenant un premier domaine constant de chaîne lourde (CH1) ou un domaine constant de chaîne légère (CL) et un second polypeptide contenant un domaine CL ou CH1, respectivement, dans laquelle le second polypeptide est lié au premier polypeptide par une liaison disulfure entre le domaine CH1 et CL,
dans laquelle la molécule de liaison d'antigène est **caractérisée en ce que** le premier polypeptide comprend deux ectodomaines de 4-1BBL comprenant la séquence d'acides aminés choisie dans le groupe constitué par la SEQ ID NO : 1, la SEQ ID NO : 2, la SEQ ID NO:3, la SEQ ID NO:4, la SEQ ID NO:5, la SEQ ID NO:6, la SEQ ID NO:7 et la SEQ ID NO : 8 qui sont reliés l'un à l'autre et au domaine CH1 ou CL par un lieur peptidique et **en ce que** le second polypeptide comprend un ectodomaine de 4-1BBL comprenant la séquence d'acides aminés choisie dans le groupe constitué par la SEQ ID NO:1, la SEQ ID NO:2, la SEQ ID NO:3, la SEQ ID NO:4, la SEQ ID NO:5, la SEQ ID NO:6, la SEQ ID NO:7 et la SEQ ID NO:8 relié au moyen d'un lieur peptidique au domaine CL ou CH1 dudit polypeptide, et
(c) un domaine Fc composé d'une première et d'une seconde sous-unité capables d'une association stable, dans laquelle le domaine Fc est un domaine Fc d'IgG1 comprenant les substitutions d'acides aminés L234A, L235A et P329G (numérotation selon l'index EU de Kabat).

2. Molécule de liaison d'antigène contenant un trimère de 4-1BBL selon la revendication 1, dans laquelle l'ectodomaine de 4-1BBL comprend la séquence d'acides aminés de SEQ ID NO:1 ou de SEQ ID NO:5.

3. Molécule de liaison d'antigène contenant un trimère de 4-1BBL selon la revendication 1, comprenant
(a) au moins une molécule Fab capable de se lier spécifiquement à PD1 comprenant un domaine VH comprenant (i) une HVR-H1 comprenant la séquence d'acides aminés de SEQ ID NO:13, (ii) une HVR-H2 comprenant la séquence d'acides aminés de SEQ ID NO:14 et (iii) une HVR-H3 comprenant la séquence d'acides aminés de SEQ ID NO:15, et un domaine VL comprenant (iv) une HVR-L1 comprenant la séquence d'acides aminés de SEQ ID NO:16, (v) une HVR-L2 comprenant la séquence d'acides aminés de SEQ ID NO:17 et (vi) une HVR-L3 comprenant la séquence d'acides aminés de SEQ ID NO:18, et
(b) un premier et un second polypeptide qui sont liés l'un à l'autre par une liaison disulfure, dans laquelle la molécule de liaison d'antigène est **caractérisée en ce que** le premier polypeptide comprend la séquence d'acides aminés choisie dans le groupe constitué par la SEQ ID NO:9, la SEQ ID NO : 11, la SEQ ID NO:12 et la SEQ ID NO : 10 et **en ce que** le second polypeptide comprend la séquence d'acides aminés choisie dans le groupe constitué par la SEQ ID NO:1, la SEQ ID NO:3, la SEQ ID NO:4 et la SEQ ID NO:5.

4. Molécule de liaison d'antigène contenant un trimère de 4-1BBL selon l'une quelconque des revendications 1 à 3, dans laquelle la molécule Fab capable de se lier spécifiquement à PD1 comprend
(a) un domaine VH comprenant une séquence d'acides aminés de SEQ ID NO:19 et un domaine VL comprenant une séquence d'acides aminés de SEQ ID NO:20,
(b) un domaine VH comprenant une séquence d'acides aminés de SEQ ID NO:21 et un domaine VL comprenant une séquence d'acides aminés de SEQ ID NO:22,
(c) un domaine VH comprenant une séquence d'acides aminés de SEQ ID NO:21 et un domaine VL comprenant une séquence d'acides aminés de SEQ ID NO:23,
(d) un domaine VH comprenant une séquence d'acides aminés de SEQ ID NO:21 et un domaine VL comprenant une séquence d'acides aminés de SEQ ID NO:24, ou
(e) un domaine VH comprenant une séquence d'acides aminés de SEQ ID NO:21 et un domaine VL comprenant une séquence d'acides aminés de SEQ ID NO:25.

5. Molécule de liaison d'antigène contenant un trimère de 4-1BBL selon l'une quelconque des revendications 1 à 4, dans laquelle la molécule de liaison d'antigène comprend
une première chaîne lourde et une première chaîne légère, toutes deux comprenant la molécule Fab capable de se lier spécifiquement à PD1,
un premier peptide comprenant deux ectodomaines de 4-1BBL comprenant la séquence d'acides aminés choisie dans le groupe constitué par la SEQ ID NO : 1, la SEQ ID NO : 2, la SEQ ID NO:3, la SEQ ID NO:4, la SEQ ID NO:5, la SEQ ID NO:6, la SEQ ID NO : 7 et la SEQ ID NO : 8 reliés l'un à l'autre par un premier lieur peptidique fusionné au niveau de son extrémité C-terminale par un deuxième lieur peptidique à une seconde chaîne lourde ou légère,
et un second peptide comprenant un ectodomaine de 4-1BBL comprenant la séquence d'acides aminés choisie dans le groupe constitué par la SEQ ID NO : 1, la SEQ ID NO : 2, la SEQ ID NO:3, la SEQ ID NO:4, la SEQ ID NO:5, la SEQ ID NO:6, la SEQ ID NO:7 et la SEQ ID NO : 8 fusionné au niveau de son extrémité C-terminale par un troisième lieur peptidique à une seconde chaîne légère ou lourde, respectivement.

6. Molécule de liaison d'antigène contenant un trimère de 4-1BBL selon l'une quelconque des revendications 1 à 4, dans laquelle le premier peptide comprenant deux ectodomaines de 4-1BBL comprenant la séquence d'acides aminés choisie dans le groupe constitué par la SEQ ID NO:1, la SEQ ID NO:2, la SEQ ID NO:3, la SEQ ID NO:4, la SEQ ID NO:5, la SEQ ID NO:6, la SEQ ID NO:7 et la SEQ ID NO : 8 reliés l'un à l'autre par un premier lieur peptidique est fusionné au niveau de son extrémité C-terminale par un deuxième lieur peptidique à un domaine CL qui fait partie d'une chaîne lourde,
et le second peptide comprenant un ectodomaine de 4-1BBL comprenant la séquence d'acides aminés choisie dans le groupe constitué par la SEQ ID NO : 1, la SEQ ID NO : 2, la SEQ ID NO:3, la SEQ ID NO:4, la SEQ ID NO:5, la SEQ ID NO:6, la SEQ ID NO:7 et la SEQ ID NO : 8 est fusionné au niveau de son extrémité C-terminale par un troisième lieur peptidique à un domaine CH1 qui fait partie d'une chaîne légère.

7. Molécule de liaison d'antigène contenant un trimère de 4-1BBL selon l'une quelconque des revendications 1 à 6, dans laquelle le domaine Fc comprend des modifications de type protubérance dans cavité favorisant l'association de la première et de la seconde sous-unité du domaine Fc.

8. Molécule de liaison d'antigène contenant un trimère de 4-1BBL selon l'une quelconque des revendications 1 à 7, dans laquelle la molécule de liaison d'antigène comprend
(i) une première chaîne lourde comprenant le domaine VH comprenant la séquence d'acides aminés de SEQ ID NO : 19 et une première chaîne légère comprenant le domaine VL comprenant la séquence d'acides aminés de SEQ ID NO:20 ou
une première chaîne lourde comprenant le domaine VH comprenant la séquence d'acides aminés de SEQ ID NO:21 et une première chaîne légère comprenant le domaine VL comprenant la séquence d'acides aminés choisie dans le groupe constitué par la SEQ ID NO : 22, la SEQ ID NO : 23, la SEQ ID NO : 24 et la SEQ ID NO : 25,
(ii) une seconde chaîne lourde comprenant la séquence d'acides aminés choisie dans le groupe constitué par la SEQ ID NO:74, la SEQ ID NO:76, la SEQ ID NO:78 et la SEQ ID NO:80, et
(iii) une seconde chaîne légère comprenant la séquence d'acides aminés choisie dans le groupe constitué par la SEQ ID NO:75, la SEQ ID NO:77, la SEQ ID NO:79 et la SEQ ID NO:81.

9. Molécule de liaison d'antigène contenant un trimère de 4-1BBL selon l'une quelconque des revendications 1 à 8, dans laquelle la molécule de liaison d'antigène comprend
(a) une première chaîne lourde comprenant la séquence d'acides aminés de SEQ ID NO : 82, une première chaîne légère comprenant la séquence d'acides aminés de SEQ ID NO:83 ou de SEQ ID NO:171, une seconde chaîne lourde comprenant la séquence d'acides aminés de SEQ ID NO:74 et une seconde chaîne légère comprenant la séquence d'acides aminés de SEQ ID NO:75,
(b) une première chaîne lourde comprenant la séquence d'acides aminés de SEQ ID NO : 82, une première chaîne légère comprenant la séquence d'acides aminés de SEQ ID NO:83 ou de SEQ ID NO:171, une seconde chaîne lourde comprenant la séquence d'acides aminés de SEQ ID NO:76 et une seconde chaîne légère comprenant la séquence d'acides aminés de SEQ ID NO:77,
(c) une première chaîne lourde comprenant la séquence d'acides aminés de SEQ ID NO : 82, une première chaîne légère comprenant la séquence d'acides aminés de SEQ ID NO:83 ou de SEQ ID NO:171, une seconde chaîne lourde comprenant la séquence d'acides aminés de SEQ ID NO:78 et une seconde chaîne légère comprenant la séquence d'acides aminés de SEQ ID NO:79, ou
(d) une première chaîne lourde comprenant la séquence d'acides aminés de SEQ ID NO : 82, une première chaîne légère comprenant la séquence d'acides aminés de SEQ ID NO:83 ou de SEQ ID NO:171, une seconde chaîne lourde comprenant la séquence d'acides aminés de SEQ ID NO:80 et une seconde chaîne légère comprenant la séquence d'acides aminés de SEQ ID NO:81.

10. Polynucléotide isolé codant pour la molécule de liaison d'antigène contenant un trimère de 4-1BBL selon l'une quelconque des revendications 1 à 9.

11. Vecteur, en particulier un vecteur d'expression, comprenant le polynucléotide isolé selon la revendication 10.

12. Cellule hôte comprenant le polynucléotide isolé selon la revendication 10 ou le vecteur selon la revendication 11.

13. Procédé de production de la molécule de liaison d'antigène contenant un trimère de 4-1BBL selon l'une quelconque des revendications 1 à 9, comprenant la culture de la cellule hôte selon la revendication 12 dans des conditions appropriées pour l'expression de la molécule de liaison d'antigène contenant un trimère de 4-1BBL et l'isolement de la molécule de liaison d'antigène contenant un trimère de 4-1BBL.

14. Composition pharmaceutique comprenant la molécule de liaison d'antigène contenant un trimère de 4-1BBL selon l'une quelconque des revendications 1 à 9 et au moins un excipient pharmaceutiquement acceptable.

15. Molécule de liaison d'antigène contenant un trimère de 4-1BBL selon l'une quelconque des revendications 1 à 9, ou composition pharmaceutique selon la revendication 14, pour une utilisation comme médicament.

16. Molécule de liaison d'antigène contenant un trimère de 4-1BBL selon l'une quelconque des revendications 1 à 9 pour une utilisation dans le traitement d'un cancer.


### SEQUENCE LISTING

<110> F. Hoffmann-La Roche AG
<120> Antigen Binding Molecules comprising a TNF family ligand trimer and PD1 binding moiety
<130> P33618-EP
<160> 170
<170> PatentIn version 3.5
<210> 1
   <211> 184
   <212> PRT
   <213> Homo sapiens
<400> 1 Gly Leu Pro Ser Pro Arg Ser Glu 180
<210> 2
   <211> 170
   <212> PRT
   <213> Homo sapiens
<400> 2
   Leu Asp Leu Arg Gln Gly Met Phe Ala Gln Leu Val Ala Gln Asn Val
   1               5                   10                  15
   Leu Leu Ile Asp Gly Pro Leu Ser Trp Tyr Ser Asp Pro Gly Leu Ala
               20                  25                  30
   Gly Val Ser Leu Thr Gly Gly Leu Ser Tyr Lys Glu Asp Thr Lys Glu
           35                  40                  45
   Leu Val Val Ala Lys Ala Gly Val Tyr Tyr Val Phe Phe Gln Leu Glu
       50                  55                  60
   Leu Arg Arg Val Val Ala Gly Glu Gly Ser Gly Ser Val Ser Leu Ala
   65                  70                  75                  80
   Leu His Leu Gln Pro Leu Arg Ser Ala Ala Gly Ala Ala Ala Leu Ala
                   85                  90                  95
   Leu Thr Val Asp Leu Pro Pro Ala Ser Ser Glu Ala Arg Asn Ser Ala
               100                 105                 110
   Phe Gly Phe Gln Gly Arg Leu Leu His Leu Ser Ala Gly Gln Arg Leu
           115                 120                 125
   Gly Val His Leu His Thr Glu Ala Arg Ala Arg His Ala Trp Gln Leu
       130                 135                 140
   Thr Gln Gly Ala Thr Val Leu Gly Leu Phe Arg Val Thr Pro Glu Ile
   145                 150                 155                 160
   Pro Ala Gly Leu Pro Ser Pro Arg Ser Glu
                   165                 170
<210> 3
   <211> 175
   <212> PRT
   <213> Homo sapiens
<400> 3
   Asp Pro Ala Gly Leu Leu Asp Leu Arg Gln Gly Met Phe Ala Gln Leu
   1               5                   10                  15
   Val Ala Gln Asn Val Leu Leu Ile Asp Gly Pro Leu Ser Trp Tyr Ser
               20                  25                  30
   Asp Pro Gly Leu Ala Gly Val Ser Leu Thr Gly Gly Leu Ser Tyr Lys
           35                  40                  45
   Glu Asp Thr Lys Glu Leu Val Val Ala Lys Ala Gly Val Tyr Tyr Val
       50                  55                  60
   Phe Phe Gln Leu Glu Leu Arg Arg Val Val Ala Gly Glu Gly Ser Gly
   65                  70                  75                  80
   Ser Val Ser Leu Ala Leu His Leu Gln Pro Leu Arg Ser Ala Ala Gly
                   85                  90                  95
   Ala Ala Ala Leu Ala Leu Thr Val Asp Leu Pro Pro Ala Ser Ser Glu
               100                 105                 110
   Ala Arg Asn Ser Ala Phe Gly Phe Gln Gly Arg Leu Leu His Leu Ser
           115                 120                 125
   Ala Gly Gln Arg Leu Gly Val His Leu His Thr Glu Ala Arg Ala Arg
       130                 135                 140
   His Ala Trp Gln Leu Thr Gln Gly Ala Thr Val Leu Gly Leu Phe Arg
   145                 150                 155                 160
   Val Thr Pro Glu Ile Pro Ala Gly Leu Pro Ser Pro Arg Ser Glu
                   165                 170                 175
<210> 4
   <211> 203
   <212> PRT
   <213> Homo sapiens
<400> 4
   Pro Trp Ala Val Ser Gly Ala Arg Ala Ser Pro Gly Ser Ala Ala Ser
   1               5                   10                  15
   Pro Arg Leu Arg Glu Gly Pro Glu Leu Ser Pro Asp Asp Pro Ala Gly
               20                  25                  30
   Leu Leu Asp Leu Arg Gln Gly Met Phe Ala Gln Leu Val Ala Gln Asn
           35                  40                  45
   Val Leu Leu Ile Asp Gly Pro Leu Ser Trp Tyr Ser Asp Pro Gly Leu
   50 55 60
   Ala Gly Val Ser Leu Thr Gly Gly Leu Ser Tyr Lys Glu Asp Thr Lys
   65 70 75 80
   Glu Leu Val Val Ala Lys Ala Gly Val Tyr Tyr Val Phe Phe Gln Leu
   85 90 95
   Glu Leu Arg Arg Val Val Ala Gly Glu Gly Ser Gly Ser Val Ser Leu
   100 105 110
   Ala Leu His Leu Gln Pro Leu Arg Ser Ala Ala Gly Ala Ala Ala Leu
   115 120 125
   Ala Leu Thr Val Asp Leu Pro Pro Ala Ser Ser Glu Ala Arg Asn Ser
   130 135 140
   Ala Phe Gly Phe Gln Gly Arg Leu Leu His Leu Ser Ala Gly Gln Arg
   145 150 155 160
   Leu Gly Val His Leu His Thr Glu Ala Arg Ala Arg His Ala Trp Gln
   165 170 175
   Leu Thr Gln Gly Ala Thr Val Leu Gly Leu Phe Arg Val Thr Pro Glu
   180 185 190
   Ile Pro Ala Gly Leu Pro Ser Pro Arg Ser Glu
   195 200
<210> 5
   <211> 178
   <212> PRT
   <213> Homo sapiens
<400> 5
   Arg Glu Gly Pro Glu Leu Ser Pro Asp Asp Pro Ala Gly Leu Leu Asp
   1 5 10 15
   Leu Arg Gln Gly Met Phe Ala Gln Leu Val Ala Gln Asn Val Leu Leu
   20 25 30
   Ile Asp Gly Pro Leu Ser Trp Tyr Ser Asp Pro Gly Leu Ala Gly Val
   35 40 45
   Ser Leu Thr Gly Gly Leu Ser Tyr Lys Glu Asp Thr Lys Glu Leu Val
   50 55 60
   Val Ala Lys Ala Gly Val Tyr Tyr Val Phe Phe Gln Leu Glu Leu Arg
   65                  70                  75                  80
   Arg Val Val Ala Gly Glu Gly Ser Gly Ser Val Ser Leu Ala Leu His
                   85                  90                  95
   Leu Gln Pro Leu Arg Ser Ala Ala Gly Ala Ala Ala Leu Ala Leu Thr
               100                 105                 110
   Val Asp Leu Pro Pro Ala Ser Ser Glu Ala Arg Asn Ser Ala Phe Gly
           115                 120                 125
   Phe Gln Gly Arg Leu Leu His Leu Ser Ala Gly Gln Arg Leu Gly Val
       130                 135                 140
   His Leu His Thr Glu Ala Arg Ala Arg His Ala Trp Gln Leu Thr Gln
   145                 150                 155                 160
   Gly Ala Thr Val Leu Gly Leu Phe Arg Val Thr Pro Glu Ile Pro Ala
                   165                 170                 175
   Gly Leu
<210> 6
   <211> 164
   <212> PRT
   <213> Homo sapiens
<400> 6
   Leu Asp Leu Arg Gln Gly Met Phe Ala Gln Leu Val Ala Gln Asn Val
   1               5                   10                  15
   Leu Leu Ile Asp Gly Pro Leu Ser Trp Tyr Ser Asp Pro Gly Leu Ala
               20                  25                  30
   Gly Val Ser Leu Thr Gly Gly Leu Ser Tyr Lys Glu Asp Thr Lys Glu
           35                  40                  45
   Leu Val Val Ala Lys Ala Gly Val Tyr Tyr Val Phe Phe Gln Leu Glu
       50                  55                  60
   Leu Arg Arg Val Val Ala Gly Glu Gly Ser Gly Ser Val Ser Leu Ala
   65                  70                  75                  80
   Leu His Leu Gln Pro Leu Arg Ser Ala Ala Gly Ala Ala Ala Leu Ala
                   85                  90                  95
   Leu Thr Val Asp Leu Pro Pro Ala Ser Ser Glu Ala Arg Asn Ser Ala
   100 105 110
   Phe Gly Phe Gln Gly Arg Leu Leu His Leu Ser Ala Gly Gln Arg Leu
   115 120 125
   Gly Val His Leu His Thr Glu Ala Arg Ala Arg His Ala Trp Gln Leu
   130 135 140
   Thr Gln Gly Ala Thr Val Leu Gly Leu Phe Arg Val Thr Pro Glu Ile
   145 150 155 160
   Pro Ala Gly Leu
<210> 7
   <211> 169
   <212> PRT
   <213> Homo sapiens
<400> 7
   Asp Pro Ala Gly Leu Leu Asp Leu Arg Gln Gly Met Phe Ala Gln Leu
   1 5 10 15
   Val Ala Gln Asn Val Leu Leu Ile Asp Gly Pro Leu Ser Trp Tyr Ser
   20 25 30
   Asp Pro Gly Leu Ala Gly Val Ser Leu Thr Gly Gly Leu Ser Tyr Lys
   35 40 45
   Glu Asp Thr Lys Glu Leu Val Val Ala Lys Ala Gly Val Tyr Tyr Val
   50 55 60
   Phe Phe Gln Leu Glu Leu Arg Arg Val Val Ala Gly Glu Gly Ser Gly
   65 70 75 80
   Ser Val Ser Leu Ala Leu His Leu Gln Pro Leu Arg Ser Ala Ala Gly
   85 90 95
   Ala Ala Ala Leu Ala Leu Thr Val Asp Leu Pro Pro Ala Ser Ser Glu
   100 105 110
   Ala Arg Asn Ser Ala Phe Gly Phe Gln Gly Arg Leu Leu His Leu Ser
   115 120 125
   Ala Gly Gln Arg Leu Gly Val His Leu His Thr Glu Ala Arg Ala Arg
   130 135 140
   His Ala Trp Gln Leu Thr Gln Gly Ala Thr Val Leu Gly Leu Phe Arg
   145                 150                 155                 160
   Val Thr Pro Glu Ile Pro Ala Gly Leu
                   165
<210> 8
   <211> 197
   <212> PRT
   <213> Homo sapiens
<400> 8
   Pro Trp Ala Val Ser Gly Ala Arg Ala Ser Pro Gly Ser Ala Ala Ser
   1               5                   10                  15
   Pro Arg Leu Arg Glu Gly Pro Glu Leu Ser Pro Asp Asp Pro Ala Gly
               20                  25                  30
   Leu Leu Asp Leu Arg Gln Gly Met Phe Ala Gln Leu Val Ala Gln Asn
           35                  40                  45
   Val Leu Leu Ile Asp Gly Pro Leu Ser Trp Tyr Ser Asp Pro Gly Leu
       50                  55                  60
   Ala Gly Val Ser Leu Thr Gly Gly Leu Ser Tyr Lys Glu Asp Thr Lys
   65                  70                  75                  80
   Glu Leu Val Val Ala Lys Ala Gly Val Tyr Tyr Val Phe Phe Gln Leu
                   85                  90                  95
   Glu Leu Arg Arg Val Val Ala Gly Glu Gly Ser Gly Ser Val Ser Leu
               100                 105                 110
   Ala Leu His Leu Gln Pro Leu Arg Ser Ala Ala Gly Ala Ala Ala Leu
           115                 120                 125
   Ala Leu Thr Val Asp Leu Pro Pro Ala Ser Ser Glu Ala Arg Asn Ser
       130                 135                 140
   Ala Phe Gly Phe Gln Gly Arg Leu Leu His Leu Ser Ala Gly Gln Arg
   145                 150                 155                 160
   Leu Gly Val His Leu His Thr Glu Ala Arg Ala Arg His Ala Trp Gln
                   165                 170                 175
   Leu Thr Gln Gly Ala Thr Val Leu Gly Leu Phe Arg Val Thr Pro Glu
               180                 185                 190
   Ile Pro Ala Gly Leu
   195
<210> 9
   <211> 378
   <212> PRT
   <213> Artificial Sequence
<220>
   <223> dimeric hu 4-1BBL (71-254) connected by (G4S)2 linker
<400> 9
   Arg Glu Gly Pro Glu Leu Ser Pro Asp Asp Pro Ala Gly Leu Leu Asp
   1               5                   10                  15
   Leu Arg Gln Gly Met Phe Ala Gln Leu Val Ala Gln Asn Val Leu Leu
               20                  25                  30
   Ile Asp Gly Pro Leu Ser Trp Tyr Ser Asp Pro Gly Leu Ala Gly Val
           35                  40                  45
   Ser Leu Thr Gly Gly Leu Ser Tyr Lys Glu Asp Thr Lys Glu Leu Val
       50                  55                  60
   Val Ala Lys Ala Gly Val Tyr Tyr Val Phe Phe Gln Leu Glu Leu Arg
   65                  70                  75                  80
   Arg Val Val Ala Gly Glu Gly Ser Gly Ser Val Ser Leu Ala Leu His
                   85                  90                  95
   Leu Gln Pro Leu Arg Ser Ala Ala Gly Ala Ala Ala Leu Ala Leu Thr
               100                 105                 110
   Val Asp Leu Pro Pro Ala Ser Ser Glu Ala Arg Asn Ser Ala Phe Gly
           115                 120                 125
   Phe Gln Gly Arg Leu Leu His Leu Ser Ala Gly Gln Arg Leu Gly Val
       130                 135                 140
   His Leu His Thr Glu Ala Arg Ala Arg His Ala Trp Gln Leu Thr Gln
   145                 150                 155                 160
   Gly Ala Thr Val Leu Gly Leu Phe Arg Val Thr Pro Glu Ile Pro Ala
                   165                 170                 175
   Gly Leu Pro Ser Pro Arg Ser Glu Gly Gly Gly Gly Ser Gly Gly Gly
               180                 185                 190
   Gly Ser Arg Glu Gly Pro Glu Leu Ser Pro Asp Asp Pro Ala Gly Leu
   195 200 205
   Leu Asp Leu Arg Gln Gly Met Phe Ala Gln Leu Val Ala Gln Asn Val
   210 215 220
   Leu Leu Ile Asp Gly Pro Leu Ser Trp Tyr Ser Asp Pro Gly Leu Ala
   225 230 235 240
   Gly Val Ser Leu Thr Gly Gly Leu Ser Tyr Lys Glu Asp Thr Lys Glu
   245 250 255
   Leu Val Val Ala Lys Ala Gly Val Tyr Tyr Val Phe Phe Gln Leu Glu
   260 265 270
   Leu Arg Arg Val Val Ala Gly Glu Gly Ser Gly Ser Val Ser Leu Ala
   275 280 285
   Leu His Leu Gln Pro Leu Arg Ser Ala Ala Gly Ala Ala Ala Leu Ala
   290 295 300
   Leu Thr Val Asp Leu Pro Pro Ala Ser Ser Glu Ala Arg Asn Ser Ala
   305 310 315 320
   Phe Gly Phe Gln Gly Arg Leu Leu His Leu Ser Ala Gly Gln Arg Leu
   325 330 335
   Gly Val His Leu His Thr Glu Ala Arg Ala Arg His Ala Trp Gln Leu
   340 345 350
   Thr Gln Gly Ala Thr Val Leu Gly Leu Phe Arg Val Thr Pro Glu Ile
   355 360 365
   Pro Ala Gly Leu Pro Ser Pro Arg Ser Glu
   370 375
<210> 10
   <211> 366
   <212> PRT
   <213> Artificial Sequence
<220>
   <223> dimeric hu 4-1BBL (71-248) connected by (G4S)2 linker
<400> 10
   Arg Glu Gly Pro Glu Leu Ser Pro Asp Asp Pro Ala Gly Leu Leu Asp
   1               5                   10                  15
   Leu Arg Gln Gly Met Phe Ala Gln Leu Val Ala Gln Asn Val Leu Leu
               20                  25                  30
   Ile Asp Gly Pro Leu Ser Trp Tyr Ser Asp Pro Gly Leu Ala Gly Val
           35                  40                  45
   Ser Leu Thr Gly Gly Leu Ser Tyr Lys Glu Asp Thr Lys Glu Leu Val
       50                  55                  60
   Val Ala Lys Ala Gly Val Tyr Tyr Val Phe Phe Gln Leu Glu Leu Arg
   65                  70                  75                  80
   Arg Val Val Ala Gly Glu Gly Ser Gly Ser Val Ser Leu Ala Leu His
                   85                  90                  95
   Leu Gln Pro Leu Arg Ser Ala Ala Gly Ala Ala Ala Leu Ala Leu Thr
               100                 105                 110
   Val Asp Leu Pro Pro Ala Ser Ser Glu Ala Arg Asn Ser Ala Phe Gly
           115                 120                 125
   Phe Gln Gly Arg Leu Leu His Leu Ser Ala Gly Gln Arg Leu Gly Val
       130                 135                 140
   His Leu His Thr Glu Ala Arg Ala Arg His Ala Trp Gln Leu Thr Gln
   145                 150                 155                 160
   Gly Ala Thr Val Leu Gly Leu Phe Arg Val Thr Pro Glu Ile Pro Ala
                   165                 170                 175
   Gly Leu Gly Gly Gly Gly Ser Gly Gly Gly Gly Ser Arg Glu Gly Pro
               180                 185                 190
   Glu Leu Ser Pro Asp Asp Pro Ala Gly Leu Leu Asp Leu Arg Gln Gly
           195                 200                 205
   Met Phe Ala Gln Leu Val Ala Gln Asn Val Leu Leu Ile Asp Gly Pro
       210                 215                 220
   Leu Ser Trp Tyr Ser Asp Pro Gly Leu Ala Gly Val Ser Leu Thr Gly
   225                 230                 235                 240
   Gly Leu Ser Tyr Lys Glu Asp Thr Lys Glu Leu Val Val Ala Lys Ala
                   245                 250                 255
   Gly Val Tyr Tyr Val Phe Phe Gln Leu Glu Leu Arg Arg Val Val Ala
               260                 265                 270
   Gly Glu Gly Ser Gly Ser Val Ser Leu Ala Leu His Leu Gln Pro Leu
   275 280 285
   Arg Ser Ala Ala Gly Ala Ala Ala Leu Ala Leu Thr Val Asp Leu Pro
   290 295 300
   Pro Ala Ser Ser Glu Ala Arg Asn Ser Ala Phe Gly Phe Gln Gly Arg
   305 310 315 320
   Leu Leu His Leu Ser Ala Gly Gln Arg Leu Gly Val His Leu His Thr
   325 330 335
   Glu Ala Arg Ala Arg His Ala Trp Gln Leu Thr Gln Gly Ala Thr Val
   340 345 350
   Leu Gly Leu Phe Arg Val Thr Pro Glu Ile Pro Ala Gly Leu
   355 360 365
<210> 11
   <211> 360
   <212> PRT
   <213> Artificial Sequence
<220>
   <223> dimeric hu 4-1BBL (80-254) connected by (G4S)2 linker
<400> 11
   Asp Pro Ala Gly Leu Leu Asp Leu Arg Gln Gly Met Phe Ala Gln Leu
   1               5                   10                  15
   Val Ala Gln Asn Val Leu Leu Ile Asp Gly Pro Leu Ser Trp Tyr Ser
               20                  25                  30
   Asp Pro Gly Leu Ala Gly Val Ser Leu Thr Gly Gly Leu Ser Tyr Lys
           35                  40                  45
   Glu Asp Thr Lys Glu Leu Val Val Ala Lys Ala Gly Val Tyr Tyr Val
       50                  55                  60
   Phe Phe Gln Leu Glu Leu Arg Arg Val Val Ala Gly Glu Gly Ser Gly
   65                  70                  75                  80
   Ser Val Ser Leu Ala Leu His Leu Gln Pro Leu Arg Ser Ala Ala Gly
                   85                  90                  95
   Ala Ala Ala Leu Ala Leu Thr Val Asp Leu Pro Pro Ala Ser Ser Glu
               100                 105                 110
   Ala Arg Asn Ser Ala Phe Gly Phe Gln Gly Arg Leu Leu His Leu Ser
           115                 120                 125
   Ala Gly Gln Arg Leu Gly Val His Leu His Thr Glu Ala Arg Ala Arg
   130 135 140
   His Ala Trp Gln Leu Thr Gln Gly Ala Thr Val Leu Gly Leu Phe Arg
   145 150 155 160
   Val Thr Pro Glu Ile Pro Ala Gly Leu Pro Ser Pro Arg Ser Glu Gly
   165 170 175
   Gly Gly Gly Ser Gly Gly Gly Gly Ser Asp Pro Ala Gly Leu Leu Asp
   180 185 190
   Leu Arg Gln Gly Met Phe Ala Gln Leu Val Ala Gln Asn Val Leu Leu
   195 200 205
   Ile Asp Gly Pro Leu Ser Trp Tyr Ser Asp Pro Gly Leu Ala Gly Val
   210 215 220
   Ser Leu Thr Gly Gly Leu Ser Tyr Lys Glu Asp Thr Lys Glu Leu Val
   225 230 235 240
   Val Ala Lys Ala Gly Val Tyr Tyr Val Phe Phe Gln Leu Glu Leu Arg
   245 250 255
   Arg Val Val Ala Gly Glu Gly Ser Gly Ser Val Ser Leu Ala Leu His
   260 265 270
   Leu Gln Pro Leu Arg Ser Ala Ala Gly Ala Ala Ala Leu Ala Leu Thr
   275 280 285
   Val Asp Leu Pro Pro Ala Ser Ser Glu Ala Arg Asn Ser Ala Phe Gly
   290 295 300
   Phe Gln Gly Arg Leu Leu His Leu Ser Ala Gly Gln Arg Leu Gly Val
   305 310 315 320
   His Leu His Thr Glu Ala Arg Ala Arg His Ala Trp Gln Leu Thr Gln
   325 330 335
   Gly Ala Thr Val Leu Gly Leu Phe Arg Val Thr Pro Glu Ile Pro Ala
   340 345 350
   Gly Leu Pro Ser Pro Arg Ser Glu
   355 360
<210> 12
   <211> 416
   <212> PRT
   <213> Artificial Sequence
<220>
   <223> dimeric hu 4-1BBL (52-254) connected by (G4S)2 linker
<400> 12
   Pro Trp Ala Val Ser Gly Ala Arg Ala Ser Pro Gly Ser Ala Ala Ser
   1               5                   10                  15
   Pro Arg Leu Arg Glu Gly Pro Glu Leu Ser Pro Asp Asp Pro Ala Gly
               20                  25                  30
   Leu Leu Asp Leu Arg Gln Gly Met Phe Ala Gln Leu Val Ala Gln Asn
           35                  40                  45
   Val Leu Leu Ile Asp Gly Pro Leu Ser Trp Tyr Ser Asp Pro Gly Leu
       50                  55                  60
   Ala Gly Val Ser Leu Thr Gly Gly Leu Ser Tyr Lys Glu Asp Thr Lys
   65                  70                  75                  80
   Glu Leu Val Val Ala Lys Ala Gly Val Tyr Tyr Val Phe Phe Gln Leu
                   85                  90                  95
   Glu Leu Arg Arg Val Val Ala Gly Glu Gly Ser Gly Ser Val Ser Leu
               100                 105                 110
   Ala Leu His Leu Gln Pro Leu Arg Ser Ala Ala Gly Ala Ala Ala Leu
           115                 120                 125
   Ala Leu Thr Val Asp Leu Pro Pro Ala Ser Ser Glu Ala Arg Asn Ser
       130                 135                 140
   Ala Phe Gly Phe Gln Gly Arg Leu Leu His Leu Ser Ala Gly Gln Arg
   145                 150                 155                 160
   Leu Gly Val His Leu His Thr Glu Ala Arg Ala Arg His Ala Trp Gln
                   165                 170                 175
   Leu Thr Gln Gly Ala Thr Val Leu Gly Leu Phe Arg Val Thr Pro Glu
               180                 185                 190
   Ile Pro Ala Gly Leu Pro Ser Pro Arg Ser Glu Gly Gly Gly Gly Ser
           195                 200                 205
   Gly Gly Gly Gly Ser Pro Trp Ala Val Ser Gly Ala Arg Ala Ser Pro
       210                 215                 220
   Gly Ser Ala Ala Ser Pro Arg Leu Arg Glu Gly Pro Glu Leu Ser Pro
   225 230 235 240
   Asp Asp Pro Ala Gly Leu Leu Asp Leu Arg Gln Gly Met Phe Ala Gln
   245 250 255
   Leu Val Ala Gln Asn Val Leu Leu Ile Asp Gly Pro Leu Ser Trp Tyr
   260 265 270
   Ser Asp Pro Gly Leu Ala Gly Val Ser Leu Thr Gly Gly Leu Ser Tyr
   275 280 285
   Lys Glu Asp Thr Lys Glu Leu Val Val Ala Lys Ala Gly Val Tyr Tyr
   290 295 300
   Val Phe Phe Gln Leu Glu Leu Arg Arg Val Val Ala Gly Glu Gly Ser
   305 310 315 320
   Gly Ser Val Ser Leu Ala Leu His Leu Gln Pro Leu Arg Ser Ala Ala
   325 330 335
   Gly Ala Ala Ala Leu Ala Leu Thr Val Asp Leu Pro Pro Ala Ser Ser
   340 345 350
   Glu Ala Arg Asn Ser Ala Phe Gly Phe Gln Gly Arg Leu Leu His Leu
   355 360 365
   Ser Ala Gly Gln Arg Leu Gly Val His Leu His Thr Glu Ala Arg Ala
   370 375 380
   Arg His Ala Trp Gln Leu Thr Gln Gly Ala Thr Val Leu Gly Leu Phe
   385 390 395 400
   Arg Val Thr Pro Glu Ile Pro Ala Gly Leu Pro Ser Pro Arg Ser Glu
   405 410 415
<210> 13
   <211> 7
   <212> PRT
   <213> Mus Musculus
<400> 13
   Gly Phe Ser Phe Ser Ser Tyr
   1 5
<210> 14
   <211> 3
   <212> PRT
   <213> Mus Musculus
<400> 14
   Gly Gly Arg
   1
<210> 15
   <211> 9
   <212> PRT
   <213> Mus Musculus
<400> 15
   Thr Gly Arg Val Tyr Phe Ala Leu Asp
   1               5
<210> 16
   <211> 11
   <212> PRT
   <213> Mus Musculus
<400> 16
   Ser Glu Ser Val Asp Thr Ser Asp Asn Ser Phe
   1               5                   10
<210> 17
   <211> 3
   <212> PRT
   <213> Mus Musculus
<400> 17
   Arg Ser Ser
   1
<210> 18
   <211> 6
   <212> PRT
   <213> Mus Musculus
<400> 18
   Asn Tyr Asp Val Pro Trp
   1               5
<210> 19
   <211> 120
   <212> PRT
   <213> Mus Musculus
<400> 19
   Glu Val Ile Leu Val Glu Ser Gly Gly Gly Leu Val Lys Pro Gly Gly
   1               5                   10                  15
   Ser Leu Lys Leu Ser Cys Ala Ala Ser Gly Phe Ser Phe Ser Ser Tyr
   20 25 30
   Thr Met Ser Trp Val Arg Gln Thr Pro Glu Lys Arg Leu Asp Trp Val
   35 40 45
   Ala Thr Ile Ser Gly Gly Gly Arg Asp Ile Tyr Tyr Pro Asp Ser Val
   50 55 60
   Lys Gly Arg Phe Thr Ile Ser Arg Asp Asn Ala Lys Asn Thr Leu Tyr
   65 70 75 80
   Leu Glu Met Ser Ser Leu Met Ser Glu Asp Thr Ala Leu Tyr Tyr Cys
   85 90 95
   Val Leu Leu Thr Gly Arg Val Tyr Phe Ala Leu Asp Ser Trp Gly Gln
   100 105 110
   Gly Thr Ser Val Thr Val Ser Ser
   115 120
<210> 20
   <211> 111
   <212> PRT
   <213> Mus Musculus
<400> 20
   Lys Ile Val Leu Thr Gln Ser Pro Ala Ser Leu Pro Val Ser Leu Gly
   1 5 10 15
   Gln Arg Ala Thr Ile Ser Cys Arg Ala Ser Glu Ser Val Asp Thr Ser
   20 25 30
   Asp Asn Ser Phe Ile His Trp Tyr Gln Gln Arg Pro Gly Gln Ser Pro
   35 40 45
   Lys Leu Leu Ile Tyr Arg Ser Ser Thr Leu Glu Ser Gly Val Pro Ala
   50 55 60
   Arg Phe Ser Gly Ser Gly Ser Arg Thr Asp Phe Thr Leu Thr Ile Asp
   65 70 75 80
   Pro Val Glu Ala Asp Asp Val Ala Thr Tyr Tyr Cys Gln Gln Asn Tyr
   85 90 95
   Asp Val Pro Trp Thr Phe Gly Gly Gly Thr Lys Leu Glu Ile Lys
   100 105 110
<210> 21
   <211> 120
   <212> PRT
   <213> Artificial Sequence
<220>
   <223> humanized variant -heavy chain variable domain VH of PD1-0103-0312, -0313, -0314 and -0315
<400> 21
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Ser Phe Ser Ser Tyr
               20                  25                  30
   Thr Met Ser Trp Val Arg Gln Ala Pro Gly Lys Gly Leu Glu Trp Val
           35                  40                  45
   Ala Thr Ile Ser Gly Gly Gly Arg Asp Ile Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Leu Leu Thr Gly Arg Val Tyr Phe Ala Leu Asp Ser Trp Gly Gln
               100                 105                 110
   Gly Thr Leu Val Thr Val Ser Ser
           115                 120
<210> 22
   <211> 111
   <212> PRT
   <213> Artificial Sequence
<220>
   <223> humanized variant -light chain variable domain VL of PD1-0103-0312
<400> 22
   Asp Ile Val Met Thr Gln Ser Pro Asp Ser Leu Ala Val Ser Leu Gly
   1               5                   10                  15
   Glu Arg Ala Thr Ile Asn Cys Lys Ala Ser Glu Ser Val Asp Thr Ser
               20                  25                  30
   Asp Asn Ser Phe Ile His Trp Tyr Gln Gln Lys Pro Gly Gln Ser Pro
           35                  40                  45
   Lys Leu Leu Ile Tyr Arg Ser Ser Thr Leu Glu Ser Gly Val Pro Asp
       50                  55                  60
   Arg Phe Ser Gly Ser Gly Ser Gly Thr Asp Phe Thr Leu Thr Ile Ser
   65                  70                  75                  80
   Ser Leu Gln Ala Glu Asp Val Ala Val Tyr Tyr Cys Gln Gln Asn Tyr
                   85                  90                  95
   Asp Val Pro Trp Thr Phe Gly Gln Gly Thr Lys Val Glu Ile Lys
               100                 105                 110
<210> 23
   <211> 111
   <212> PRT
   <213> Artificial Sequence
<220>
   <223> humanized variant -light chain variable domain VL of PD1-0103-0313
<400> 23
   Asp Val Val Met Thr Gln Ser Pro Leu Ser Leu Pro Val Thr Leu Gly
   1               5                   10                  15
   Gln Pro Ala Ser Ile Ser Cys Arg Ala Ser Glu Ser Val Asp Thr Ser
               20                  25                  30
   Asp Asn Ser Phe Ile His Trp Tyr Gln Gln Arg Pro Gly Gln Ser Pro
           35                  40                  45
   Arg Leu Leu Ile Tyr Arg Ser Ser Thr Leu Glu Ser Gly Val Pro Asp
       50                  55                  60
   Arg Phe Ser Gly Ser Gly Ser Gly Thr Asp Phe Thr Leu Lys Ile Ser
   65                  70                  75                  80
   Arg Val Glu Ala Glu Asp Val Gly Val Tyr Tyr Cys Gln Gln Asn Tyr
                   85                  90                  95
   Asp Val Pro Trp Thr Phe Gly Gln Gly Thr Lys Val Glu Ile Lys
               100                 105                 110
<210> 24
   <211> 111
   <212> PRT
   <213> Artificial Sequence
<220>
   <223> humanized variant -light chain variable domain VL of PD1-0103-0314
<400> 24
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Glu Ser Val Asp Thr Ser
               20                  25                  30
   Asp Asn Ser Phe Ile His Trp Tyr Gln Gln Lys Pro Gly Gln Ser Pro
           35                  40                  45
   Arg Leu Leu Ile Tyr Arg Ser Ser Thr Leu Glu Ser Gly Ile Pro Ala
       50                  55                  60
   Arg Phe Ser Gly Ser Gly Ser Gly Thr Asp Phe Thr Leu Thr Ile Ser
   65                  70                  75                  80
   Ser Leu Glu Pro Glu Asp Phe Ala Val Tyr Tyr Cys Gln Gln Asn Tyr
                   85                  90                  95
   Asp Val Pro Trp Thr Phe Gly Gln Gly Thr Lys Val Glu Ile Lys
               100                 105                 110
<210> 25
   <211> 111
   <212> PRT
   <213> Artificial Sequence
<220>
   <223> humanized variant -light chain variable domain VL of PD1-0103-0315
<400> 25
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Glu Ser Val Asp Thr Ser
               20                  25                  30
   Asp Asn Ser Phe Ile His Trp Tyr Gln Gln Lys Pro Gly Gln Ser Pro
           35                  40                  45
   Arg Leu Leu Ile Tyr Arg Ser Ser Thr Leu Glu Ser Gly Ile Pro Ala
       50                  55                  60
   Arg Phe Ser Gly Ser Gly Ser Gly Thr Asp Phe Thr Leu Thr Ile Ser
   65 70 75 80
   Ser Leu Glu Pro Glu Asp Phe Ala Val Tyr Tyr Cys Gln Gln Asn Tyr
   85 90 95
   Asp Val Pro Trp Thr Phe Gly Gln Gly Thr Lys Val Glu Ile Lys
   100 105 110
<210> 26
   <211> 8
   <212> PRT
   <213> Artificial Sequence
<220>
   <223> heavy chain HVR-H1, PD1-0098
<400> 26
   Gly Tyr Ser Ile Thr Ser Asp Tyr
   1 5
<210> 27
   <211> 3
   <212> PRT
   <213> Artificial Sequence
<220>
   <223> heavy chain HVR-H2, PD1-0098
<400> 27
   Tyr Ser Gly
   1
<210> 28
   <211> 9
   <212> PRT
   <213> Artificial Sequence
<220>
   <223> heavy chain HVR-H3, PD1-0098
<400> 28
   His Gly Ser Ala Pro Trp Tyr Phe Asp
   1 5
<210> 29
   <211> 12
   <212> PRT
   <213> Artificial Sequence
<220>
   <223> light chain HVR-L1, PD1-0098
<400> 29
   Ser Gln Asn Ile Val His Ser Asp Gly Asn Thr Tyr
   1 5 10
<210> 30
   <211> 3
   <212> PRT
   <213> Artificial Sequence
<220>
   <223> light chain HVR-L2, PD1-0098
<400> 30
   Lys Val Ser
   1
<210> 31
   <211> 6
   <212> PRT
   <213> Artificial Sequence
<220>
   <223> light chain HVR-L3, PD1-0098
<400> 31
   Gly Ser His Phe Pro Leu
   1 5
<210> 32
   <211> 120
   <212> PRT
   <213> Artificial Sequence
<220>
   <223> heavy chain variable domain VH, PD1-0098
<400> 32
   Asp Val Gln Leu Gln Glu Ser Gly Pro Gly Leu Val Lys Pro Ser Gln
   1 5 10 15
   Ser Leu Ser Leu Thr Cys Thr Val Thr Gly Tyr Ser Ile Thr Ser Asp
   20 25 30
   Tyr Ala Trp Asn Trp Ile Arg Gln Phe Pro Gly Asp Lys Leu Glu Trp
   35 40 45
   Leu Gly Tyr Ile Thr Tyr Ser Gly Phe Thr Asn Tyr Asn Pro Ser Leu
   50 55 60
   Lys Ser Arg Ile Ser Ile Ser Arg Asp Thr Ser Lys Asn Gln Phe Phe
   65 70 75 80
   Leu Gln Leu Asn Ser Val Ala Thr Glu Asp Thr Ala Thr Tyr Tyr Cys
   85 90 95
   Ala Arg Trp His Gly Ser Ala Pro Trp Tyr Phe Asp Tyr Trp Gly Arg
   100 105 110
   Gly Thr Thr Leu Thr Val Ser Ser
   115 120
<210> 33
   <211> 112
   <212> PRT
   <213> Artificial Sequence
<220>
   <223> light chain variable domain VL, PD1-0098
<400> 33
   Asp Val Leu Met Thr Gln Thr Pro Leu Ser Leu Pro Val Ser Leu Gly
   1 5 10 15
   Asp Gln Ala Ser Ile Ser Cys Arg Ser Ser Gln Asn Ile Val His Ser
   20 25 30
   Asp Gly Asn Thr Tyr Leu Glu Trp Tyr Leu Gln Lys Pro Gly Gln Ser
   35 40 45
   Pro Asn Leu Leu Ile Tyr Lys Val Ser Arg Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Phe Pro Leu Thr Phe Gly Ala Gly Thr Lys Leu Glu Leu Lys
   100 105 110
<210> 34
   <211> 8
   <212> PRT
   <213> Artificial Sequence
<220>
   <223> heavy chain HVR-H1, PD1-0050
<400> 34
   Gly Tyr Ser Ile Thr Ser Asp Tyr
   1 5
<210> 35
   <211> 3
   <212> PRT
   <213> Artificial Sequence
<220>
   <223> heavy chain HVR-H2, PD1-0050
<400> 35
   Tyr Thr Gly
   1
<210> 36
   <211> 9
   <212> PRT
   <213> Artificial Sequence
<220>
   <223> heavy chain HVR-H3, PD1-0050
<400> 36
   Met Asp Tyr Tyr Gly Ser Thr Leu Asp
   1               5
<210> 37
   <211> 11
   <212> PRT
   <213> Artificial Sequence
<220>
   <223> light chain HVR-L1, PD1-0050
<400> 37
   Ser Glu Ser Val Asp Arg Tyr Gly Asn Ser Phe
   1               5                   10
<210> 38
   <211> 3
   <212> PRT
   <213> Artificial Sequence
<220>
   <223> light chain HVR-L2, PD1-0050
<400> 38
   Arg Ala Ser
   1
<210> 39
   <211> 6
   <212> PRT
   <213> Artificial Sequence
<220>
   <223> light chain HVR-L3, PD1-0050
<400> 39
   Asn Asn Glu Asp Pro Tyr
   1               5
<210> 40
   <211> 120
   <212> PRT
   <213> Artificial Sequence
<220>
   <223> heavy chain variable domain VH, PD1-0050
<400> 40
   Asp Val Gln Leu Gln Glu Ser Gly Pro Gly Leu Val Lys Pro Ser Gln
   1 5 10 15
   Ser Leu Ser Leu Thr Cys Thr Val Thr Gly Tyr Ser Ile Thr Ser Asp
   20 25 30
   Tyr Ala Trp Asn Trp Ile Arg Gln Phe Pro Gly Asn Lys Leu Glu Trp
   35 40 45
   Met Gly Tyr Ile Thr Tyr Thr Gly Arg Thr Ser Tyr Asn Pro Ser Leu
   50 55 60
   Lys Ser Arg Ile Ser Ile Thr Arg Asp Thr Ser Lys Asn Gln Phe Phe
   65 70 75 80
   Leu Gln Leu Asn Ser Val Thr Thr Glu Asp Thr Ala Thr Tyr Tyr Cys
   85 90 95
   Ala Arg Glu Met Asp Tyr Tyr Gly Ser Thr Leu Asp Tyr Trp Gly Gln
   100 105 110
   Gly Thr Thr Leu Thr Val Ser Ser
   115 120
<210> 41
   <211> 111
   <212> PRT
   <213> Artificial Sequence
<220>
   <223> light chain variable domain VL, PD1-0050
<400> 41
   Lys Ile Val Leu Thr Gln Ser Pro Ala Ser Leu Ala Val Ser Leu Arg
   1 5 10 15
   Gln Arg Ala Thr Ile Ser Cys Arg Ala Ser Glu Ser Val Asp Arg Tyr
               20                  25                  30
   Gly Asn Ser Phe Ile His Trp Tyr Gln Gln Lys Pro Gly Gln Pro Pro
           35                  40                  45
   Lys Val Leu Ile Tyr Arg Ala Ser Asn Leu Glu Ser Gly Phe Pro Ala
       50                  55                  60
   Arg Phe Ser Gly Ser Gly Ser Arg Thr Asp Phe Thr Leu Thr Ile Asp
   65                  70                  75                  80
   Pro Val Glu Ala Asp Asp Ala Ala Thr Tyr Tyr Cys Gln Gln Asn Asn
                   85                  90                  95
   Glu Asp Pro Tyr Thr Phe Gly Ser Gly Thr Lys Leu Glu Ile Lys
               100                 105                 110
<210> 42
   <211> 7
   <212> PRT
   <213> Artificial Sequence
<220>
   <223> heavy chain HVR-H1, PD1-0069
<400> 42
   Gly Tyr Thr Phe Thr Asp Tyr
   1               5
<210> 43
   <211> 3
   <212> PRT
   <213> Artificial Sequence
<220>
   <223> heavy chain HVR-H2, PD1-0069
<400> 43
   Tyr Ser Gly
   1
<210> 44
   <211> 7
   <212> PRT
   <213> Artificial Sequence
<220>
   <223> heavy chain HVR-H3, PD1-0069
<400> 44
   Gly Ile Thr Thr Gly Phe Ala
   1 5
<210> 45
   <211> 11
   <212> PRT
   <213> Artificial Sequence
<220>
   <223> light chain HVR-L1, PD1-0069
<400> 45
   Ser Lys Gly Val Ser Thr Ser Ser Tyr Ser Phe
   1 5 10
<210> 46
   <211> 3
   <212> PRT
   <213> Artificial Sequence
<220>
   <223> light chain HVR-L2, PD1-0069
<400> 46
   Tyr Ala Ser
   1
<210> 47
   <211> 6
   <212> PRT
   <213> Artificial Sequence
<220>
   <223> light chain HVR-L3, PD1-0069
<400> 47
   Ser Arg Glu Phe Pro Trp
   1 5
<210> 48
   <211> 118
   <212> PRT
   <213> Artificial Sequence
<220>
   <223> heavy chain variable domain VH, PD1-0069
<400> 48
   Gln Val Gln Leu Gln Gln Ser Gly Pro Glu Leu Val Arg Pro Gly Val
   1               5                   10                  15
   Ser Val Lys Ile Ser Cys Lys Gly Ser Gly Tyr Thr Phe Thr Asp Tyr
               20                  25                  30
   Ala Met His Trp Val Lys Gln Ser His Ala Arg Thr Leu Glu Trp Ile
           35                  40                  45
   Gly Val Ile Ser Thr Tyr Ser Gly Asp Thr Asn Tyr Asn Gln Lys Phe
       50                  55                  60
   Lys Asp Lys Ala Thr Met Thr Val Asp Lys Ser Ser Ser Thr Ala Tyr
   65                  70                  75                  80
   Leu Glu Leu Ala Arg Met Thr Ser Glu Asp Ser Ala Ile Tyr Tyr Cys
                   85                  90                  95
   Ala Arg Leu Gly Ile Thr Thr Gly Phe Ala Tyr Trp Gly Gln Gly Thr
               100                 105                 110
   Leu Val Thr Val Ser Ala
           115
<210> 49
   <211> 111
   <212> PRT
   <213> Artificial Sequence
<220>
   <223> light chain variable domain VL, PD1-0069
<400> 49
   Asp Ile Val Leu Thr Gln Ser Pro Ala Ser Leu Ala Val Ser Leu Gly
   1 5 10 15
   Gln Arg Ala Thr Ile Ser Cys Arg Ala Ser Lys Gly Val Ser Thr Ser
   20 25 30
   Ser Tyr Ser Phe Met His Trp Tyr Gln Gln Lys Pro Arg Gln Pro Pro
   35 40 45
   Lys Leu Leu Ile Lys Tyr Ala Ser Tyr Leu Glu Ser Gly Val Pro Ala
   50 55 60
   Arg Phe Ser Gly Ser Gly Ser Gly Thr Asp Phe Thr Leu Asn Ile His
   65 70 75 80
   Pro Val Glu Glu Glu Asp Ala Ala Thr Tyr Tyr Cys His His Ser Arg
   85 90 95
   Glu Phe Pro Trp Thr Phe Gly Gly Gly Thr Lys Leu Glu Ile Lys
   100 105 110
<210> 50
   <211> 7
   <212> PRT
   <213> Artificial Sequence
<220>
   <223> heavy chain HVR-H1, PD1-0073
<400> 50
   Gly Phe Thr Phe Ser Asn Tyr
   1 5
<210> 51
   <211> 3
   <212> PRT
   <213> Artificial Sequence
<220>
   <223> heavy chain HVR-H2, PD1-0073
<400> 51
   Gly Gly Arg
   1
<210> 52
   <211> 5
   <212> PRT
   <213> Artificial Sequence
<220>
   <223> heavy chain HVR-H3, PD1-0073
<400> 52
   Tyr Tyr Gly Ile Asp
   1 5
<210> 53
   <211> 7
   <212> PRT
   <213> Artificial Sequence
<220>
   <223> light chain HVR-L1, PD1-0073
<400> 53
   Ser Gln Asp Val Thr Thr Ala
   1 5
<210> 54
   <211> 3
   <212> PRT
   <213> Artificial Sequence
<220>
   <223> light chain HVR-L2, PD1-0073
<400> 54
   Trp Ala Ser
   1
<210> 55
   <211> 6
   <212> PRT
   <213> Artificial Sequence
<220>
   <223> light chain HVR-L3, PD1-0073
<400> 55
   His Tyr Ser Ile Pro Trp
   1 5
<210> 56
   <211> 116
   <212> PRT
   <213> Artificial Sequence
<220>
   <223> heavy chain variable domain VH, PD1-0073
<400> 56
   Glu Val Lys Leu Val Glu Ser Gly Gly Gly Leu Val Lys Pro Gly Gly
   1               5                   10                  15
   Ser Leu Lys Leu Ser Cys Ala Ala Ser Gly Phe Thr Phe Ser Asn Tyr
               20                  25                  30
   Gly Met Ser Trp Ile Arg Gln Thr Pro Glu Lys Gly Leu Glu Trp Val
           35                  40                  45
   Ala Thr Ile Ser Gly Gly Gly Arg Asp Thr Tyr Tyr Pro Asp Ser Val
       50                  55                  60
   Lys Gly Arg Phe Thr Ile Ser Arg Asp Asn Val Lys Asn Asn Leu Tyr
   65                  70                  75                  80
   Leu Gln Met Ser Ser Leu Arg Ser Glu Asp Thr Ala Phe Tyr Tyr Cys
                   85                  90                  95
   Ala Ser Tyr Tyr Tyr Gly Ile Asp Tyr Trp Gly Gln Gly Thr Ser Val
               100                 105                 110
   Thr Val Ser Ser
           115
<210> 57
   <211> 107
   <212> PRT
   <213> Artificial Sequence
<220>
   <223> light chain variable domain VL, PD1-0073
<400> 57
   Asp Ile Val Met Thr Gln Pro His Lys Phe Met Ser Thr Ser Val Gly
   1 5 10 15
   Asp Arg Val Arg Ile Thr Cys Lys Ala Ser Gln Asp Val Thr Thr Ala
   20 25 30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Gly Ser Gly Thr Glu Phe Thr Leu Thr Ile Ser Ser Val Gln Ala
   65 70 75 80
   Glu Asp Leu Ala Leu Tyr Tyr Cys Gln Gln His Tyr Ser Ile Pro Trp
   85 90 95
   Thr Phe Gly Gly Gly Thr Lys Leu Glu Ile Lys
   100 105
<210> 58
   <211> 7
   <212> PRT
   <213> Artificial Sequence
<220>
   <223> heavy chain HVR-H1, PD1-0078
<400> 58
   Gly Tyr Thr Phe Thr Ser Thr
   1 5
<210> 59
   <211> 3
   <212> PRT
   <213> Artificial Sequence
<220>
   <223> heavy chain HVR-H2, PD1-0078
<400> 59
   Ser Asp Ser
   1
<210> 60
   <211> 3
   <212> PRT
   <213> Artificial Sequence
<220>
   <223> heavy chain HVR-H3, PD1-0078
<400> 60
   Pro Phe Asp
   1
<210> 61
   <211> 7
   <212> PRT
   <213> Artificial Sequence
<220>
   <223> light chain HVR-L1, PD1-0078
<400> 61
   Ser Gln Asp Val Ser Thr Ala
   1 5
<210> 62
   <211> 3
   <212> PRT
   <213> Artificial Sequence
<220>
   <223> light chain HVR-L2, PD1-0078
<400> 62
   Ser Ala Ser
   1
<210> 63
   <211> 6
   <212> PRT
   <213> Artificial Sequence
<220>
   <223> light chain HVR-L3, PD1-0078
<400> 63
   His Tyr Ser His Pro Phe
   1 5
<210> 64
   <211> 114
   <212> PRT
   <213> Artificial Sequence
<220>
   <223> heavy chain variable domain VH, PD1-0078
<400> 64
   Gln Val Gln Leu Gln Gln Pro Gly Ala Glu Leu Val Lys Pro Gly Ala
   1 5 10 15
   Ser Val Lys Met Ser Cys Lys Ala Ser Gly Tyr Thr Phe Thr Ser Thr
   20 25 30
   Trp Met His Trp Val Lys Gln Arg Pro Gly Gln Gly Leu Glu Trp Ile
   35 40 45
   Gly Ala Ile Asp Pro Ser Asp Ser Tyr Thr Thr Tyr Asn Gln Lys Phe
   50 55 60
   Lys Gly Lys Ala Thr Leu Thr Val Asp Thr Ser Ser Thr Thr Ala Tyr
   65 70 75 80
   Met Gln Leu Ser Ser Leu Thr Ser Glu Asp Ser Ala Val Tyr Tyr Cys
   85 90 95
   Thr Arg Ser Pro Phe Asp Tyr Trp Gly Gln Gly Thr Thr Leu Thr Val
   100 105 110
   Ser Ser
<210> 65
   <211> 107
   <212> PRT
   <213> Artificial Sequence
<220>
   <223> light chain variable domain VL, PD1-0078
<400> 65
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Ser Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Thr Gly Val Pro Asp Arg Phe Thr Gly
   50 55 60
   Ser Gly Ser Gly Thr Asp Phe Thr Phe Ala Ile Ser Ser Val Gln Ala
   65 70 75 80
   Glu Asp Leu Ala Val Tyr Tyr Cys Gln Gln His Tyr Ser His Pro Phe
   85 90 95
   Thr Phe Gly Ser Gly Thr Lys Leu Glu Ile Lys
   100 105
<210> 66
   <211> 8
   <212> PRT
   <213> Artificial Sequence
<220>
   <223> heavy chain HVR-H1, PD1-0102
<400> 66
   Gly Tyr Ser Ile Thr Ser Gly Tyr
   1 5
<210> 67
   <211> 3
   <212> PRT
   <213> Artificial Sequence
<220>
   <223> heavy chain HVR-H2, PD1-0102
<400> 67
   Ser Ser Gly
   1
<210> 68
   <211> 6
   <212> PRT
   <213> Artificial Sequence
<220>
   <223> heavy chain HVR-H3, PD1-0102
<400> 68
   Arg Asn Trp Tyr Phe Asp
   1 5
<210> 69
   <211> 13
   <212> PRT
   <213> Artificial Sequence
<220>
   <223> light chain HVR-L1, PD1-0102
<400> 69
   Ser Gln Ser Leu Leu Asn Ser Gly Thr Gln Lys Asn Tyr
   1               5                   10
<210> 70
   <211> 3
   <212> PRT
   <213> Artificial Sequence
<220>
   <223> light chain HVR-L2, PD1-0102
<400> 70
   Trp Ala Ser
   1
<210> 71
   <211> 6
   <212> PRT
   <213> Artificial Sequence
<220>
   <223> light chain HVR-L3, PD1-0102
<400> 71
   Asp Tyr Thr Phe Pro Leu
   1               5
<210> 72
   <211> 117
   <212> PRT
   <213> Artificial Sequence
<220>
   <223> heavy chain variable domain VH, PD1-0102
<400> 72
   Asp Val Gln Leu Gln Glu Ser Gly Pro Asp Leu Val Lys Pro Ser Gln
   1               5                   10                  15
   Ser Leu Ser Leu Thr Cys Thr Val Thr Gly Tyr Ser Ile Thr Ser Gly
               20                  25                  30
   Tyr Ser Trp His Trp Ile Arg Gln Phe Pro Gly Asn Lys Leu Glu Trp
           35                  40                  45
   Met Gly Phe Ile His Ser Ser Gly Asp Thr Asn Tyr Asn Pro Ser Leu
       50                  55                  60
   Lys Ser Arg Ile Ser Phe Thr Arg Asp Thr Ser Lys Asn Gln Phe Phe
   65                  70                  75                  80
   Leu Gln Leu Ser Ser Leu Thr Asp Glu Asp Thr Ala Thr Tyr Tyr Cys
   85 90 95
   Ala Thr Tyr Arg Asn Trp Tyr Phe Asp Val Trp Gly Ala Gly Thr Thr
   100 105 110
   Val Thr Val Ser Ser
   115
<210> 73
   <211> 113
   <212> PRT
   <213> Artificial Sequence
<220>
   <223> light chain variable domain VL, PD1-0102
<400> 73
   Asp Ile Val Met Thr Gln Ser Pro Ser Ser Leu Thr Val Thr Ala Gly
   1 5 10 15
   Glu Lys Val Thr Met Arg Cys Lys Ser Ser Gln Ser Leu Leu Asn Ser
   20 25 30
   Gly Thr Gln Lys Asn Tyr Leu Thr Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asn Arg Phe Thr Gly Ser Gly Ser Gly Thr Asp Phe Thr Leu Thr
   65 70 75 80
   Ile Ser Ser Val Gln Ala Glu Asp Leu Ser Val Tyr Tyr Cys Gln Ser
   85 90 95
   Asp Tyr Thr Phe Pro Leu Thr Phe Gly Gly Gly Thr Lys Leu Glu Leu
   100 105 110
   Lys
<210> 74
   <211> 722
   <212> PRT
   <213> Artificial Sequence
<220>
   <223> Dimeric hu 4-1BBL (71-254) - CL* Fc knob chain
<400> 74
   Arg Glu Gly Pro Glu Leu Ser Pro Asp Asp Pro Ala Gly Leu Leu Asp
   1               5                   10                  15
   Leu Arg Gln Gly Met Phe Ala Gln Leu Val Ala Gln Asn Val Leu Leu
               20                  25                  30
   Ile Asp Gly Pro Leu Ser Trp Tyr Ser Asp Pro Gly Leu Ala Gly Val
           35                  40                  45
   Ser Leu Thr Gly Gly Leu Ser Tyr Lys Glu Asp Thr Lys Glu Leu Val
       50                  55                  60
   Val Ala Lys Ala Gly Val Tyr Tyr Val Phe Phe Gln Leu Glu Leu Arg
   65                  70                  75                  80
   Arg Val Val Ala Gly Glu Gly Ser Gly Ser Val Ser Leu Ala Leu His
                   85                  90                  95
   Leu Gln Pro Leu Arg Ser Ala Ala Gly Ala Ala Ala Leu Ala Leu Thr
               100                 105                 110
   Val Asp Leu Pro Pro Ala Ser Ser Glu Ala Arg Asn Ser Ala Phe Gly
           115                 120                 125
   Phe Gln Gly Arg Leu Leu His Leu Ser Ala Gly Gln Arg Leu Gly Val
       130                 135                 140
   His Leu His Thr Glu Ala Arg Ala Arg His Ala Trp Gln Leu Thr Gln
   145                 150                 155                 160
   Gly Ala Thr Val Leu Gly Leu Phe Arg Val Thr Pro Glu Ile Pro Ala
                   165                 170                 175
   Gly Leu Pro Ser Pro Arg Ser Glu Gly Gly Gly Gly Ser Gly Gly Gly
               180                 185                 190
   Gly Ser Arg Glu Gly Pro Glu Leu Ser Pro Asp Asp Pro Ala Gly Leu
           195                 200                 205
   Leu Asp Leu Arg Gln Gly Met Phe Ala Gln Leu Val Ala Gln Asn Val
       210                 215                 220
   Leu Leu Ile Asp Gly Pro Leu Ser Trp Tyr Ser Asp Pro Gly Leu Ala
   225                 230                 235                 240
   Gly Val Ser Leu Thr Gly Gly Leu Ser Tyr Lys Glu Asp Thr Lys Glu
                   245                 250                 255
   Leu Val Val Ala Lys Ala Gly Val Tyr Tyr Val Phe Phe Gln Leu Glu
   260 265 270
   Leu Arg Arg Val Val Ala Gly Glu Gly Ser Gly Ser Val Ser Leu Ala
   275 280 285
   Leu His Leu Gln Pro Leu Arg Ser Ala Ala Gly Ala Ala Ala Leu Ala
   290 295 300
   Leu Thr Val Asp Leu Pro Pro Ala Ser Ser Glu Ala Arg Asn Ser Ala
   305 310 315 320
   Phe Gly Phe Gln Gly Arg Leu Leu His Leu Ser Ala Gly Gln Arg Leu
   325 330 335
   Gly Val His Leu His Thr Glu Ala Arg Ala Arg His Ala Trp Gln Leu
   340 345 350
   Thr Gln Gly Ala Thr Val Leu Gly Leu Phe Arg Val Thr Pro Glu Ile
   355 360 365
   Pro Ala Gly Leu Pro Ser Pro Arg Ser Glu Gly Gly Gly Gly Ser Gly
   370 375 380
   Gly Gly Gly Ser Arg Thr Val Ala Ala Pro Ser Val Phe Ile Phe Pro
   385 390 395 400
   Pro Ser Asp Arg Lys Leu Lys Ser Gly Thr Ala Ser Val Val Cys Leu
   405 410 415
   Leu Asn Asn Phe Tyr Pro Arg Glu Ala Lys Val Gln Trp Lys Val Asp
   420 425 430
   Asn Ala Leu Gln Ser Gly Asn Ser Gln Glu Ser Val Thr Glu Gln Asp
   435 440 445
   Ser Lys Asp Ser Thr Tyr Ser Leu Ser Ser Thr Leu Thr Leu Ser Lys
   450 455 460
   Ala Asp Tyr Glu Lys His Lys Val Tyr Ala Cys Glu Val Thr His Gln
   465 470 475 480
   Gly Leu Ser Ser Pro Val Thr Lys Ser Phe Asn Arg Gly Glu Cys Asp
   485 490 495
   Lys Thr His Thr Cys Pro Pro Cys Pro Ala Pro Glu Ala Ala Gly Gly
   500 505 510
   Pro Ser Val Phe Leu Phe Pro Pro Lys Pro Lys Asp Thr Leu Met Ile
   515 520 525
   Ser Arg Thr Pro Glu Val Thr Cys Val Val Val Asp Val Ser His Glu
   530 535 540
   Asp Pro Glu Val Lys Phe Asn Trp Tyr Val Asp Gly Val Glu Val His
   545 550 555 560
   Asn Ala Lys Thr Lys Pro Arg Glu Glu Gln Tyr Asn Ser Thr Tyr Arg
   565 570 575
   Val Val Ser Val Leu Thr Val Leu His Gln Asp Trp Leu Asn Gly Lys
   580 585 590
   Glu Tyr Lys Cys Lys Val Ser Asn Lys Ala Leu Gly Ala Pro Ile Glu
   595 600 605
   Lys Thr Ile Ser Lys Ala Lys Gly Gln Pro Arg Glu Pro Gln Val Tyr
   610 615 620
   Thr Leu Pro Pro Cys Arg Asp Glu Leu Thr Lys Asn Gln Val Ser Leu
   625 630 635 640
   Trp Cys Leu Val Lys Gly Phe Tyr Pro Ser Asp Ile Ala Val Glu Trp
   645 650 655
   Glu Ser Asn Gly Gln Pro Glu Asn Asn Tyr Lys Thr Thr Pro Pro Val
   660 665 670
   Leu Asp Ser Asp Gly Ser Phe Phe Leu Tyr Ser Lys Leu Thr Val Asp
   675 680 685
   Lys Ser Arg Trp Gln Gln Gly Asn Val Phe Ser Cys Ser Val Met His
   690 695 700
   Glu Ala Leu His Asn His Tyr Thr Gln Lys Ser Leu Ser Leu Ser Pro
   705 710 715 720
   Gly Lys
<210> 75
   <211> 297
   <212> PRT
   <213> Artificial Sequence
<220>
   <223> Monomeric hu 4-1BBL (71-254) - CH1*
<400> 75
   Arg Glu Gly Pro Glu Leu Ser Pro Asp Asp Pro Ala Gly Leu Leu Asp
   1               5                   10                  15
   Leu Arg Gln Gly Met Phe Ala Gln Leu Val Ala Gln Asn Val Leu Leu
               20                  25                  30
   Ile Asp Gly Pro Leu Ser Trp Tyr Ser Asp Pro Gly Leu Ala Gly Val
           35                  40                  45
   Ser Leu Thr Gly Gly Leu Ser Tyr Lys Glu Asp Thr Lys Glu Leu Val
       50                  55                  60
   Val Ala Lys Ala Gly Val Tyr Tyr Val Phe Phe Gln Leu Glu Leu Arg
   65                  70                  75                  80
   Arg Val Val Ala Gly Glu Gly Ser Gly Ser Val Ser Leu Ala Leu His
                   85                  90                  95
   Leu Gln Pro Leu Arg Ser Ala Ala Gly Ala Ala Ala Leu Ala Leu Thr
               100                 105                 110
   Val Asp Leu Pro Pro Ala Ser Ser Glu Ala Arg Asn Ser Ala Phe Gly
           115                 120                 125
   Phe Gln Gly Arg Leu Leu His Leu Ser Ala Gly Gln Arg Leu Gly Val
       130                 135                 140
   His Leu His Thr Glu Ala Arg Ala Arg His Ala Trp Gln Leu Thr Gln
   145                 150                 155                 160
   Gly Ala Thr Val Leu Gly Leu Phe Arg Val Thr Pro Glu Ile Pro Ala
                   165                 170                 175
   Gly Leu Pro Ser Pro Arg Ser Glu Gly Gly Gly Gly Ser Gly Gly Gly
               180                 185                 190
   Gly Ser Ala Ser Thr Lys Gly Pro Ser Val Phe Pro Leu Ala Pro Ser
           195                 200                 205
   Ser Lys Ser Thr Ser Gly Gly Thr Ala Ala Leu Gly Cys Leu Val Glu
       210                 215                 220
   Asp Tyr Phe Pro Glu Pro Val Thr Val Ser Trp Asn Ser Gly Ala Leu
   225 230 235 240
   Thr Ser Gly Val His Thr Phe Pro Ala Val Leu Gln Ser Ser Gly Leu
   245 250 255
   Tyr Ser Leu Ser Ser Val Val Thr Val Pro Ser Ser Ser Leu Gly Thr
   260 265 270
   Gln Thr Tyr Ile Cys Asn Val Asn His Lys Pro Ser Asn Thr Lys Val
   275 280 285
   Asp Glu Lys Val Glu Pro Lys Ser Cys
   290 295
<210> 76
   <211> 722
   <212> PRT
   <213> Artificial Sequence
<220>
   <223> Dimeric hu 4-1BBL (71-254) - CL Fc knob chain
<400> 76
   Arg Glu Gly Pro Glu Leu Ser Pro Asp Asp Pro Ala Gly Leu Leu Asp
   1               5                   10                  15
   Leu Arg Gln Gly Met Phe Ala Gln Leu Val Ala Gln Asn Val Leu Leu
               20                  25                  30
   Ile Asp Gly Pro Leu Ser Trp Tyr Ser Asp Pro Gly Leu Ala Gly Val
           35                  40                  45
   Ser Leu Thr Gly Gly Leu Ser Tyr Lys Glu Asp Thr Lys Glu Leu Val
       50                  55                  60
   Val Ala Lys Ala Gly Val Tyr Tyr Val Phe Phe Gln Leu Glu Leu Arg
   65                  70                  75                  80
   Arg Val Val Ala Gly Glu Gly Ser Gly Ser Val Ser Leu Ala Leu His
                   85                  90                  95
   Leu Gln Pro Leu Arg Ser Ala Ala Gly Ala Ala Ala Leu Ala Leu Thr
               100                 105                 110
   Val Asp Leu Pro Pro Ala Ser Ser Glu Ala Arg Asn Ser Ala Phe Gly
           115                 120                 125
   Phe Gln Gly Arg Leu Leu His Leu Ser Ala Gly Gln Arg Leu Gly Val
       130                 135                 140
   His Leu His Thr Glu Ala Arg Ala Arg His Ala Trp Gln Leu Thr Gln
   145 150 155 160
   Gly Ala Thr Val Leu Gly Leu Phe Arg Val Thr Pro Glu Ile Pro Ala
   165 170 175
   Gly Leu Pro Ser Pro Arg Ser Glu Gly Gly Gly Gly Ser Gly Gly Gly
   180 185 190
   Gly Ser Arg Glu Gly Pro Glu Leu Ser Pro Asp Asp Pro Ala Gly Leu
   195 200 205
   Leu Asp Leu Arg Gln Gly Met Phe Ala Gln Leu Val Ala Gln Asn Val
   210 215 220
   Leu Leu Ile Asp Gly Pro Leu Ser Trp Tyr Ser Asp Pro Gly Leu Ala
   225 230 235 240
   Gly Val Ser Leu Thr Gly Gly Leu Ser Tyr Lys Glu Asp Thr Lys Glu
   245 250 255
   Leu Val Val Ala Lys Ala Gly Val Tyr Tyr Val Phe Phe Gln Leu Glu
   260 265 270
   Leu Arg Arg Val Val Ala Gly Glu Gly Ser Gly Ser Val Ser Leu Ala
   275 280 285
   Leu His Leu Gln Pro Leu Arg Ser Ala Ala Gly Ala Ala Ala Leu Ala
   290 295 300
   Leu Thr Val Asp Leu Pro Pro Ala Ser Ser Glu Ala Arg Asn Ser Ala
   305 310 315 320
   Phe Gly Phe Gln Gly Arg Leu Leu His Leu Ser Ala Gly Gln Arg Leu
   325 330 335
   Gly Val His Leu His Thr Glu Ala Arg Ala Arg His Ala Trp Gln Leu
   340 345 350
   Thr Gln Gly Ala Thr Val Leu Gly Leu Phe Arg Val Thr Pro Glu Ile
   355 360 365
   Pro Ala Gly Leu Pro Ser Pro Arg Ser Glu Gly Gly Gly Gly Ser Gly
   370 375 380
   Gly Gly Gly Ser Arg Thr Val Ala Ala Pro Ser Val Phe Ile Phe Pro
   385 390 395 400
   Pro Ser Asp Glu Gln Leu Lys Ser Gly Thr Ala Ser Val Val Cys Leu
   405 410 415
   Leu Asn Asn Phe Tyr Pro Arg Glu Ala Lys Val Gln Trp Lys Val Asp
   420 425 430
   Asn Ala Leu Gln Ser Gly Asn Ser Gln Glu Ser Val Thr Glu Gln Asp
   435 440 445
   Ser Lys Asp Ser Thr Tyr Ser Leu Ser Ser Thr Leu Thr Leu Ser Lys
   450 455 460
   Ala Asp Tyr Glu Lys His Lys Val Tyr Ala Cys Glu Val Thr His Gln
   465 470 475 480
   Gly Leu Ser Ser Pro Val Thr Lys Ser Phe Asn Arg Gly Glu Cys Asp
   485 490 495
   Lys Thr His Thr Cys Pro Pro Cys Pro Ala Pro Glu Ala Ala Gly Gly
   500 505 510
   Pro Ser Val Phe Leu Phe Pro Pro Lys Pro Lys Asp Thr Leu Met Ile
   515 520 525
   Ser Arg Thr Pro Glu Val Thr Cys Val Val Val Asp Val Ser His Glu
   530 535 540
   Asp Pro Glu Val Lys Phe Asn Trp Tyr Val Asp Gly Val Glu Val His
   545 550 555 560
   Asn Ala Lys Thr Lys Pro Arg Glu Glu Gln Tyr Asn Ser Thr Tyr Arg
   565 570 575
   Val Val Ser Val Leu Thr Val Leu His Gln Asp Trp Leu Asn Gly Lys
   580 585 590
   Glu Tyr Lys Cys Lys Val Ser Asn Lys Ala Leu Gly Ala Pro Ile Glu
   595 600 605
   Lys Thr Ile Ser Lys Ala Lys Gly Gln Pro Arg Glu Pro Gln Val Tyr
   610 615 620
   Thr Leu Pro Pro Cys Arg Asp Glu Leu Thr Lys Asn Gln Val Ser Leu
   625 630 635 640
   Trp Cys Leu Val Lys Gly Phe Tyr Pro Ser Asp Ile Ala Val Glu Trp
   645 650 655
   Glu Ser Asn Gly Gln Pro Glu Asn Asn Tyr Lys Thr Thr Pro Pro Val
   660 665 670
   Leu Asp Ser Asp Gly Ser Phe Phe Leu Tyr Ser Lys Leu Thr Val Asp
   675 680 685
   Lys Ser Arg Trp Gln Gln Gly Asn Val Phe Ser Cys Ser Val Met His
   690 695 700
   Glu Ala Leu His Asn His Tyr Thr Gln Lys Ser Leu Ser Leu Ser Pro
   705 710 715 720
   Gly Lys
<210> 77
   <211> 297
   <212> PRT
   <213> Artificial Sequence
<220>
   <223> Monomeric hu 4-1BBL (71-254) - CH1
<400> 77
   Arg Glu Gly Pro Glu Leu Ser Pro Asp Asp Pro Ala Gly Leu Leu Asp
   1               5                   10                  15
   Leu Arg Gln Gly Met Phe Ala Gln Leu Val Ala Gln Asn Val Leu Leu
               20                  25                  30
   Ile Asp Gly Pro Leu Ser Trp Tyr Ser Asp Pro Gly Leu Ala Gly Val
           35                  40                  45
   Ser Leu Thr Gly Gly Leu Ser Tyr Lys Glu Asp Thr Lys Glu Leu Val
       50                  55                  60
   Val Ala Lys Ala Gly Val Tyr Tyr Val Phe Phe Gln Leu Glu Leu Arg
   65                  70                  75                  80
   Arg Val Val Ala Gly Glu Gly Ser Gly Ser Val Ser Leu Ala Leu His
                   85                  90                  95
   Leu Gln Pro Leu Arg Ser Ala Ala Gly Ala Ala Ala Leu Ala Leu Thr
               100                 105                 110
   Val Asp Leu Pro Pro Ala Ser Ser Glu Ala Arg Asn Ser Ala Phe Gly
               115                 120                 125
   Phe Gln Gly Arg Leu Leu His Leu Ser Ala Gly Gln Arg Leu Gly Val
       130                 135                 140
   His Leu His Thr Glu Ala Arg Ala Arg His Ala Trp Gln Leu Thr Gln
   145                 150                 155                 160
   Gly Ala Thr Val Leu Gly Leu Phe Arg Val Thr Pro Glu Ile Pro Ala
                   165                 170                 175
   Gly Leu Pro Ser Pro Arg Ser Glu Gly Gly Gly Gly Ser Gly Gly Gly
               180                 185                 190
   Gly Ser Ala Ser Thr Lys Gly Pro Ser Val Phe Pro Leu Ala Pro Ser
           195                 200                 205
   Ser Lys Ser Thr Ser Gly Gly Thr Ala Ala Leu Gly Cys Leu Val Lys
       210                 215                 220
   Asp Tyr Phe Pro Glu Pro Val Thr Val Ser Trp Asn Ser Gly Ala Leu
   225                 230                 235                 240
   Thr Ser Gly Val His Thr Phe Pro Ala Val Leu Gln Ser Ser Gly Leu
                   245                 250                 255
   Tyr Ser Leu Ser Ser Val Val Thr Val Pro Ser Ser Ser Leu Gly Thr
               260                 265                 270
   Gln Thr Tyr Ile Cys Asn Val Asn His Lys Pro Ser Asn Thr Lys Val
           275                 280                 285
   Asp Lys Lys Val Glu Pro Lys Ser Cys
       290                 295
<210> 78
   <211> 710
   <212> PRT
   <213> Artificial Sequence
<220>
   <223> Dimeric hu 4-1BBL (71-248) - CL* Fc knob chain
<400> 78
   Arg Glu Gly Pro Glu Leu Ser Pro Asp Asp Pro Ala Gly Leu Leu Asp
   1               5                   10                  15
   Leu Arg Gln Gly Met Phe Ala Gln Leu Val Ala Gln Asn Val Leu Leu
               20                  25                  30
   Ile Asp Gly Pro Leu Ser Trp Tyr Ser Asp Pro Gly Leu Ala Gly Val
           35                  40                  45
   Ser Leu Thr Gly Gly Leu Ser Tyr Lys Glu Asp Thr Lys Glu Leu Val
       50                  55                  60
   Val Ala Lys Ala Gly Val Tyr Tyr Val Phe Phe Gln Leu Glu Leu Arg
   65                  70                  75                  80
   Arg Val Val Ala Gly Glu Gly Ser Gly Ser Val Ser Leu Ala Leu His
                   85                  90                  95
   Leu Gln Pro Leu Arg Ser Ala Ala Gly Ala Ala Ala Leu Ala Leu Thr
               100                 105                 110
   Val Asp Leu Pro Pro Ala Ser Ser Glu Ala Arg Asn Ser Ala Phe Gly
           115                 120                 125
   Phe Gln Gly Arg Leu Leu His Leu Ser Ala Gly Gln Arg Leu Gly Val
       130                 135                 140
   His Leu His Thr Glu Ala Arg Ala Arg His Ala Trp Gln Leu Thr Gln
   145                 150                 155                 160
   Gly Ala Thr Val Leu Gly Leu Phe Arg Val Thr Pro Glu Ile Pro Ala
                   165                 170                 175
   Gly Leu Gly Gly Gly Gly Ser Gly Gly Gly Gly Ser Arg Glu Gly Pro
               180                 185                 190
   Glu Leu Ser Pro Asp Asp Pro Ala Gly Leu Leu Asp Leu Arg Gln Gly
           195                 200                 205
   Met Phe Ala Gln Leu Val Ala Gln Asn Val Leu Leu Ile Asp Gly Pro
       210                 215                 220
   Leu Ser Trp Tyr Ser Asp Pro Gly Leu Ala Gly Val Ser Leu Thr Gly
   225                 230                 235                 240
   Gly Leu Ser Tyr Lys Glu Asp Thr Lys Glu Leu Val Val Ala Lys Ala
                   245                 250                 255
   Gly Val Tyr Tyr Val Phe Phe Gln Leu Glu Leu Arg Arg Val Val Ala
               260                 265                 270
   Gly Glu Gly Ser Gly Ser Val Ser Leu Ala Leu His Leu Gln Pro Leu
   275 280 285
   Arg Ser Ala Ala Gly Ala Ala Ala Leu Ala Leu Thr Val Asp Leu Pro
   290 295 300
   Pro Ala Ser Ser Glu Ala Arg Asn Ser Ala Phe Gly Phe Gln Gly Arg
   305 310 315 320
   Leu Leu His Leu Ser Ala Gly Gln Arg Leu Gly Val His Leu His Thr
   325 330 335
   Glu Ala Arg Ala Arg His Ala Trp Gln Leu Thr Gln Gly Ala Thr Val
   340 345 350
   Leu Gly Leu Phe Arg Val Thr Pro Glu Ile Pro Ala Gly Leu Gly Gly
   355 360 365
   Gly Gly Ser Gly Gly Gly Gly Ser Arg Thr Val Ala Ala Pro Ser Val
   370 375 380
   Phe Ile Phe Pro Pro Ser Asp Arg Lys Leu Lys Ser Gly Thr Ala Ser
   385 390 395 400
   Val Val Cys Leu Leu Asn Asn Phe Tyr Pro Arg Glu Ala Lys Val Gln
   405 410 415
   Trp Lys Val Asp Asn Ala Leu Gln Ser Gly Asn Ser Gln Glu Ser Val
   420 425 430
   Thr Glu Gln Asp Ser Lys Asp Ser Thr Tyr Ser Leu Ser Ser Thr Leu
   435 440 445
   Thr Leu Ser Lys Ala Asp Tyr Glu Lys His Lys Val Tyr Ala Cys Glu
   450 455 460
   Val Thr His Gln Gly Leu Ser Ser Pro Val Thr Lys Ser Phe Asn Arg
   465 470 475 480
   Gly Glu Cys Asp Lys Thr His Thr Cys Pro Pro Cys Pro Ala Pro Glu
   485 490 495
   Ala Ala Gly Gly Pro Ser Val Phe Leu Phe Pro Pro Lys Pro Lys Asp
   500 505 510
   Thr Leu Met Ile Ser Arg Thr Pro Glu Val Thr Cys Val Val Val Asp
   515 520 525
   Val Ser His Glu Asp Pro Glu Val Lys Phe Asn Trp Tyr Val Asp Gly
   530 535 540
   Val Glu Val His Asn Ala Lys Thr Lys Pro Arg Glu Glu Gln Tyr Asn
   545 550 555 560
   Ser Thr Tyr Arg Val Val Ser Val Leu Thr Val Leu His Gln Asp Trp
   565 570 575
   Leu Asn Gly Lys Glu Tyr Lys Cys Lys Val Ser Asn Lys Ala Leu Gly
   580 585 590
   Ala Pro Ile Glu Lys Thr Ile Ser Lys Ala Lys Gly Gln Pro Arg Glu
   595 600 605
   Pro Gln Val Tyr Thr Leu Pro Pro Cys Arg Asp Glu Leu Thr Lys Asn
   610 615 620
   Gln Val Ser Leu Trp Cys Leu Val Lys Gly Phe Tyr Pro Ser Asp Ile
   625 630 635 640
   Ala Val Glu Trp Glu Ser Asn Gly Gln Pro Glu Asn Asn Tyr Lys Thr
   645 650 655
   Thr Pro Pro Val Leu Asp Ser Asp Gly Ser Phe Phe Leu Tyr Ser Lys
   660 665 670
   Leu Thr Val Asp Lys Ser Arg Trp Gln Gln Gly Asn Val Phe Ser Cys
   675 680 685
   Ser Val Met His Glu Ala Leu His Asn His Tyr Thr Gln Lys Ser Leu
   690 695 700
   Ser Leu Ser Pro Gly Lys
   705 710
<210> 79
   <211> 291
   <212> PRT
   <213> Artificial Sequence
<220>
   <223> Monomeric hu 4-1BBL (71-248) - CH1*
<400> 79
   Arg Glu Gly Pro Glu Leu Ser Pro Asp Asp Pro Ala Gly Leu Leu Asp
   1               5                   10                  15
   Leu Arg Gln Gly Met Phe Ala Gln Leu Val Ala Gln Asn Val Leu Leu
   20 25 30
   Ile Asp Gly Pro Leu Ser Trp Tyr Ser Asp Pro Gly Leu Ala Gly Val
   35 40 45
   Ser Leu Thr Gly Gly Leu Ser Tyr Lys Glu Asp Thr Lys Glu Leu Val
   50 55 60
   Val Ala Lys Ala Gly Val Tyr Tyr Val Phe Phe Gln Leu Glu Leu Arg
   65 70 75 80
   Arg Val Val Ala Gly Glu Gly Ser Gly Ser Val Ser Leu Ala Leu His
   85 90 95
   Leu Gln Pro Leu Arg Ser Ala Ala Gly Ala Ala Ala Leu Ala Leu Thr
   100 105 110
   Val Asp Leu Pro Pro Ala Ser Ser Glu Ala Arg Asn Ser Ala Phe Gly
   115 120 125
   Phe Gln Gly Arg Leu Leu His Leu Ser Ala Gly Gln Arg Leu Gly Val
   130 135 140
   His Leu His Thr Glu Ala Arg Ala Arg His Ala Trp Gln Leu Thr Gln
   145 150 155 160
   Gly Ala Thr Val Leu Gly Leu Phe Arg Val Thr Pro Glu Ile Pro Ala
   165 170 175
   Gly Leu Gly Gly Gly Gly Ser Gly Gly Gly Gly Ser Ala Ser Thr Lys
   180 185 190
   Gly Pro Ser Val Phe Pro Leu Ala Pro Ser Ser Lys Ser Thr Ser Gly
   195 200 205
   Gly Thr Ala Ala Leu Gly Cys Leu Val Glu Asp Tyr Phe Pro Glu Pro
   210 215 220
   Val Thr Val Ser Trp Asn Ser Gly Ala Leu Thr Ser Gly Val His Thr
   225 230 235 240
   Phe Pro Ala Val Leu Gln Ser Ser Gly Leu Tyr Ser Leu Ser Ser Val
   245 250 255
   Val Thr Val Pro Ser Ser Ser Leu Gly Thr Gln Thr Tyr Ile Cys Asn
   260 265 270
   Val Asn His Lys Pro Ser Asn Thr Lys Val Asp Glu Lys Val Glu Pro
   275 280 285
   Lys Ser Cys
   290
<210> 80
   <211> 710
   <212> PRT
   <213> Artificial Sequence
<220>
   <223> Dimeric hu 4-1BBL (71-248) - CL Fc knob chain
<400> 80
   Arg Glu Gly Pro Glu Leu Ser Pro Asp Asp Pro Ala Gly Leu Leu Asp
   1               5                   10                  15
   Leu Arg Gln Gly Met Phe Ala Gln Leu Val Ala Gln Asn Val Leu Leu
               20                  25                  30
   Ile Asp Gly Pro Leu Ser Trp Tyr Ser Asp Pro Gly Leu Ala Gly Val
           35                  40                  45
   Ser Leu Thr Gly Gly Leu Ser Tyr Lys Glu Asp Thr Lys Glu Leu Val
       50                  55                  60
   Val Ala Lys Ala Gly Val Tyr Tyr Val Phe Phe Gln Leu Glu Leu Arg
   65                  70                  75                  80
   Arg Val Val Ala Gly Glu Gly Ser Gly Ser Val Ser Leu Ala Leu His
                   85                  90                  95
   Leu Gln Pro Leu Arg Ser Ala Ala Gly Ala Ala Ala Leu Ala Leu Thr
               100                 105                 110
   Val Asp Leu Pro Pro Ala Ser Ser Glu Ala Arg Asn Ser Ala Phe Gly
           115                 120                 125
   Phe Gln Gly Arg Leu Leu His Leu Ser Ala Gly Gln Arg Leu Gly Val
       130                 135                 140
   His Leu His Thr Glu Ala Arg Ala Arg His Ala Trp Gln Leu Thr Gln
   145                 150                 155                 160
   Gly Ala Thr Val Leu Gly Leu Phe Arg Val Thr Pro Glu Ile Pro Ala
                   165                 170                 175
   Gly Leu Gly Gly Gly Gly Ser Gly Gly Gly Gly Ser Arg Glu Gly Pro
   180 185 190
   Glu Leu Ser Pro Asp Asp Pro Ala Gly Leu Leu Asp Leu Arg Gln Gly
   195 200 205
   Met Phe Ala Gln Leu Val Ala Gln Asn Val Leu Leu Ile Asp Gly Pro
   210 215 220
   Leu Ser Trp Tyr Ser Asp Pro Gly Leu Ala Gly Val Ser Leu Thr Gly
   225 230 235 240
   Gly Leu Ser Tyr Lys Glu Asp Thr Lys Glu Leu Val Val Ala Lys Ala
   245 250 255
   Gly Val Tyr Tyr Val Phe Phe Gln Leu Glu Leu Arg Arg Val Val Ala
   260 265 270
   Gly Glu Gly Ser Gly Ser Val Ser Leu Ala Leu His Leu Gln Pro Leu
   275 280 285
   Arg Ser Ala Ala Gly Ala Ala Ala Leu Ala Leu Thr Val Asp Leu Pro
   290 295 300
   Pro Ala Ser Ser Glu Ala Arg Asn Ser Ala Phe Gly Phe Gln Gly Arg
   305 310 315 320
   Leu Leu His Leu Ser Ala Gly Gln Arg Leu Gly Val His Leu His Thr
   325 330 335
   Glu Ala Arg Ala Arg His Ala Trp Gln Leu Thr Gln Gly Ala Thr Val
   340 345 350
   Leu Gly Leu Phe Arg Val Thr Pro Glu Ile Pro Ala Gly Leu Gly Gly
   355 360 365
   Gly Gly Ser Gly Gly Gly Gly Ser Arg Thr Val Ala Ala Pro Ser Val
   370 375 380
   Phe Ile Phe Pro Pro Ser Asp Glu Gln Leu Lys Ser Gly Thr Ala Ser
   385 390 395 400
   Val Val Cys Leu Leu Asn Asn Phe Tyr Pro Arg Glu Ala Lys Val Gln
   405 410 415
   Trp Lys Val Asp Asn Ala Leu Gln Ser Gly Asn Ser Gln Glu Ser Val
   420 425 430
   Thr Glu Gln Asp Ser Lys Asp Ser Thr Tyr Ser Leu Ser Ser Thr Leu
           435                 440                 445
   Thr Leu Ser Lys Ala Asp Tyr Glu Lys His Lys Val Tyr Ala Cys Glu
       450                 455                 460
   Val Thr His Gln Gly Leu Ser Ser Pro Val Thr Lys Ser Phe Asn Arg
   465                 470                 475                 480
   Gly Glu Cys Asp Lys Thr His Thr Cys Pro Pro Cys Pro Ala Pro Glu
                   485                 490                 495
   Ala Ala Gly Gly Pro Ser Val Phe Leu Phe Pro Pro Lys Pro Lys Asp
               500                 505                 510
   Thr Leu Met Ile Ser Arg Thr Pro Glu Val Thr Cys Val Val Val Asp
           515                 520                 525
   Val Ser His Glu Asp Pro Glu Val Lys Phe Asn Trp Tyr Val Asp Gly
       530                 535                 540
   Val Glu Val His Asn Ala Lys Thr Lys Pro Arg Glu Glu Gln Tyr Asn
   545                 550                 555                 560
   Ser Thr Tyr Arg Val Val Ser Val Leu Thr Val Leu His Gln Asp Trp
                   565                 570                 575
   Leu Asn Gly Lys Glu Tyr Lys Cys Lys Val Ser Asn Lys Ala Leu Gly
               580                 585                 590
   Ala Pro Ile Glu Lys Thr Ile Ser Lys Ala Lys Gly Gln Pro Arg Glu
           595                 600                 605
   Pro Gln Val Tyr Thr Leu Pro Pro Cys Arg Asp Glu Leu Thr Lys Asn
       610                 615                 620
   Gln Val Ser Leu Trp Cys Leu Val Lys Gly Phe Tyr Pro Ser Asp Ile
   625                 630                 635                 640
   Ala Val Glu Trp Glu Ser Asn Gly Gln Pro Glu Asn Asn Tyr Lys Thr
                   645                 650                 655
   Thr Pro Pro Val Leu Asp Ser Asp Gly Ser Phe Phe Leu Tyr Ser Lys
               660                 665                 670
   Leu Thr Val Asp Lys Ser Arg Trp Gln Gln Gly Asn Val Phe Ser Cys
           675                 680                 685
   Ser Val Met His Glu Ala Leu His Asn His Tyr Thr Gln Lys Ser Leu
   690 695 700
   Ser Leu Ser Pro Gly Lys
   705 710
<210> 81
   <211> 291
   <212> PRT
   <213> Artificial Sequence
<220>
   <223> Monomeric hu 4-1BBL (71-248) - CH1
<400> 81
   Arg Glu Gly Pro Glu Leu Ser Pro Asp Asp Pro Ala Gly Leu Leu Asp
   1 5 10 15
   Leu Arg Gln Gly Met Phe Ala Gln Leu Val Ala Gln Asn Val Leu Leu
   20 25 30
   Ile Asp Gly Pro Leu Ser Trp Tyr Ser Asp Pro Gly Leu Ala Gly Val
   35 40 45
   Ser Leu Thr Gly Gly Leu Ser Tyr Lys Glu Asp Thr Lys Glu Leu Val
   50 55 60
   Val Ala Lys Ala Gly Val Tyr Tyr Val Phe Phe Gln Leu Glu Leu Arg
   65 70 75 80
   Arg Val Val Ala Gly Glu Gly Ser Gly Ser Val Ser Leu Ala Leu His
   85 90 95
   Leu Gln Pro Leu Arg Ser Ala Ala Gly Ala Ala Ala Leu Ala Leu Thr
   100 105 110
   Val Asp Leu Pro Pro Ala Ser Ser Glu Ala Arg Asn Ser Ala Phe Gly
   115 120 125
   Phe Gln Gly Arg Leu Leu His Leu Ser Ala Gly Gln Arg Leu Gly Val
   130 135 140
   His Leu His Thr Glu Ala Arg Ala Arg His Ala Trp Gln Leu Thr Gln
   145 150 155 160
   Gly Ala Thr Val Leu Gly Leu Phe Arg Val Thr Pro Glu Ile Pro Ala
   165 170 175
   Gly Leu Gly Gly Gly Gly Ser Gly Gly Gly Gly Ser Ala Ser Thr Lys
   180 185 190
   Gly Pro Ser Val Phe Pro Leu Ala Pro Ser Ser Lys Ser Thr Ser Gly
   195 200 205
   Gly Thr Ala Ala Leu Gly Cys Leu Val Lys Asp Tyr Phe Pro Glu Pro
   210 215 220
   Val Thr Val Ser Trp Asn Ser Gly Ala Leu Thr Ser Gly Val His Thr
   225 230 235 240
   Phe Pro Ala Val Leu Gln Ser Ser Gly Leu Tyr Ser Leu Ser Ser Val
   245 250 255
   Val Thr Val Pro Ser Ser Ser Leu Gly Thr Gln Thr Tyr Ile Cys Asn
   260 265 270
   Val Asn His Lys Pro Ser Asn Thr Lys Val Asp Lys Lys Val Glu Pro
   275 280 285
   Lys Ser Cys
   290
<210> 82
   <211> 450
   <212> PRT
   <213> Artificial Sequence
<220>
   <223> anti-PD1 (0314) Fc hole chain
<400> 82
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Ser Phe Ser Ser Tyr
               20                  25                  30
   Thr Met Ser Trp Val Arg Gln Ala Pro Gly Lys Gly Leu Glu Trp Val
           35                  40                  45
   Ala Thr Ile Ser Gly Gly Gly Arg Asp Ile Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Leu Leu Thr Gly Arg Val Tyr Phe Ala Leu Asp Ser Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Ala Ala Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Gly Ala Pro Ile Glu
                   325                 330                 335
   Lys Thr Ile Ser Lys Ala Lys Gly Gln Pro Arg Glu Pro Gln Val Cys
               340                 345                 350
   Thr Leu Pro Pro Ser Arg Asp Glu Leu Thr Lys Asn Gln Val Ser Leu
           355                 360                 365
   Ser Cys Ala Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Val Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 83
   <211> 218
   <212> PRT
   <213> Artificial Sequence
<220>
   <223> anti-PD1 (0314) light chain
<400> 83
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Glu Ser Val Asp Thr Ser
   20 25 30
   Asp Asn Ser Phe Ile His Trp Tyr Gln Gln Lys Pro Gly Gln Ser Pro
   35 40 45
   Arg Leu Leu Ile Tyr Arg Ser Ser Thr Leu Glu Ser Gly Ile Pro Ala
   50 55 60
   Arg Phe Ser Gly Ser Gly Ser Gly Thr Asp Phe Thr Leu Thr Ile Ser
   65 70 75 80
   Ser Leu Glu Pro Glu Asp Phe Ala Val Tyr Tyr Cys Gln Gln Asn Tyr
   85 90 95
   Asp Val Pro Trp Thr Phe Gly Gln Gly Thr Lys Val Glu Ile Lys Arg
   100 105 110
   Thr Val Ala Ala Pro Ser Val Phe Ile Phe Pro Pro Ser Asp Glu Gln
   115 120 125
   Leu Lys Ser Gly Thr Ala Ser Val Val Cys Leu Leu Asn Asn Phe Tyr
   130 135 140
   Pro Arg Glu Ala Lys Val Gln Trp Lys Val Asp Asn Ala Leu Gln Ser
   145 150 155 160
   Gly Asn Ser Gln Glu Ser Val Thr Glu Gln Asp Ser Lys Asp Ser Thr
   165 170 175
   Tyr Ser Leu Ser Ser Thr Leu Thr Leu Ser Lys Ala Asp Tyr Glu Lys
   180 185 190
   His Lys Val Tyr Ala Cys Glu Val Thr His Gln Gly Leu Ser Ser Pro
   195 200 205
   Val Thr Lys Ser Phe Asn Arg Gly Glu Cys
   210 215
<210> 84
   <211> 837
   <212> PRT
   <213> Artificial Sequence
<220>
   <223> anti-PD1(0314) Fc hole chain fused to dimeric hu 4-1BBL (71-254)
<400> 84
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Ser Phe Ser Ser Tyr
               20                  25                  30
   Thr Met Ser Trp Val Arg Gln Ala Pro Gly Lys Gly Leu Glu Trp Val
           35                  40                  45
   Ala Thr Ile Ser Gly Gly Gly Arg Asp Ile Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
   85 90 95
   Val Leu Leu Thr Gly Arg Val Tyr Phe Ala Leu Asp Ser Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Lys Val Glu Pro Lys Ser Cys Asp
   210 215 220
   Lys Thr His Thr Cys Pro Pro Cys Pro Ala Pro Glu Ala Ala Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Gly Ala Pro Ile Glu
   325 330 335
   Lys Thr Ile Ser Lys Ala Lys Gly Gln Pro Arg Glu Pro Gln Val Cys
   340 345 350
   Thr Leu Pro Pro Ser Arg Asp Glu Leu Thr Lys Asn Gln Val Ser Leu
   355 360 365
   Ser Cys Ala Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Val Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Gly Gly Gly Gly Ser Gly Gly Gly Gly Ser Arg Glu Gly Pro Glu
   450 455 460
   Leu Ser Pro Asp Asp Pro Ala Gly Leu Leu Asp Leu Arg Gln Gly Met
   465 470 475 480
   Phe Ala Gln Leu Val Ala Gln Asn Val Leu Leu Ile Asp Gly Pro Leu
   485 490 495
   Ser Trp Tyr Ser Asp Pro Gly Leu Ala Gly Val Ser Leu Thr Gly Gly
   500 505 510
   Leu Ser Tyr Lys Glu Asp Thr Lys Glu Leu Val Val Ala Lys Ala Gly
   515 520 525
   Val Tyr Tyr Val Phe Phe Gln Leu Glu Leu Arg Arg Val Val Ala Gly
   530 535 540
   Glu Gly Ser Gly Ser Val Ser Leu Ala Leu His Leu Gln Pro Leu Arg
   545 550 555 560
   Ser Ala Ala Gly Ala Ala Ala Leu Ala Leu Thr Val Asp Leu Pro Pro
   565 570 575
   Ala Ser Ser Glu Ala Arg Asn Ser Ala Phe Gly Phe Gln Gly Arg Leu
   580 585 590
   Leu His Leu Ser Ala Gly Gln Arg Leu Gly Val His Leu His Thr Glu
   595 600 605
   Ala Arg Ala Arg His Ala Trp Gln Leu Thr Gln Gly Ala Thr Val Leu
   610 615 620
   Gly Leu Phe Arg Val Thr Pro Glu Ile Pro Ala Gly Leu Pro Ser Pro
   625 630 635 640
   Arg Ser Glu Gly Gly Gly Gly Ser Gly Gly Gly Gly Ser Arg Glu Gly
   645 650 655
   Pro Glu Leu Ser Pro Asp Asp Pro Ala Gly Leu Leu Asp Leu Arg Gln
   660 665 670
   Gly Met Phe Ala Gln Leu Val Ala Gln Asn Val Leu Leu Ile Asp Gly
   675 680 685
   Pro Leu Ser Trp Tyr Ser Asp Pro Gly Leu Ala Gly Val Ser Leu Thr
   690 695 700
   Gly Gly Leu Ser Tyr Lys Glu Asp Thr Lys Glu Leu Val Val Ala Lys
   705 710 715 720
   Ala Gly Val Tyr Tyr Val Phe Phe Gln Leu Glu Leu Arg Arg Val Val
   725 730 735
   Ala Gly Glu Gly Ser Gly Ser Val Ser Leu Ala Leu His Leu Gln Pro
   740 745 750
   Leu Arg Ser Ala Ala Gly Ala Ala Ala Leu Ala Leu Thr Val Asp Leu
   755 760 765
   Pro Pro Ala Ser Ser Glu Ala Arg Asn Ser Ala Phe Gly Phe Gln Gly
   770 775 780
   Arg Leu Leu His Leu Ser Ala Gly Gln Arg Leu Gly Val His Leu His
   785 790 795 800
   Thr Glu Ala Arg Ala Arg His Ala Trp Gln Leu Thr Gln Gly Ala Thr
   805 810 815
   Val Leu Gly Leu Phe Arg Val Thr Pro Glu Ile Pro Ala Gly Leu Pro
   820 825 830
   Ser Pro Arg Ser Glu
   835
<210> 85
   <211> 643
   <212> PRT
   <213> Artificial Sequence
<220>
   <223> anti-PD1(0314) Fc knob chain fused to monomeric hu 4-1BBL (71-254)
<400> 85
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Ser Phe Ser Ser Tyr
               20                  25                  30
   Thr Met Ser Trp Val Arg Gln Ala Pro Gly Lys Gly Leu Glu Trp Val
           35                  40                  45
   Ala Thr Ile Ser Gly Gly Gly Arg Asp Ile Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Leu Leu Thr Gly Arg Val Tyr Phe Ala Leu Asp Ser Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
               195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Ala Ala Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Gly Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Cys Arg Asp Glu Leu Thr Lys Asn Gln Val Ser Leu
           355                 360                 365
   Trp Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Gly Gly Gly Gly Ser Gly Gly Gly Gly Ser Arg Glu Gly Pro Glu
   450 455 460
   Leu Ser Pro Asp Asp Pro Ala Gly Leu Leu Asp Leu Arg Gln Gly Met
   465 470 475 480
   Phe Ala Gln Leu Val Ala Gln Asn Val Leu Leu Ile Asp Gly Pro Leu
   485 490 495
   Ser Trp Tyr Ser Asp Pro Gly Leu Ala Gly Val Ser Leu Thr Gly Gly
   500 505 510
   Leu Ser Tyr Lys Glu Asp Thr Lys Glu Leu Val Val Ala Lys Ala Gly
   515 520 525
   Val Tyr Tyr Val Phe Phe Gln Leu Glu Leu Arg Arg Val Val Ala Gly
   530 535 540
   Glu Gly Ser Gly Ser Val Ser Leu Ala Leu His Leu Gln Pro Leu Arg
   545 550 555 560
   Ser Ala Ala Gly Ala Ala Ala Leu Ala Leu Thr Val Asp Leu Pro Pro
   565 570 575
   Ala Ser Ser Glu Ala Arg Asn Ser Ala Phe Gly Phe Gln Gly Arg Leu
   580 585 590
   Leu His Leu Ser Ala Gly Gln Arg Leu Gly Val His Leu His Thr Glu
   595 600 605
   Ala Arg Ala Arg His Ala Trp Gln Leu Thr Gln Gly Ala Thr Val Leu
   610 615 620
   Gly Leu Phe Arg Val Thr Pro Glu Ile Pro Ala Gly Leu Pro Ser Pro
   625 630 635 640
   Arg Ser Glu
<210> 86
   <211> 825
   <212> PRT
   <213> Artificial Sequence
<220>
   <223> anti-PD1(0314) Fc hole chain fused to dimeric hu 4-1BBL (71-248)
<400> 86
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Ser Phe Ser Ser Tyr
               20                  25                  30
   Thr Met Ser Trp Val Arg Gln Ala Pro Gly Lys Gly Leu Glu Trp Val
           35                  40                  45
   Ala Thr Ile Ser Gly Gly Gly Arg Asp Ile Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Leu Leu Thr Gly Arg Val Tyr Phe Ala Leu Asp Ser Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Ala Ala Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Gly Ala Pro Ile Glu
   325 330 335
   Lys Thr Ile Ser Lys Ala Lys Gly Gln Pro Arg Glu Pro Gln Val Cys
   340 345 350
   Thr Leu Pro Pro Ser Arg Asp Glu Leu Thr Lys Asn Gln Val Ser Leu
   355 360 365
   Ser Cys Ala Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Val Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Gly Gly Gly Gly Ser Gly Gly Gly Gly Ser Arg Glu Gly Pro Glu
   450 455 460
   Leu Ser Pro Asp Asp Pro Ala Gly Leu Leu Asp Leu Arg Gln Gly Met
   465 470 475 480
   Phe Ala Gln Leu Val Ala Gln Asn Val Leu Leu Ile Asp Gly Pro Leu
   485 490 495
   Ser Trp Tyr Ser Asp Pro Gly Leu Ala Gly Val Ser Leu Thr Gly Gly
   500 505 510
   Leu Ser Tyr Lys Glu Asp Thr Lys Glu Leu Val Val Ala Lys Ala Gly
   515 520 525
   Val Tyr Tyr Val Phe Phe Gln Leu Glu Leu Arg Arg Val Val Ala Gly
   530 535 540
   Glu Gly Ser Gly Ser Val Ser Leu Ala Leu His Leu Gln Pro Leu Arg
   545 550 555 560
   Ser Ala Ala Gly Ala Ala Ala Leu Ala Leu Thr Val Asp Leu Pro Pro
   565 570 575
   Ala Ser Ser Glu Ala Arg Asn Ser Ala Phe Gly Phe Gln Gly Arg Leu
   580 585 590
   Leu His Leu Ser Ala Gly Gln Arg Leu Gly Val His Leu His Thr Glu
   595 600 605
   Ala Arg Ala Arg His Ala Trp Gln Leu Thr Gln Gly Ala Thr Val Leu
   610 615 620
   Gly Leu Phe Arg Val Thr Pro Glu Ile Pro Ala Gly Leu Gly Gly Gly
   625 630 635 640
   Gly Ser Gly Gly Gly Gly Ser Arg Glu Gly Pro Glu Leu Ser Pro Asp
   645 650 655
   Asp Pro Ala Gly Leu Leu Asp Leu Arg Gln Gly Met Phe Ala Gln Leu
   660 665 670
   Val Ala Gln Asn Val Leu Leu Ile Asp Gly Pro Leu Ser Trp Tyr Ser
   675 680 685
   Asp Pro Gly Leu Ala Gly Val Ser Leu Thr Gly Gly Leu Ser Tyr Lys
   690 695 700
   Glu Asp Thr Lys Glu Leu Val Val Ala Lys Ala Gly Val Tyr Tyr Val
   705 710 715 720
   Phe Phe Gln Leu Glu Leu Arg Arg Val Val Ala Gly Glu Gly Ser Gly
   725 730 735
   Ser Val Ser Leu Ala Leu His Leu Gln Pro Leu Arg Ser Ala Ala Gly
   740 745 750
   Ala Ala Ala Leu Ala Leu Thr Val Asp Leu Pro Pro Ala Ser Ser Glu
   755 760 765
   Ala Arg Asn Ser Ala Phe Gly Phe Gln Gly Arg Leu Leu His Leu Ser
   770 775 780
   Ala Gly Gln Arg Leu Gly Val His Leu His Thr Glu Ala Arg Ala Arg
   785 790 795 800
   His Ala Trp Gln Leu Thr Gln Gly Ala Thr Val Leu Gly Leu Phe Arg
   805 810 815
   Val Thr Pro Glu Ile Pro Ala Gly Leu
   820 825
<210> 87
   <211> 637
   <212> PRT
   <213> Artificial Sequence
<220>
   <223> anti-PD1(0314) Fc knob chain fused to monomeric hu 4-1BBL (71-248)
<400> 87
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Ser Phe Ser Ser Tyr
   20 25 30
   Thr Met Ser Trp Val Arg Gln Ala Pro Gly Lys Gly Leu Glu Trp Val
   35 40 45
   Ala Thr Ile Ser Gly Gly Gly Arg Asp Ile Tyr Tyr Pro Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Val Leu Leu Thr Gly Arg Val Tyr Phe Ala Leu Asp Ser Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Ala Ala Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Gly Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Cys Arg Asp Glu Leu Thr Lys Asn Gln Val Ser Leu
           355                 360                 365
   Trp Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Gly Gly Gly Gly Ser Gly Gly Gly Gly Ser Arg Glu Gly Pro Glu
   450 455 460
   Leu Ser Pro Asp Asp Pro Ala Gly Leu Leu Asp Leu Arg Gln Gly Met
   465 470 475 480
   Phe Ala Gln Leu Val Ala Gln Asn Val Leu Leu Ile Asp Gly Pro Leu
   485 490 495
   Ser Trp Tyr Ser Asp Pro Gly Leu Ala Gly Val Ser Leu Thr Gly Gly
   500 505 510
   Leu Ser Tyr Lys Glu Asp Thr Lys Glu Leu Val Val Ala Lys Ala Gly
   515 520 525
   Val Tyr Tyr Val Phe Phe Gln Leu Glu Leu Arg Arg Val Val Ala Gly
   530 535 540
   Glu Gly Ser Gly Ser Val Ser Leu Ala Leu His Leu Gln Pro Leu Arg
   545 550 555 560
   Ser Ala Ala Gly Ala Ala Ala Leu Ala Leu Thr Val Asp Leu Pro Pro
   565 570 575
   Ala Ser Ser Glu Ala Arg Asn Ser Ala Phe Gly Phe Gln Gly Arg Leu
   580 585 590
   Leu His Leu Ser Ala Gly Gln Arg Leu Gly Val His Leu His Thr Glu
   595 600 605
   Ala Arg Ala Arg His Ala Trp Gln Leu Thr Gln Gly Ala Thr Val Leu
   610 615 620
   Gly Leu Phe Arg Val Thr Pro Glu Ile Pro Ala Gly Leu
   625 630 635
<210> 88
   <211> 602
   <212> PRT
   <213> Artificial Sequence
<220>
   <223> Fc hole dimeric ligand (71-248) chain
<400> 88
   Asp Lys Thr His Thr Cys Pro Pro Cys Pro Ala Pro Glu Ala Ala Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Gly Ala Pro Ile
               100                 105                 110
   Glu Lys Thr Ile Ser Lys Ala Lys Gly Gln Pro Arg Glu Pro Gln Val
           115                 120                 125
   Cys Thr Leu Pro Pro Ser Arg Asp Glu Leu Thr Lys Asn Gln Val Ser
       130                 135                 140
   Leu Ser Cys Ala Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Val Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
   210 215 220
   Pro Gly Gly Gly Gly Gly Ser Gly Gly Gly Gly Ser Arg Glu Gly Pro
   225 230 235 240
   Glu Leu Ser Pro Asp Asp Pro Ala Gly Leu Leu Asp Leu Arg Gln Gly
   245 250 255
   Met Phe Ala Gln Leu Val Ala Gln Asn Val Leu Leu Ile Asp Gly Pro
   260 265 270
   Leu Ser Trp Tyr Ser Asp Pro Gly Leu Ala Gly Val Ser Leu Thr Gly
   275 280 285
   Gly Leu Ser Tyr Lys Glu Asp Thr Lys Glu Leu Val Val Ala Lys Ala
   290 295 300
   Gly Val Tyr Tyr Val Phe Phe Gln Leu Glu Leu Arg Arg Val Val Ala
   305 310 315 320
   Gly Glu Gly Ser Gly Ser Val Ser Leu Ala Leu His Leu Gln Pro Leu
   325 330 335
   Arg Ser Ala Ala Gly Ala Ala Ala Leu Ala Leu Thr Val Asp Leu Pro
   340 345 350
   Pro Ala Ser Ser Glu Ala Arg Asn Ser Ala Phe Gly Phe Gln Gly Arg
   355 360 365
   Leu Leu His Leu Ser Ala Gly Gln Arg Leu Gly Val His Leu His Thr
   370 375 380
   Glu Ala Arg Ala Arg His Ala Trp Gln Leu Thr Gln Gly Ala Thr Val
   385 390 395 400
   Leu Gly Leu Phe Arg Val Thr Pro Glu Ile Pro Ala Gly Leu Gly Gly
   405 410 415
   Gly Gly Ser Gly Gly Gly Gly Ser Arg Glu Gly Pro Glu Leu Ser Pro
   420 425 430
   Asp Asp Pro Ala Gly Leu Leu Asp Leu Arg Gln Gly Met Phe Ala Gln
   435 440 445
   Leu Val Ala Gln Asn Val Leu Leu Ile Asp Gly Pro Leu Ser Trp Tyr
   450 455 460
   Ser Asp Pro Gly Leu Ala Gly Val Ser Leu Thr Gly Gly Leu Ser Tyr
   465 470 475 480
   Lys Glu Asp Thr Lys Glu Leu Val Val Ala Lys Ala Gly Val Tyr Tyr
   485 490 495
   Val Phe Phe Gln Leu Glu Leu Arg Arg Val Val Ala Gly Glu Gly Ser
   500 505 510
   Gly Ser Val Ser Leu Ala Leu His Leu Gln Pro Leu Arg Ser Ala Ala
   515 520 525
   Gly Ala Ala Ala Leu Ala Leu Thr Val Asp Leu Pro Pro Ala Ser Ser
   530 535 540
   Glu Ala Arg Asn Ser Ala Phe Gly Phe Gln Gly Arg Leu Leu His Leu
   545 550 555 560
   Ser Ala Gly Gln Arg Leu Gly Val His Leu His Thr Glu Ala Arg Ala
   565 570 575
   Arg His Ala Trp Gln Leu Thr Gln Gly Ala Thr Val Leu Gly Leu Phe
   580 585 590
   Arg Val Thr Pro Glu Ile Pro Ala Gly Leu
   595 600
<210> 89
   <211> 404
   <212> PRT
   <213> Artificial Sequence
<220>
   <223> Fc hole monomeric ligand (71-248) chain
<400> 89
   Asp Lys Thr His Thr Cys Pro Pro Cys Pro Ala Pro Glu Ala Ala Gly
   1 5 10 15
   Gly Pro Ser Val Phe Leu Phe Pro Pro Lys Pro Lys Asp Thr Leu Met
   20 25 30
   Ile Ser Arg Thr Pro Glu Val Thr Cys Val Val Val Asp Val Ser His
   35 40 45
   Glu Asp Pro Glu Val Lys Phe Asn Trp Tyr Val Asp Gly Val Glu Val
   50 55 60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Gly Ala Pro Ile
               100                 105                 110
   Glu Lys Thr Ile Ser Lys Ala Lys Gly Gln Pro Arg Glu Pro Gln Val
           115                 120                 125
   Cys Thr Leu Pro Pro Ser Arg Asp Glu Leu Thr Lys Asn Gln Val Ser
       130                 135                 140
   Leu Ser Cys Ala Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Val Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Arg Glu Gly Pro Glu Leu Ser Pro Asp Asp Pro Ala Gly Leu
   225                 230                 235                 240
   Leu Asp Leu Arg Gln Gly Met Phe Ala Gln Leu Val Ala Gln Asn Val
                   245                 250                 255
   Leu Leu Ile Asp Gly Pro Leu Ser Trp Tyr Ser Asp Pro Gly Leu Ala
               260                 265                 270
   Gly Val Ser Leu Thr Gly Gly Leu Ser Tyr Lys Glu Asp Thr Lys Glu
           275                 280                 285
   Leu Val Val Ala Lys Ala Gly Val Tyr Tyr Val Phe Phe Gln Leu Glu
       290                 295                 300
   Leu Arg Arg Val Val Ala Gly Glu Gly Ser Gly Ser Val Ser Leu Ala
   305                 310                 315                 320
   Leu His Leu Gln Pro Leu Arg Ser Ala Ala Gly Ala Ala Ala Leu Ala
   325 330 335
   Leu Thr Val Asp Leu Pro Pro Ala Ser Ser Glu Ala Arg Asn Ser Ala
   340 345 350
   Phe Gly Phe Gln Gly Arg Leu Leu His Leu Ser Ala Gly Gln Arg Leu
   355 360 365
   Gly Val His Leu His Thr Glu Ala Arg Ala Arg His Ala Trp Gln Leu
   370 375 380
   Thr Gln Gly Ala Thr Val Leu Gly Leu Phe Arg Val Thr Pro Glu Ile
   385 390 395 400
   Pro Ala Gly Leu
<210> 90
   <211> 815
   <212> PRT
   <213> Artificial Sequence
<220>
   <223> anti-PD1(0314) Fc knob chain fused to dimeric hu 4-1BBL (71-248)
<400> 90
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Ser Phe Ser Ser Tyr
   20 25 30
   Thr Met Ser Trp Val Arg Gln Ala Pro Gly Lys Gly Leu Glu Trp Val
   35 40 45
   Ala Thr Ile Ser Gly Gly Gly Arg Asp Ile Tyr Tyr Pro Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Val Leu Leu Thr Gly Arg Val Tyr Phe Ala Leu Asp Ser Trp Gly Gln
   100 105 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Ala Ala Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Gly Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Cys Arg Asp Glu Leu Thr Lys Asn Gln Val Ser Leu
           355                 360                 365
   Trp Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Arg Glu Gly Pro Glu Leu Ser Pro Asp Asp Pro Ala Gly Leu Leu
   450 455 460
   Asp Leu Arg Gln Gly Met Phe Ala Gln Leu Val Ala Gln Asn Val Leu
   465 470 475 480
   Leu Ile Asp Gly Pro Leu Ser Trp Tyr Ser Asp Pro Gly Leu Ala Gly
   485 490 495
   Val Ser Leu Thr Gly Gly Leu Ser Tyr Lys Glu Asp Thr Lys Glu Leu
   500 505 510
   Val Val Ala Lys Ala Gly Val Tyr Tyr Val Phe Phe Gln Leu Glu Leu
   515 520 525
   Arg Arg Val Val Ala Gly Glu Gly Ser Gly Ser Val Ser Leu Ala Leu
   530 535 540
   His Leu Gln Pro Leu Arg Ser Ala Ala Gly Ala Ala Ala Leu Ala Leu
   545 550 555 560
   Thr Val Asp Leu Pro Pro Ala Ser Ser Glu Ala Arg Asn Ser Ala Phe
   565 570 575
   Gly Phe Gln Gly Arg Leu Leu His Leu Ser Ala Gly Gln Arg Leu Gly
   580 585 590
   Val His Leu His Thr Glu Ala Arg Ala Arg His Ala Trp Gln Leu Thr
   595 600 605
   Gln Gly Ala Thr Val Leu Gly Leu Phe Arg Val Thr Pro Glu Ile Pro
   610 615 620
   Ala Gly Leu Gly Gly Gly Gly Ser Gly Gly Gly Gly Ser Arg Glu Gly
   625 630 635 640
   Pro Glu Leu Ser Pro Asp Asp Pro Ala Gly Leu Leu Asp Leu Arg Gln
   645 650 655
   Gly Met Phe Ala Gln Leu Val Ala Gln Asn Val Leu Leu Ile Asp Gly
   660 665 670
   Pro Leu Ser Trp Tyr Ser Asp Pro Gly Leu Ala Gly Val Ser Leu Thr
   675 680 685
   Gly Gly Leu Ser Tyr Lys Glu Asp Thr Lys Glu Leu Val Val Ala Lys
   690 695 700
   Ala Gly Val Tyr Tyr Val Phe Phe Gln Leu Glu Leu Arg Arg Val Val
   705 710 715 720
   Ala Gly Glu Gly Ser Gly Ser Val Ser Leu Ala Leu His Leu Gln Pro
   725 730 735
   Leu Arg Ser Ala Ala Gly Ala Ala Ala Leu Ala Leu Thr Val Asp Leu
   740 745 750
   Pro Pro Ala Ser Ser Glu Ala Arg Asn Ser Ala Phe Gly Phe Gln Gly
   755 760 765
   Arg Leu Leu His Leu Ser Ala Gly Gln Arg Leu Gly Val His Leu His
   770 775 780
   Thr Glu Ala Arg Ala Arg His Ala Trp Gln Leu Thr Gln Gly Ala Thr
   785 790 795 800
   Val Leu Gly Leu Phe Arg Val Thr Pro Glu Ile Pro Ala Gly Leu
   805 810 815
<210> 91
   <211> 414
   <212> PRT
   <213> Artificial Sequence
<220>
   <223> Fc knob chain fused to monomeric hu 4-1BBL (71-248)
<400> 91
   Asp Lys Thr His Thr Cys Pro Pro Cys Pro Ala Pro Glu Ala Ala Gly
   1               5                   10                  15
   Gly Pro Ser Val Phe Leu Phe Pro Pro Lys Pro Lys Asp Thr Leu Met
   20 25 30
   Ile Ser Arg Thr Pro Glu Val Thr Cys Val Val Val Asp Val Ser His
   35 40 45
   Glu Asp Pro Glu Val Lys Phe Asn Trp Tyr Val Asp Gly Val Glu Val
   50 55 60
   His Asn Ala Lys Thr Lys Pro Arg Glu Glu Gln Tyr Asn Ser Thr Tyr
   65 70 75 80
   Arg Val Val Ser Val Leu Thr Val Leu His Gln Asp Trp Leu Asn Gly
   85 90 95
   Lys Glu Tyr Lys Cys Lys Val Ser Asn Lys Ala Leu Gly Ala Pro Ile
   100 105 110
   Glu Lys Thr Ile Ser Lys Ala Lys Gly Gln Pro Arg Glu Pro Gln Val
   115 120 125
   Tyr Thr Leu Pro Pro Cys Arg Asp Glu Leu Thr Lys Asn Gln Val Ser
   130 135 140
   Leu Trp Cys Leu Val Lys Gly Phe Tyr Pro Ser Asp Ile Ala Val Glu
   145 150 155 160
   Trp Glu Ser Asn Gly Gln Pro Glu Asn Asn Tyr Lys Thr Thr Pro Pro
   165 170 175
   Val Leu Asp Ser Asp Gly Ser Phe Phe Leu Tyr Ser Lys Leu Thr Val
   180 185 190
   Asp Lys Ser Arg Trp Gln Gln Gly Asn Val Phe Ser Cys Ser Val Met
   195 200 205
   His Glu Ala Leu His Asn His Tyr Thr Gln Lys Ser Leu Ser Leu Ser
   210 215 220
   Pro Gly Gly Gly Gly Gly Ser Gly Gly Gly Gly Ser Arg Glu Gly Pro
   225 230 235 240
   Glu Leu Ser Pro Asp Asp Pro Ala Gly Leu Leu Asp Leu Arg Gln Gly
   245 250 255
   Met Phe Ala Gln Leu Val Ala Gln Asn Val Leu Leu Ile Asp Gly Pro
                   260                 265                 270
   Leu Ser Trp Tyr Ser Asp Pro Gly Leu Ala Gly Val Ser Leu Thr Gly
           275                 280                 285
   Gly Leu Ser Tyr Lys Glu Asp Thr Lys Glu Leu Val Val Ala Lys Ala
       290                 295                 300
   Gly Val Tyr Tyr Val Phe Phe Gln Leu Glu Leu Arg Arg Val Val Ala
   305                 310                 315                 320
   Gly Glu Gly Ser Gly Ser Val Ser Leu Ala Leu His Leu Gln Pro Leu
                   325                 330                 335
   Arg Ser Ala Ala Gly Ala Ala Ala Leu Ala Leu Thr Val Asp Leu Pro
               340                 345                 350
   Pro Ala Ser Ser Glu Ala Arg Asn Ser Ala Phe Gly Phe Gln Gly Arg
           355                 360                 365
   Leu Leu His Leu Ser Ala Gly Gln Arg Leu Gly Val His Leu His Thr
       370                 375                 380
   Glu Ala Arg Ala Arg His Ala Trp Gln Leu Thr Gln Gly Ala Thr Val
   385                 390                 395                 400
   Leu Gly Leu Phe Arg Val Thr Pro Glu Ile Pro Ala Gly Leu
                   405                 410
<210> 92
   <211> 637
   <212> PRT
   <213> Artificial Sequence
<220>
   <223> anti-PD1(0314) Fc hole monomeric ligand (71-248) chain
<400> 92
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Ser Phe Ser Ser Tyr
               20                  25                  30
   Thr Met Ser Trp Val Arg Gln Ala Pro Gly Lys Gly Leu Glu Trp Val
           35                  40                  45
   Ala Thr Ile Ser Gly Gly Gly Arg Asp Ile Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Leu Leu Thr Gly Arg Val Tyr Phe Ala Leu Asp Ser Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Ala Ala Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Gly Ala Pro Ile Glu
   325 330 335
   Lys Thr Ile Ser Lys Ala Lys Gly Gln Pro Arg Glu Pro Gln Val Cys
   340 345 350
   Thr Leu Pro Pro Ser Arg Asp Glu Leu Thr Lys Asn Gln Val Ser Leu
   355 360 365
   Ser Cys Ala Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Val Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Gly Gly Gly Gly Ser Gly Gly Gly Gly Ser Arg Glu Gly Pro Glu
   450 455 460
   Leu Ser Pro Asp Asp Pro Ala Gly Leu Leu Asp Leu Arg Gln Gly Met
   465 470 475 480
   Phe Ala Gln Leu Val Ala Gln Asn Val Leu Leu Ile Asp Gly Pro Leu
   485 490 495
   Ser Trp Tyr Ser Asp Pro Gly Leu Ala Gly Val Ser Leu Thr Gly Gly
   500 505 510
   Leu Ser Tyr Lys Glu Asp Thr Lys Glu Leu Val Val Ala Lys Ala Gly
   515 520 525
   Val Tyr Tyr Val Phe Phe Gln Leu Glu Leu Arg Arg Val Val Ala Gly
   530 535 540
   Glu Gly Ser Gly Ser Val Ser Leu Ala Leu His Leu Gln Pro Leu Arg
   545 550 555 560
   Ser Ala Ala Gly Ala Ala Ala Leu Ala Leu Thr Val Asp Leu Pro Pro
   565 570 575
   Ala Ser Ser Glu Ala Arg Asn Ser Ala Phe Gly Phe Gln Gly Arg Leu
   580 585 590
   Leu His Leu Ser Ala Gly Gln Arg Leu Gly Val His Leu His Thr Glu
   595 600 605
   Ala Arg Ala Arg His Ala Trp Gln Leu Thr Gln Gly Ala Thr Val Leu
   610 615 620
   Gly Leu Phe Arg Val Thr Pro Glu Ile Pro Ala Gly Leu
   625 630 635
<210> 93
   <211> 602
   <212> PRT
   <213> Artificial Sequence
<220>
   <223> Fc knob chain fused to dimeric hu 4-1BBL (71-248)
<400> 93
   Asp Lys Thr His Thr Cys Pro Pro Cys Pro Ala Pro Glu Ala Ala Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Gly Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Cys Arg Asp Glu Leu Thr Lys Asn Gln Val Ser
   130 135 140
   Leu Trp Cys Leu Val Lys Gly Phe Tyr Pro Ser Asp Ile Ala Val Glu
   145 150 155 160
   Trp Glu Ser Asn Gly Gln Pro Glu Asn Asn Tyr Lys Thr Thr Pro Pro
   165 170 175
   Val Leu Asp Ser Asp Gly Ser Phe Phe Leu Tyr Ser Lys Leu Thr Val
   180 185 190
   Asp Lys Ser Arg Trp Gln Gln Gly Asn Val Phe Ser Cys Ser Val Met
   195 200 205
   His Glu Ala Leu His Asn His Tyr Thr Gln Lys Ser Leu Ser Leu Ser
   210 215 220
   Pro Gly Gly Gly Gly Gly Ser Gly Gly Gly Gly Ser Arg Glu Gly Pro
   225 230 235 240
   Glu Leu Ser Pro Asp Asp Pro Ala Gly Leu Leu Asp Leu Arg Gln Gly
   245 250 255
   Met Phe Ala Gln Leu Val Ala Gln Asn Val Leu Leu Ile Asp Gly Pro
   260 265 270
   Leu Ser Trp Tyr Ser Asp Pro Gly Leu Ala Gly Val Ser Leu Thr Gly
   275 280 285
   Gly Leu Ser Tyr Lys Glu Asp Thr Lys Glu Leu Val Val Ala Lys Ala
   290 295 300
   Gly Val Tyr Tyr Val Phe Phe Gln Leu Glu Leu Arg Arg Val Val Ala
   305 310 315 320
   Gly Glu Gly Ser Gly Ser Val Ser Leu Ala Leu His Leu Gln Pro Leu
   325 330 335
   Arg Ser Ala Ala Gly Ala Ala Ala Leu Ala Leu Thr Val Asp Leu Pro
   340 345 350
   Pro Ala Ser Ser Glu Ala Arg Asn Ser Ala Phe Gly Phe Gln Gly Arg
   355 360 365
   Leu Leu His Leu Ser Ala Gly Gln Arg Leu Gly Val His Leu His Thr
   370 375 380
   Glu Ala Arg Ala Arg His Ala Trp Gln Leu Thr Gln Gly Ala Thr Val
   385 390 395 400
   Leu Gly Leu Phe Arg Val Thr Pro Glu Ile Pro Ala Gly Leu Gly Gly
   405 410 415
   Gly Gly Ser Gly Gly Gly Gly Ser Arg Glu Gly Pro Glu Leu Ser Pro
   420 425 430
   Asp Asp Pro Ala Gly Leu Leu Asp Leu Arg Gln Gly Met Phe Ala Gln
   435 440 445
   Leu Val Ala Gln Asn Val Leu Leu Ile Asp Gly Pro Leu Ser Trp Tyr
   450 455 460
   Ser Asp Pro Gly Leu Ala Gly Val Ser Leu Thr Gly Gly Leu Ser Tyr
   465 470 475 480
   Lys Glu Asp Thr Lys Glu Leu Val Val Ala Lys Ala Gly Val Tyr Tyr
   485 490 495
   Val Phe Phe Gln Leu Glu Leu Arg Arg Val Val Ala Gly Glu Gly Ser
   500 505 510
   Gly Ser Val Ser Leu Ala Leu His Leu Gln Pro Leu Arg Ser Ala Ala
   515 520 525
   Gly Ala Ala Ala Leu Ala Leu Thr Val Asp Leu Pro Pro Ala Ser Ser
   530 535 540
   Glu Ala Arg Asn Ser Ala Phe Gly Phe Gln Gly Arg Leu Leu His Leu
   545 550 555 560
   Ser Ala Gly Gln Arg Leu Gly Val His Leu His Thr Glu Ala Arg Ala
   565 570 575
   Arg His Ala Trp Gln Leu Thr Gln Gly Ala Thr Val Leu Gly Leu Phe
   580 585 590
   Arg Val Thr Pro Glu Ile Pro Ala Gly Leu
   595 600
<210> 94
   <211> 288
   <212> PRT
   <213> Homo sapiens
<400> 94
   Met Gln Ile Pro Gln Ala Pro Trp Pro Val Val Trp Ala Val Leu Gln
   1               5                   10                  15
   Leu Gly Trp Arg Pro Gly Trp Phe Leu Asp Ser Pro Asp Arg Pro Trp
               20                  25                  30
   Asn Pro Pro Thr Phe Ser Pro Ala Leu Leu Val Val Thr Glu Gly Asp
           35                  40                  45
   Asn Ala Thr Phe Thr Cys Ser Phe Ser Asn Thr Ser Glu Ser Phe Val
       50                  55                  60
   Leu Asn Trp Tyr Arg Met Ser Pro Ser Asn Gln Thr Asp Lys Leu Ala
   65                  70                  75                  80
   Ala Phe Pro Glu Asp Arg Ser Gln Pro Gly Gln Asp Cys Arg Phe Arg
                   85                  90                  95
   Val Thr Gln Leu Pro Asn Gly Arg Asp Phe His Met Ser Val Val Arg
               100                 105                 110
   Ala Arg Arg Asn Asp Ser Gly Thr Tyr Leu Cys Gly Ala Ile Ser Leu
           115                 120                 125
   Ala Pro Lys Ala Gln Ile Lys Glu Ser Leu Arg Ala Glu Leu Arg Val
       130                 135                 140
   Thr Glu Arg Arg Ala Glu Val Pro Thr Ala His Pro Ser Pro Ser Pro
   145                 150                 155                 160
   Arg Pro Ala Gly Gln Phe Gln Thr Leu Val Val Gly Val Val Gly Gly
                   165                 170                 175
   Leu Leu Gly Ser Leu Val Leu Leu Val Trp Val Leu Ala Val Ile Cys
               180                 185                 190
   Ser Arg Ala Ala Arg Gly Thr Ile Gly Ala Arg Arg Thr Gly Gln Pro
           195                 200                 205
   Leu Lys Glu Asp Pro Ser Ala Val Pro Val Phe Ser Val Asp Tyr Gly
       210                 215                 220
   Glu Leu Asp Phe Gln Trp Arg Glu Lys Thr Pro Glu Pro Pro Val Pro
   225                 230                 235                 240
   Cys Val Pro Glu Gln Thr Glu Tyr Ala Thr Ile Val Phe Pro Ser Gly
                   245                 250                 255
   Met Gly Thr Ser Ser Pro Ala Arg Arg Gly Ser Ala Asp Gly Pro Arg
   260 265 270
   Ser Ala Gln Pro Leu Arg Pro Glu Asp Gly His Cys Ser Trp Pro Leu
   275 280 285
<210> 95
   <211> 205
   <212> PRT
   <213> Homo sapiens
<400> 95
   Met Thr Pro Pro Glu Arg Leu Phe Leu Pro Arg Val Cys Gly Thr Thr
   1 5 10 15
   Leu His Leu Leu Leu Leu Gly Leu Leu Leu Val Leu Leu Pro Gly Ala
   20 25 30
   Gln Gly Leu Pro Gly Val Gly Leu Thr Pro Ser Ala Ala Gln Thr Ala
   35 40 45
   Arg Gln His Pro Lys Met His Leu Ala His Ser Thr Leu Lys Pro Ala
   50 55 60
   Ala His Leu Ile Gly Asp Pro Ser Lys Gln Asn Ser Leu Leu Trp Arg
   65 70 75 80
   Ala Asn Thr Asp Arg Ala Phe Leu Gln Asp Gly Phe Ser Leu Ser Asn
   85 90 95
   Asn Ser Leu Leu Val Pro Thr Ser Gly Ile Tyr Phe Val Tyr Ser Gln
   100 105 110
   Val Val Phe Ser Gly Lys Ala Tyr Ser Pro Lys Ala Thr Ser Ser Pro
   115 120 125
   Leu Tyr Leu Ala His Glu Val Gln Leu Phe Ser Ser Gln Tyr Pro Phe
   130 135 140
   His Val Pro Leu Leu Ser Ser Gln Lys Met Val Tyr Pro Gly Leu Gln
   145 150 155 160
   Glu Pro Trp Leu His Ser Met Tyr His Gly Ala Ala Phe Gln Leu Thr
   165 170 175
   Gln Gly Asp Gln Leu Ser Thr His Thr Asp Gly Ile Pro His Leu Val
   180 185 190
   Leu Ser Pro Ser Thr Val Phe Phe Gly Ala Phe Ala Leu
           195                 200                 205
<210> 96
   <211> 233
   <212> PRT
   <213> Homo sapiens
<400> 96
   Met Ser Thr Glu Ser Met Ile Arg Asp Val Glu Leu Ala Glu Glu Ala
   1               5                   10                  15
   Leu Pro Lys Lys Thr Gly Gly Pro Gln Gly Ser Arg Arg Cys Leu Phe
               20                  25                  30
   Leu Ser Leu Phe Ser Phe Leu Ile Val Ala Gly Ala Thr Thr Leu Phe
           35                  40                  45
   Cys Leu Leu His Phe Gly Val Ile Gly Pro Gln Arg Glu Glu Phe Pro
       50                  55                  60
   Arg Asp Leu Ser Leu Ile Ser Pro Leu Ala Gln Ala Val Arg Ser Ser
   65                  70                  75                  80
   Ser Arg Thr Pro Ser Asp Lys Pro Val Ala His Val Val Ala Asn Pro
                   85                  90                  95
   Gln Ala Glu Gly Gln Leu Gln Trp Leu Asn Arg Arg Ala Asn Ala Leu
               100                 105                 110
   Leu Ala Asn Gly Val Glu Leu Arg Asp Asn Gln Leu Val Val Pro Ser
           115                 120                 125
   Glu Gly Leu Tyr Leu Ile Tyr Ser Gln Val Leu Phe Lys Gly Gln Gly
       130                 135                 140
   Cys Pro Ser Thr His Val Leu Leu Thr His Thr Ile Ser Arg Ile Ala
   145                 150                 155                 160
   Val Ser Tyr Gln Thr Lys Val Asn Leu Leu Ser Ala Ile Lys Ser Pro
                   165                 170                 175
   Cys Gln Arg Glu Thr Pro Glu Gly Ala Glu Ala Lys Pro Trp Tyr Glu
               180                 185                 190
   Pro Ile Tyr Leu Gly Gly Val Phe Gln Leu Glu Lys Gly Asp Arg Leu
           195                 200                 205
   Ser Ala Glu Ile Asn Arg Pro Asp Tyr Leu Asp Phe Ala Glu Ser Gly
   210 215 220
   Gln Val Tyr Phe Gly Ile Ile Ala Leu
   225 230
<210> 97
   <211> 244
   <212> PRT
   <213> Homo sapiens
<400> 97
   Met Gly Ala Leu Gly Leu Glu Gly Arg Gly Gly Arg Leu Gln Gly Arg
   1 5 10 15
   Gly Ser Leu Leu Leu Ala Val Ala Gly Ala Thr Ser Leu Val Thr Leu
   20 25 30
   Leu Leu Ala Val Pro Ile Thr Val Leu Ala Val Leu Ala Leu Val Pro
   35 40 45
   Gln Asp Gln Gly Gly Leu Val Thr Glu Thr Ala Asp Pro Gly Ala Gln
   50 55 60
   Ala Gln Gln Gly Leu Gly Phe Gln Lys Leu Pro Glu Glu Glu Pro Glu
   65 70 75 80
   Thr Asp Leu Ser Pro Gly Leu Pro Ala Ala His Leu Ile Gly Ala Pro
   85 90 95
   Leu Lys Gly Gln Gly Leu Gly Trp Glu Thr Thr Lys Glu Gln Ala Phe
   100 105 110
   Leu Thr Ser Gly Thr Gln Phe Ser Asp Ala Glu Gly Leu Ala Leu Pro
   115 120 125
   Gln Asp Gly Leu Tyr Tyr Leu Tyr Cys Leu Val Gly Tyr Arg Gly Arg
   130 135 140
   Ala Pro Pro Gly Gly Gly Asp Pro Gln Gly Arg Ser Val Thr Leu Arg
   145 150 155 160
   Ser Ser Leu Tyr Arg Ala Gly Gly Ala Tyr Gly Pro Gly Thr Pro Glu
   165 170 175
   Leu Leu Leu Glu Gly Ala Glu Thr Val Thr Pro Val Leu Asp Pro Ala
   180 185 190
   Arg Arg Gln Gly Tyr Gly Pro Leu Trp Tyr Thr Ser Val Gly Phe Gly
   195 200 205
   Gly Leu Val Gln Leu Arg Arg Gly Glu Arg Val Tyr Val Asn Ile Ser
   210 215 220
   His Pro Asp Met Val Asp Phe Ala Arg Gly Lys Thr Phe Phe Gly Ala
   225 230 235 240
   Val Met Val Gly
<210> 98
   <211> 183
   <212> PRT
   <213> Homo sapiens
<400> 98
   Met Glu Arg Val Gln Pro Leu Glu Glu Asn Val Gly Asn Ala Ala Arg
   1 5 10 15
   Pro Arg Phe Glu Arg Asn Lys Leu Leu Leu Val Ala Ser Val Ile Gln
   20 25 30
   Gly Leu Gly Leu Leu Leu Cys Phe Thr Tyr Ile Cys Leu His Phe Ser
   35 40 45
   Ala Leu Gln Val Ser His Arg Tyr Pro Arg Ile Gln Ser Ile Lys Val
   50 55 60
   Gln Phe Thr Glu Tyr Lys Lys Glu Lys Gly Phe Ile Leu Thr Ser Gln
   65 70 75 80
   Lys Glu Asp Glu Ile Met Lys Val Gln Asn Asn Ser Val Ile Ile Asn
   85 90 95
   Cys Asp Gly Phe Tyr Leu Ile Ser Leu Lys Gly Tyr Phe Ser Gln Glu
   100 105 110
   Val Asn Ile Ser Leu His Tyr Gln Lys Asp Glu Glu Pro Leu Phe Gln
   115 120 125
   Leu Lys Lys Val Arg Ser Val Asn Ser Leu Met Val Ala Ser Leu Thr
   130 135 140
   Tyr Lys Asp Lys Val Tyr Leu Asn Val Thr Thr Asp Asn Thr Ser Leu
   145 150 155 160
   Asp Asp Phe His Val Asn Gly Gly Glu Leu Ile Leu Ile His Gln Asn
   165 170 175
   Pro Gly Glu Phe Cys Val Leu
   180
<210> 99
   <211> 261
   <212> PRT
   <213> Homo sapiens
<400> 99
<210> 100
   <211> 281
   <212> PRT
   <213> Homo sapiens
<400> 100
   Met Gln Gln Pro Phe Asn Tyr Pro Tyr Pro Gln Ile Tyr Trp Val Asp
   1 5 10 15
   Ser Ser Ala Ser Ser Pro Trp Ala Pro Pro Gly Thr Val Leu Pro Cys
   20 25 30
   Pro Thr Ser Val Pro Arg Arg Pro Gly Gln Arg Arg Pro Pro Pro Pro
   35 40 45
   Pro Pro Pro Pro Pro Leu Pro Pro Pro Pro Pro Pro Pro Pro Leu Pro
   50 55 60
   Pro Leu Pro Leu Pro Pro Leu Lys Lys Arg Gly Asn His Ser Thr Gly
   65 70 75 80
   Leu Cys Leu Leu Val Met Phe Phe Met Val Leu Val Ala Leu Val Gly
   85 90 95
   Leu Gly Leu Gly Met Phe Gln Leu Phe His Leu Gln Lys Glu Leu Ala
   100 105 110
   Glu Leu Arg Glu Ser Thr Ser Gln Met His Thr Ala Ser Ser Leu Glu
   115 120 125
   Lys Gln Ile Gly His Pro Ser Pro Pro Pro Glu Lys Lys Glu Leu Arg
   130 135 140
   Lys Val Ala His Leu Thr Gly Lys Ser Asn Ser Arg Ser Met Pro Leu
   145 150 155 160
   Glu Trp Glu Asp Thr Tyr Gly Ile Val Leu Leu Ser Gly Val Lys Tyr
   165 170 175
   Lys Lys Gly Gly Leu Val Ile Asn Glu Thr Gly Leu Tyr Phe Val Tyr
   180 185 190
   Ser Lys Val Tyr Phe Arg Gly Gln Ser Cys Asn Asn Leu Pro Leu Ser
   195 200 205
   His Lys Val Tyr Met Arg Asn Ser Lys Tyr Pro Gln Asp Leu Val Met
   210 215 220
   Met Glu Gly Lys Met Met Ser Tyr Cys Thr Thr Gly Gln Met Trp Ala
   225 230 235 240
   Arg Ser Ser Tyr Leu Gly Ala Val Phe Asn Leu Thr Ser Ala Asp His
   245 250 255
   Leu Tyr Val Asn Val Ser Glu Leu Ser Leu Val Asn Phe Glu Glu Ser
   260 265 270
   Gln Thr Phe Phe Gly Leu Tyr Lys Leu
   275 280
<210> 101
   <211> 193
   <212> PRT
   <213> Homo sapiens
<400> 101
   Met Pro Glu Glu Gly Ser Gly Cys Ser Val Arg Arg Arg Pro Tyr Gly
   1 5 10 15
   Cys Val Leu Arg Ala Ala Leu Val Pro Leu Val Ala Gly Leu Val Ile
   20 25 30
   Cys Leu Val Val Cys Ile Gln Arg Phe Ala Gln Ala Gln Gln Gln Leu
   35 40 45
   Pro Leu Glu Ser Leu Gly Trp Asp Val Ala Glu Leu Gln Leu Asn His
   50 55 60
   Thr Gly Pro Gln Gln Asp Pro Arg Leu Tyr Trp Gln Gly Gly Pro Ala
   65 70 75 80
   Leu Gly Arg Ser Phe Leu His Gly Pro Glu Leu Asp Lys Gly Gln Leu
   85 90 95
   Arg Ile His Arg Asp Gly Ile Tyr Met Val His Ile Gln Val Thr Leu
   100 105 110
   Ala Ile Cys Ser Ser Thr Thr Ala Ser Arg His His Pro Thr Thr Leu
   115 120 125
   Ala Val Gly Ile Cys Ser Pro Ala Ser Arg Ser Ile Ser Leu Leu Arg
   130 135 140
   Leu Ser Phe His Gln Gly Cys Thr Ile Ala Ser Gln Arg Leu Thr Pro
   145 150 155 160
   Leu Ala Arg Gly Asp Thr Leu Cys Thr Asn Leu Thr Gly Thr Leu Leu
   165 170 175
   Pro Ser Arg Asn Thr Asp Glu Thr Phe Phe Gly Val Gln Trp Val Arg
   180 185 190
   Pro
<210> 102
   <211> 234
   <212> PRT
   <213> Homo sapiens
<400> 102
   Met Asp Pro Gly Leu Gln Gln Ala Leu Asn Gly Met Ala Pro Pro Gly
   1 5 10 15
   Asp Thr Ala Met His Val Pro Ala Gly Ser Val Ala Ser His Leu Gly
   20 25 30
   Thr Thr Ser Arg Ser Tyr Phe Tyr Leu Thr Thr Ala Thr Leu Ala Leu
   35 40 45
   Cys Leu Val Phe Thr Val Ala Thr Ile Met Val Leu Val Val Gln Arg
   50 55 60
   Thr Asp Ser Ile Pro Asn Ser Pro Asp Asn Val Pro Leu Lys Gly Gly
   65 70 75 80
   Asn Cys Ser Glu Asp Leu Leu Cys Ile Leu Lys Arg Ala Pro Phe Lys
   85 90 95
   Lys Ser Trp Ala Tyr Leu Gln Val Ala Lys His Leu Asn Lys Thr Lys
   100 105 110
   Leu Ser Trp Asn Lys Asp Gly Ile Leu His Gly Val Arg Tyr Gln Asp
   115 120 125
   Gly Asn Leu Val Ile Gln Phe Pro Gly Leu Tyr Phe Ile Ile Cys Gln
   130 135 140
   Leu Gln Phe Leu Val Gln Cys Pro Asn Asn Ser Val Asp Leu Lys Leu
   145 150 155 160
   Glu Leu Leu Ile Asn Lys His Ile Lys Lys Gln Ala Leu Val Thr Val
   165 170 175
   Cys Glu Ser Gly Met Gln Thr Lys His Val Tyr Gln Asn Leu Ser Gln
   180 185 190
   Phe Leu Leu Asp Tyr Leu Gln Val Asn Thr Thr Ile Ser Val Asn Val
   195 200 205
   Asp Thr Phe Gln Tyr Ile Asp Thr Ser Thr Phe Pro Leu Glu Asn Val
   210 215 220
   Leu Ser Ile Phe Leu Tyr Ser Asn Ser Asp
   225 230
<210> 103
   <211> 254
   <212> PRT
   <213> Homo sapiens
<400> 103
   Met Glu Tyr Ala Ser Asp Ala Ser Leu Asp Pro Glu Ala Pro Trp Pro
   1 5 10 15
   Pro Ala Pro Arg Ala Arg Ala Cys Arg Val Leu Pro Trp Ala Leu Val
   20 25 30
   Ala Gly Leu Leu Leu Leu Leu Leu Leu Ala Ala Ala Cys Ala Val Phe
   35 40 45
   Leu Ala Cys Pro Trp Ala Val Ser Gly Ala Arg Ala Ser Pro Gly Ser
   50 55 60
   Ala Ala Ser Pro Arg Leu Arg Glu Gly Pro Glu Leu Ser Pro Asp Asp
   65 70 75 80
   Pro Ala Gly Leu Leu Asp Leu Arg Gln Gly Met Phe Ala Gln Leu Val
                   85                  90                  95
   Ala Gln Asn Val Leu Leu Ile Asp Gly Pro Leu Ser Trp Tyr Ser Asp
               100                 105                 110
   Pro Gly Leu Ala Gly Val Ser Leu Thr Gly Gly Leu Ser Tyr Lys Glu
           115                 120                 125
   Asp Thr Lys Glu Leu Val Val Ala Lys Ala Gly Val Tyr Tyr Val Phe
       130                 135                 140
   Phe Gln Leu Glu Leu Arg Arg Val Val Ala Gly Glu Gly Ser Gly Ser
   145                 150                 155                 160
   Val Ser Leu Ala Leu His Leu Gln Pro Leu Arg Ser Ala Ala Gly Ala
                   165                 170                 175
   Ala Ala Leu Ala Leu Thr Val Asp Leu Pro Pro Ala Ser Ser Glu Ala
               180                 185                 190
   Arg Asn Ser Ala Phe Gly Phe Gln Gly Arg Leu Leu His Leu Ser Ala
           195                 200                 205
   Gly Gln Arg Leu Gly Val His Leu His Thr Glu Ala Arg Ala Arg His
       210                 215                 220
   Ala Trp Gln Leu Thr Gln Gly Ala Thr Val Leu Gly Leu Phe Arg Val
   225                 230                 235                 240
   Thr Pro Glu Ile Pro Ala Gly Leu Pro Ser Pro Arg Ser Glu
                   245                 250
<210> 104
   <211> 281
   <212> PRT
   <213> Homo sapiens
<400> 104
<210> 105
   <211> 317
   <212> PRT
   <213> Homo sapiens
<400> 105
   Met Arg Arg Ala Ser Arg Asp Tyr Thr Lys Tyr Leu Arg Gly Ser Glu
   1               5                   10                  15
   Glu Met Gly Gly Gly Pro Gly Ala Pro His Glu Gly Pro Leu His Ala
               20                  25                  30
   Pro Pro Pro Pro Ala Pro His Gln Pro Pro Ala Ala Ser Arg Ser Met
           35                  40                  45
   Phe Val Ala Leu Leu Gly Leu Gly Leu Gly Gln Val Val Cys Ser Val
       50                  55                  60
   Ala Leu Phe Phe Tyr Phe Arg Ala Gln Met Asp Pro Asn Arg Ile Ser
   65                  70                  75                  80
   Glu Asp Gly Thr His Cys Ile Tyr Arg Ile Leu Arg Leu His Glu Asn
                   85                  90                  95
   Ala Asp Phe Gln Asp Thr Thr Leu Glu Ser Gln Asp Thr Lys Leu Ile
               100                 105                 110
   Pro Asp Ser Cys Arg Arg Ile Lys Gln Ala Phe Gln Gly Ala Val Gln
           115                 120                 125
   Lys Glu Leu Gln His Ile Val Gly Ser Gln His Ile Arg Ala Glu Lys
       130                 135                 140
   Ala Met Val Asp Gly Ser Trp Leu Asp Leu Ala Lys Arg Ser Lys Leu
   145                 150                 155                 160
   Glu Ala Gln Pro Phe Ala His Leu Thr Ile Asn Ala Thr Asp Ile Pro
                   165                 170                 175
   Ser Gly Ser His Lys Val Ser Leu Ser Ser Trp Tyr His Asp Arg Gly
               180                 185                 190
   Trp Ala Lys Ile Ser Asn Met Thr Phe Ser Asn Gly Lys Leu Ile Val
           195                 200                 205
   Asn Gln Asp Gly Phe Tyr Tyr Leu Tyr Ala Asn Ile Cys Phe Arg His
       210                 215                 220
   His Glu Thr Ser Gly Asp Leu Ala Thr Glu Tyr Leu Gln Leu Met Val
   225                 230                 235                 240
   Tyr Val Thr Lys Thr Ser Ile Lys Ile Pro Ser Ser His Thr Leu Met
   245 250 255
   Lys Gly Gly Ser Thr Lys Tyr Trp Ser Gly Asn Ser Glu Phe His Phe
   260 265 270
   Tyr Ser Ile Asn Val Gly Gly Phe Phe Lys Leu Arg Ser Gly Glu Glu
   275 280 285
   Ile Ser Ile Glu Val Ser Asn Pro Ser Leu Leu Asp Pro Asp Gln Asp
   290 295 300
   Ala Thr Tyr Phe Gly Ala Phe Lys Val Arg Asp Ile Asp
   305 310 315
<210> 106
   <211> 249
   <212> PRT
   <213> Homo sapiens
<400> 106
   Met Ala Ala Arg Arg Ser Gln Arg Arg Arg Gly Arg Arg Gly Glu Pro
   1 5 10 15
   Gly Thr Ala Leu Leu Val Pro Leu Ala Leu Gly Leu Gly Leu Ala Leu
   20 25 30
   Ala Cys Leu Gly Leu Leu Leu Ala Val Val Ser Leu Gly Ser Arg Ala
   35 40 45
   Ser Leu Ser Ala Gln Glu Pro Ala Gln Glu Glu Leu Val Ala Glu Glu
   50 55 60
   Asp Gln Asp Pro Ser Glu Leu Asn Pro Gln Thr Glu Glu Ser Gln Asp
   65 70 75 80
   Pro Ala Pro Phe Leu Asn Arg Leu Val Arg Pro Arg Arg Ser Ala Pro
   85 90 95
   Lys Gly Arg Lys Thr Arg Ala Arg Arg Ala Ile Ala Ala His Tyr Glu
   100 105 110
   Val His Pro Arg Pro Gly Gln Asp Gly Ala Gln Ala Gly Val Asp Gly
   115 120 125
   Thr Val Ser Gly Trp Glu Glu Ala Arg Ile Asn Ser Ser Ser Pro Leu
   130 135 140
   Arg Tyr Asn Arg Gln Ile Gly Glu Phe Ile Val Thr Arg Ala Gly Leu
   145                 150                 155                 160
   Tyr Tyr Leu Tyr Cys Gln Val His Phe Asp Glu Gly Lys Ala Val Tyr
                   165                 170                 175
   Leu Lys Leu Asp Leu Leu Val Asp Gly Val Leu Ala Leu Arg Cys Leu
               180                 185                 190
   Glu Glu Phe Ser Ala Thr Ala Ala Ser Ser Leu Gly Pro Gln Leu Arg
           195                 200                 205
   Leu Cys Gln Val Ser Gly Leu Leu Ala Leu Arg Pro Gly Ser Ser Leu
       210                 215                 220
   Arg Ile Arg Thr Leu Pro Trp Ala His Leu Lys Ala Ala Pro Phe Leu
   225                 230                 235                 240
   Thr Tyr Phe Gly Leu Phe Gln Val His
                   245
<210> 107
   <211> 250
   <212> PRT
   <213> Homo sapiens
<400> 107
<210> 108
   <211> 285
   <212> PRT
   <213> Homo sapiens
<400> 108
   Met Asp Asp Ser Thr Glu Arg Glu Gln Ser Arg Leu Thr Ser Cys Leu
   1 5 10 15
   Lys Lys Arg Glu Glu Met Lys Leu Lys Glu Cys Val Ser Ile Leu Pro
   20 25 30
   Arg Lys Glu Ser Pro Ser Val Arg Ser Ser Lys Asp Gly Lys Leu Leu
   35 40 45
   Ala Ala Thr Leu Leu Leu Ala Leu Leu Ser Cys Cys Leu Thr Val Val
   50 55 60
   Ser Phe Tyr Gln Val Ala Ala Leu Gln Gly Asp Leu Ala Ser Leu Arg
   65 70 75 80
   Ala Glu Leu Gln Gly His His Ala Glu Lys Leu Pro Ala Gly Ala Gly
   85 90 95
   Ala Pro Lys Ala Gly Leu Glu Glu Ala Pro Ala Val Thr Ala Gly Leu
   100 105 110
   Lys Ile Phe Glu Pro Pro Ala Pro Gly Glu Gly Asn Ser Ser Gln Asn
   115 120 125
   Ser Arg Asn Lys Arg Ala Val Gln Gly Pro Glu Glu Thr Val Thr Gln
   130 135 140
   Asp Cys Leu Gln Leu Ile Ala Asp Ser Glu Thr Pro Thr Ile Gln Lys
   145 150 155 160
   Gly Ser Tyr Thr Phe Val Pro Trp Leu Leu Ser Phe Lys Arg Gly Ser
   165 170 175
   Ala Leu Glu Glu Lys Glu Asn Lys Ile Leu Val Lys Glu Thr Gly Tyr
   180 185 190
   Phe Phe Ile Tyr Gly Gln Val Leu Tyr Thr Asp Lys Thr Tyr Ala Met
   195 200 205
   Gly His Leu Ile Gln Arg Lys Lys Val His Val Phe Gly Asp Glu Leu
   210 215 220
   Ser Leu Val Thr Leu Phe Arg Cys Ile Gln Asn Met Pro Glu Thr Leu
   225 230 235 240
   Pro Asn Asn Ser Cys Tyr Ser Ala Gly Ile Ala Lys Leu Glu Glu Gly
   245 250 255
   Asp Glu Leu Gln Leu Ala Ile Pro Arg Glu Asn Ala Gln Ile Ser Leu
   260 265 270
   Asp Gly Asp Val Thr Phe Phe Gly Ala Leu Lys Leu Leu
   275 280 285
<210> 109
   <211> 240
   <212> PRT
   <213> Homo sapiens
<400> 109
   Met Glu Glu Ser Val Val Arg Pro Ser Val Phe Val Val Asp Gly Gln
   1 5 10 15
   Thr Asp Ile Pro Phe Thr Arg Leu Gly Arg Ser His Arg Arg Gln Ser
   20 25 30
   Cys Ser Val Ala Arg Val Gly Leu Gly Leu Leu Leu Leu Leu Met Gly
   35 40 45
   Ala Gly Leu Ala Val Gln Gly Trp Phe Leu Leu Gln Leu His Trp Arg
   50 55 60
   Leu Gly Glu Met Val Thr Arg Leu Pro Asp Gly Pro Ala Gly Ser Trp
   65 70 75 80
   Glu Gln Leu Ile Gln Glu Arg Arg Ser His Glu Val Asn Pro Ala Ala
   85 90 95
   His Leu Thr Gly Ala Asn Ser Ser Leu Thr Gly Ser Gly Gly Pro Leu
   100 105 110
   Leu Trp Glu Thr Gln Leu Gly Leu Ala Phe Leu Arg Gly Leu Ser Tyr
   115 120 125
   His Asp Gly Ala Leu Val Val Thr Lys Ala Gly Tyr Tyr Tyr Ile Tyr
   130 135 140
   Ser Lys Val Gln Leu Gly Gly Val Gly Cys Pro Leu Gly Leu Ala Ser
   145 150 155 160
   Thr Ile Thr His Gly Leu Tyr Lys Arg Thr Pro Arg Tyr Pro Glu Glu
   165 170 175
   Leu Glu Leu Leu Val Ser Gln Gln Ser Pro Cys Gly Arg Ala Thr Ser
   180 185 190
   Ser Ser Arg Val Trp Trp Asp Ser Ser Phe Leu Gly Gly Val Val His
   195 200 205
   Leu Glu Ala Gly Glu Lys Val Val Val Arg Val Leu Asp Glu Arg Leu
   210 215 220
   Val Arg Leu Arg Asp Gly Thr Arg Ser Tyr Phe Gly Ala Phe Met Val
   225 230 235 240
<210> 110
   <211> 251
   <212> PRT
   <213> Homo sapiens
<400> 110
   Met Ala Glu Asp Leu Gly Leu Ser Phe Gly Glu Thr Ala Ser Val Glu
   1               5                   10                  15
   Met Leu Pro Glu His Gly Ser Cys Arg Pro Lys Ala Arg Ser Ser Ser
               20                  25                  30
   Ala Arg Trp Ala Leu Thr Cys Cys Leu Val Leu Leu Pro Phe Leu Ala
           35                  40                  45
   Gly Leu Thr Thr Tyr Leu Leu Val Ser Gln Leu Arg Ala Gln Gly Glu
       50                  55                  60
   Ala Cys Val Gln Phe Gln Ala Leu Lys Gly Gln Glu Phe Ala Pro Ser
   65                  70                  75                  80
   His Gln Gln Val Tyr Ala Pro Leu Arg Ala Asp Gly Asp Lys Pro Arg
                   85                  90                  95
   Ala His Leu Thr Val Val Arg Gln Thr Pro Thr Gln His Phe Lys Asn
               100                 105                 110
   Gln Phe Pro Ala Leu His Trp Glu His Glu Leu Gly Leu Ala Phe Thr
           115                 120                 125
   Lys Asn Arg Met Asn Tyr Thr Asn Lys Phe Leu Leu Ile Pro Glu Ser
       130                 135                 140
   Gly Asp Tyr Phe Ile Tyr Ser Gln Val Thr Phe Arg Gly Met Thr Ser
   145                 150                 155                 160
   Glu Cys Ser Glu Ile Arg Gln Ala Gly Arg Pro Asn Lys Pro Asp Ser
                   165                 170                 175
   Ile Thr Val Val Ile Thr Lys Val Thr Asp Ser Tyr Pro Glu Pro Thr
               180                 185                 190
   Gln Leu Leu Met Gly Thr Lys Ser Val Cys Glu Val Gly Ser Asn Trp
           195                 200                 205
   Phe Gln Pro Ile Tyr Leu Gly Ala Met Phe Ser Leu Gln Glu Gly Asp
       210                 215                 220
   Lys Leu Met Val Asn Val Ser Asp Ile Ser Leu Val Asp Tyr Thr Lys
   225                 230                 235                 240
   Glu Asp Lys Thr Phe Phe Gly Ala Phe Leu Leu
   245 250
<210> 111
   <211> 199
   <212> PRT
   <213> Homo sapiens
<400> 111
   Met Thr Leu His Pro Ser Pro Ile Thr Cys Glu Phe Leu Phe Ser Thr
   1               5                   10                  15
   Ala Leu Ile Ser Pro Lys Met Cys Leu Ser His Leu Glu Asn Met Pro
               20                  25                  30
   Leu Ser His Ser Arg Thr Gln Gly Ala Gln Arg Ser Ser Trp Lys Leu
           35                  40                  45
   Trp Leu Phe Cys Ser Ile Val Met Leu Leu Phe Leu Cys Ser Phe Ser
       50                  55                  60
   Trp Leu Ile Phe Ile Phe Leu Gln Leu Glu Thr Ala Lys Glu Pro Cys
   65                  70                  75                  80
   Met Ala Lys Phe Gly Pro Leu Pro Ser Lys Trp Gln Met Ala Ser Ser
                   85                  90                  95
   Glu Pro Pro Cys Val Asn Lys Val Ser Asp Trp Lys Leu Glu Ile Leu
               100                 105                 110
   Gln Asn Gly Leu Tyr Leu Ile Tyr Gly Gln Val Ala Pro Asn Ala Asn
           115                 120                 125
   Tyr Asn Asp Val Ala Pro Phe Glu Val Arg Leu Tyr Lys Asn Lys Asp
       130                 135                 140
   Met Ile Gln Thr Leu Thr Asn Lys Ser Lys Ile Gln Asn Val Gly Gly
   145                 150                 155                 160
   Thr Tyr Glu Leu His Val Gly Asp Thr Ile Asp Leu Ile Phe Asn Ser
                   165                 170                 175
   Glu His Gln Val Leu Lys Asn Asn Thr Tyr Trp Gly Ile Ile Leu Leu
               180                 185                 190
   Ala Asn Pro Gln Phe Ile Ser
           195
<210> 112
   <211> 391
   <212> PRT
   <213> Homo sapiens
<400> 112
<210> 113
   <211> 205
   <212> PRT
   <213> Homo sapiens
<400> 113
   Ala Cys Pro Trp Ala Val Ser Gly Ala Arg Ala Ser Pro Gly Ser Ala
   1               5                   10                  15
   Ala Ser Pro Arg Leu Arg Glu Gly Pro Glu Leu Ser Pro Asp Asp Pro
               20                  25                  30
   Ala Gly Leu Leu Asp Leu Arg Gln Gly Met Phe Ala Gln Leu Val Ala
           35                  40                  45
   Gln Asn Val Leu Leu Ile Asp Gly Pro Leu Ser Trp Tyr Ser Asp Pro
       50                  55                  60
   Gly Leu Ala Gly Val Ser Leu Thr Gly Gly Leu Ser Tyr Lys Glu Asp
   65                  70                  75                  80
   Thr Lys Glu Leu Val Val Ala Lys Ala Gly Val Tyr Tyr Val Phe Phe
                   85                  90                  95
   Gln Leu Glu Leu Arg Arg Val Val Ala Gly Glu Gly Ser Gly Ser Val
               100                 105                 110
   Ser Leu Ala Leu His Leu Gln Pro Leu Arg Ser Ala Ala Gly Ala Ala
           115                 120                 125
   Ala Leu Ala Leu Thr Val Asp Leu Pro Pro Ala Ser Ser Glu Ala Arg
       130                 135                 140
   Asn Ser Ala Phe Gly Phe Gln Gly Arg Leu Leu His Leu Ser Ala Gly
   145                 150                 155                 160
   Gln Arg Leu Gly Val His Leu His Thr Glu Ala Arg Ala Arg His Ala
                   165                 170                 175
   Trp Gln Leu Thr Gln Gly Ala Thr Val Leu Gly Leu Phe Arg Val Thr
               180                 185                 190
   Pro Glu Ile Pro Ala Gly Leu Pro Ser Pro Arg Ser Glu
           195                 200                 205
<210> 114
   <211> 133
   <212> PRT
   <213> Homo sapiens
<400> 114
   Gln Val Ser His Arg Tyr Pro Arg Ile Gln Ser Ile Lys Val Gln Phe
   1               5                   10                  15
   Thr Glu Tyr Lys Lys Glu Lys Gly Phe Ile Leu Thr Ser Gln Lys Glu
               20                  25                  30
   Asp Glu Ile Met Lys Val Gln Asn Asn Ser Val Ile Ile Asn Cys Asp
           35                  40                  45
   Gly Phe Tyr Leu Ile Ser Leu Lys Gly Tyr Phe Ser Gln Glu Val Asn
       50                  55                  60
   Ile Ser Leu His Tyr Gln Lys Asp Glu Glu Pro Leu Phe Gln Leu Lys
   65                  70                  75                  80
   Lys Val Arg Ser Val Asn Ser Leu Met Val Ala Ser Leu Thr Tyr Lys
                   85                  90                  95
   Asp Lys Val Tyr Leu Asn Val Thr Thr Asp Asn Thr Ser Leu Asp Asp
               100                 105                 110
   Phe His Val Asn Gly Gly Glu Leu Ile Leu Ile His Gln Asn Pro Gly
           115                 120                 125
   Glu Phe Cys Val Leu
       130
<210> 115
   <211> 132
   <212> PRT
   <213> Homo sapiens
<400> 115
   Val Ser His Arg Tyr Pro Arg Ile Gln Ser Ile Lys Val Gln Phe Thr
   1               5                   10                  15
   Glu Tyr Lys Lys Glu Lys Gly Phe Ile Leu Thr Ser Gln Lys Glu Asp
               20                  25                  30
   Glu Ile Met Lys Val Gln Asn Asn Ser Val Ile Ile Asn Cys Asp Gly
           35                  40                  45
   Phe Tyr Leu Ile Ser Leu Lys Gly Tyr Phe Ser Gln Glu Val Asn Ile
       50                  55                  60
   Ser Leu His Tyr Gln Lys Asp Glu Glu Pro Leu Phe Gln Leu Lys Lys
   65                  70                  75                  80
   Val Arg Ser Val Asn Ser Leu Met Val Ala Ser Leu Thr Tyr Lys Asp
                   85                  90                  95
   Lys Val Tyr Leu Asn Val Thr Thr Asp Asn Thr Ser Leu Asp Asp Phe
               100                 105                 110
   His Val Asn Gly Gly Glu Leu Ile Leu Ile His Gln Asn Pro Gly Glu
           115                 120                 125
   Phe Cys Val Leu
       130
<210> 116
   <211> 5
   <212> PRT
   <213> Artificial Sequence
<220>
   <223> Peptide linker G4S
<400> 116
   Gly Gly Gly Gly Ser
   1               5
<210> 117
   <211> 10
   <212> PRT
   <213> Artificial Sequence
<220>
   <223> Peptide linker (G4S)2
<400> 117
   Gly Gly Gly Gly Ser Gly Gly Gly Gly Ser
   1               5                   10
<210> 118
   <211> 10
   <212> PRT
   <213> Artificial Sequence
<220>
   <223> Peptide linker (SG4)2
<400> 118
   Ser Gly Gly Gly Gly Ser Gly Gly Gly Gly
   1               5                   10
<210> 119
   <211> 15
   <212> PRT
   <213> Artificial Sequence
<220>
   <223> Peptide linker (G4S)3
<400> 119
   Gly Gly Gly Gly Ser Gly Gly Gly Gly Ser Gly Gly Gly Gly Ser
   1               5                   10                  15
<210> 120
   <211> 14
   <212> PRT
   <213> Artificial Sequence
<220>
   <223> Peptide linker G4(SG4)2
<400> 120
   Gly Gly Gly Gly Ser Gly Gly Gly Gly Ser Gly Gly Gly Gly
   1               5                   10
<210> 121
   <211> 20
   <212> PRT
   <213> Artificial Sequence
<220>
   <223> Peptide linker (G4S)4
<400> 121
   Gly Gly Gly Gly Ser Gly Gly Gly Gly Ser Gly Gly Gly Gly Ser Gly
   1               5                   10                  15
   Gly Gly Gly Ser
               20
<210> 122
   <211> 10
   <212> PRT
   <213> Artificial Sequence
<220>
   <223> Peptide linker GSPGSSSSGS
<400> 122
   Gly Ser Pro Gly Ser Ser Ser Ser Gly Ser
   1 5 10
<210> 123
   <211> 8
   <212> PRT
   <213> Artificial Sequence
<220>
   <223> Peptide linker GSGSGSGS
<400> 123
   Gly Ser Gly Ser Gly Ser Gly Ser
   1 5
<210> 124
   <211> 8
   <212> PRT
   <213> Artificial Sequence
<220>
   <223> Peptide linker GSGSGNGS
<400> 124
   Gly Ser Gly Ser Gly Asn Gly Ser
   1 5
<210> 125
   <211> 8
   <212> PRT
   <213> Artificial Sequence
<220>
   <223> Peptide linker GGSGSGSG
<400> 125
   Gly Gly Ser Gly Ser Gly Ser Gly
   1 5
<210> 126
   <211> 6
   <212> PRT
   <213> Artificial Sequence
<220>
   <223> Peptide linker GGSGSG
<400> 126
   Gly Gly Ser Gly Ser Gly
   1               5
<210> 127
   <211> 4
   <212> PRT
   <213> Artificial Sequence
<220>
   <223> Peptide linker GGSG
<400> 127
   Gly Gly Ser Gly
   1
<210> 128
   <211> 8
   <212> PRT
   <213> Artificial Sequence
<220>
   <223> GGSGNGSG
<400> 128
   Gly Gly Ser Gly Asn Gly Ser Gly
   1               5
<210> 129
   <211> 8
   <212> PRT
   <213> Artificial Sequence
<220>
   <223> Peptide inker GGNGSGSG
<400> 129
   Gly Gly Asn Gly Ser Gly Ser Gly
   1               5
<210> 130
   <211> 6
   <212> PRT
   <213> Artificial Sequence
<220>
   <223> Peptide linker GGNGSG
<400> 130
   Gly Gly Asn Gly Ser Gly
   1               5
<210> 131
   <211> 2166
   <212> DNA
   <213> Artificial Sequence
<220>
   <223> nucleotide sequence of dimeric hu 4-1BBL (71-254) - CL* Fc knob chain
<400> 131
   agagagggcc ctgagctgag ccccgatgat cctgctggac tgctggacct gcggcagggc     60
   atgtttgctc agctggtggc ccagaacgtg ctgctgatcg atggccccct gtcctggtac    120
   agcgatcctg gactggctgg cgtgtcactg acaggcggcc tgagctacaa agaggacacc    180
   aaagaactgg tggtggccaa ggccggcgtg tactacgtgt tctttcagct ggaactgcgg    240
   agagtggtgg ccggcgaagg atctggctct gtgtctctgg ccctgcatct gcagcctctg    300
   agaagcgctg ctggcgctgc agctctggca ctgacagtgg atctgcctcc tgccagctcc    360
   gaggcccgga atagcgcatt tgggtttcaa ggcaggctgc tgcacctgtc tgccggccag    420
   aggctgggag tgcatctgca cacagaggcc agggctagac acgcctggca gctgacacag    480
   ggcgctacag tgctgggcct gttcagagtg acccccgaga ttccagccgg cctgccttct    540
   ccaagaagcg aaggcggagg cggatctggc ggcggaggat ctagagaggg acccgaactg    600
   tcccctgacg atccagccgg gctgctggat ctgagacagg gaatgttcgc ccagctggtg    660
   gctcagaatg tgctgctgat tgacggacct ctgagctggt actccgaccc agggctggca    720
   ggggtgtccc tgactggggg actgtcctac aaagaagata caaaagaact ggtggtggct    780
   aaagctgggg tgtactatgt gttttttcag ctggaactga ggcgggtggt ggctggggag    840
   ggctcaggat ctgtgtccct ggctctgcat ctgcagccac tgcgctctgc tgctggcgca    900
   gctgcactgg ctctgactgt ggacctgcca ccagcctcta gcgaggccag aaacagcgcc 960
   ttcgggttcc aaggacgcct gctgcatctg agcgccggac agcgcctggg agtgcatctg 1020
   catactgaag ccagagcccg gcatgcttgg cagctgactc agggggcaac tgtgctggga 1080
   ctgtttcgcg tgacacctga gatccctgcc ggactgccaa gccctagatc agaagggggc 1140
   ggaggttccg gagggggagg atctcgtacg gtggctgcac catctgtctt tatcttccca 1200
   cccagcgacc ggaagctgaa gtctggcaca gccagcgtcg tgtgcctgct gaataacttc 1260
   tacccccgcg aggccaaggt gcagtggaag gtggacaatg ccctgcagag cggcaacagc 1320
   caggaaagcg tgaccgagca ggacagcaag gactccacct acagcctgag cagcaccctg 1380
   accctgagca aggccgacta cgagaagcac aaggtgtacg cctgcgaagt gacccaccag 1440
   ggcctgtcta gccccgtgac caagagcttc aaccggggcg agtgcgacaa gacccacacc 1500
   tgtcctccat gccctgcccc tgaagctgct ggcggcccta gcgtgttcct gttcccccca 1560
   aagcccaagg acaccctgat gatcagccgg acccctgaag tgacctgcgt ggtggtggat 1620
   gtgtcccacg aggaccctga agtgaagttc aattggtacg tggacggcgt ggaagtgcac 1680
   aatgccaaga ccaagccgcg ggaggagcag tacaacagca cgtaccgtgt ggtcagcgtc 1740
   ctcaccgtcc tgcaccagga ctggctgaat ggcaaggagt acaagtgcaa ggtctccaac 1800
   aaagccctcg gcgcccccat cgagaaaacc atctccaaag ccaaagggca gccccgagaa 1860
   ccacaggtgt acaccctgcc cccatgccgg gatgagctga ccaagaacca ggtcagcctg 1920
   tggtgcctgg tcaaaggctt ctatcccagc gacatcgccg tggagtggga gagcaatggg 1980
   cagccggaga acaactacaa gaccacgcct cccgtgctgg actccgacgg ctccttcttc 2040
   ctctacagca agctcaccgt ggacaagagc aggtggcagc aggggaacgt cttctcatgc 2100
   tccgtgatgc atgaggctct gcacaaccac tacacgcaga agagcctctc cctgtctccg 2160
   ggtaaa 2166
<210> 132
   <211> 891
   <212> DNA
   <213> Artificial Sequence
<220>
   <223> nucleotide sequence of monomeric hu 4-1BBL (71-254) - CH1*
<400> 132
   agagagggcc ctgagctgag ccccgatgat cctgctggac tgctggacct gcggcagggc 60
   atgtttgctc agctggtggc ccagaacgtg ctgctgatcg atggccccct gtcctggtac 120
   agcgatcctg gactggctgg cgtgtcactg acaggcggcc tgagctacaa agaggacacc 180
   aaagaactgg tggtggccaa ggccggcgtg tactacgtgt tctttcagct ggaactgcgg 240
   agagtggtgg ccggcgaagg atctggctct gtgtctctgg ccctgcatct gcagcctctg 300
   agaagcgctg ctggcgctgc agctctggct ctgacagtgg atctgcctcc tgccagctcc 360
   gaggcccgga atagcgcatt tgggtttcaa ggccggctgc tgcacctgtc tgccggccag 420
   agactgggag tgcatctgca cacagaggcc agagccaggc acgcctggca gctgacacag 480
   ggcgctacag tgctgggcct gttcagagtg acccccgaga ttcctgccgg cctgcctagc 540
   cctagatctg aaggcggcgg aggttccgga ggcggaggat ctgctagcac aaagggcccc 600
   agcgtgttcc ctctggcccc tagcagcaag agcacatctg gcggaacagc cgccctgggc 660
   tgcctggtgg aagattactt ccccgagccc gtgaccgtgt cctggaattc tggcgccctg 720
   acaagcggcg tgcacacctt tccagccgtg ctgcagagca gcggcctgta ctctctgagc 780
   agcgtcgtga cagtgcccag cagctctctg ggcacccaga cctacatctg caacgtgaac 840
   cacaagccca gcaacaccaa ggtggacgag aaggtggaac ccaagtcctg c 891
<210> 133
   <211> 1350
   <212> DNA
   <213> Artificial Sequence
<220>
   <223> nucleotide sequence of anti-PD1(0314) Fc hole chain
<400> 133
   gaagtgcagc tgctcgaaag cggcggggga ctggtccagc ccggcggttc cctgaggctg 60
   tcttgcgccg cttcagggtt cagcttttcc tcttacacca tgagttgggt cagacaggca 120
   cctggcaagg gactggagtg ggtcgccaca atcagcggtg gcgggcgcga catttattac 180
   ccagattccg tgaaaggacg gttcaccatc tctagggaca actcaaagaa tactctgtat 240
   ttgcagatga acagcctgag agctgaggat acagcagttt actactgtgt gctcctgacc 300
   ggccgcgtct attttgccct tgactcctgg ggacaaggca ctctggtgac cgtatctagt 360
   gctagcacca agggcccctc cgtgttcccc ctggccccca gcagcaagag caccagcggc 420
   ggcacagccg ctctgggctg cctggtcaag gactacttcc ccgagcccgt gaccgtgtcc 480
   tggaacagcg gagccctgac ctccggcgtg cacaccttcc ccgccgtgct gcagagttct 540
   ggcctgtata gcctgagcag cgtggtcacc gtgccttcta gcagcctggg cacccagacc 600
   tacatctgca acgtgaacca caagcccagc aacaccaagg tggacaagaa ggtggagccc 660
   aagagctgcg acaaaactca cacatgccca ccgtgcccag cacctgaagc tgcaggggga 720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacaac 900
   agcacgtacc gtgtggtcag cgtcctcacc gtcctgcacc aggactggct gaatggcaag 960
   gagtacaagt gcaaggtctc caacaaagcc ctcggcgccc ccatcgagaa aaccatctcc 1020
   aaagccaaag ggcagccccg agaaccacag gtgtgcaccc tgcccccatc ccgggatgag 1080
   ctgaccaaga accaggtcag cctctcgtgc gcagtcaaag gcttctatcc cagcgacatc 1140
   gccgtggagt gggagagcaa tgggcagccg gagaacaact acaagaccac gcctcccgtg 1200
   ctggactccg acggctcctt cttcctcgtg agcaagctca ccgtggacaa gagcaggtgg 1260
   cagcagggga acgtcttctc atgctccgtg atgcatgagg ctctgcacaa ccactacacg 1320
   cagaagagcc tctccctgtc tccgggtaaa 1350
<210> 134
   <211> 654
   <212> DNA
   <213> Artificial Sequence
<220>
   <223> nucleotide sequence of anti-PD1 (0314) light chain
<400> 134
   gagatcgtgc tgacccagag ccccgccaca ctctccctgt ctcctggcga aagggctacc      60
   ctttcatgca gagcaagcga gtccgtggac acttctgata acagtttcat tcactggtat     120
   cagcagaagc cagggcagag cccccgcctg ctcatctatc ggtcctctac actggagtca     180
   gggattcctg ccaggtttag cggctccggt tctgggaccg acttcacttt gaccatcagt     240
   agcctggaac cagaggattt tgctgtgtac tattgtcaac agaattacga cgtcccctgg     300
   acattcggcc agggaaccaa agtggagata aagcgtacgg tggctgcacc atctgtcttc     360
   atcttcccgc catctgatga gcagttgaaa tctggaactg cctctgttgt gtgcctgctg     420
   aataacttct atcccagaga ggccaaagta cagtggaagg tggataacgc cctccaatcg     480
   ggtaactccc aggagagtgt cacagagcag gacagcaagg acagcaccta cagcctcagc     540
   agcaccctga cgctgagcaa agcagactac gagaaacaca aagtctacgc ctgcgaagtc     600
   acccatcagg gcctgagctc gcccgtcaca aagagcttca acaggggaga gtgt           654
<210> 135
   <211> 2166
   <212> DNA
   <213> Artificial Sequence
<220>
   <223> nucleotide sequence of dimeric hu 4-1BBL (71-254) - CL Fc knob chain
<400> 135
   agagagggcc ctgagctgag ccccgatgat cctgctggac tgctggacct gcggcagggc      60
   atgtttgctc agctggtggc ccagaacgtg ctgctgatcg atggccccct gtcctggtac     120
   agcgatcctg gactggctgg cgtgtcactg acaggcggcc tgagctacaa agaggacacc     180
   aaagaactgg tggtggccaa ggccggcgtg tactacgtgt tctttcagct ggaactgcgg 240
   agagtggtgg ccggcgaagg atctggctct gtgtctctgg ccctgcatct gcagcctctg 300
   agaagcgctg ctggcgctgc agctctggca ctgacagtgg atctgcctcc tgccagctcc 360
   gaggcccgga atagcgcatt tgggtttcaa ggcaggctgc tgcacctgtc tgccggccag 420
   aggctgggag tgcatctgca cacagaggcc agggctagac acgcctggca gctgacacag 480
   ggcgctacag tgctgggcct gttcagagtg acccccgaga ttccagccgg cctgccttct 540
   ccaagaagcg aaggcggagg cggatctggc ggcggaggat ctagagaggg acccgaactg 600
   tcccctgacg atccagccgg gctgctggat ctgagacagg gaatgttcgc ccagctggtg 660
   gctcagaatg tgctgctgat tgacggacct ctgagctggt actccgaccc agggctggca 720
   ggggtgtccc tgactggggg actgtcctac aaagaagata caaaagaact ggtggtggct 780
   aaagctgggg tgtactatgt gttttttcag ctggaactga ggcgggtggt ggctggggag 840
   ggctcaggat ctgtgtccct ggctctgcat ctgcagccac tgcgctctgc tgctggcgca 900
   gctgcactgg ctctgactgt ggacctgcca ccagcctcta gcgaggccag aaacagcgcc 960
   ttcgggttcc aaggacgcct gctgcatctg agcgccggac agcgcctggg agtgcatctg 1020
   catactgaag ccagagcccg gcatgcttgg cagctgactc agggggcaac tgtgctggga 1080
   ctgtttcgcg tgacacctga gatccctgcc ggactgccaa gccctagatc agaagggggc 1140
   ggaggttccg gagggggagg atctcgtacg gtggccgctc cctccgtgtt tatctttccc 1200
   ccatccgatg aacagctgaa aagcggcacc gcctccgtcg tgtgtctgct gaacaatttt 1260
   taccctaggg aagctaaagt gcagtggaaa gtggataacg cactgcagtc cggcaactcc 1320
   caggaatctg tgacagaaca ggactccaag gacagcacct actccctgtc ctccaccctg 1380
   acactgtcta aggctgatta tgagaaacac aaagtctacg cctgcgaagt cacccatcag 1440
   ggcctgagct cgcccgtcac aaagagcttc aacaggggag agtgtgacaa gacccacacc 1500
   tgtccccctt gtcctgcccc tgaagctgct ggcggccctt ctgtgttcct gttcccccca 1560
   aagcccaagg acaccctgat gatcagccgg acccccgaag tgacctgcgt ggtggtggat 1620
   gtgtcccacg aggaccctga agtgaagttc aattggtacg tggacggcgt ggaagtgcac 1680
   aatgccaaga ccaagccgcg ggaggagcag tacaacagca cgtaccgtgt ggtcagcgtc 1740
   ctcaccgtcc tgcaccagga ctggctgaat ggcaaggagt acaagtgcaa ggtctccaac 1800
   aaagccctcg gcgcccccat cgagaaaacc atctccaaag ccaaagggca gccccgagaa 1860
   ccacaggtgt acaccctgcc cccatgccgg gatgagctga ccaagaacca ggtcagcctg 1920
   tggtgcctgg tcaaaggctt ctatcccagc gacatcgccg tggagtggga gagcaatggg 1980
   cagccggaga acaactacaa gaccacgcct cccgtgctgg actccgacgg ctccttcttc 2040
   ctctacagca agctcaccgt ggacaagagc aggtggcagc aggggaacgt cttctcatgc 2100
   tccgtgatgc atgaggctct gcacaaccac tacacgcaga agagcctctc cctgtctccg 2160
   ggtaaa 2166
<210> 136
   <211> 891
   <212> DNA
   <213> Artificial Sequence
<220>
   <223> nucleotide sequence of monomeric hu 4-1BBL (71-254) - CH1
<400> 136
   agagagggcc ctgagctgag ccccgatgat cctgctggac tgctggacct gcggcagggc 60
   atgtttgctc agctggtggc ccagaacgtg ctgctgatcg atggccccct gtcctggtac 120
   agcgatcctg gactggctgg cgtgtcactg acaggcggcc tgagctacaa agaggacacc 180
   aaagaactgg tggtggccaa ggccggcgtg tactacgtgt tctttcagct ggaactgcgg 240
   agagtggtgg ccggcgaagg atctggctct gtgtctctgg ccctgcatct gcagcctctg 300
   agaagcgctg ctggcgctgc agctctggct ctgacagtgg atctgcctcc tgccagctcc 360
   gaggcccgga atagcgcatt tgggtttcaa ggccggctgc tgcacctgtc tgccggccag 420
   agactgggag tgcatctgca cacagaggcc agagccaggc acgcctggca gctgacacag 480
   ggcgctacag tgctgggcct gttcagagtg acccccgaga ttcctgccgg cctgcctagc 540
   cctagatctg aaggcggcgg aggttccgga ggcggaggat ctgctagcac caaaggccct 600
   tccgtgtttc ctctggctcc tagctccaag tccacctctg gaggcaccgc tgctctcgga 660
   tgcctcgtga aggattattt tcctgagcct gtgacagtgt cctggaatag cggagcactg 720
   acctctggag tgcatacttt ccccgctgtg ctgcagtcct ctggactgta cagcctgagc 780
   agcgtggtga cagtgcccag cagcagcctg ggcacccaga cctacatctg caacgtgaac 840
   cacaagccca gcaacaccaa ggtggacaag aaggtggaac ccaagtcttg t 891
<210> 137
   <211> 2511
   <212> DNA
   <213> Artificial Sequence
<220>
   <223> nucleotide sequence of anti-PD1(0314) Fc hole chain fused to dimeric hu 4-1BBL (71-254)
<400> 137
   gaagtgcagc tgctcgaaag cggcggggga ctggtccagc ccggcggttc cctgaggctg        60
   tcttgcgccg cttcagggtt cagcttttcc tcttacacca tgagttgggt cagacaggca       120
   cctggcaagg gactggagtg ggtcgccaca atcagcggtg gcgggcgcga catttattac       180
   ccagattccg tgaaaggacg gttcaccatc tctagggaca actcaaagaa tactctgtat 240
   ttgcagatga acagcctgag agctgaggat acagcagttt actactgtgt gctcctgacc 300
   ggccgcgtct attttgccct tgactcctgg ggacaaggca ctctggtgac cgtatctagt 360
   gctagcacca agggcccctc cgtgttcccc ctggccccca gcagcaagag caccagcggc 420
   ggcacagccg ctctgggctg cctggtcaag gactacttcc ccgagcccgt gaccgtgtcc 480
   tggaacagcg gagccctgac ctccggcgtg cacaccttcc ccgccgtgct gcagagttct 540
   ggcctgtata gcctgagcag cgtggtcacc gtgccttcta gcagcctggg cacccagacc 600
   tacatctgca acgtgaacca caagcccagc aacaccaagg tggacaagaa ggtggagccc 660
   aagagctgcg acaaaactca cacatgccca ccgtgcccag cacctgaagc tgcaggggga 720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacaac 900
   agcacgtacc gtgtggtcag cgtcctcacc gtcctgcacc aggactggct gaatggcaag 960
   gagtacaagt gcaaggtctc caacaaagcc ctcggcgccc ccatcgagaa aaccatctcc 1020
   aaagccaaag ggcagccccg agaaccacag gtgtgcaccc tgcccccatc ccgggatgag 1080
   ctgaccaaga accaggtcag cctctcgtgc gcagtcaaag gcttctatcc cagcgacatc 1140
   gccgtggagt gggagagcaa tgggcagccg gagaacaact acaagaccac gcctcccgtg 1200
   ctggactccg acggctcctt cttcctcgtg agcaagctca ccgtggacaa gagcaggtgg 1260
   cagcagggga acgtcttctc atgctccgtg atgcatgagg ctctgcacaa ccactacacg 1320
   cagaagagcc tctccctgtc tccgggtgga ggcggcggaa gcggaggagg aggatccaga 1380
   gagggccctg agctgagccc cgatgatcct gctggactgc tggacctgcg gcagggcatg 1440
   tttgctcagc tggtggccca gaacgtgctg ctgatcgatg gccccctgtc ctggtacagc 1500
   gatcctggac tggctggcgt gtcactgaca ggcggcctga gctacaaaga ggacaccaaa 1560
   gaactggtgg tggccaaggc cggcgtgtac tacgtgttct ttcagctgga actgcggaga 1620
   gtggtggccg gcgaaggatc tggctctgtg tctctggccc tgcatctgca gcctctgaga 1680
   agcgctgctg gcgctgcagc tctggcactg acagtggatc tgcctcctgc cagctccgag 1740
   gcccggaata gcgcatttgg gtttcaaggc aggctgctgc acctgtctgc cggccagagg 1800
   ctgggagtgc atctgcacac agaggccagg gctagacacg cctggcagct gacacagggc 1860
   gctacagtgc tgggcctgtt cagagtgacc cccgagattc cagccggcct gccttctcca 1920
   agaagcgaag gcggaggcgg atctggcggc ggaggatcta gagagggacc cgaactgtcc 1980
   cctgacgatc cagccgggct gctggatctg agacagggaa tgttcgccca gctggtggct 2040
   cagaatgtgc tgctgattga cggacctctg agctggtact ccgacccagg gctggcaggg 2100
   gtgtccctga ctgggggact gtcctacaaa gaagatacaa aagaactggt ggtggctaaa 2160
   gctggggtgt actatgtgtt ttttcagctg gaactgaggc gggtggtggc tggggagggc 2220
   tcaggatctg tgtccctggc tctgcatctg cagccactgc gctctgctgc tggcgcagct 2280
   gcactggctc tgactgtgga cctgccacca gcctctagcg aggccagaaa cagcgccttc 2340
   gggttccaag gacgcctgct gcatctgagc gccggacagc gcctgggagt gcatctgcat 2400
   actgaagcca gagcccggca tgcttggcag ctgactcagg gggcaactgt gctgggactg 2460
   tttcgcgtga cacctgagat ccctgccgga ctgccaagcc ctagatcaga a 2511
<210> 138
   <211> 1929
   <212> DNA
   <213> Artificial Sequence
<220>
   <223> nucleotide sequence of anti-PD1(0314) Fc knob chain fused to monomeric hu 4-1BBL (71-254)
<400> 138
   gaagtgcagc tgctcgaaag cggcggggga ctggtccagc ccggcggttc cctgaggctg      60
   tcttgcgccg cttcagggtt cagcttttcc tcttacacca tgagttgggt cagacaggca     120
   cctggcaagg gactggagtg ggtcgccaca atcagcggtg gcgggcgcga catttattac     180
   ccagattccg tgaaaggacg gttcaccatc tctagggaca actcaaagaa tactctgtat     240
   ttgcagatga acagcctgag agctgaggat acagcagttt actactgtgt gctcctgacc     300
   ggccgcgtct attttgccct tgactcctgg ggacaaggca ctctggtgac cgtatctagt     360
   gctagcacca agggcccatc ggtcttcccc ctggcaccct cctccaagag cacctctggg     420
   ggcacagcgg ccctgggctg cctggtcaag gactacttcc ccgaaccggt gacggtgtcg     480
   tggaactcag gcgccctgac cagcggcgtg cacaccttcc cggctgtcct acagtcctca     540
   ggactctact ccctcagcag cgtggtgacc gtgccctcca gcagcttggg cacccagacc     600
   tacatctgca acgtgaatca caagcccagc aacaccaagg tggacaagaa agttgagccc     660
   aaatcttgtg acaaaactca cacatgccca ccgtgcccag cacctgaagc tgcaggggga     720
   ccgtcagtct tcctcttccc cccaaaaccc aaggacaccc tcatgatctc ccggacccct     780
   gaggtcacat gcgtggtggt ggacgtgagc cacgaagacc ctgaggtcaa gttcaactgg     840
   tacgtggacg gcgtggaggt gcataatgcc aagacaaagc cgcgggagga gcagtacaac     900
   agcacgtacc gtgtggtcag cgtcctcacc gtcctgcacc aggactggct gaatggcaag     960
   gagtacaagt gcaaggtctc caacaaagcc ctcggcgccc ccatcgagaa aaccatctcc    1020
   aaagccaaag ggcagccccg agaaccacag gtgtacaccc tgcccccctg cagagatgag    1080
   ctgaccaaga accaggtgtc cctgtggtgt ctggtcaagg gcttctaccc cagcgatatc    1140
   gccgtggagt gggagagcaa cggccagcct gagaacaact acaagaccac cccccctgtg      1200
   ctggacagcg acggcagctt cttcctgtac tccaaactga ccgtggacaa gagccggtgg      1260
   cagcagggca acgtgttcag ctgcagcgtg atgcacgagg ccctgcacaa ccactacacc      1320
   cagaagtccc tgagcctgag ccccggcgga ggcggcggaa gcggaggagg aggatccaga      1380
   gagggccctg agctgagccc cgatgatcct gctggactgc tggacctgcg gcagggcatg      1440
   tttgctcagc tggtggccca gaacgtgctg ctgatcgatg gccccctgtc ctggtacagc      1500
   gatcctggac tggctggcgt gtcactgaca ggcggcctga gctacaaaga ggacaccaaa      1560
   gaactggtgg tggccaaggc cggcgtgtac tacgtgttct ttcagctgga actgcggaga      1620
   gtggtggccg gcgaaggatc tggctctgtg tctctggccc tgcatctgca gcctctgaga      1680
   agcgctgctg gcgctgcagc tctggcactg acagtggatc tgcctcctgc cagctccgag      1740
   gcccggaata gcgcatttgg gtttcaaggc aggctgctgc acctgtctgc cggccagagg      1800
   ctgggagtgc atctgcacac agaggccagg gctagacacg cctggcagct gacacagggc      1860
   gctacagtgc tgggcctgtt cagagtgacc cccgagattc cagccggcct gccttctcca      1920
   agaagcgaa                                                                1929
<210> 139
   <211> 2130
   <212> DNA
   <213> Artificial Sequence
<220>
   <223> nucleotide sequence of dimeric hu 4-1BBL (71-248) - CL* Fc knob chain
<400> 139
   agagagggcc ctgagctgag ccccgatgat cctgctggac tgctggacct gcggcagggc      60
   atgtttgctc agctggtggc ccagaacgtg ctgctgatcg atggccccct gtcctggtac     120
   agcgatcctg gactggctgg cgtgtcactg acaggcggcc tgagctacaa agaggacacc     180
   aaagaactgg tggtggccaa ggccggcgtg tactacgtgt tctttcagct ggaactgcgg     240
   agagtggtgg ccggcgaagg atctggctct gtgtctctgg ccctgcatct gcagcctctg     300
   agatctgctg ctggcgccgc tgctctggca ctgacagtgg atctgcctcc tgccagcagc     360
   gaggcccgga atagcgcatt tgggtttcaa ggcaggctgc tgcacctgtc tgccggccag     420
   aggctgggag tgcatctgca cacagaggcc agggctagac acgcctggca gctgacacag     480
   ggcgctacag tgctgggcct gttcagagtg acccccgaga ttccagccgg actgggaggc     540
   ggcggatctg gcggcggagg atctagagaa ggacccgagc tgtcccctga cgatccagcc     600
   gggctgctgg atctgagaca gggaatgttc gcccagctgg tggctcagaa tgtgctgctg     660
   attgacggac ctctgagctg gtactccgac ccagggctgg caggggtgtc cctgactggg     720
   ggactgtcct acaaagaaga tacaaaagaa ctggtggtgg ctaaagctgg ggtgtactat 780
   gtgttttttc agctggaact gaggcgggtg gtggctgggg agggctcagg atctgtgtcc 840
   ctggctctgc atctgcagcc actgcgctct gcagcagggg ctgcagcact ggccctgact 900
   gtggacctgc ccccagcttc ttccgaggcc agaaacagcg ccttcgggtt ccaaggacgc 960
   ctgctgcatc tgagcgccgg acagcgcctg ggagtgcatc tgcatactga agccagagcc 1020
   cggcatgctt ggcagctgac tcagggggca actgtgctgg gactgtttcg cgtgacacct 1080
   gagatccccg ctggactggg cggaggcggt tccggagggg gaggatctcg tacggtggct 1140
   gcaccatctg tctttatctt cccacccagc gaccggaagc tgaagtctgg cacagccagc 1200
   gtcgtgtgcc tgctgaataa cttctacccc cgcgaggcca aggtgcagtg gaaggtggac 1260
   aatgccctgc agagcggcaa cagccaggaa agcgtgaccg agcaggacag caaggactcc 1320
   acctacagcc tgagcagcac cctgaccctg agcaaggccg actacgagaa gcacaaggtg 1380
   tacgcctgcg aagtgaccca ccagggcctg tctagccccg tgaccaagag cttcaaccgg 1440
   ggcgagtgcg acaagaccca cacctgtcct ccatgccctg cccctgaagc tgctggcggc 1500
   cctagcgtgt tcctgttccc cccaaagccc aaggacaccc tgatgatcag ccggacccct 1560
   gaagtgacct gcgtggtggt ggatgtgtcc cacgaggacc ctgaagtgaa gttcaattgg 1620
   tacgtggacg gcgtggaagt gcacaatgcc aagaccaagc cgcgggagga gcagtacaac 1680
   agcacgtacc gtgtggtcag cgtcctcacc gtcctgcacc aggactggct gaatggcaag 1740
   gagtacaagt gcaaggtctc caacaaagcc ctcggcgccc ccatcgagaa aaccatctcc 1800
   aaagccaaag ggcagccccg agaaccacag gtgtacaccc tgcccccatg ccgggatgag 1860
   ctgaccaaga accaggtcag cctgtggtgc ctggtcaaag gcttctatcc cagcgacatc 1920
   gccgtggagt gggagagcaa tgggcagccg gagaacaact acaagaccac gcctcccgtg 1980
   ctggactccg acggctcctt cttcctctac agcaagctca ccgtggacaa gagcaggtgg 2040
   cagcagggga acgtcttctc atgctccgtg atgcatgagg ctctgcacaa ccactacacg 2100
   cagaagagcc tctccctgtc tccgggtaaa 2130
<210> 140
   <211> 873
   <212> DNA
   <213> Artificial Sequence
<220>
   <223> nucleotide sequence of monomeric hu 4-1BBL (71-248) - CH1*
<400> 140
   agagagggcc ctgagctgag ccccgatgat cctgctggac tgctggacct gcggcagggc 60
   atgtttgctc agctggtggc ccagaacgtg ctgctgatcg atggccccct gtcctggtac 120
   agcgatcctg gactggctgg cgtgtcactg acaggcggcc tgagctacaa agaggacacc 180
   aaagaactgg tggtggccaa ggccggcgtg tactacgtgt tctttcagct ggaactgcgg 240
   agagtggtgg ccggcgaagg atctggctct gtgtctctgg ccctgcatct gcagcctctg 300
   agatctgctg ctggcgccgc tgctctggca ctgacagtgg atctgcctcc tgccagcagc 360
   gaggcccgga atagcgcatt tgggtttcaa ggcaggctgc tgcacctgtc tgccggccag 420
   aggctgggag tgcatctgca cacagaggcc agggctagac acgcctggca gctgacacag 480
   ggcgctacag tgctgggcct gttcagagtg acccccgaga ttccagccgg actgggaggc 540
   ggaggttccg gaggcggagg atctgctagc acaaagggcc ccagcgtgtt ccctctggcc 600
   cctagcagca agagcacatc tggcggaaca gccgccctgg gctgcctggt ggaagattac 660
   ttccccgagc ccgtgaccgt gtcctggaat tctggcgccc tgacaagcgg cgtgcacacc 720
   tttccagccg tgctgcagag cagcggcctg tactctctga gcagcgtcgt gacagtgccc 780
   agcagctctc tgggcaccca gacctacatc tgcaacgtga accacaagcc cagcaacacc 840
   aaggtggacg agaaggtgga acccaagtcc tgc 873
<210> 141
   <211> 2130
   <212> DNA
   <213> Artificial Sequence
<220>
   <223> nucleotide sequence of dimeric hu 4-1BBL (71-248) - CL Fc knob chain
<400> 141
   agagagggcc ctgagctgag ccccgatgat cctgctggac tgctggacct gcggcagggc 60
   atgtttgctc agctggtggc ccagaacgtg ctgctgatcg atggccccct gtcctggtac 120
   agcgatcctg gactggctgg cgtgtcactg acaggcggcc tgagctacaa agaggacacc 180
   aaagaactgg tggtggccaa ggccggcgtg tactacgtgt tctttcagct ggaactgcgg 240
   agagtggtgg ccggcgaagg atctggctct gtgtctctgg ccctgcatct gcagcctctg 300
   agatctgctg ctggcgccgc tgctctggca ctgacagtgg atctgcctcc tgccagcagc 360
   gaggcccgga atagcgcatt tgggtttcaa ggcaggctgc tgcacctgtc tgccggccag 420
   aggctgggag tgcatctgca cacagaggcc agggctagac acgcctggca gctgacacag 480
   ggcgctacag tgctgggcct gttcagagtg acccccgaga ttccagccgg actgggaggc 540
   ggcggatctg gcggcggagg atctagagaa ggacccgagc tgtcccctga cgatccagcc 600
   gggctgctgg atctgagaca gggaatgttc gcccagctgg tggctcagaa tgtgctgctg 660
   attgacggac ctctgagctg gtactccgac ccagggctgg caggggtgtc cctgactggg 720
   ggactgtcct acaaagaaga tacaaaagaa ctggtggtgg ctaaagctgg ggtgtactat 780
   gtgttttttc agctggaact gaggcgggtg gtggctgggg agggctcagg atctgtgtcc 840
   ctggctctgc atctgcagcc actgcgctct gcagcagggg ctgcagcact ggccctgact 900
   gtggacctgc ccccagcttc ttccgaggcc agaaacagcg ccttcgggtt ccaaggacgc 960
   ctgctgcatc tgagcgccgg acagcgcctg ggagtgcatc tgcatactga agccagagcc 1020
   cggcatgctt ggcagctgac tcagggggca actgtgctgg gactgtttcg cgtgacacct 1080
   gagatccccg ctggactggg cggaggcggt tccggagggg gaggatctcg tacggtggcc 1140
   gctccctccg tgtttatctt tcccccatcc gatgaacagc tgaaaagcgg caccgcctcc 1200
   gtcgtgtgtc tgctgaacaa tttttaccct agggaagcta aagtgcagtg gaaagtggat 1260
   aacgcactgc agtccggcaa ctcccaggaa tctgtgacag aacaggactc caaggacagc 1320
   acctactccc tgtcctccac cctgacactg tctaaggctg attatgagaa acacaaagtc 1380
   tacgcctgcg aagtcaccca tcagggcctg agctcgcccg tcacaaagag cttcaacagg 1440
   ggagagtgtg acaagaccca cacctgtccc ccttgtcctg cccctgaagc tgctggcggc 1500
   ccttctgtgt tcctgttccc cccaaagccc aaggacaccc tgatgatcag ccggaccccc 1560
   gaagtgacct gcgtggtggt ggatgtgtcc cacgaggacc ctgaagtgaa gttcaattgg 1620
   tacgtggacg gcgtggaagt gcacaatgcc aagaccaagc cgcgggagga gcagtacaac 1680
   agcacgtacc gtgtggtcag cgtcctcacc gtcctgcacc aggactggct gaatggcaag 1740
   gagtacaagt gcaaggtctc caacaaagcc ctcggcgccc ccatcgagaa aaccatctcc 1800
   aaagccaaag ggcagccccg agaaccacag gtgtacaccc tgcccccatg ccgggatgag 1860
   ctgaccaaga accaggtcag cctgtggtgc ctggtcaaag gcttctatcc cagcgacatc 1920
   gccgtggagt gggagagcaa tgggcagccg gagaacaact acaagaccac gcctcccgtg 1980
   ctggactccg acggctcctt cttcctctac agcaagctca ccgtggacaa gagcaggtgg 2040
   cagcagggga acgtcttctc atgctccgtg atgcatgagg ctctgcacaa ccactacacg 2100
   cagaagagcc tctccctgtc tccgggtaaa 2130
<210> 142
   <211> 873
   <212> DNA
   <213> Artificial Sequence
<220>
   <223> nucleotide sequence of monomeric hu 4-1BBL (71-248) - CH1
<400> 142
   agagagggcc ctgagctgag ccccgatgat cctgctggac tgctggacct gcggcagggc      60
   atgtttgctc agctggtggc ccagaacgtg ctgctgatcg atggccccct gtcctggtac     120
   agcgatcctg gactggctgg cgtgtcactg acaggcggcc tgagctacaa agaggacacc     180
   aaagaactgg tggtggccaa ggccggcgtg tactacgtgt tctttcagct ggaactgcgg     240
   agagtggtgg ccggcgaagg atctggctct gtgtctctgg ccctgcatct gcagcctctg     300
   agatctgctg ctggcgccgc tgctctggca ctgacagtgg atctgcctcc tgccagcagc     360
   gaggcccgga atagcgcatt tgggtttcaa ggcaggctgc tgcacctgtc tgccggccag     420
   aggctgggag tgcatctgca cacagaggcc agggctagac acgcctggca gctgacacag     480
   ggcgctacag tgctgggcct gttcagagtg acccccgaga ttccagccgg actgggaggc     540
   ggaggttccg gaggcggagg atctgctagc accaaaggcc cttccgtgtt tcctctggct     600
   cctagctcca agtccacctc tggaggcacc gctgctctcg gatgcctcgt gaaggattat     660
   tttcctgagc ctgtgacagt gtcctggaat agcggagcac tgacctctgg agtgcatact     720
   ttccccgctg tgctgcagtc ctctggactg tacagcctga gcagcgtggt gacagtgccc     780
   agcagcagcc tgggcaccca gacctacatc tgcaacgtga accacaagcc cagcaacacc     840
   aaggtggaca agaaggtgga acccaagtct tgt                                  873
<210> 143
   <211> 2475
   <212> DNA
   <213> Artificial Sequence
<220>
   <223> nucleotide sequence of anti-PD1(0314) Fc hole chain fused to dimeric hu 4-1BBL (71-248)
<400> 143
   gaagtgcagc tgctcgaaag cggcggggga ctggtccagc ccggcggttc cctgaggctg 60
   tcttgcgccg cttcagggtt cagcttttcc tcttacacca tgagttgggt cagacaggca 120
   cctggcaagg gactggagtg ggtcgccaca atcagcggtg gcgggcgcga catttattac 180
   ccagattccg tgaaaggacg gttcaccatc tctagggaca actcaaagaa tactctgtat 240
   ttgcagatga acagcctgag agctgaggat acagcagttt actactgtgt gctcctgacc 300
   ggccgcgtct attttgccct tgactcctgg ggacaaggca ctctggtgac cgtatctagt 360
   gctagcacca agggcccctc cgtgttcccc ctggccccca gcagcaagag caccagcggc 420
   ggcacagccg ctctgggctg cctggtcaag gactacttcc ccgagcccgt gaccgtgtcc 480
   tggaacagcg gagccctgac ctccggcgtg cacaccttcc ccgccgtgct gcagagttct 540
   ggcctgtata gcctgagcag cgtggtcacc gtgccttcta gcagcctggg cacccagacc 600
   tacatctgca acgtgaacca caagcccagc aacaccaagg tggacaagaa ggtggagccc 660
   aagagctgcg acaaaactca cacatgccca ccgtgcccag cacctgaagc tgcaggggga 720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacaac 900
   agcacgtacc gtgtggtcag cgtcctcacc gtcctgcacc aggactggct gaatggcaag 960
   gagtacaagt gcaaggtctc caacaaagcc ctcggcgccc ccatcgagaa aaccatctcc 1020
   aaagccaaag ggcagccccg agaaccacag gtgtgcaccc tgcccccatc ccgggatgag 1080
   ctgaccaaga accaggtcag cctctcgtgc gcagtcaaag gcttctatcc cagcgacatc 1140
   gccgtggagt gggagagcaa tgggcagccg gagaacaact acaagaccac gcctcccgtg 1200
   ctggactccg acggctcctt cttcctcgtg agcaagctca ccgtggacaa gagcaggtgg 1260
   cagcagggga acgtcttctc atgctccgtg atgcatgagg ctctgcacaa ccactacacg 1320
   cagaagagcc tctccctgtc tccgggtgga ggcggcggaa gcggaggagg aggatccaga 1380
   gagggccctg agctgagccc tgatgatcct gccggactgc tggacctgcg gcagggaatg 1440
   tttgcccagc tggtggccca gaacgtgctg ctgatcgatg gccccctgtc ctggtacagc 1500
   gatcctggac tggctggcgt gtcactgaca ggcggcctga gctacaaaga ggacaccaaa 1560
   gaactggtgg tggccaaggc cggcgtgtac tacgtgttct ttcagctgga actgcggaga 1620
   gtggtggccg gcgaaggatc tggctctgtg tctctggccc tgcatctgca gcctctgaga 1680
   tctgctgctg gcgccgctgc tctggcactg acagtggatc tgcctcctgc cagcagcgag 1740
   gcccggaata gcgcatttgg gtttcaaggc aggctgctgc acctgtctgc cggccagagg 1800
   ctgggagtgc atctgcacac agaggccagg gctagacacg cctggcagct gacacagggc 1860
   gctacagtgc tgggcctgtt cagagtgacc cccgagattc cagcaggcct gggaggcggc 1920
   ggatctggcg gcggaggatc tagagaagga cccgagctgt cccccgacga tcccgctggg 1980
   ctgctggatc tgagacaggg catgttcgct cagctggtgg ctcagaatgt gctgctgatt 2040
   gacggacctc tgagctggta ctccgaccca gggctggcag gggtgtccct gactggggga 2100
   ctgtcctaca aagaagatac aaaagaactg gtggtggcta aagctggggt gtactatgtg 2160
   ttttttcagc tggaactgag gcgggtggtg gctggggagg gctcaggatc tgtgtccctg 2220
   gctctgcatc tgcagccact gcgctctgca gcaggggctg cagcactggc cctgactgtg 2280
   gacctgcccc cagcttcttc cgaggccaga aacagcgcct tcgggttcca aggacgcctg 2340
   ctgcatctga gcgccggaca gcgcctggga gtgcatctgc atactgaagc cagagcccgg 2400
   catgcttggc agctgactca gggggcaact gtgctgggac tgtttcgcgt gacacctgag 2460
   atcccagccg ggctc 2475
<210> 144
   <211> 1911
   <212> DNA
   <213> Artificial Sequence
<220>
   <223> nucleotide sequence anti-PD1(0314) Fc knob chain fused to monomeric hu 4-1BBL (71-248)
<400> 144
   gaagtgcagc tgctcgaaag cggcggggga ctggtccagc ccggcggttc cctgaggctg 60
   tcttgcgccg cttcagggtt cagcttttcc tcttacacca tgagttgggt cagacaggca 120
   cctggcaagg gactggagtg ggtcgccaca atcagcggtg gcgggcgcga catttattac 180
   ccagattccg tgaaaggacg gttcaccatc tctagggaca actcaaagaa tactctgtat 240
   ttgcagatga acagcctgag agctgaggat acagcagttt actactgtgt gctcctgacc 300
   ggccgcgtct attttgccct tgactcctgg ggacaaggca ctctggtgac cgtatctagt 360
   gctagcacca agggcccatc ggtcttcccc ctggcaccct cctccaagag cacctctggg 420
   ggcacagcgg ccctgggctg cctggtcaag gactacttcc ccgaaccggt gacggtgtcg 480
   tggaactcag gcgccctgac cagcggcgtg cacaccttcc cggctgtcct acagtcctca 540
   ggactctact ccctcagcag cgtggtgacc gtgccctcca gcagcttggg cacccagacc 600
   tacatctgca acgtgaatca caagcccagc aacaccaagg tggacaagaa agttgagccc 660
   aaatcttgtg acaaaactca cacatgccca ccgtgcccag cacctgaagc tgcaggggga 720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacaac 900
   agcacgtacc gtgtggtcag cgtcctcacc gtcctgcacc aggactggct gaatggcaag 960
   gagtacaagt gcaaggtctc caacaaagcc ctcggcgccc ccatcgagaa aaccatctcc 1020
   aaagccaaag ggcagccccg agaaccacag gtgtacaccc tgcccccctg cagagatgag 1080
   ctgaccaaga accaggtgtc cctgtggtgt ctggtcaagg gcttctaccc cagcgatatc 1140
   gccgtggagt gggagagcaa cggccagcct gagaacaact acaagaccac cccccctgtg 1200
   ctggacagcg acggcagctt cttcctgtac tccaaactga ccgtggacaa gagccggtgg 1260
   cagcagggca acgtgttcag ctgcagcgtg atgcacgagg ccctgcacaa ccactacacc 1320
   cagaagtccc tgagcctgag ccccggcgga ggcggcggaa gcggaggagg aggatccaga 1380
   gagggccctg agctgagccc tgatgatcct gccggactgc tggacctgcg gcagggaatg 1440
   tttgcccagc tggtggccca gaacgtgctg ctgatcgatg gccccctgtc ctggtacagc 1500
   gatcctggac tggctggcgt gtcactgaca ggcggcctga gctacaaaga ggacaccaaa 1560
   gaactggtgg tggccaaggc cggcgtgtac tacgtgttct ttcagctgga actgcggaga 1620
   gtggtggccg gcgaaggatc tggctctgtg tctctggccc tgcatctgca gcctctgaga 1680
   tctgctgctg gcgccgctgc tctggcactg acagtggatc tgcctcctgc cagcagcgag 1740
   gcccggaata gcgcatttgg gtttcaaggc aggctgctgc acctgtctgc cggccagagg 1800
   ctgggagtgc atctgcacac agaggccagg gctagacacg cctggcagct gacacagggc 1860
   gctacagtgc tgggcctgtt cagagtgacc cccgagattc ctgccgggct c 1911
<210> 145
   <211> 1806
   <212> DNA
   <213> Artificial Sequence
<220>
   <223> nucleotide sequence of Fc hole dimeric ligand (71-248) chain
<400> 145
   gacaaaactc acacatgccc accgtgccca gcacctgaag ctgcaggggg accgtcagtc 60
   ttcctcttcc ccccaaaacc caaggacacc ctcatgatct cccggacccc tgaggtcaca 120
   tgcgtggtgg tggacgtgag ccacgaagac cctgaggtca agttcaactg gtacgtggac 180
   ggcgtggagg tgcataatgc caagacaaag ccgcgggagg agcagtacaa cagcacgtac 240
   cgtgtggtca gcgtcctcac cgtcctgcac caggactggc tgaatggcaa ggagtacaag 300
   tgcaaggtct ccaacaaagc cctcggcgcc cccatcgaga aaaccatctc caaagccaaa 360
   gggcagcccc gagaaccaca ggtgtgcacc ctgcccccat cccgggatga gctgaccaag 420
   aaccaggtca gcctctcgtg cgcagtcaaa ggcttctatc ccagcgacat cgccgtggag 480
   tgggagagca atgggcagcc ggagaacaac tacaagacca cgcctcccgt gctggactcc 540
   gacggctcct tcttcctcgt gagcaagctc accgtggaca agagcaggtg gcagcagggg 600
   aacgtcttct catgctccgt gatgcatgag gctctgcaca accactacac gcagaagagc 660
   ctctccctgt ctccgggtgg aggcggcgga agcggaggag gaggatccag agagggccct 720
   gagctgagcc ctgatgatcc tgccggactg ctggacctgc ggcagggaat gtttgcccag 780
   ctggtggccc agaacgtgct gctgatcgat ggccccctgt cctggtacag cgatcctgga 840
   ctggctggcg tgtcactgac aggcggcctg agctacaaag aggacaccaa agaactggtg 900
   gtggccaagg ccggcgtgta ctacgtgttc tttcagctgg aactgcggag agtggtggcc 960
   ggcgaaggat ctggctctgt gtctctggcc ctgcatctgc agcctctgag atctgctgct 1020
   ggcgccgctg ctctggcact gacagtggat ctgcctcctg ccagcagcga ggcccggaat 1080
   agcgcatttg ggtttcaagg caggctgctg cacctgtctg ccggccagag gctgggagtg 1140
   catctgcaca cagaggccag ggctagacac gcctggcagc tgacacaggg cgctacagtg 1200
   ctgggcctgt tcagagtgac ccccgagatt ccagcaggcc tgggaggcgg cggatctggc 1260
   ggcggaggat ctagagaagg acccgagctg tcccccgacg atcccgctgg gctgctggat 1320
   ctgagacagg gcatgttcgc tcagctggtg gctcagaatg tgctgctgat tgacggacct 1380
   ctgagctggt actccgaccc agggctggca ggggtgtccc tgactggggg actgtcctac 1440
   aaagaagata caaaagaact ggtggtggct aaagctgggg tgtactatgt gttttttcag 1500
   ctggaactga ggcgggtggt ggctggggag ggctcaggat ctgtgtccct ggctctgcat 1560
   ctgcagccac tgcgctctgc agcaggggct gcagcactgg ccctgactgt ggacctgccc 1620
   ccagcttctt ccgaggccag aaacagcgcc ttcgggttcc aaggacgcct gctgcatctg 1680
   agcgccggac agcgcctggg agtgcatctg catactgaag ccagagcccg gcatgcttgg 1740
   cagctgactc agggggcaac tgtgctggga ctgtttcgcg tgacacctga gatcccagcc 1800
   gggctc 1806
<210> 146
   <211> 1212
   <212> DNA
   <213> Artificial Sequence
<220>
   <223> nucleotide sequence of Fc hole monomeric ligand (71-248) chain
<400> 146
   gacaaaactc acacatgccc accgtgccca gcacctgaag ctgcaggggg accgtcagtc      60
   ttcctcttcc ccccaaaacc caaggacacc ctcatgatct cccggacccc tgaggtcaca     120
   tgcgtggtgg tggacgtgag ccacgaagac cctgaggtca agttcaactg gtacgtggac     180
   ggcgtggagg tgcataatgc caagacaaag ccgcgggagg agcagtacaa cagcacgtac     240
   cgtgtggtca gcgtcctcac cgtcctgcac caggactggc tgaatggcaa ggagtacaag     300
   tgcaaggtct ccaacaaagc cctcggcgcc cccatcgaga aaaccatctc caaagccaaa     360
   gggcagcccc gagaaccaca ggtgtgcacc ctgcccccat cccgggatga gctgaccaag     420
   aaccaggtca gcctctcgtg cgcagtcaaa ggcttctatc ccagcgacat cgccgtggag     480
   tgggagagca atgggcagcc ggagaacaac tacaagacca cgcctcccgt gctggactcc     540
   gacggctcct tcttcctcgt gagcaagctc accgtggaca agagcaggtg gcagcagggg     600
   aacgtcttct catgctccgt gatgcatgag gctctgcaca accactacac gcagaagagc     660
   ctctccctgt ctccgggtag agagggccct gagctgagcc ctgatgatcc tgccggactg     720
   ctggacctgc ggcagggaat gtttgcccag ctggtggccc agaacgtgct gctgatcgat     780
   ggccccctgt cctggtacag cgatcctgga ctggctggcg tgtcactgac aggcggcctg     840
   agctacaaag aggacaccaa agaactggtg gtggccaagg ccggcgtgta ctacgtgttc     900
   tttcagctgg aactgcggag agtggtggcc ggcgaaggat ctggctctgt gtctctggcc     960
   ctgcatctgc agcctctgag atctgctgct ggcgccgctg ctctggcact gacagtggat    1020
   ctgcctcctg ccagcagcga ggcccggaat agcgcatttg ggtttcaagg caggctgctg    1080
   cacctgtctg ccggccagag gctgggagtg catctgcaca cagaggccag ggctagacac    1140
   gcctggcagc tgacacaggg cgctacagtg ctgggcctgt tcagagtgac ccccgagatt    1200
   cctgccgggc tc                                                        1212
<210> 147
   <211> 2445
   <212> DNA
   <213> Artificial Sequence
<220>
   <223> nucleotide sequence of anti-PD1(0314) Fc knob chain fused to dimeric hu 4-1BBL (71-248)
<400> 147
   gaagtgcagc tgctcgaaag cggcggggga ctggtccagc ccggcggttc cctgaggctg 60
   tcttgcgccg cttcagggtt cagcttttcc tcttacacca tgagttgggt cagacaggca 120
   cctggcaagg gactggagtg ggtcgccaca atcagcggtg gcgggcgcga catttattac 180
   ccagattccg tgaaaggacg gttcaccatc tctagggaca actcaaagaa tactctgtat 240
   ttgcagatga acagcctgag agctgaggat acagcagttt actactgtgt gctcctgacc 300
   ggccgcgtct attttgccct tgactcctgg ggacaaggca ctctggtgac cgtatctagt 360
   gctagcacca agggcccatc ggtcttcccc ctggcaccct cctccaagag cacctctggg 420
   ggcacagcgg ccctgggctg cctggtcaag gactacttcc ccgaaccggt gacggtgtcg 480
   tggaactcag gcgccctgac cagcggcgtg cacaccttcc cggctgtcct acagtcctca 540
   ggactctact ccctcagcag cgtggtgacc gtgccctcca gcagcttggg cacccagacc 600
   tacatctgca acgtgaatca caagcccagc aacaccaagg tggacaagaa agttgagccc     660
   aaatcttgtg acaaaactca cacatgccca ccgtgcccag cacctgaagc tgcaggggga     720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacaac 900
   agcacgtacc gtgtggtcag cgtcctcacc gtcctgcacc aggactggct gaatggcaag 960
   gagtacaagt gcaaggtctc caacaaagcc ctcggcgccc ccatcgagaa aaccatctcc 1020
   aaagccaaag ggcagccccg agaaccacag gtgtacaccc tgcccccctg cagagatgag 1080
   ctgaccaaga accaggtgtc cctgtggtgt ctggtcaagg gcttctaccc cagcgatatc 1140
   gccgtggagt gggagagcaa cggccagcct gagaacaact acaagaccac cccccctgtg 1200
   ctggacagcg acggcagctt cttcctgtac tccaaactga ccgtggacaa gagccggtgg 1260
   cagcagggca acgtgttcag ctgcagcgtg atgcacgagg ccctgcacaa ccactacacc 1320
   cagaagtccc tgagcctgag ccccggcaga gagggccctg agctgagccc tgatgatcct 1380
   gccggactgc tggacctgcg gcagggaatg tttgcccagc tggtggccca gaacgtgctg    1440
   ctgatcgatg gccccctgtc ctggtacagc gatcctggac tggctggcgt gtcactgaca    1500
   ggcggcctga gctacaaaga ggacaccaaa gaactggtgg tggccaaggc cggcgtgtac 1560
   tacgtgttct ttcagctgga actgcggaga gtggtggccg gcgaaggatc tggctctgtg 1620
   tctctggccc tgcatctgca gcctctgaga tctgctgctg gcgccgctgc tctggcactg 1680
   acagtggatc tgcctcctgc cagcagcgag gcccggaata gcgcatttgg gtttcaaggc 1740
   aggctgctgc acctgtctgc cggccagagg ctgggagtgc atctgcacac agaggccagg 1800
   gctagacacg cctggcagct gacacagggc gctacagtgc tgggcctgtt cagagtgacc 1860
   cccgagattc cagcaggcct gggaggcggc ggatctggcg gcggaggatc tagagaagga 1920
   cccgagctgt cccccgacga tcccgctggg ctgctggatc tgagacaggg catgttcgct 1980
   cagctggtgg ctcagaatgt gctgctgatt gacggacctc tgagctggta ctccgaccca 2040
   gggctggcag gggtgtccct gactggggga ctgtcctaca aagaagatac aaaagaactg 2100
   gtggtggcta aagctggggt gtactatgtg ttttttcagc tggaactgag gcgggtggtg 2160
   gctggggagg gctcaggatc tgtgtccctg gctctgcatc tgcagccact gcgctctgca 2220
   gcaggggctg cagcactggc cctgactgtg gacctgcccc cagcttcttc cgaggccaga 2280
   aacagcgcct tcgggttcca aggacgcctg ctgcatctga gcgccggaca gcgcctggga 2340
   gtgcatctgc atactgaagc cagagcccgg catgcttggc agctgactca gggggcaact 2400
   gtgctgggac tgtttcgcgt gacacctgag atcccagccg ggctc 2445
<210> 148
   <211> 1242
   <212> DNA
   <213> Artificial Sequence
<220>
   <223> nucleotide sequence of Fc knob chain fused to monomeric hu 4-1BBL (71-248)
<400> 148
   gacaaaactc acacatgccc accgtgccca gcacctgaag ctgcaggggg accgtcagtc        60
   ttcctcttcc ccccaaaacc caaggacacc ctcatgatct cccggacccc tgaggtcaca       120
   tgcgtggtgg tggacgtgag ccacgaagac cctgaggtca agttcaactg gtacgtggac       180
   ggcgtggagg tgcataatgc caagacaaag ccgcgggagg agcagtacaa cagcacgtac       240
   cgtgtggtca gcgtcctcac cgtcctgcac caggactggc tgaatggcaa ggagtacaag       300
   tgcaaggtct ccaacaaagc cctcggcgcc cccatcgaga aaaccatctc caaagccaaa       360
   gggcagcccc gagaaccaca ggtgtacacc ctgcccccct gcagagatga gctgaccaag       420
   aaccaggtgt ccctgtggtg tctggtcaag ggcttctacc ccagcgatat cgccgtggag       480
   tgggagagca acggccagcc tgagaacaac tacaagacca ccccccctgt gctggacagc       540
   gacggcagct tcttcctgta ctccaaactg accgtggaca agagccggtg gcagcagggc       600
   aacgtgttca gctgcagcgt gatgcacgag gccctgcaca accactacac ccagaagtcc       660
   ctgagcctga gccccggcgg aggcggcgga agcggaggag gaggatccag agagggccct       720
   gagctgagcc ctgatgatcc tgccggactg ctggacctgc ggcagggaat gtttgcccag       780
   ctggtggccc agaacgtgct gctgatcgat ggccccctgt cctggtacag cgatcctgga       840
   ctggctggcg tgtcactgac aggcggcctg agctacaaag aggacaccaa agaactggtg       900
   gtggccaagg ccggcgtgta ctacgtgttc tttcagctgg aactgcggag agtggtggcc       960
   ggcgaaggat ctggctctgt gtctctggcc ctgcatctgc agcctctgag atctgctgct      1020
   ggcgccgctg ctctggcact gacagtggat ctgcctcctg ccagcagcga ggcccggaat      1080
   agcgcatttg ggtttcaagg caggctgctg cacctgtctg ccggccagag gctgggagtg      1140
   catctgcaca cagaggccag ggctagacac gcctggcagc tgacacaggg cgctacagtg      1200
   ctgggcctgt tcagagtgac ccccgagatt cctgccgggc tc                         1242
<210> 149
   <211> 1911
   <212> DNA
   <213> Artificial Sequence
<220>
   <223> nucleotide sequence of anti-PD1(0314) Fc hole monomeric ligand (71-248) chain
<400> 149
   gaagtgcagc tgctcgaaag cggcggggga ctggtccagc ccggcggttc cctgaggctg 60
   tcttgcgccg cttcagggtt cagcttttcc tcttacacca tgagttgggt cagacaggca 120
   cctggcaagg gactggagtg ggtcgccaca atcagcggtg gcgggcgcga catttattac 180
   ccagattccg tgaaaggacg gttcaccatc tctagggaca actcaaagaa tactctgtat 240
   ttgcagatga acagcctgag agctgaggat acagcagttt actactgtgt gctcctgacc 300
   ggccgcgtct attttgccct tgactcctgg ggacaaggca ctctggtgac cgtatctagt 360
   gctagcacca agggcccctc cgtgttcccc ctggccccca gcagcaagag caccagcggc 420
   ggcacagccg ctctgggctg cctggtcaag gactacttcc ccgagcccgt gaccgtgtcc 480
   tggaacagcg gagccctgac ctccggcgtg cacaccttcc ccgccgtgct gcagagttct 540
   ggcctgtata gcctgagcag cgtggtcacc gtgccttcta gcagcctggg cacccagacc 600
   tacatctgca acgtgaacca caagcccagc aacaccaagg tggacaagaa ggtggagccc 660
   aagagctgcg acaaaactca cacatgccca ccgtgcccag cacctgaagc tgcaggggga 720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacaac 900
   agcacgtacc gtgtggtcag cgtcctcacc gtcctgcacc aggactggct gaatggcaag 960
   gagtacaagt gcaaggtctc caacaaagcc ctcggcgccc ccatcgagaa aaccatctcc 1020
   aaagccaaag ggcagccccg agaaccacag gtgtgcaccc tgcccccatc ccgggatgag 1080
   ctgaccaaga accaggtcag cctctcgtgc gcagtcaaag gcttctatcc cagcgacatc 1140
   gccgtggagt gggagagcaa tgggcagccg gagaacaact acaagaccac gcctcccgtg 1200
   ctggactccg acggctcctt cttcctcgtg agcaagctca ccgtggacaa gagcaggtgg 1260
   cagcagggga acgtcttctc atgctccgtg atgcatgagg ctctgcacaa ccactacacg 1320
   cagaagagcc tctccctgtc tccgggtgga ggcggcggaa gcggaggagg aggatccaga 1380
   gagggccctg agctgagccc tgatgatcct gccggactgc tggacctgcg gcagggaatg 1440
   tttgcccagc tggtggccca gaacgtgctg ctgatcgatg gccccctgtc ctggtacagc 1500
   gatcctggac tggctggcgt gtcactgaca ggcggcctga gctacaaaga ggacaccaaa 1560
   gaactggtgg tggccaaggc cggcgtgtac tacgtgttct ttcagctgga actgcggaga 1620
   gtggtggccg gcgaaggatc tggctctgtg tctctggccc tgcatctgca gcctctgaga 1680
   tctgctgctg gcgccgctgc tctggcactg acagtggatc tgcctcctgc cagcagcgag 1740
   gcccggaata gcgcatttgg gtttcaaggc aggctgctgc acctgtctgc cggccagagg 1800
   ctgggagtgc atctgcacac agaggccagg gctagacacg cctggcagct gacacagggc 1860
   gctacagtgc tgggcctgtt cagagtgacc cccgagattc ctgccgggct c 1911
<210> 150
   <211> 1806
   <212> DNA
   <213> Artificial Sequence
<220>
   <223> nucleotide sequence of Fc knob chain fused to dimeric hu 4-1BBL (71-248)
<400> 150
   gacaaaactc acacatgccc accgtgccca gcacctgaag ctgcaggggg accgtcagtc 60
   ttcctcttcc ccccaaaacc caaggacacc ctcatgatct cccggacccc tgaggtcaca 120
   tgcgtggtgg tggacgtgag ccacgaagac cctgaggtca agttcaactg gtacgtggac 180
   ggcgtggagg tgcataatgc caagacaaag ccgcgggagg agcagtacaa cagcacgtac 240
   cgtgtggtca gcgtcctcac cgtcctgcac caggactggc tgaatggcaa ggagtacaag 300
   tgcaaggtct ccaacaaagc cctcggcgcc cccatcgaga aaaccatctc caaagccaaa 360
   gggcagcccc gagaaccaca ggtgtacacc ctgcccccct gcagagatga gctgaccaag 420
   aaccaggtgt ccctgtggtg tctggtcaag ggcttctacc ccagcgatat cgccgtggag 480
   tgggagagca acggccagcc tgagaacaac tacaagacca ccccccctgt gctggacagc 540
   gacggcagct tcttcctgta ctccaaactg accgtggaca agagccggtg gcagcagggc 600
   aacgtgttca gctgcagcgt gatgcacgag gccctgcaca accactacac ccagaagtcc 660
   ctgagcctga gccccggcgg aggcggcgga agcggaggag gaggatccag agagggccct 720
   gagctgagcc ctgatgatcc tgccggactg ctggacctgc ggcagggaat gtttgcccag 780
   ctggtggccc agaacgtgct gctgatcgat ggccccctgt cctggtacag cgatcctgga 840
   ctggctggcg tgtcactgac aggcggcctg agctacaaag aggacaccaa agaactggtg 900
   gtggccaagg ccggcgtgta ctacgtgttc tttcagctgg aactgcggag agtggtggcc 960
   ggcgaaggat ctggctctgt gtctctggcc ctgcatctgc agcctctgag atctgctgct 1020
   ggcgccgctg ctctggcact gacagtggat ctgcctcctg ccagcagcga ggcccggaat 1080
   agcgcatttg ggtttcaagg caggctgctg cacctgtctg ccggccagag gctgggagtg 1140
   catctgcaca cagaggccag ggctagacac gcctggcagc tgacacaggg cgctacagtg 1200
   ctgggcctgt tcagagtgac ccccgagatt ccagcaggcc tgggaggcgg cggatctggc 1260
   ggcggaggat ctagagaagg acccgagctg tcccccgacg atcccgctgg gctgctggat 1320
   ctgagacagg gcatgttcgc tcagctggtg gctcagaatg tgctgctgat tgacggacct 1380
   ctgagctggt actccgaccc agggctggca ggggtgtccc tgactggggg actgtcctac 1440
   aaagaagata caaaagaact ggtggtggct aaagctgggg tgtactatgt gttttttcag 1500
   ctggaactga ggcgggtggt ggctggggag ggctcaggat ctgtgtccct ggctctgcat 1560
   ctgcagccac tgcgctctgc agcaggggct gcagcactgg ccctgactgt ggacctgccc 1620
   ccagcttctt ccgaggccag aaacagcgcc ttcgggttcc aaggacgcct gctgcatctg 1680
   agcgccggac agcgcctggg agtgcatctg catactgaag ccagagcccg gcatgcttgg 1740
   cagctgactc agggggcaac tgtgctggga ctgtttcgcg tgacacctga gatcccagcc 1800
   gggctc 1806
<210> 151
   <211> 1335
   <212> DNA
   <213> Artificial Sequence
<220>
   <223> nucleotide sequence of DP47 Fc hole chain
<400> 151
   gaggtgcaat tgttggagtc tgggggaggc ttggtacagc ctggggggtc cctgagactc      60
   tcctgtgcag cctccggatt cacctttagc agttatgcca tgagctgggt ccgccaggct     120
   ccagggaagg ggctggagtg ggtctcagct attagtggta gtggtggtag cacatactac     180
   gcagactccg tgaagggccg gttcaccatc tccagagaca attccaagaa cacgctgtat     240
   ctgcagatga acagcctgag agccgaggac acggccgtat attactgtgc gaaaggcagc     300
   ggatttgact actggggcca aggaaccctg gtcaccgtct cgagtgctag caccaagggc     360
   ccatcggtct tccccctggc accctcctcc aagagcacct ctgggggcac agcggccctg     420
   ggctgcctgg tcaaggacta cttccccgaa ccggtgacgg tgtcgtggaa ctcaggcgcc     480
   ctgaccagcg gcgtgcacac cttcccggct gtcctacagt cctcaggact ctactccctc     540
   agcagcgtgg tgaccgtgcc ctccagcagc ttgggcaccc agacctacat ctgcaacgtg     600
   aatcacaagc ccagcaacac caaggtggac aagaaagttg agcccaaatc ttgtgacaaa     660
   actcacacat gcccaccgtg cccagcacct gaagctgcag ggggaccgtc agtcttcctc    720
   ttccccccaa aacccaagga caccctcatg atctcccgga cccctgaggt cacatgcgtg     780
   gtggtggacg tgagccacga agaccctgag gtcaagttca actggtacgt ggacggcgtg     840
   gaggtgcata atgccaagac aaagccgcgg gaggagcagt acaacagcac gtaccgtgtg     900
   gtcagcgtcc tcaccgtcct gcaccaggac tggctgaatg gcaaggagta caagtgcaag     960
   gtctccaaca aagccctcgg cgcccccatc gagaaaacca tctccaaagc caaagggcag    1020
   ccccgagaac cacaggtgtg caccctgccc ccatcccggg atgagctgac caagaaccag    1080
   gtcagcctct cgtgcgcagt caaaggcttc tatcccagcg acatcgccgt ggagtgggag    1140
   agcaatgggc agccggagaa caactacaag accacgcctc ccgtgctgga ctccgacggc    1200
   tccttcttcc tcgtgagcaa gctcaccgtg gacaagagca ggtggcagca ggggaacgtc    1260
   ttctcatgct ccgtgatgca tgaggctctg cacaaccact acacgcagaa gagcctctcc    1320
   ctgtctccgg gtaaa                                                     1335
<210> 152
   <211> 645
   <212> DNA
   <213> Artificial Sequence
<220>
   <223> nucleotide sequence of DP47 light chain
<400> 152
   gaaatcgtgt taacgcagtc tccaggcacc ctgtctttgt ctccagggga aagagccacc      60
   ctctcttgca gggccagtca gagtgttagc agcagctact tagcctggta ccagcagaaa     120
   cctggccagg ctcccaggct cctcatctat ggagcatcca gcagggccac tggcatccca     180
   gacaggttca gtggcagtgg atccgggaca gacttcactc tcaccatcag cagactggag     240
   cctgaagatt ttgcagtgta ttactgtcag cagtatggta gctcaccgct gacgttcggc     300
   caggggacca aagtggaaat caaacgtacg gtggctgcac catctgtctt catcttcccg     360
   ccatctgatg agcagttgaa atctggaact gcctctgttg tgtgcctgct gaataacttc     420
   tatcccagag aggccaaagt acagtggaag gtggataacg ccctccaatc gggtaactcc     480
   caggagagtg tcacagagca ggacagcaag gacagcacct acagcctcag cagcaccctg     540
   acgctgagca aagcagacta cgagaaacac aaagtctacg cctgcgaagt cacccatcag     600
   ggcctgagct cgcccgtcac aaagagcttc aacaggggag agtgt                     645
<210> 153
   <211> 445
   <212> PRT
   <213> Artificial Sequence
<220>
   <223> DP47 Fc hole chain
<400> 153
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ser Gly Phe Asp Tyr Trp Gly Gln Gly Thr Leu Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
   195 200 205
   Val Asp Lys Lys Val Glu Pro Lys Ser Cys Asp Lys Thr His Thr Cys
   210 215 220
   Pro Pro Cys Pro Ala Pro Glu Ala Ala Gly Gly Pro Ser Val Phe Leu
   225 230 235 240
   Phe Pro Pro Lys Pro Lys Asp Thr Leu Met Ile Ser Arg Thr Pro Glu
   245 250 255
   Val Thr Cys Val Val Val Asp Val Ser His Glu Asp Pro Glu Val Lys
   260 265 270
   Phe Asn Trp Tyr Val Asp Gly Val Glu Val His Asn Ala Lys Thr Lys
   275 280 285
   Pro Arg Glu Glu Gln Tyr Asn Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Ala Leu Gly Ala Pro Ile Glu Lys Thr Ile Ser Lys
   325 330 335
   Ala Lys Gly Gln Pro Arg Glu Pro Gln Val Cys Thr Leu Pro Pro Ser
   340 345 350
   Arg Asp Glu Leu Thr Lys Asn Gln Val Ser Leu Ser Cys Ala Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Val Ser Lys Leu Thr Val Asp Lys Ser Arg Trp Gln
   405 410 415
   Gln Gly Asn Val Phe Ser Cys Ser Val Met His Glu Ala Leu His Asn
   420 425 430
   His Tyr Thr Gln Lys Ser Leu Ser Leu Ser Pro Gly Lys
   435 440 445
<210> 154
   <211> 215
   <212> PRT
   <213> Artificial Sequence
<220>
   <223> DP47 light chain
<400> 154
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Leu Thr Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 155
   <211> 2496
   <212> DNA
   <213> Artificial Sequence
<220>
   <223> nucleotide sequence of DP47 Fc hole chain fused to dimeric hu 4-1BBL (71-254)
<400> 155
   gaggtgcaat tgttggagtc tgggggaggc ttggtacagc ctggggggtc cctgagactc      60
   tcctgtgcag cctccggatt cacctttagc agttatgcca tgagctgggt ccgccaggct     120
   ccagggaagg ggctggagtg ggtctcagct attagtggta gtggtggtag cacatactac     180
   gcagactccg tgaagggccg gttcaccatc tccagagaca attccaagaa cacgctgtat     240
   ctgcagatga acagcctgag agccgaggac acggccgtat attactgtgc gaaaggcagc     300
   ggatttgact actggggcca aggaaccctg gtcaccgtct cgagtgctag caccaagggc     360
   ccctccgtgt tccccctggc ccccagcagc aagagcacca gcggcggcac agccgctctg     420
   ggctgcctgg tcaaggacta cttccccgag cccgtgaccg tgtcctggaa cagcggagcc     480
   ctgacctccg gcgtgcacac cttccccgcc gtgctgcaga gttctggcct gtatagcctg     540
   agcagcgtgg tcaccgtgcc ttctagcagc ctgggcaccc agacctacat ctgcaacgtg     600
   aaccacaagc ccagcaacac caaggtggac aagaaggtgg agcccaagag ctgcgacaaa     660
   actcacacat gcccaccgtg cccagcacct gaagctgcag ggggaccgtc agtcttcctc      720
   ttccccccaa aacccaagga caccctcatg atctcccgga cccctgaggt cacatgcgtg     780
   gtggtggacg tgagccacga agaccctgag gtcaagttca actggtacgt ggacggcgtg     840
   gaggtgcata atgccaagac aaagccgcgg gaggagcagt acaacagcac gtaccgtgtg     900
   gtcagcgtcc tcaccgtcct gcaccaggac tggctgaatg gcaaggagta caagtgcaag     960
   gtctccaaca aagccctcgg cgcccccatc gagaaaacca tctccaaagc caaagggcag    1020
   ccccgagaac cacaggtgtg caccctgccc ccatcccggg atgagctgac caagaaccag    1080
   gtcagcctct cgtgcgcagt caaaggcttc tatcccagcg acatcgccgt ggagtgggag    1140
   agcaatgggc agccggagaa caactacaag accacgcctc ccgtgctgga ctccgacggc    1200
   tccttcttcc tcgtgagcaa gctcaccgtg gacaagagca ggtggcagca ggggaacgtc    1260
   ttctcatgct ccgtgatgca tgaggctctg cacaaccact acacgcagaa gagcctctcc    1320
   ctgtctccgg gtggaggcgg cggaagcgga ggaggaggat ccagagaggg ccctgagctg    1380
   agccccgatg atcctgctgg actgctggac ctgcggcagg gcatgtttgc tcagctggtg    1440
   gcccagaacg tgctgctgat cgatggcccc ctgtcctggt acagcgatcc tggactggct 1500
   ggcgtgtcac tgacaggcgg cctgagctac aaagaggaca ccaaagaact ggtggtggcc 1560
   aaggccggcg tgtactacgt gttctttcag ctggaactgc ggagagtggt ggccggcgaa 1620
   ggatctggct ctgtgtctct ggccctgcat ctgcagcctc tgagaagcgc tgctggcgct 1680
   gcagctctgg cactgacagt ggatctgcct cctgccagct ccgaggcccg gaatagcgca 1740
   tttgggtttc aaggcaggct gctgcacctg tctgccggcc agaggctggg agtgcatctg 1800
   cacacagagg ccagggctag acacgcctgg cagctgacac agggcgctac agtgctgggc 1860
   ctgttcagag tgacccccga gattccagcc ggcctgcctt ctccaagaag cgaaggcgga 1920
   ggcggatctg gcggcggagg atctagagag ggacccgaac tgtcccctga cgatccagcc 1980
   gggctgctgg atctgagaca gggaatgttc gcccagctgg tggctcagaa tgtgctgctg 2040
   attgacggac ctctgagctg gtactccgac ccagggctgg caggggtgtc cctgactggg 2100
   ggactgtcct acaaagaaga tacaaaagaa ctggtggtgg ctaaagctgg ggtgtactat 2160
   gtgttttttc agctggaact gaggcgggtg gtggctgggg agggctcagg atctgtgtcc 2220
   ctggctctgc atctgcagcc actgcgctct gctgctggcg cagctgcact ggctctgact 2280
   gtggacctgc caccagcctc tagcgaggcc agaaacagcg ccttcgggtt ccaaggacgc 2340
   ctgctgcatc tgagcgccgg acagcgcctg ggagtgcatc tgcatactga agccagagcc 2400
   cggcatgctt ggcagctgac tcaggggcca actgtgctgg gactgtttcg cgtgacacct 2460
   gagatccctg ccggactgcc aagccctaga tcagaa 2496
<210> 156
   <211> 1914
   <212> DNA
   <213> Artificial Sequence
<220>
   <223> nucleotide sequence of DP47 Fc knob chain fused to monomeric hu 4-1BBL (71-254)
<400> 156
   gaggtgcaat tgttggagtc tgggggaggc ttggtacagc ctggggggtc cctgagactc 60
   tcctgtgcag cctccggatt cacctttagc agttatgcca tgagctgggt ccgccaggct 120
   ccagggaagg ggctggagtg ggtctcagct attagtggta gtggtggtag cacatactac 180
   gcagactccg tgaagggccg gttcaccatc tccagagaca attccaagaa cacgctgtat 240
   ctgcagatga acagcctgag agccgaggac acggccgtat attactgtgc gaaaggcagc 300
   ggatttgact actggggcca aggaaccctg gtcaccgtct cgagtgctag caccaagggc 360
   ccatcggtct tccccctggc accctcctcc aagagcacct ctgggggcac agcggccctg 420
   ggctgcctgg tcaaggacta cttccccgaa ccggtgacgg tgtcgtggaa ctcaggcgcc 480
   ctgaccagcg gcgtgcacac cttcccggct gtcctacagt cctcaggact ctactccctc      540
   agcagcgtgg tgaccgtgcc ctccagcagc ttgggcaccc agacctacat ctgcaacgtg      600
   aatcacaagc ccagcaacac caaggtggac aagaaagttg agcccaaatc ttgtgacaaa      660
   actcacacat gcccaccgtg cccagcacct gaagctgcag ggggaccgtc agtcttcctc     720
   ttccccccaa aacccaagga caccctcatg atctcccgga cccctgaggt cacatgcgtg      780
   gtggtggacg tgagccacga agaccctgag gtcaagttca actggtacgt ggacggcgtg      840
   gaggtgcata atgccaagac aaagccgcgg gaggagcagt acaacagcac gtaccgtgtg      900
   gtcagcgtcc tcaccgtcct gcaccaggac tggctgaatg gcaaggagta caagtgcaag      960
   gtctccaaca aagccctcgg cgcccccatc gagaaaacca tctccaaagc caaagggcag     1020
   ccccgagaac cacaggtgta caccctgccc ccctgcagag atgagctgac caagaaccag     1080
   gtgtccctgt ggtgtctggt caagggcttc taccccagcg atatcgccgt ggagtgggag     1140
   agcaacggcc agcctgagaa caactacaag accacccccc ctgtgctgga cagcgacggc     1200
   agcttcttcc tgtactccaa actgaccgtg gacaagagcc ggtggcagca gggcaacgtg     1260
   ttcagctgca gcgtgatgca cgaggccctg cacaaccact acacccagaa gtccctgagc     1320
   ctgagccccg gcggaggcgg cggaagcgga ggaggaggat ccagagaggg ccctgagctg     1380
   agccccgatg atcctgctgg actgctggac ctgcggcagg gcatgtttgc tcagctggtg     1440
   gcccagaacg tgctgctgat cgatggcccc ctgtcctggt acagcgatcc tggactggct     1500
   ggcgtgtcac tgacaggcgg cctgagctac aaagaggaca ccaaagaact ggtggtggcc     1560
   aaggccggcg tgtactacgt gttctttcag ctggaactgc ggagagtggt ggccggcgaa     1620
   ggatctggct ctgtgtctct ggccctgcat ctgcagcctc tgagaagcgc tgctggcgct     1680
   gcagctctgg cactgacagt ggatctgcct cctgccagct ccgaggcccg gaatagcgca     1740
   tttgggtttc aaggcaggct gctgcacctg tctgccggcc agaggctggg agtgcatctg     1800
   cacacagagg ccagggctag acacgcctgg cagctgacac agggcgctac agtgctgggc     1860
   ctgttcagag tgacccccga gattccagcc ggcctgcctt ctccaagaag cgaa           1914
<210> 157
   <211> 832
   <212> PRT
   <213> Artificial Sequence
<220>
   <223> DP47 Fc hole chain fused to dimeric hu 4-1BBL (71-254)
<400> 157
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ser Gly Phe Asp Tyr Trp Gly Gln Gly Thr Leu Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
           195                 200                 205
   Val Asp Lys Lys Val Glu Pro Lys Ser Cys Asp Lys Thr His Thr Cys
       210                 215                 220
   Pro Pro Cys Pro Ala Pro Glu Ala Ala Gly Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser His Glu Asp Pro Glu Val Lys
               260                 265                 270
   Phe Asn Trp Tyr Val Asp Gly Val Glu Val His Asn Ala Lys Thr Lys
   275 280 285
   Pro Arg Glu Glu Gln Tyr Asn Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Ala Leu Gly Ala Pro Ile Glu Lys Thr Ile Ser Lys
   325 330 335
   Ala Lys Gly Gln Pro Arg Glu Pro Gln Val Cys Thr Leu Pro Pro Ser
   340 345 350
   Arg Asp Glu Leu Thr Lys Asn Gln Val Ser Leu Ser Cys Ala Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Val Ser Lys Leu Thr Val Asp Lys Ser Arg Trp Gln
   405 410 415
   Gln Gly Asn Val Phe Ser Cys Ser Val Met His Glu Ala Leu His Asn
   420 425 430
   His Tyr Thr Gln Lys Ser Leu Ser Leu Ser Pro Gly Gly Gly Gly Gly
   435 440 445
   Ser Gly Gly Gly Gly Ser Arg Glu Gly Pro Glu Leu Ser Pro Asp Asp
   450 455 460
   Pro Ala Gly Leu Leu Asp Leu Arg Gln Gly Met Phe Ala Gln Leu Val
   465 470 475 480
   Ala Gln Asn Val Leu Leu Ile Asp Gly Pro Leu Ser Trp Tyr Ser Asp
   485 490 495
   Pro Gly Leu Ala Gly Val Ser Leu Thr Gly Gly Leu Ser Tyr Lys Glu
   500 505 510
   Asp Thr Lys Glu Leu Val Val Ala Lys Ala Gly Val Tyr Tyr Val Phe
   515 520 525
   Phe Gln Leu Glu Leu Arg Arg Val Val Ala Gly Glu Gly Ser Gly Ser
   530 535 540
   Val Ser Leu Ala Leu His Leu Gln Pro Leu Arg Ser Ala Ala Gly Ala
   545 550 555 560
   Ala Ala Leu Ala Leu Thr Val Asp Leu Pro Pro Ala Ser Ser Glu Ala
   565 570 575
   Arg Asn Ser Ala Phe Gly Phe Gln Gly Arg Leu Leu His Leu Ser Ala
   580 585 590
   Gly Gln Arg Leu Gly Val His Leu His Thr Glu Ala Arg Ala Arg His
   595 600 605
   Ala Trp Gln Leu Thr Gln Gly Ala Thr Val Leu Gly Leu Phe Arg Val
   610 615 620
   Thr Pro Glu Ile Pro Ala Gly Leu Pro Ser Pro Arg Ser Glu Gly Gly
   625 630 635 640
   Gly Gly Ser Gly Gly Gly Gly Ser Arg Glu Gly Pro Glu Leu Ser Pro
   645 650 655
   Asp Asp Pro Ala Gly Leu Leu Asp Leu Arg Gln Gly Met Phe Ala Gln
   660 665 670
   Leu Val Ala Gln Asn Val Leu Leu Ile Asp Gly Pro Leu Ser Trp Tyr
   675 680 685
   Ser Asp Pro Gly Leu Ala Gly Val Ser Leu Thr Gly Gly Leu Ser Tyr
   690 695 700
   Lys Glu Asp Thr Lys Glu Leu Val Val Ala Lys Ala Gly Val Tyr Tyr
   705 710 715 720
   Val Phe Phe Gln Leu Glu Leu Arg Arg Val Val Ala Gly Glu Gly Ser
   725 730 735
   Gly Ser Val Ser Leu Ala Leu His Leu Gln Pro Leu Arg Ser Ala Ala
   740 745 750
   Gly Ala Ala Ala Leu Ala Leu Thr Val Asp Leu Pro Pro Ala Ser Ser
   755 760 765
   Glu Ala Arg Asn Ser Ala Phe Gly Phe Gln Gly Arg Leu Leu His Leu
       770                 775                 780
   Ser Ala Gly Gln Arg Leu Gly Val His Leu His Thr Glu Ala Arg Ala
   785                 790                 795                 800
   Arg His Ala Trp Gln Leu Thr Gln Gly Ala Thr Val Leu Gly Leu Phe
                   805                 810                 815
   Arg Val Thr Pro Glu Ile Pro Ala Gly Leu Pro Ser Pro Arg Ser Glu
               820                 825                 830
<210> 158
   <211> 638
   <212> PRT
   <213> Artificial Sequence
<220>
   <223> DP47 Fc knob chain fused to monomeric hu 4-1BBL (71-254)
<400> 158
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ser Gly Phe Asp Tyr Trp Gly Gln Gly Thr Leu Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145 150 155 160
   Leu Thr Ser Gly Val His Thr Phe Pro Ala Val Leu Gln Ser Ser Gly
   165 170 175
   Leu Tyr Ser Leu Ser Ser Val Val Thr Val Pro Ser Ser Ser Leu Gly
   180 185 190
   Thr Gln Thr Tyr Ile Cys Asn Val Asn His Lys Pro Ser Asn Thr Lys
   195 200 205
   Val Asp Lys Lys Val Glu Pro Lys Ser Cys Asp Lys Thr His Thr Cys
   210 215 220
   Pro Pro Cys Pro Ala Pro Glu Ala Ala Gly Gly Pro Ser Val Phe Leu
   225 230 235 240
   Phe Pro Pro Lys Pro Lys Asp Thr Leu Met Ile Ser Arg Thr Pro Glu
   245 250 255
   Val Thr Cys Val Val Val Asp Val Ser His Glu Asp Pro Glu Val Lys
   260 265 270
   Phe Asn Trp Tyr Val Asp Gly Val Glu Val His Asn Ala Lys Thr Lys
   275 280 285
   Pro Arg Glu Glu Gln Tyr Asn Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Ala Leu Gly Ala Pro Ile Glu Lys Thr Ile Ser Lys
   325 330 335
   Ala Lys Gly Gln Pro Arg Glu Pro Gln Val Tyr Thr Leu Pro Pro Cys
   340 345 350
   Arg Asp Glu Leu Thr Lys Asn Gln Val Ser Leu Trp Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Lys Leu Thr Val Asp Lys Ser Arg Trp Gln
   405 410 415
   Gln Gly Asn Val Phe Ser Cys Ser Val Met His Glu Ala Leu His Asn
   420 425 430
   His Tyr Thr Gln Lys Ser Leu Ser Leu Ser Pro Gly Gly Gly Gly Gly
   435 440 445
   Ser Gly Gly Gly Gly Ser Arg Glu Gly Pro Glu Leu Ser Pro Asp Asp
   450 455 460
   Pro Ala Gly Leu Leu Asp Leu Arg Gln Gly Met Phe Ala Gln Leu Val
   465 470 475 480
   Ala Gln Asn Val Leu Leu Ile Asp Gly Pro Leu Ser Trp Tyr Ser Asp
   485 490 495
   Pro Gly Leu Ala Gly Val Ser Leu Thr Gly Gly Leu Ser Tyr Lys Glu
   500 505 510
   Asp Thr Lys Glu Leu Val Val Ala Lys Ala Gly Val Tyr Tyr Val Phe
   515 520 525
   Phe Gln Leu Glu Leu Arg Arg Val Val Ala Gly Glu Gly Ser Gly Ser
   530 535 540
   Val Ser Leu Ala Leu His Leu Gln Pro Leu Arg Ser Ala Ala Gly Ala
   545 550 555 560
   Ala Ala Leu Ala Leu Thr Val Asp Leu Pro Pro Ala Ser Ser Glu Ala
   565 570 575
   Arg Asn Ser Ala Phe Gly Phe Gln Gly Arg Leu Leu His Leu Ser Ala
   580 585 590
   Gly Gln Arg Leu Gly Val His Leu His Thr Glu Ala Arg Ala Arg His
   595 600 605
   Ala Trp Gln Leu Thr Gln Gly Ala Thr Val Leu Gly Leu Phe Arg Val
   610 615 620
   Thr Pro Glu Ile Pro Ala Gly Leu Pro Ser Pro Arg Ser Glu
   625 630 635
<210> 159
   <211> 1335
   <212> DNA
   <213> Artificial Sequence
<220>
   <223> nucleotide sequence of DP47 heavy chain (hu IgG1 PGLALA)
<400> 159
   gaggtgcaat tgttggagtc tgggggaggc ttggtacagc ctggggggtc cctgagactc      60
   tcctgtgcag cctccggatt cacctttagc agttatgcca tgagctgggt ccgccaggct     120
   ccagggaagg ggctggagtg ggtctcagct attagtggta gtggtggtag cacatactac     180
   gcagactccg tgaagggccg gttcaccatc tccagagaca attccaagaa cacgctgtat     240
   ctgcagatga acagcctgag agccgaggac acggccgtat attactgtgc gaaaggcagc     300
   ggatttgact actggggcca aggaaccctg gtcaccgtct cgagtgctag caccaagggc     360
   ccatcggtct tccccctggc accctcctcc aagagcacct ctgggggcac agcggccctg     420
   ggctgcctgg tcaaggacta cttccccgaa ccggtgacgg tgtcgtggaa ctcaggcgcc     480
   ctgaccagcg gcgtgcacac cttcccggct gtcctacagt cctcaggact ctactccctc     540
   agcagcgtgg tgaccgtgcc ctccagcagc ttgggcaccc agacctacat ctgcaacgtg     600
   aatcacaagc ccagcaacac caaggtggac aagaaagttg agcccaaatc ttgtgacaaa     660
   actcacacat gcccaccgtg cccagcacct gaagctgcag ggggaccgtc agtcttcctc     720
   ttccccccaa aacccaagga caccctcatg atctcccgga cccctgaggt cacatgcgtg     780
   gtggtggacg tgagccacga agaccctgag gtcaagttca actggtacgt ggacggcgtg     840
   gaggtgcata atgccaagac aaagccgcgg gaggagcagt acaacagcac gtaccgtgtg     900
   gtcagcgtcc tcaccgtcct gcaccaggac tggctgaatg gcaaggagta caagtgcaag     960
   gtctccaaca aagccctcgg cgcccccatc gagaaaacca tctccaaagc caaagggcag    1020
   ccccgagaac cacaggtgta caccctgccc ccatcccggg atgagctgac caagaaccag    1080
   gtcagcctga cctgcctggt caaaggcttc tatcccagcg acatcgccgt ggagtgggag    1140
   agcaatgggc agccggagaa caactacaag accacgcctc ccgtgctgga ctccgacggc    1200
   tccttcttcc tctacagcaa gctcaccgtg gacaagagca ggtggcagca ggggaacgtc    1260
   ttctcatgct ccgtgatgca tgaggctctg cacaaccact acacgcagaa gagcctctcc    1320
   ctgtctccgg gtaaa                                                     1335
<210> 160
   <211> 445
   <212> PRT
   <213> Artificial Sequence
<220>
   <223> DP47 heavy chain (hu IgG1 PGLALA)
<400> 160
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Ser Gly Phe Asp Tyr Trp Gly Gln Gly Thr Leu Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
           195                 200                 205
   Val Asp Lys Lys Val Glu Pro Lys Ser Cys Asp Lys Thr His Thr Cys
       210                 215                 220
   Pro Pro Cys Pro Ala Pro Glu Ala Ala Gly Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser His Glu Asp Pro Glu Val Lys
   260 265 270
   Phe Asn Trp Tyr Val Asp Gly Val Glu Val His Asn Ala Lys Thr Lys
   275 280 285
   Pro Arg Glu Glu Gln Tyr Asn Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Ala Leu Gly Ala Pro Ile Glu Lys Thr Ile Ser Lys
   325 330 335
   Ala Lys Gly Gln Pro Arg Glu Pro Gln Val Tyr Thr Leu Pro Pro Ser
   340 345 350
   Arg Asp Glu Leu Thr Lys Asn Gln Val Ser Leu Thr Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Lys Leu Thr Val Asp Lys Ser Arg Trp Gln
   405 410 415
   Gln Gly Asn Val Phe Ser Cys Ser Val Met His Glu Ala Leu His Asn
   420 425 430
   His Tyr Thr Gln Lys Ser Leu Ser Leu Ser Pro Gly Lys
   435 440 445
<210> 161
   <211> 1350
   <212> DNA
   <213> Artificial Sequence
<220>
   <223> nucleotide sequence of PD1(0314) heavy chain (huIgG1 PGLALA)
<400> 161
   gaagtgcagc tgctcgaaag cggcggggga ctggtccagc ccggcggttc cctgaggctg        60
   tcttgcgccg cttcagggtt cagcttttcc tcttacacca tgagttgggt cagacaggca      120
   cctggcaagg gactggagtg ggtcgccaca atcagcggtg gcgggcgcga catttattac      180
   ccagattccg tgaaaggacg gttcaccatc tctagggaca actcaaagaa tactctgtat      240
   ttgcagatga acagcctgag agctgaggat acagcagttt actactgtgt gctcctgacc      300
   ggccgcgtct attttgccct tgactcctgg ggacaaggca ctctggtgac cgtatctagt      360
   gctagcacca agggcccatc ggtcttcccc ctggcaccct cctccaagag cacctctggg      420
   ggcacagcgg ccctgggctg cctggtcaag gactacttcc ccgaaccggt gacggtgtcg      480
   tggaactcag gcgccctgac cagcggcgtg cacaccttcc cggctgtcct acagtcctca      540
   ggactctact ccctcagcag cgtggtgacc gtgccctcca gcagcttggg cacccagacc      600
   tacatctgca acgtgaatca caagcccagc aacaccaagg tggacaagaa agttgagccc      660
   aaatcttgtg acaaaactca cacatgccca ccgtgcccag cacctgaagc tgcaggggga      720
   ccgtcagtct tcctcttccc cccaaaaccc aaggacaccc tcatgatctc ccggacccct      780
   gaggtcacat gcgtggtggt ggacgtgagc cacgaagacc ctgaggtcaa gttcaactgg      840
   tacgtggacg gcgtggaggt gcataatgcc aagacaaagc cgcgggagga gcagtacaac      900
   agcacgtacc gtgtggtcag cgtcctcacc gtcctgcacc aggactggct gaatggcaag      960
   gagtacaagt gcaaggtctc caacaaagcc ctcggcgccc ccatcgagaa aaccatctcc     1020
   aaagccaaag ggcagccccg agaaccacag gtgtacaccc tgcccccatc ccgggatgag     1080
   ctgaccaaga accaggtcag cctgacctgc ctggtcaaag gcttctatcc cagcgacatc     1140
   gccgtggagt gggagagcaa tgggcagccg gagaacaact acaagaccac gcctcccgtg     1200
   ctggactccg acggctcctt cttcctctac agcaagctca ccgtggacaa gagcaggtgg     1260
   cagcagggga acgtcttctc atgctccgtg atgcatgagg ctctgcacaa ccactacacg     1320
   cagaagagcc tctccctgtc tccgggtaaa                                       1350
<210> 162
   <211> 450
   <212> PRT
   <213> Artificial Sequence
<220>
   <223> PD1(0314) heavy chain (huIgG1 PGLALA)
<400> 162
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Ser Phe Ser Ser Tyr
               20                  25                  30
   Thr Met Ser Trp Val Arg Gln Ala Pro Gly Lys Gly Leu Glu Trp Val
           35                  40                  45
   Ala Thr Ile Ser Gly Gly Gly Arg Asp Ile Tyr Tyr Pro Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Leu Leu Thr Gly Arg Val Tyr Phe Ala Leu Asp Ser Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Ala Ala Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Gly Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 163
   <211> 163
   <212> PRT
   <213> Homo sapiens
<400> 163
   Leu Gln Asp Pro Cys Ser Asn Cys Pro Ala Gly Thr Phe Cys Asp Asn
   1 5 10 15
   Asn Arg Asn Gln Ile Cys Ser Pro Cys Pro Pro Asn Ser Phe Ser Ser
   20 25 30
   Ala Gly Gly Gln Arg Thr Cys Asp Ile Cys Arg Gln Cys Lys Gly Val
   35 40 45
   Phe Arg Thr Arg Lys Glu Cys Ser Ser Thr Ser Asn Ala Glu Cys Asp
   50 55 60
   Cys Thr Pro Gly Phe His Cys Leu Gly Ala Gly Cys Ser Met Cys Glu
   65 70 75 80
   Gln Asp Cys Lys Gln Gly Gln Glu Leu Thr Lys Lys Gly Cys Lys Asp
   85 90 95
   Cys Cys Phe Gly Thr Phe Asn Asp Gln Lys Arg Gly Ile Cys Arg Pro
   100 105 110
   Trp Thr Asn Cys Ser Leu Asp Gly Lys Ser Val Leu Val Asn Gly Thr
   115 120 125
   Lys Glu Arg Asp Val Val Cys Gly Pro Ser Pro Ala Asp Leu Ser Pro
   130 135 140
   Gly Ala Ser Ser Val Thr Pro Pro Ala Pro Ala Arg Glu Pro Gly His
   145 150 155 160
   Ser Pro Gln
<210> 164
   <211> 681
   <212> DNA
   <213> Artificial Sequence
<220>
   <223> Nucleotide sequence of Fc hole chain
<400> 164
   gacaaaactc acacatgccc accgtgccca gcacctgaac tcctgggggg accgtcagtc 60
   ttcctcttcc ccccaaaacc caaggacacc ctcatgatct cccggacccc tgaggtcaca 120
   tgcgtggtgg tggacgtgag ccacgaagac cctgaggtca agttcaactg gtacgtggac 180
   ggcgtggagg tgcataatgc caagacaaag ccgcgggagg agcagtacaa cagcacgtac 240
   cgtgtggtca gcgtcctcac cgtcctgcac caggactggc tgaatggcaa ggagtacaag 300
   tgcaaggtct ccaacaaagc cctcccagcc cccatcgaga aaaccatctc caaagccaaa 360
   gggcagcccc gagaaccaca ggtgtgcacc ctgcccccat cccgggatga gctgaccaag 420
   aaccaggtca gcctctcgtg cgcagtcaaa ggcttctatc ccagcgacat cgccgtggag 480
   tgggagagca atgggcagcc ggagaacaac tacaagacca cgcctcccgt gctggactcc 540
   gacggctcct tcttcctcgt gagcaagctc accgtggaca agagcaggtg gcagcagggg 600
   aacgtcttct catgctccgt gatgcatgag gctctgcaca accactacac gcagaagagc 660
   ctctccctgt ctccgggtaa a 681
<210> 165
   <211> 1266
   <212> DNA
   <213> Artificial Sequence
<220>
   <223> Nucleotide sequence of human 4-1BB antigen Fc knob chain
<400> 165
   ctgcaggacc cctgcagcaa ctgccctgcc ggcaccttct gcgacaacaa ccggaaccag 60
   atctgcagcc cctgcccccc caacagcttc agctctgccg gcggacagcg gacctgcgac 120
   atctgcagac agtgcaaggg cgtgttcaga acccggaaag agtgcagcag caccagcaac 180
   gccgagtgcg actgcacccc cggcttccat tgtctgggag ccggctgcag catgtgcgag 240
   caggactgca agcagggcca ggaactgacc aagaagggct gcaaggactg ctgcttcggc 300
   accttcaacg accagaagcg gggcatctgc cggccctgga ccaactgtag cctggacggc 360
   aagagcgtgc tggtcaacgg caccaaagaa cgggacgtcg tgtgcggccc cagccctgct 420
   gatctgtctc ctggggccag cagcgtgacc cctcctgccc ctgccagaga gcctggccac 480
   tctcctcagg tcgacgaaca gttatatttt cagggcggct cacccaaatc tgcagacaaa 540
   actcacacat gcccaccgtg cccagcacct gaactcctgg ggggaccgtc agtcttcctc 600
   ttccccccaa aacccaagga caccctcatg atctcccgga cccctgaggt cacatgcgtg 660
   gtggtggacg tgagccacga agaccctgag gtcaagttca actggtacgt ggacggcgtg 720
   gaggtgcata atgccaagac aaagccgcgg gaggagcagt acaacagcac gtaccgtgtg 780
   gtcagcgtcc tcaccgtcct gcaccaggac tggctgaatg gcaaggagta caagtgcaag 840
   gtctccaaca aagccctccc agcccccatc gagaaaacca tctccaaagc caaagggcag 900
   ccccgagaac cacaggtgta caccctgccc ccatgccggg atgagctgac caagaaccag 960
   gtcagcctgt ggtgcctggt caaaggcttc tatcccagcg acatcgccgt ggagtgggag 1020
   agcaatgggc agccggagaa caactacaag accacgcctc ccgtgctgga ctccgacggc 1080
   tccttcttcc tctacagcaa gctcaccgtg gacaagagca ggtggcagca ggggaacgtc 1140
   ttctcatgct ccgtgatgca tgaggctctg cacaaccact acacgcagaa gagcctctcc 1200
   ctgtctccgg gtaaatccgg aggcctgaac gacatcttcg aggcccagaa gattgaatgg 1260
   cacgag 1266
<210> 166
   <211> 227
   <212> PRT
   <213> Artificial Sequence
<220>
   <223> Fc hole chain
<400> 166
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Cys Thr Leu Pro Pro Ser Arg Asp Glu Leu Thr Lys Asn Gln Val Ser
       130                 135                 140
   Leu Ser Cys Ala Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Val Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225
<210> 167
   <211> 422
   <212> PRT
   <213> Artificial Sequence
<220>
   <223> human 4-1BB antigen Fc knob chain
<400> 167
   Leu Gln Asp Pro Cys Ser Asn Cys Pro Ala Gly Thr Phe Cys Asp Asn
   1               5                   10                  15
   Asn Arg Asn Gln Ile Cys Ser Pro Cys Pro Pro Asn Ser Phe Ser Ser
               20                  25                  30
   Ala Gly Gly Gln Arg Thr Cys Asp Ile Cys Arg Gln Cys Lys Gly Val
           35                  40                  45
   Phe Arg Thr Arg Lys Glu Cys Ser Ser Thr Ser Asn Ala Glu Cys Asp
       50                  55                  60
   Cys Thr Pro Gly Phe His Cys Leu Gly Ala Gly Cys Ser Met Cys Glu
   65                  70                  75                  80
   Gln Asp Cys Lys Gln Gly Gln Glu Leu Thr Lys Lys Gly Cys Lys Asp
                   85                  90                  95
   Cys Cys Phe Gly Thr Phe Asn Asp Gln Lys Arg Gly Ile Cys Arg Pro
               100                 105                 110
   Trp Thr Asn Cys Ser Leu Asp Gly Lys Ser Val Leu Val Asn Gly Thr
           115                 120                 125
   Lys Glu Arg Asp Val Val Cys Gly Pro Ser Pro Ala Asp Leu Ser Pro
       130                 135                 140
   Gly Ala Ser Ser Val Thr Pro Pro Ala Pro Ala Arg Glu Pro Gly His
   145                 150                 155                 160
   Ser Pro Gln Val Asp Glu Gln Leu Tyr Phe Gln Gly Gly Ser Pro Lys
                   165                 170                 175
   Ser Ala Asp Lys Thr His Thr Cys Pro Pro Cys Pro Ala Pro Glu Leu
               180                 185                 190
   Leu Gly Gly Pro Ser Val Phe Leu Phe Pro Pro Lys Pro Lys Asp Thr
           195                 200                 205
   Leu Met Ile Ser Arg Thr Pro Glu Val Thr Cys Val Val Val Asp Val
       210                 215                 220
   Ser His Glu Asp Pro Glu Val Lys Phe Asn Trp Tyr Val Asp Gly Val
   225 230 235 240
   Glu Val His Asn Ala Lys Thr Lys Pro Arg Glu Glu Gln Tyr Asn Ser
   245 250 255
   Thr Tyr Arg Val Val Ser Val Leu Thr Val Leu His Gln Asp Trp Leu
   260 265 270
   Asn Gly Lys Glu Tyr Lys Cys Lys Val Ser Asn Lys Ala Leu Pro Ala
   275 280 285
   Pro Ile Glu Lys Thr Ile Ser Lys Ala Lys Gly Gln Pro Arg Glu Pro
   290 295 300
   Gln Val Tyr Thr Leu Pro Pro Cys Arg Asp Glu Leu Thr Lys Asn Gln
   305 310 315 320
   Val Ser Leu Trp Cys Leu Val Lys Gly Phe Tyr Pro Ser Asp Ile Ala
   325 330 335
   Val Glu Trp Glu Ser Asn Gly Gln Pro Glu Asn Asn Tyr Lys Thr Thr
   340 345 350
   Pro Pro Val Leu Asp Ser Asp Gly Ser Phe Phe Leu Tyr Ser Lys Leu
   355 360 365
   Thr Val Asp Lys Ser Arg Trp Gln Gln Gly Asn Val Phe Ser Cys Ser
   370 375 380
   Val Met His Glu Ala Leu His Asn His Tyr Thr Gln Lys Ser Leu Ser
   385 390 395 400
   Leu Ser Pro Gly Lys Ser Gly Gly Leu Asn Asp Ile Phe Glu Ala Gln
   405 410 415
   Lys Ile Glu Trp His Glu
   420
<210> 168
   <211> 15
   <212> PRT
   <213> Artificial Sequence
<220>
   <223> avi tag
<400> 168
   Gly Leu Asn Asp Ile Phe Glu Ala Gln Lys Ile Glu Trp His Glu
   1 5 10 15
<210> 169
   <211> 594
   <212> DNA
   <213> Artificial Sequence
<220>
   <223> nucleotide sequence of human 4-1BB His
<400> 169
   ctgcaggacc cctgcagcaa ctgccctgcc ggcaccttct gcgacaacaa ccggaaccag 60
   atctgcagcc cctgcccccc caacagcttc agctctgccg gcggacagcg gacctgcgac 120
   atctgcagac agtgcaaggg cgtgttcaga acccggaaag agtgcagcag caccagcaac 180
   gccgagtgcg actgcacccc cggcttccat tgtctgggag ccggctgcag catgtgcgag 240
   caggactgca agcagggcca ggaactgacc aagaagggct gcaaggactg ctgcttcggc 300
   accttcaacg accagaagcg gggcatctgc cggccctgga ccaactgtag cctggacggc 360
   aagagcgtgc tggtcaacgg caccaaagaa cgggacgtcg tgtgcggccc cagccctgct 420
   gatctgtctc ctggggccag cagcgtgacc cctcctgccc ctgccagaga gcctggccac 480
   tctcctcagg tcgacgaaca gttatatttt cagggcggct caggcctgaa cgacatcttc 540
   gaggcccaga agatcgagtg gcacgaggct cgagctcacc accatcacca tcac 594
<210> 170
   <211> 198
   <212> PRT
   <213> Artificial Sequence
<220>
   <223> human 4-1BB His
<400> 170
   Leu Gln Asp Pro Cys Ser Asn Cys Pro Ala Gly Thr Phe Cys Asp Asn
   1               5                   10                  15
   Asn Arg Asn Gln Ile Cys Ser Pro Cys Pro Pro Asn Ser Phe Ser Ser
               20                  25                  30
   Ala Gly Gly Gln Arg Thr Cys Asp Ile Cys Arg Gln Cys Lys Gly Val
           35                  40                  45
   Phe Arg Thr Arg Lys Glu Cys Ser Ser Thr Ser Asn Ala Glu Cys Asp
       50                  55                  60
   Cys Thr Pro Gly Phe His Cys Leu Gly Ala Gly Cys Ser Met Cys Glu
   65                  70                  75                  80
   Gln Asp Cys Lys Gln Gly Gln Glu Leu Thr Lys Lys Gly Cys Lys Asp
                   85                  90                  95
   Cys Cys Phe Gly Thr Phe Asn Asp Gln Lys Arg Gly Ile Cys Arg Pro
               100                 105                 110
   Trp Thr Asn Cys Ser Leu Asp Gly Lys Ser Val Leu Val Asn Gly Thr
           115                 120                 125
   Lys Glu Arg Asp Val Val Cys Gly Pro Ser Pro Ala Asp Leu Ser Pro
       130                 135                 140
   Gly Ala Ser Ser Val Thr Pro Pro Ala Pro Ala Arg Glu Pro Gly His
   145                 150                 155                 160
   Ser Pro Gln Val Asp Glu Gln Leu Tyr Phe Gln Gly Gly Ser Gly Leu
                   165                 170                 175
   Asn Asp Ile Phe Glu Ala Gln Lys Ile Glu Trp His Glu Ala Arg Ala
               180                 185                 190
   His His His His His His
           195